(19) Europäisches Patentamt
European Patent Office
Office européen des brevets

(11) EP 1 981 504 B1

(12) **EUROPEAN PATENT SPECIFICATION**

(45) Date of publication and mention of the grant of the patent:
**21.07.2010 Bulletin 2010/29**

(21) Application number: **07711455.1**

(22) Date of filing: **07.02.2007**

(51) Int Cl.:
*A61K 31/496* (2006.01) *C07D 285/12* (2006.01)
*C07C 257/14* (2006.01) *A61P 25/00* (2006.01)

(86) International application number:
**PCT/EP2007/001022**

(87) International publication number:
**WO 2007/090617 (16.08.2007 Gazette 2007/33)**

(54) **THIADIAZOLE DERIVATIVES FOR THE TREATMENT OF NEURO DEGENERATIVE DISEASES**

THIADIAZOL-DERIVATE ZUR BEHANDLUNG VON NEURODEGENERATIVEN ERKRANKUNGEN

DERIVES DE THIADIAZOLE POUR LE TRAITEMENT DE MALADIES NEURODEGENERATIVES

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.02.2006 GB 0602335**

(43) Date of publication of application:
**22.10.2008 Bulletin 2008/43**

(73) Proprietor: **NV reMYND**
**3001 Leuven-Heverlee (BE)**

(72) Inventors:
- **GRIFFIOEN, Gerard**
  **3210 Linden (BE)**
- **COUPET, Kristel Marie Edith**
  **1700 Dilbeek (BE)**
- **DUHAMEL, Hein Roger**
  **3010 Kessel-Lo (BE)**
- **WERA, Stefaan**
  **3360 Bierbeek (BE)**
- **GOMME, Ellen**
  **3078 Kortenberg (BE)**
- **VAN DAMME, Nele**
  **3010 Kessel-Lo (BE)**
- **VAN DER AUWERA, Ingrid**
  **3201 Langdorp (BE)**
- **LOX, Marleen**
  **3300 Tienen (BE)**
- **VAN DOOREN, Tom**
  **2600 Berchem (BE)**
- **DECRUY, Tine**
  **8340 Moerkerke-Damme (BE)**

(74) Representative: **Bird, William Edward**
**Bird Goën & Co.**
**Klein Dalenstraat 42A**
**3020 Winksele (BE)**

(56) References cited:
**WO-A-2005/020991 WO-A-2005/095368**
**WO-A-2006/002981**

- **DATABASE CHEMCATS [Online] Comgenex Product list 15 April 2005 (2005-04-15), XP002442116 retrieved from STN Database accession no. 2022059786**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 981 504 B1

Printed by Jouve, 75001 PARIS (FR)

## Description

### FIELD OF THE INVENTION

The present invention relates to novel thiadiazole derivatives useful for treating certain neurological disorders characterised by cytotoxic α-synuclein amyloidogenesis. The invention further relates to methods of treatment or prevention of such neurological disorders by the administration of a pharmaceutical composition, comprising one or more thiadiazole derivatives in an amount which alleviates or prevents the cytotoxic properties of α-synuclein. The invention further relates to methods of preparing novel thiadiazole derivatives, as well as certain classes of intermediates useful in such preparation.

### BACKGROUND OF THE INVENTION

α-Synuclein is a neuronal protein which originally has been associated with neuronal plasticity during Zebra finch song learning. Although its role at the molecular level is at present largely elusive it appears to have lipid bi-layer (or membrane) with binding properties important for preserving proper transport of neurotransmitter vesicles to the axonal ends of neurons presumably to ensure proper signalling at the synapse. Apart from its physiological role in brain cells, human α-synuclein also possesses pathological features that underlies a plethora of neurodegenerative diseases including Parkinson's disease, diffuse Lewy body disease, traumatic brain injury, amyotrophic lateral sclerosis, Niemann-Pick disease, Hallervorden-Spatz syndrome, Down syndrome, neuroaxonal dystrophy, multiple system atrophy and Alzheimer's disease. These neurological disorders are characterised by the presence of insoluble α-synuclein polymers or aggregates usually residing within neuronal cells, although in the case of Alzheimer's disease α-synuclein (or proteolytic fragments thereof) constitutes the non-amyloid component of extracellular " amyloid-β plaques ". It is widely believed that the amyloidogenic properties α-synuclein disrupt cellular integrity leading to dysfunctioning or death of affected neurons resulting in cognitive and/or motoric decline as it is found in patients suffering from such diseases. The aggregation of α-synuclein is at present very poorly defined, but constitutes most likely a multi-step process wherein self-polymerization of α-synuclein into insoluble aggregates is preceded by the formation of soluble protofibrils of α-synuclein monomers. Self-association may be triggered by the formation of alternative conformations of α-synuclein monomers with high propensity to polymerize. Several studies using neuronal cell lines or whole animals have shown that formation of reactive oxygen species (hereinafter abbreviated as ROS) appear to stimulate noxious α-synuclein amyloidogenesis. For instance paraquat (an agent stimulating ROS formation within the cell) has been recognized as a stimulator of α-synuclein aggregation. Like in animals, exposure to paraquat is believed to induce the formation of synuclein inclusions, and consequently neurodegeneration, especially of dopaminergic neurons in humans. Dopaminergic neurons appear to be particularly sensitive because the concurrent dopamine metabolism may on the one hand contribute significantly to the oxidative stress load but may on the other hand result in kinetic stabilisation of highly toxic protofibrillar α-synuclein species by dopamine (or its metabolic derivatives). Parkinson's disease is characterised by a selective loss of dopaminergic *substantia nigra* cells and therefore treatment of animals (or neuronal cells) with paraquat is a common well-accepted experimental set-up for studying synucleopathies, in particular Parkinson's disease.

Apart from ROS, mutations in the coding region of the α-synuclein gene have also been identified as stimulators of self-polymerization resulting in early disease onset as it is observed in families afflicted by such mutations. Finally, increased expression of α-synuclein also promotes early disease onset as evidenced by a duplication or triplication of the α-synuclein gene in the genome of some individuals. The molecular mechanism by which α-synuclein self-association triggers cellular degeneration is at present largely unknown. Although it has been speculated that insoluble aggregates affect cellular integrity, it has recently been suggested that soluble protofibrillar intermediates of the aggregation process are particularly toxic for the cell as opposed to mature insoluble fibrils which may be inert end-products or may even serve as cytoprotective reservoirs of otherwise harmful soluble species. Therapeutic attempts to inhibit formation of insoluble aggregates may therefore be conceptually wrong, possibly even promoting disease progress.

While the identification of pathological α-synuclein mutations unequivocally revealed a causative factor of a plethora of neurodegenerative disorders, treatments ensuring suppression of toxic α-synuclein amyloidogenesis are presently not available. Only symptomatic treatments of Parkinson's disease exist, which aim e.g. at increasing dopamine levels in order to replenish its lowered level due to degeneration of dopaminergic neurons, for instance by administrating L-DOPA or inhibitors of dopamine breakdown. Although such treatments suppress disease symptoms to some extent, they are only temporarily effective and certainly do not slow down ongoing neuronal degeneration.

Thus there is a need in the art for designing new drugs for therapeutic treatments that target the underlying molecular mechanism of α-synuclein related pathologies in order to reduce neuronal cell death and/or degeneration.

WO 99/51584 discloses 5-piperazinyl-1,2,4-thiadiazoles as inhibitors of proton pump $H^+/K^+$-ATPase and therefor useful in the treatment of peptic ulcer. However these compounds are not suggested for use in the prevention or treatment of neurodegenerative disorders.

SUMMARY OF THE INVENTION

The present invention relates to a class of 1,2,4-thiadiazote derivatives that have been shown to effectively counteract or inhibit the toxic properties of α-synuclein . Administration of these compounds to patients suffering from a neurodegenerative disease characterised by noxious α-synuclein amyloidogenesis therefore constitutes an effective therapeutic and/or prophylactic method of treatment.

According to a first aspect, the present invention provides a class of novel 1,2,4-thiadiazole derivatives, which are capable of inhibiting or significantly reducing α-synuclein-instigated loss of neuronal cell integrity according to claim 11. According to a second aspect, the present invention provides 1,2,4-thiadiazole derivatives having the structural formula of claim 1 for use as medicaments, more particularly for use in the treatment of α-synucleopathies according to claim 13 and claim 14 dependent thereon. According to this aspect, the invention also provides pharmaceutical compositions comprising an effective amount of one or more 1,2,4-thiadiazole derivatives according to claim 1 and claims 2 to 10 and 12 dependent thereon, said compositions being useful for the prevention and/or treatment of an α-synucleopathy such as, but not limited to, Parkinson's disease, diffuse Lewy body disease, multiple system atrophy and Alzheimer's disease. Accordingly, the present invention also relates to the use of the 1,2,4-thiadiazole derivatives of the present invention in the manufacture of a medicament for the treatment and/or prevention of α-synucleopathies, such as, but not limited to Parkinson's disease, diffuse Lewy body disease, multiple system atrophy and Alzheimer's disease. In a third aspect, the present invention provides a method for preparing such novel 1,2.4-thiadiazole derivatives in a limited number of steps and starting from commercially available materials or easily obtainable analogues thereof.

DEFINITIONS

As used herein with respect to a substituting group, and unless otherwise stated, the therm " $C_{1-4}$ alkyl " means straight and branched chain saturated acyclic hydrocarbon monovalent groups having from 1 to 4 carbon atoms such as, for example, methyl, ethyl, propyl, n-butyl, 1-methylethyl (isopropyl), 2-methylpropyl (isobutyl) and 1,1-dimethylethyl (ter-butyl). By analogy, the term " $C_{1-6}$ alkyl " refers to such radicals having from 1 to 6 carbon atoms, including 2-methylbutyl, n-pentyl, dimethylpropyl, n-hexyl, 2-methylpentyl, 3-methylpentyl, and the like.

As used herein with respect to a linking group, and unless otherwise stated, the term " $C_{1-6}$ alkylene " means a divalent hydrocarbon radical corresponding to the above defined $C_{1-6}$ alkyl, such as methylene, bis(methylene), tris (methylene), tetramethylene, hexamethylene and the like.

As used herein with respect to a substituting group, and unless otherwise stated, the term " $C_{3-6}$ cycloalkyl " means a mono- or polycyclic saturated hydrocarbon monovalent group having from 3 to 6 carbon atoms, such as for instance cyclopropyl, cyclobutyl, cyclopentyl, cyclohexyl, and the like.

As used herein with respect to a substituting group, and unless otherwise stated, the term " aryl " designate any mono- or polycyclic aromatic monovalent hydrocarbon group having from 6 up to 30 carbon atoms such as but not limited to phenyl, naphthyl, anthracenyl, phenantracyl, fluoranthenyl, chrysenyl, pyrenyl, biphenylyl, terphenyl, picenyl, indenyl, biphenyl, indacenyl, benzocyclobutenyl, benzocyclooctenyl and the like, including fused benzo-$C_{4-8}$ cycloalkyl groups such as, for instance, indanyl, tetrahydronaphtyl, fluorenyl and the like, all of the said radicals being optionally substituted with one or more substituents independently selected from the group consisting of halogen, amino, trifluoromethyl, hydroxyl, sulfhydryl and nitro, such as for instance 4-fluorophenyl, 4-chlorophenyl, 3,4-dichlorophenyl, 4-cyanophenyl, 2,6-dichlorophenyl, 2-fluorophenyl, 3-chlorophenyl, 3,5-dichlorophenyl and the like.

As used herein with respect to a substituting group , and unless otherwise stated, the terms " $C_{1-4}$ alkoxy ", " $C_{1-6}$ alkoxy " and " aryloxy ", refer to substituents wherein a carbon atom of a $C_{1-4}$ alkyl, respectively a $C_{1-6}$ alkyl or an aryl group (each of them such as defined herein), is attached to an oxygen atom through a single bond such as, but not limited to, methoxy, ethoxy, propoxy, butoxy, pentoxy, isopropoxy, sec-butoxy, tert-butoxy, isopentoxy, phenyloxy, and the like.

As used herein and unless otherwise stated, the term " stereoisomer " refers to all possible different isomeric as well as conformational forms which the compounds of structural formula (A) may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a derivative of this invention with a suitable inorganic solvent (e.g. hydrates) or organic solvent, such as but not limited to alcohols, ketones, esters, ethers, nitriles and the like.

The term " α-synucleopathy " as used herein, unless otherwise stated, refers to a disease characterised by the presence of pathological deposition of insoluble α-synuclein polymers or aggregates intracellularly and/or extracellularly. Such diseases include, but are not limited to, Parkinson's disease, diffuse Lewy body disease, traumatic brain injury, amyotrophic lateral sclerosis, Niemann-Pick disease, Hallervorden-Spatz syndrome, Down syndrome, neuroaxonal dystrophy, multiple system atrophy and Alzheimer's disease.

As used herein, the term " Parkinson's disease " refers to a chronic progressive nervous disease characterised by neurodegeneration, especially degeneration of dopaminergic neurons. Symptoms include stooped posture, resting tremor, weakness of resting muscles, a shuffling gait, speech impediments, movement difficulties and an eventual slowing of mental processes and dementia.

The term " neuroprotective " agent, as used herein, refers to drugs or chemical agents intended to prevent neurodegeneration, including drugs that slow down or stop the progression of neuronal degeneration.

## DETAILED DESCRIPTION OF THE INVENTION

The present invention relates, in a first aspect, to a group 1,2,4-thiadiazole derivatives which have desirable biological properties such as an inhibitory effect on α-synuclein mediated toxicity. Based on this inhibitory activity, and the fact that these compounds are not toxic to neural cells, these compounds are useful in the prevention and/or treatment of α-synucleopathies. In the broadest expression, the class of 1,2,4-thiadiazole derivatives of this invention may be represented by the structural formula (A) of claim 1, including stereoisomers, solvates and salts thereof. This broad class may be sub-divided into several sub-classes wherein each substituent $R_1$ to $R_{13}$, the divalent group (A'), and the linking moiety X may be defined in a more restricted manner, at will and independently from each other. Exemplary but non-limiting embodiments of such sub-classes are defined in claims 2 to 11.

The ability of the compounds of the invention to inhibit α-synuclein mediated toxicity is based on their activity in the α-synuclein cytotoxicity test described in the examples section herein. Treatment of mice with mitochondrial complex I inhibitors such as paraquat or MPTP (1-methyl-4-phenyl-1,2,3,6-tetrahydropyridine) is a well-accepted and commonly used experimental set-up to study neuronal degeneration. Paraquat triggers synuclein-aggregation, which allegedly triggers a specific loss of dopaminergic neurons and ultimately a decline in the locomotion function. Briefly, one or more compounds are administered to paraquat-receiving mice and the onset of motoric dysfunction is assessed using a rotary rod device. A delay or absence in the occurrence of motoric problems in compound treated mice (compared to control mice treated with only vehicle) indicates that the compound(s) inhibit(s) synuclein-dependent degeneration of dopaminergic cells.

In the assays used herein, compounds were considered to be active when inhibiting α-synuclein cytotoxicity by more than 25% relative to controls at a concentration of 20 μg/mL or lower. Dose responses were carried out on all compounds found to be active (10 point curves in duplicate). Although the pharmacological properties of the compounds disclosed in this invention vary with structural change, active compounds particularly possess EC50's in a cell-based assay of synuclein cytotoxicity in a range from about 0.0001 to 10 μM. Based on these findings, methods for treating and preventing disorders or diseases provoked by cytotoxic intracellular α-synuclein are provided herein. These methods comprise administering to a subject suffering from or susceptible to such a disease or disorder, an effective amount of one or more inhibitors of α-synuclein cytotoxicity as defined by the broad structural formula (A) of the invention, or sub-classes thereof. As used herein, the term " effective amount " designates an amount sufficient to effect beneficial or desired clinical or biochemical results. An effective amount can be administered one or more times. For purposes of this invention, an effective amount of an inhibitor of α-synuclein cytotoxicity is an amount that is sufficient to palliate, ameliorate, stabilize, reverse, slow down or delay the progression of a disease state or condition. In a particular embodiment of the invention, the " effective amount " is defined as an amount of compound capable of preventing deposition of insoluble α-synuclein polymers or aggregates and/or capable of preventing the cytotoxic effects triggered by aggregation or polymerization of α-synuclein, and is an amount that substantially reduces the symptoms of an α-synucleopathy, such as Parkinson's disease. Other forms of effective amount may be for the treatment or prevention of the learning or memory impairment related to Alzheimer's disease. As used herein, the terms " mammal ", " subject " or " patient " for the purposes of a therapeutic or prophylactic treatment refers to any animal classified as a mammal, including humans, domestic and farm animals, such as but not limited to dogs, cats, pigs, horses, sheep, and the like. Most particularly, the mammal is a human being.

The various 1,2,4-thiadiazole compounds of the present invention can be synthesised in an effective manner according to the methods described in the following examples. These methods comprise a limited number of steps and start from commercially available, or readily accessible, materials. Generally, 1,2,4-thiadiazole compounds having the structural formula (A) can be synthesised according to schemes 1 and 2 described hereafter:

Scheme 1

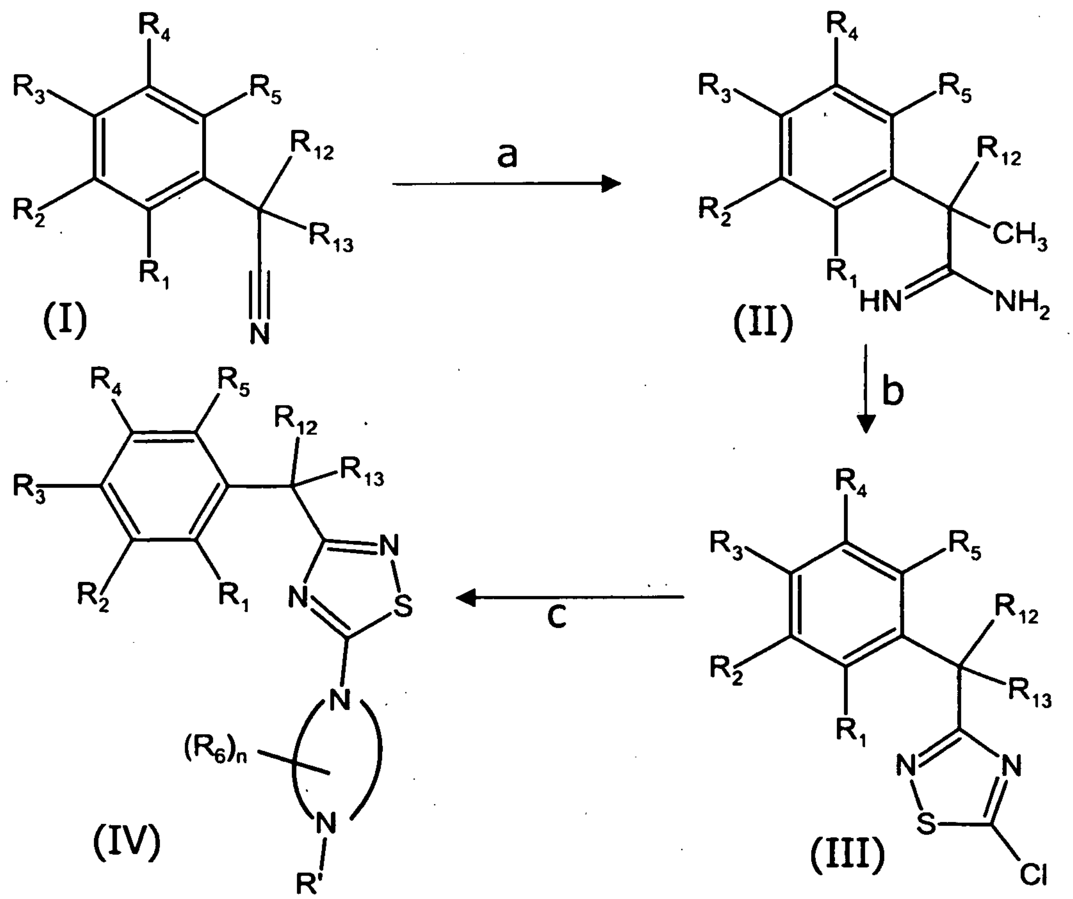


In step (a) of scheme 1, the benzonitrile derivative having the structural formula (I), optionally substituted on the aromatic ring and/or in alpha position to the cyano group, may be reacted with ammonia yielding the corresponding amidine (II).

The direct synthesis of amidines from nitriles and ammonia may be greatly facilitated by the presence of electron-withdrawing groups on the benzonitrile derivative (I). Optionally, this reaction may be carried out in the presence of an effective amount of one or more Lewis acids such as but not limited to $AlCl_3$ or $ZnCl_2$ at temperatures ranging from 20°C up to a maximum of 150-200°C. Nitriles or cyanides that may be used in step (a) include, but are not limited to, 4-Aminobenzylcyanide, 4-bromo-2,2-diphenylbutyronitrile, 3-chlorobenzyl-cyanide, 4-chlorobenzyl-cyanide, cyclohexylphenyl-acetonitrile, 4-hydroxybenzyl-cyanide, α-methylbenzylcyanide, 2-methylbenzyl-cyanide, 3-methylbenzyl-cyanide, 4-methylbenzylcyanide, 4-cyano-4-phenylcyclohexanone, 1-(2,4-dichlorophenyl)-1-cyclopropylcyanide, 4-fluorophenylacetonitrile, diphenylacetonitrile, 3,4,5-trimethoxyphenylacetonitrile, 2,2-diphenylpropionitrile, 4-bromophenylacetonitrile, 1-phenylcyclobutanecarbonitrile, 2,6-dichlorophenylacetonitrile, (3,4-dimethoxyphenyl)-acetonitrile, 4-nitrophenylacetonitrile, 1-phenyl-1-cyclopropanecarbonitrile, 1-(4-chlorophenyl)-1-cyclopropanecarbonitrile, 1-(4-methylphenyl)-1-cyclopropanecarbonitrile, 1-phenyl-1-cyclohexanecarbonitrile, 1-(4-chlorophenyl)-1-cyclohexanecarbonitrile, 1-(4-methylphenyl)-1-cyclohexanecarbonitrile, 1-(4-methoxyphenyl)-1-cyclohexanecarbonitrile, 2-nitrophenylacetonitrile, (4-methoxyphenyl)acetonitrile, 2,4-dichlorophenyl-acetonitrile, (2-methoxyphenyl)acetonitrile, benzyl cyanide, 2-chlorobenzylcyanide, 3-phenoxybenzaldehyde-cyanohydrin, 3-(trifluoromethyl)-phenylacetonitrile, (3-methoxyphenyl)-acetonitrile, 2-chloro-6-fluorophenylacetonitrile, 3,4-dichlorophenylacetonitrile, 4-amino-2-chlorodiphenylacetonitrile, 2-fluorophenylacetonitrile, 3-fluorophenylacetonitrile, 2,3,4,5,6-pentafluorophenylacetonitrile, 3,4-difluorophenylacetonitrile, 3-bromophenylacetonitrile, 2-chloro-4-fluorobenzyl cyanide, 1-(2-fluorophenyl)-cyclopentanecarbonitrile, 1-(2-fluorophenyl)-cyclohexanecarbonitrile, 1-(3-fluorophenyl)-cyclopentanecarbonitrile, 1-(3-fluorophenyl)-cyclohexanecarbonitrile, 1-(4-fluorophenyl)-cyclopentanecarbonitrile, 1-(4-fluorophenyl)-cyclohexanecarbonitrile, 1-(2-chloro-4-fluorophenyl)-cyclopentanecarbonitrile, 1-(2-chloro-4-fluorophenyl)-cyclohexanecarbonitrile, 1-(2-chloro-6-fluorophenyl)-cyclopentanecarbonitrile, 1-(2-chloro-6-fluorophenyl)-cyclohexanecarbonitrile, 2,4-difluorophenylacetonitrile, 2,5-difluorophenylacetonitrile, 2,6-difluorophenylacetonitrile, 4-(trifluoromethyl)phenylacetonitrile, 2-(trifluoromethyl)-phenylacetonitrile, 3,5-bis-(trifluoromethyl)phenylacetonitrile, 2,5-dimethylphenylacetonitrile, 2-bromophenylacetonitrile, 2,4,6-trimethylbenzylcyanide, 2,3-dichlorophenylacetonitrile, 3,4-(methylenedioxy)phenylacetonitrile, 1-(4-methoxyphenyl)-1-cyclopentanecarbonitrile, 1-(4-chlorophenyl)-1-cyclobutanecarbonitrile, 2-(4-chloro-2-fluorophenyl)-acetonitrile, 2-(3,5-difluorophenyl)-acetonitrile, 2-(4-isobutylphenyl)-propanenitrile, 2-[-4[(4-Methylbenzyl)-oxy]phenyl]acetonitrile, 1-(3-chlorophenyl)-1-cyclohexanecarbonitrile, 3-chloro-5-fluorophenylcetonitrile, 4-(trifluoromethoxy)-phenylacetonitrile, 2-phenyl-2-piperidinoacetonitrile, 4-bromo-2-fluorobenzylcyanide, 2-(4-chlorophenyl)-2-morpholinoacetonitrile, 1-(4-methoxyphenyl)-1-cyclopropanecarbonitrile, 2-(4-aminophenyl)-3-[4-(dimethylamino)phenyl] propanenitrile and 2-(4-hydroxyphenyl)-2-morpholinoacetonitrile.

According to a particular embodiment of the invention, the starting materials are selected from the group comprising 4-fluorobenzyl cyanide, 4-chlorobenzyl cyanide, 4-methylbenzyl cyanide, 3-methoxybenzyl cyanide and benzyl cyanide.

Alternatively an amidine having the structural formula (II) may be-commercially available, for example 2-(2,6-dichlorophenyl)ethanimidamide in its hydrochloride salt form, and may then be used as the starting point of scheme 1.

Subsequently, the thiadiazole core of the compounds of this invention is synthesised in step (b) in a manner similar as described in WO 99/51584. For instance, the amidine compound having the structural formula (II) may be reacted with $CCl_3SCl$ to form the corresponding 3-substituted,5-chloro-thiadiazole (III) (step (b) of scheme 1) which may then be reacted with a piperazine derivative to obtain a final compound of the invention having the structural formula (IV) wherein R' is according to formula

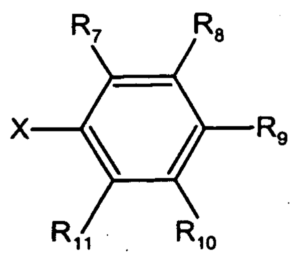

(wherein X, $R_7$, $R_8$, $R_9$, $R_{10}$, $R_{11}$ are as defined above), including intermediates having the structural formula (IV-A) (i.e. wherein R' is H) and intermediates having the structural formula (IV-B) (i.e. wherein R' is aminoethyl) shown below.

Piperazine compounds when used for the displacement reaction in step (c) directly yielding compounds of the present invention include, but are not limited to, 1-(4-nitrophenyl)piperazine, 1-(2-methoxyphenyl)piperazine, 1-(3-methoxyphenyl)-piperazine dihydrochloride, 1-phenylpiperazine, 1-(3-chlorophenyl)piperazine, 1-(4-chlorophenyl)piperazine, 1-(3,4-dichlorophenyl)piperazine, 1-(2,3-dimethylphenyl)-piperazine, 1-(2,4-dimethylphenyl)piperazine, 1-(2,5-dimethylphenyl)piperazine, 1-(3,4-dimethylphenyl)piperazine, 1-(5-chloro,2-methylphenyl)piperazine, 2-methyl-1-(3-methylphenyl)piperazine, 4-piperazinoacetophenone, 1-(4-fluorophenyl)piperazine, 1-(2-methoxyphenyl)piperazine hydrochloride, 1-(4-methoxyphenyl)piperazine, 1-(2-fluorophenyl)piperazine, 1-(3-methylphenyl)piperazine, 1-(4-methoxyphenyl)-2-methylpiperazine, 1-(2,4-di-fluorophenyl)piperazine, N-(alpha,alpha,alpha-trifluoro-p-tolyl)piperazine, 1-(4-Hydroxyphenyl)piperazine, 1-(4-methylphenyl)piperazine, 1-[2-nitro-4-(trifluoromethyl)phenyl]piperazine, 1-(2-hydroxyphenyl)piperazine, benzyl 3-oxopiperazine-1-carboxylate, 1-(2-chlorophenyl)piperazine, 1-(2-methylphenyl)-piperazine, 1-cinnamylpiperazine, trans-1-cinnamylpiperazine, 1-(4-fluorobenzyl)piperazine and 2-methyl-4-piperazinoquinoline.

Alternatively, using the same synthetic pathway, another saturated or partly unsaturated heterocyclic ring having the structural formula (A') with at least two nitrogen atoms in the said heterocyclic ring and with a total of 5 to 7 atoms such as, but not limited to, homopiperazinyl, may be introduced onto the thiadiazole core. Commercially available reagents for such synthesis, yielding final compounds of the present invention with homopiperazinyl connected to the thiadiazole core, include but are not limited to 1-(4-bromo-2-fluorobenzyl)-1,4-diazepane, 1-(4-bromo-2-fluorobenzyl)-1,4-diazepane, 1-(mesitylmethyl)-1,4-diazepane, 1-(4-bromobenzyl)-1,4-diazepane, 6-chloro-2-(1,4-diazepan-1-yl)-1,3-benzothiazole, 1-(2-chloro-6-fluorobenzyl)-1,4-diazepane, 1-(4-fluorobenzyl)-1,4-diazepane, and 5-(1,4-diazepan-1-yl)-3-phenyl-1,2,4-thiadiazole.

Scheme 2

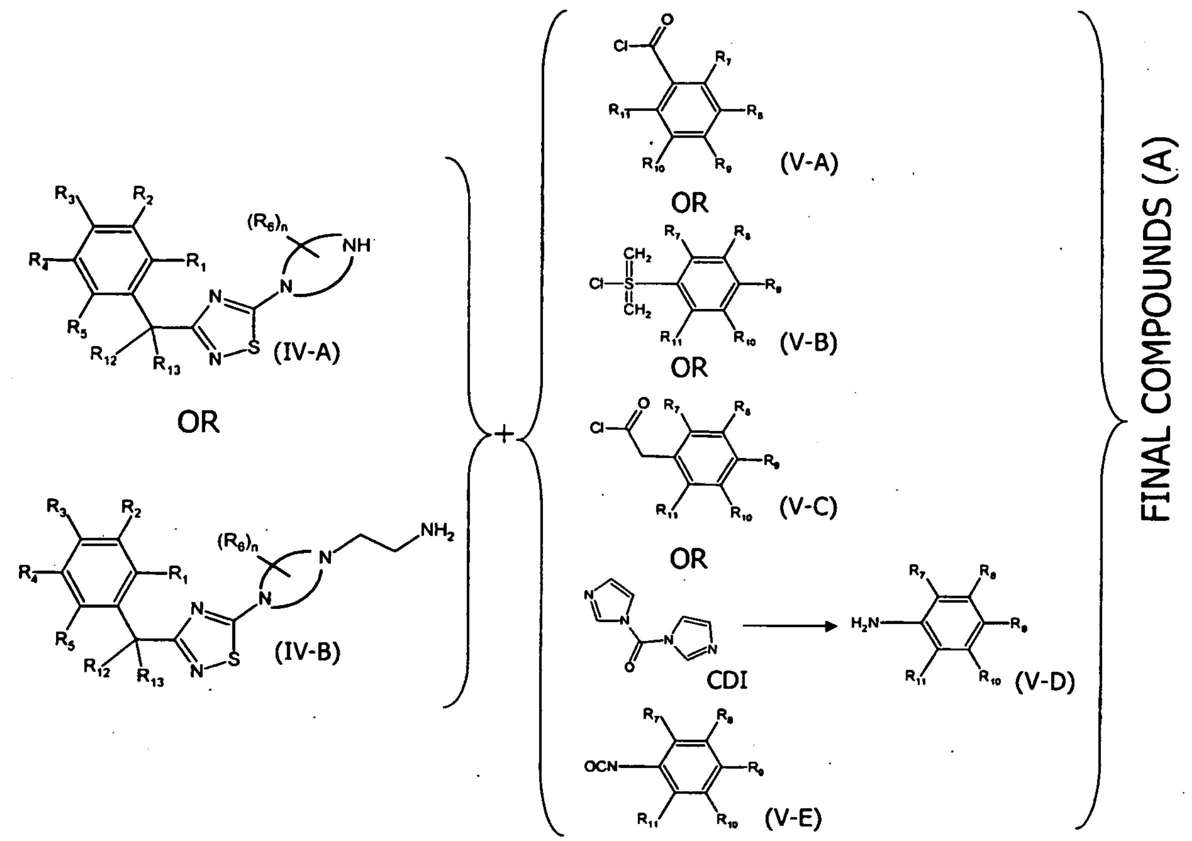


Scheme 2 schematically illustrates methods to prepare compounds according to structural formula (A) wherein step (c) comprises two subsequent reaction sub-steps. In a first sub-step, a thiadiazole compound having the structural formula (III) may first be derivatised with the optionally $R_6$-substituted heterocyclic ring with at least two nitrogen atoms in the said heterocyclic ring and with a total of 5 to 7 atoms. Examples of such heterocyclic compounds for use in step (c) for the synthesis of appropriate intermediates comprise, but are not limited to, piperazine, 2-methylpiperazine (either (R)-(-)-2-methylpiperazine or (S)-(+)-2-methylpiperazine), trans-2,5-dimethylpiperazine, homopiperazine, 1-(2-aminoethyl)piperazine. In this way, compounds as illustrated by structural formulae (IV-A) and (IV-B) in scheme 2 may be obtained.

In a second reaction sub-step comprised within step (c) (scheme 2), these intermediate compounds having the structural formulae (IV-A) and (IV-B) may be reacted with a reagent susceptible to nucleophilic attack by a non-tertiary amino group, e.g. the NH group present in compounds according to structural formula (IVA), or the terminal amino group present in compounds according to structural formula (IVB). Suitable such reagents include, but are not limited to, acid chlorides such as carbonyl chlorides or sulfonyl chlorides, or activated acids such as carboxylic acid anhydrides. Particular carbonyl chlorides for use in this reaction sub-step include benzoyl chlorides (as shown in formula V-A) and phenyl acetyl chlorides (as shown in formula V-C). Particular sulfonyl chlorides for use in this reaction sub-step include phenylsulphonyl chlorides (as shown in formula V-B).

Benzoyl chlorides (as shown in formula V-A) suitable for use in the synthesis of the compounds of the present invention include, but are not limited to, benzoyl chloride, p-anisoyl-chloride, 2-bromobenzoyl chloride, 4-bromobenzoyl chloride, 3-chlorobenzoyl chloride, pentafluorobenzoyl chloride, 2-chlorobenzoyl chloride, p-toluoyl chloride, 4-chlorobenzoyl chloride, 2,4-dichlorobenzoyl chloride, 3,4-dichlorobenzoyl chloride, 4-nitrobenzoyl chloride, 4-fluorobenzoyl chloride, 2-fluorobenzoyl chloride, o-toluoyl chloride, m-toluoyl chloride, 4-cyanobenzoyl chloride, 3-nitrobenzoyl chloride, 4-tert-butyl-benzoyl chloride, 4-biphenylcarbonyl chloride, 3,5-dimethoxybenzoyl chloride, 3-fluorobenzoyl chloride, 2,6-dichlorobenzoyl chloride, 4-butylbenzoyl chloride, 4-heptyloxybenzoyl chloride, 4-hexylbenzoyl chloride, 4-hexyloxybenzoyl chloride, 4-pentylbenzoyl chloride, m-anisoyl chloride, 2,6-difluorobenzoyl chloride, 2-nitrobenzoyl chloride, 4-chloro3nitrobenzoylchloride, 3,4-difluorobenzoyl chloride, 2-iodobenzoyl chloride, 1-naphthoyl chloride, o-anisoyl chloride, 2,4-difluorobenzoyl chloride, 4-(trifluoromethyl)benzoyl chloride, m-anisoyl chloride, 2,6-Difluorobenzoyl chloride, 2-nitrobenzoyl chloride, 4-chloro-3-nitrobenzoylchloride, 3,4-difluorobenzoyl chloride, 2-iodobenzoyl chloride, 1-naphthoyl chloride, o-anisoyl chloride, 2,4-difluorobenzoyl chloride, 4-(trifluoromethyl)benzoyl chloride, 3-(chloromethyl)-benzoyl chloride, 4-(chloromethyl)-benzoyl chloride, 3-(dichloromethyl)-benzoyl chloride, 2,3,4,5-tetrafluorobenzoyl chloride, 2,4,6-trichlorobenzoyl chloride, 2,3,4-trifluorobenzoyl chloride, 2,4,6-trifluorobenzoyl chloride, 4-bromo-2-fluorobenzoyl chloride, 2,3,5,6-tetrafluorobenzoyl chloride, 3,5-dinitrobenzoyl chloride, 4-heptylbenzoyl chloride, 4-iodobenzoyl chloride, 4-octylbenzoyl chloride, 4-pentyloxybenzoyl chloride, 4-phenylazobenzoyl chloride, 4-propylbenzoyl chloride, methyl 4-chlorocarbonylbenzoate, 3,5-dichlorobenzoyl chloride, 3-fluoro-4-(trifluoromethyl)benzoyl chloride, 2,6-dimethoxybenzoyl chloride, piperonyloyl chloride, 2,4-dimethoxybenzoyl chloride, 3,4-dihydro-2H-1,5-benzodioxepine-6-carbonyl chloride, 2,3-dihydro-1,4-benzodioxine-6-carbonyl chloride, 2,3-dihydro-1,4-benzodioxine-5-carbonyl chloride, 1-benzofuran-5-carbonyl chloride, 2,1,3-benzothiadiazole-4-carbonyl chloride, 2,1,3-Benzothiadiazole-5-carbonyl chloride, 1,2,3-benzothiadiazole-5-carbonyl chloride, 2,1,3-benzoxadiazole-5-carbonyl chloride, 6-quinoxalinecarbonyl chloride, 4-(2-thienyl)-benzoyl chloride, 4-methyl-3,4-dihydro-2H-1,4-benzoxazine-7-carbonyl chloride, 4-(1,2,3-thiadiazol-4-yl)benzoyl chloride, 4-(1H-pyrazol-1-yl)benzoyl chloride, 1-methyl-1H-1,2,3-benzotriazole-5-carbonyl chloride, 1-benzothiophene-5-carbonyl chloride, 2,2-dimethyl-2,3-dihydro-1-benzofuran-7-carbonyl chloride, 4-[(dipropylamino)sulfonyl] benzene-1-carbonyt chloride, 4-[3-(trifluoromethyl)-1H-pyrazol-1-yl]-benzoyl chloride, 2-bromo-5-methoxybenzene-1-carbonyl chloride, 5-bromo-2,3,4-trimethylbenzoyl chloride, 2-chloro-6-fluorobenzene-1-carbonyl chloride, 2,3-dimethylbenzene-1-carbonyl chloride, 3,4-dimethylbenzene-1-carbonyl chloride, 2-chloro-4-fluorobenzoyl chloride, 5,5,8,8-tetramethyl-5,6,7,8-tetrahydro-2-naphthalenecarbonyl chloride, 2-(4-methoxyphenoxy)-5-nitrobenzene-1-carbonyl chloride, 2,3-difluorobenzoyl chloride, 2-Fluoro-5-(trifluoromethyl)benzoyl chloride, 2,3,6-trifluorobenzoyl chloride, 1-isopropyl-1 h-1,2,3-benzotriazole-5-carbonyl chloride, 1-isopropyl-1H-1,2,3-benzotriazole-5-carbonyl chloride, 3-fluoro-4-methylbenzoyl chloride, 3-(cyclopentyloxy)-4-methoxybenzoyl chloride, 4-fluoro-3-(trifluoromethyl)benzoyl chloride, 2,3-dihydro-1-benzofuran-7-carbonyl chloride, 3-(2-methyl-thiazol-4-yl)-benzoyl chloride, 1-isopropyl-2-(trifluoromethyl)-1H-benzimidazole-5-carbonyl chloride, 5-bromo-2,3-dihydrobenzo[b]furan-7-carbonyl chloride, 2,4,6-trimethylbenzoyl chloride, 2-(2-thienyl)-benzoyl chloride, 3-cyanobenzoyl chloride, acetylsalicyloyl chloride, 3-(5-methyl-1,2,4-oxadiazol-3-yl)-benzoyl chloride and 4-(5-methyl-1,2,4-oxadiazol-3-yl)-benzoyl chloride.

According to a particular embodiment the benzoyl chloride reagent may be selected from the group consisting of 2-fluorobenzoyl chloride, 4-ethylbenzoyl chloride, 4-butylbenzoyl chloride, 4-methoxybenzoyl chloride, piperonyloyl chloride, 4-hexylbenzoyl chloride, 3-chlorobenzoyl chloride, 4-fluorobenzoyl chloride, p-toluoyl chloride, 3-fluorobenzoyl chloride, 4-chlorobenzoyl chloride, benzoyl chloride, 4-tert-butylbenzoyl chloride, 4-biphenylcarbonyl chloride, o-anisoyl chloride, 1-naphthoyl chloride, 2-naphthoyl chloride, 4-pentylbenzoyl chloride, 4-bromobenzoyl chloride, 2,4-dimethoxybenzoyl chloride, 3,5-dichlorobenzoyl chloride, 3-bromobenzoyl chloride, 2-bromobenzoyl chloride 3-trifluoromethylbenzoyl chloride, 4-trifluoromethylbenzoyl chloride and 2-ethylbenzoyl chloride.

Numerous other carbonyl chlorides are known to the person skilled in the art and commercially available for use as acylating reagent for use in the reaction step illustrated in scheme 2. Particular carbonyl chlorides for use in the method of the invention include, but are not limited to, cinnamoyl chloride, hydrocinnamoyl chloride, 2-phenylbutyryl chloride, phenylacetyl chloride and 4-fluorophenylacetyl chloride.

Phenylsulfonyl chlorides (structural formula V-B) suitable for use in the synthesis of the compounds of the present invention include, but are not limited to, 4-fluorobenzenesulfonyl chloride, 2-mesitylenesulfonyl chloride, 4-methoxybenzenesulfonyl chloride, p-toluenesulfonyl chloride, pentafluorobenzenesulfonyl chloride, benzenesulfonyl chloride, 4-bromobenzenesulfonyl chloride, N-acetylsulfanilyl chloride, 2,4,6-triisopropyl-benzenesulfonyl chloride 2-naphthalenesulfonylchloride, 4-chloro-benzenesulfonyl chloride 3,5-dichloro-2-hydroxy-benzenesulfonylchloride, 2,5-dichlorobenzenesulfonyl chloride, pipsyl chloride, 1-naphthalenesulfonylchloride, methyl 2-(chlorosulfonyl)-benzoate, 4-*tert*-butylbenzenesulfonyl chloride, 3-(trifluoromethyl)benzenesulfonyl chloride, 2-bromobenzenesulfonyl chloride, 4-acetylbenzenesulfonylchloride, 2-(trifluoromethyl)-benzenesulfonyl chloride, 3,4-dichlorobenzenesulfonyl chloride, 3,4-dimethoxybenzenesulfonyl chloride, 3-chlorobenzenesulfonyl chloride, 2-chloro-4-fluorobenzenesulfonyl chloride, 3,5-dichlorobenzenesulfonyl chloride, 3-chloro-4-fluorobenzenesulfonyl chloride, 2,4-dichlorobenzenesulfonyl chloride, 2,5-dimethoxybenzenesulfonyl chloride, 3-bromobenzenesulfonyl chloride, 2,3-dichlorobenzenesulfonyl chloride, 5-fluoro-2-methylbenzenesulfonyl chloride, 3-fluorobenzenesulfonyl chloride, 2,3,5,6-tetramethylbenzenesulfonyl chloride, 3-chloro-2-methylbenzenesulfonyl chloride, 2,5-Dibromo-3,6-difluorobenzenesulfonyl chloride, 2,6-difluorobenzenesulfonyl chloride, 2-Chlorobenzenesulfonyl chloride, 5-bromo-2-methoxybenzenesulfonyl chloride, 5-chloro-2-methoxybenzenesulfonyl chloride, 2,4-difluorobenzenesulfonyl chloride, 2-cyanobenzenesulfonyl chloride, 2-chloro-5-(trifluoromethyl)-benzenesulfonyl chloride, 4-bromomethylbenzenesulfonyl chloride, 2,4-dimethoxybenzenesulfonyl chloride, 4-chloro-3-nitrobenzenesulfonyl chloride, 4-(chlorosulfonyl)-benzoic acid, 3-nitrobenzenesulfonyl chloride, 4-nitrobenzenesulfonyl chloride, 2-(methylsulfonyl)-benzenesulfonyl chloride, 4-(methylsulfonyl)-benzenesulfonyl chloride, 3-(chlorosulfonyl)-benzoic acid, 2,4-dichloro-5-methylbenzenesulfonyl chloride, 4-(trifluoromethoxy)-benzenesulfonyl chloride, 2-methoxy-4-nitrobenzenesulfonyl chloride, 4-bromo-2-chlorobenienesulfonyl chloride, 2,3-dihydro-1-benzofuran-5-sulfonyl chloride, 2,3-dihydro-1,4-benzodioxine-6-sulfonyl chloride, 1,3-benzothiazole-6-sulfonyl chloride, 2,1,3-benzothiadiazole4sulfonyl chloride, 2,1,3-benzothiadiazole-5-sulfonyl chloride, 2,1,3-benzoxadiazole-4-sulfonyl chloride, 3,4-dihydro-2H-1,5-benzodioxepine-7-sulfonyl chloride, 4-methyl-3,4-dihydro-2H-1,4-benzoxazine-7-sulfonyl chloride, 4-(1,3-oxazol-5-yl)benzenesulfonyl chloride, 4-(1,2,3-thiadiazol-4-yl)benzenesulfonyl chloride, 4-(1H-pyrazol-1-yl) benzenesulfonyl chloride, 4-(3-chloro-2-cyanophenoxy)benzene-1-sulfonyl chloride, 5-chlorosulfonyl-2-hydroxybenzoic acid, 4-bromo-2,5-difluorobenzene-1-sulfonyl chloride, 4-(Acetylamino)-3-chlorobenzene-1-sulfonyl chloride, 3,5-di-(trifluoromethyl)-benzene-1-sulfonyl chloride, 2-fluorobenzenesulfonyl chloride, 4-methyl-3-nitrobenzene-1-sulfonyl chloride, 5-chloro-2,1,3-benzoxadiazole-4-sulfonyl chloride, 3-(5-methyl-1,3,4-oxadiazol-2-yl)benzenesulfonyl chloride, methyl 3-(chlorosulfonyl)-4-methoxybenzoate, 4-bromo-2-(trifluoromethyl)-benzenesulfonyl chloride, 2,2-dimethyl-6-chromanesulfonyl chloride, 4-(morpholine-4-sulfonyl)benzenesulfonyl chloride, 4-(pyrrolidine-1-sulfonyl)-benzenesulfonyl chloride, 3-(2-methyl-4-pyrimidinyl)benzenesulfonyl chloride, 2-cyano-5-methylbenzenesulfonyl chloride, 2,5-dimethylbenzene-sulfonyl chloride, 4-chloro-3-(trifluoromethyl)-benzenesulfonyl chloride, 4-bromo-2-methylbenzene-1-sulfonyl chloride, 2-chloro-4-(trifluoromethyl)-benzene-1-sulfonyl chloride, 2-chloro-4-cyanobenzene-1-sulfonyl chloride, 2,6-dichloro-4-(trifluoromethyl)benzene-1-sulfonyl chloride, 3,4-difluorobenzene-1-sulfonyl chloride, 2-lodobenzene-1-sulfonyl chloride, 4-methyl-1-naphthalenesulfonyl chloride, 4-(trifluoromethyl)benzene-1-sulfonyl chloride, 2,6-dichlorobenzene-1-sulfonyl chloride, 2-(trifluoromethoxy)benzene-1-sulfonyl chloride, 4-cyanobenzene-1-sulfonyl chloride, 4-butoxybenzene-1-sulfonyl chloride, 2,3,4-trifluorobenzene-1-sulfonyl chloride, 4-bromo-2-(trifluoromethoxy)benzene-1-sulfonyl chloride, 3-cyanobenzene-1-sulfonyl chloride, 3-chloro-4-methylbenzene-1-sulfonyl chloride, 4-bromo-2-ethylbenzene-1-sulfonyl chloride, 5,5,8,8-tetramethyl-5,6,7,8-tetrahydro-2-naphthalenesulfonyl chloride, 4-(2-Chloro-6-nitrophenoxy)benzene-1-sulfonyl chloride, 3,5-dichloro-4-(2-chloro-4-nitrophenoxy)benzene-1-sulfonyl chloride, 4-pentylbenzene-1-sulfonyl chloride, 4-ethylbenzene-1-sulfonyl chloride, 4-propylbenzene-1-sulfonyl chloride, 4-butylbenzene-1-sulfonyl chloride, 3-toluenesulfonyl chloride, 4-isopropylbenzenesulfonyl chloride, 4-(2-oxo-1-pyrrolidinyl)benzene sulfonyl chloride, 4-(2-Methoxyphenoxy)benzenesulfonyl chloride, 4-(2-Chlorophenoxy)benzenesulfonyl chloride, 4-(2-Methylphenoxy)benzenesulfonyl chloride, 4'-Chloro(1,1'-biphenyl)-4-sulfonyl chloride, 4'-Fluoro(1,1'-biphenyl)-4-sulfonyl chloride, 4'-Methoxy-(1,1'-biphenyl)-4-sulfonyl chloride, 3',4'-Dichloro-(1,1'-biphenyl)-4-sulfonyl chloride, 4-Phenoxybenzenesulfonyl chloride, 4'-Methyl-(1,1'-biphenyl)-4-sulfonyl chloride, 5-Bromo-2,3-dihydrobenzo[b]furan-7-sulphonyl chloride, 3,4,5-Trifluorobenzenesulfonyl chloride, 3-(5-methyl-1,2,4-oxadiazol-3-yl)benzenesulfonyl chloride, 4-(2-methyl-1,3-thiazol-4-yl)benzenesulfonyl chloride, 1-Acetyl-5-indolinesulfonyl chloride, 3-(2-methyl-1,3-thiazol-4-yl)benzenesulfonyl chloride and 1,3-benzodioxole-5-sulfonyl chloride.

Phenylacetyl chlorides (structural formula V-C) suitable for use in the synthesis of the compounds of the present invention include, but are not limited to, phenylacetyl chloride, 4-methoxyphenylacetyl chloride, 2-(2-naphthyl)acetyl chloride, 2-(3,5-difluorophenyl)ethanoyl chloride, 2-(1-naphthyl)ethanoyl chloride, 4-chlorophenylacetyl chloride, 3-methoxyphenylacetyl chloride, and 4-fluorophenylacetyl chloride.

The intermediate compounds having the structural formulae (IV-A) and (IV-B) may also be reacted with an isocyanate (structural formula V-E shown above) to yield final compounds wherein X comprises a urea linkage.

Isocyanates suitable for use in the synthesis of the compounds of the present invention include, but are not limited to, 4-fluorophenyl isocyanate, phenyl isocyanate, m-Tolyl isocyanate, p-Tolyl isocyanate, 4-Chlorophenyl isocyanate, Ethyl 4-isocyanatobenzoate, 2-Fluorophenyl isocyanate, 3-Fluorophenyl isocyanate, alpha,alpha,alpha-Trifluoro-o-tolyl isocyanate, Tolylene-2,4-diisocyanate, Tolylene-2,6-diisocyanate, 4-Methoxyphenyl isocyanate, 4-Bromophenyl isocyanate, 2-Methoxyphenyl isocyanate, 3-Methoxyphenyl isocyanate, 2,4-Dichlorophenyl isocyanate, o-Tolyl isocyanate, 3,4-Dichlorophenyl isocyanate, 2-Nitrophenyl isocyanate, 4-Nitrophenyl isocyanate, 2,4-Difluorophenyl isocyanate, 2-Bromophenyl isocyanate, 2,6-Difluorophenyl isocyanate, 2-(Trifluoromethoxy)phenyl isocyanate, 2-chloro-5-(trifluoromethyl)phenyl isocyanate, 4-chloro-2-(trifluoromethyl)phenyl isocyanate, 4-Chloro-3-(trifluoromethyl)phenyl isocyanate, 2,5-Difluorophenyl isocyanate, 4-(Trifluoromethoxy)phenyl isocyanate, 2-Ethoxyphenyl isocyanate, 4-Ethoxyphenyl isocyanate, 4-Isopropylphenyl isocyanate, 3-Acetylphenyl isocyanate, 2,6-Diisopropylphenyl isocyanate, 3-Bromophenyl isocyanate, 3,5-Dichlorophenyl isocyanate, 4-Fluoro-3-nitrophenyl isocyanate, 3,5-dimethylphenyl isocyanate, 3,5-bis(trifluoromethyl)phenyl isocyanate, 3-cyanophenyl isocyanate, 4-(methylthio)-phenyl isocyanate, 2-Ethylphenyl isocyanate, 2,6-Dimethylphenyl isocyanate, alpha,alpha,alpha-Trifluoro-p-tolyl isocyanate, 2,3-Dichlorophenyl isocyanate, 4-methyl-3-nitrophenyl isocyanate, 2,4-dimethoxyphenyl isocyanate, 4-(chloromethyl)-phenyl isocyanate, 4-Bromo-2-chlorophenyl isocyanate, 2-Bromo-4,6-difluorophenyl isocyanate, 4-Bromo-2-fluorophenyl isocyanate, 4-(Dimethylamino)phenyl isocyanate, 2-Fluoro-5-methylphenyl isocyanate, 4-Fluoro-2-nitrophenyl isocyanate, 2-Fluoro-3-(trifluoromethyl)phenyl isocyanate, 2-Fluoro-5-(trifluoromethyl)phenyl isocyanate, 2-fluoro-6-(trifluoromethyl)phenyl isocyanate, 4-fluoro-2-(trifluoromethyl)-phenyl isocyanate, 4-fluoro-3-(trifluoromethyl)phenyl isocyanate, 4-(heptyloxy) phenyl isocyanate, 2-lodophenyl isocyanate, 2-Naphthyl isocyanate, 2-n-Propylphenyl isocyanate, 4-(Trifluoromethylthio)phenyl isocyanate, 2,3,4-Trifluorophenyl isocyanate, 2,6-Dichlorophenyl isocyanate, 3-Nitrophenyl isocyanate, 3-Chlorophenyl isocyanate, 2-Chlorophenyl isocyanate, 1-Naphthyl isocyanate, 2,3-Dimethylphenyl isocyanate, 3-Chloro-4-fluorophenyl isocyanate, 2,5-Dimethylphenyl isocyanate, 3,4-Difluorophenyl isocyanate, 2,3-Dihydro-1-benzofuran-5-yl isocyanate, 2,3-Dihydro-1,4-benzodioxin-6-yl isocyanate, 6-Fluoro-4H-1,3-benzodioxin-8-yl isocyanate, 2,1,3-Benzothiadiazol-4-yl isocyanate, 3,4-Dihydro-2H-1,5-benzodioxepin-7-yl isocyanate, 3-(Cyclopentyloxy)-4-methoxyphenyl

isocyanate, 2-(Methylthio)phenyl isocyanate, 2-(tert-Butyl)phenyl isocyanate, 4-(tert-Butyl)phenyl isocyanate, 3-Chloro-2-methylphenyl isocyanate, 4-Butyl-2-methylphenyl isocyanate, 2-Ethyl-6-methylphenyl isocyanate, 4-Chloro-3-nitrophenyl isocyanate, 4-Bromo-2-methylphenyl isocyanate, 3-(Methylthio)phenyl isocyanate, 5,5,8,8-Tetramethyl-5,6,7,8-tetrahydro-2-naphthalenyl isocyanate, 5-Fluoro-2-methylphenyl isocyanate, 4-Phenoxyphenyl isocyanate, 4-Methoxy-2-methylphenyl isocyanate, alpha,alpha,alpha-Trifluoro-m-tolyl isocyanate, 2,6-Dibromo-4-isopropylphenyl isocyanate, 2,6-Dimethoxyphenyl isocyanate, 2-(4-Isocyanatophenyl)thiophene, 4-(3-Isocyanatophenyl)-2-methyl-1,3-thiazole, 3-(3-Isocyanatophenyl)-5-methyl-1,2,4-oxadiazole, 1-Benzothiophen-5-yl isocyanate, 1-(3-isocyanatophenyl)-1 h-pyrrole, 1-(4-isocyanatophenyl)-1H-pyrrole, 3,5-Dimethoxyphenyl isocyanate and 2,4,6-trichlorophenyl isocyanate.

Alternatively, intermediates having the structural formula (IV-A) or (IV-B) may be derivatised with a carbonylation agent such as, but not limited to, trisphosgene, carbonyl diimidazole (hereinafter abbreviated as CDI) or carbonyl ditriazole. The resulting imidazo-carbonyl compound may then be further reacted with an amino compound, particularly an aniline derivative wherein $R_7$, $R_8$, $R_9$, $R_{10}$, $R_{11}$ are as defined above, thereby providing further compounds of the invention. The carbonylation and further urea formation with an amine may be most particularly carried out in a one-pot procedure. Amino compounds suitable for the latter reaction include arylamines, arylalkylamines, arylalkenylamines, arylalkynylamines wherein said one or more of the carbon atoms in the alkyl, alkenyl or alkynyl moiety is optionally replaced by a heteroatom selected from the group comprising O, N and S, and wherein each of said amino compounds is optionally substituted.

Suitable aniline derivatives for the above reaction step include, but are not limited, to 2,6-dimethylaniline, 2-methylaniline, 3-fluoroaniline, 4-ethylaniline, 2, 4-dimethoxyaniline, 2,6-dichloroaniline, 3-cyanoaniline, and 2,4-fluoroaniline.

When step (c) comprises two sub-steps as described herein-above, the order of performing the different reactions is not critical for the invention and therefore can be changed. For example, the optionally substituted heterocyclic ring with at least two nitrogen atoms in the said heterocyclic ring and with a total of 5 to 7 atoms may first be reacted with either one of the reagents having the structural formula (V-A), (V-B), or (V-C), or CDI followed by reaction with a compound having the structural formula (V-D). The resulting compound may then be used for reaction with the 3-substituted-5-chloro-thiadiazole having the structural formula (III) to yield a final compound according to the invention.

The above description provides a general synthesis scheme for making the 1,2,4-thiadiazole compounds of the present invention. A list of exemplary, but non-limiting, compounds which have been synthesised according to the described methods is provided in Table 1 herein.

The 1,2,4-thiadiazole derivatives having the above structural formula (A) may be in the form of a pharmaceutically acceptable salt. The latter include any therapeutically active non-toxic addition salt which 1,2,4-thiadiazole compounds having the general formula (A) are able to form with a salt-forming agent. Such addition salts may conveniently be obtained by treating the said derivative of the invention with an appropriate salt-forming acid or base. For instance, derivatives having basic properties may be converted into the corresponding therapeutically active, non-toxic acid addition salt form by treating the free base form with a suitable amount of an appropriate acid following conventional procedures. Examples of such appropriate salt-forming acids include, for instance, inorganic acids resulting in forming salts such as but not limited to hydrohalides (e.g. hydrochloride and hydrobromide), sulfate, nitrate, phosphate, diphosphate, carbonate, bicarbonate, and the like; and organic monocarboxylic or dicarboxylic acids resulting in forming salts such as, for example, acetate, propanoate, hydroxyacetate, 2-hydroxypropanoate, 2-oxopropanoate, lactate, pyruvate, oxalate, malonate, succinate, maleate, fumarate, malate, tartrate, citrate, methanesulfonate, ethanesulfonate, benzoate, 2-hydroxybenzoate, 4-amino-2-hydroxybenzoate, benzenesulfonate, p-toluenesulfonate, salicylate, p-aminosalicylate, pamoate, bitartrate, camphorsulfonate, edetate, 1,2-ethanedisulfonate, fumarate, glucoheptonate, gluconate, glutamate, hexylresorcinate, hydroxynaphtoate, hydroxyethanesulfonate, mandelate, methylsulfate, pantothenate, stearate, as well as salts derived from ethanedioic, propanedioic, butanedioic, (Z)-2-butenedioic, (E)2-butenedioic, 2-hydroxybutanedioic, 2,3-dihydroxybutane-dioic, 2-hydroxy-1,2,3-propanetricarboxylic and cyclohexanesulfamic acids and the like.

The 1,2,4-thiadiazole derivatives of the general formula (A) having acidic properties may be converted in a similar manner into the corresponding therapeutically active, non-toxic base addition salt form. Examples of appropriate salt-forming bases include, for instance, inorganic bases like metallic hydroxides such as but not limited to those of alkali and alkaline-earth metals like calcium, lithium, magnesium, potassium and sodium, or zinc, resulting in the corresponding metal salt; organic bases such as but not limited to ammonia, alkylamines, benzathine, hydrabamine, arginine, lysine, N,N'-dibenzylethylenediamine, chloroprocaine, choline, diethanolamine, ethylene-diamine, N-methylglucamine, procaine and the like.

Reaction conditions for treating the 1,2,4-thiadiazole derivatives having the structural formula (A) of this invention with an appropriate salt-forming acid or base are similar to standard conditions involving the same acid or base but different organic compounds with basic or acidic properties, respectively. Preferably, in view of its use in a pharmaceutical composition or in the manufacture of a medicament for treating specific diseases, the pharmaceutically acceptable salt will be designed, i.e. the salt-forming acid or base-will be selected so as to impart greater water-solubility, lower toxicity, greater stability and/or slower dissolution rate to the derivative of this invention.

In order to suitably use a 1,2,4-thiadiazole compound disclosed in this invention or a pharmaceutically acceptable salt, pro-drug or solvate thereof, for the therapeutic treatment (including prophylactic treatment) of mammals including humans, it is usually formulated in accordance with standard pharmaceutical practice as a pharmaceutical composition including one or more appropriate pharmaceutically acceptable excipients.

In another embodiment, this invention provides combinations, preferably synergistic combinations, of one or more derivatives represented by the general formula (A) with one or more biologically-active drugs being preferably selected from the group consisting of neuro-protective agents and α-synuclein deposition inhibitors. As is conventional in the art, the evaluation of a synergistic effect in a drug combination may be made by analysing the quantification of the interactions between individual drugs, using the median effect principle described by Chou et al. in Adv. Enzyme Reg. (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a, 2a), or in combination with the second or respectively first drug (1c, 2c), which is needed to produce a given effect. The said first and second drug have synergistic or additive or antagonistic effects depending upon $CI < 1$, $CI = 1$, or $CI > 1$, respectively. As will be explained in more detail herein below, this principle may be applied to a number of desirable effects such as, but not limited to, an activity against neurodegenerative disorders.

The term " pharmaceutically acceptable carrier or excipient " as used herein in relation to pharmaceutical compositions and combined preparations means any material or substance with which the active principle, i.e. the 1,2,4-thiadiazole derivative of the general formula (A), and optionally the neuro-protective agent or α-synuclein deposition inhibitor, may be formulated in order to facilitate its application or dissemination to the locus to be treated, for instance by dissolving, dispersing or diffusing the said composition, and / or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art. There is no particular restriction to their selection within the present invention although, due to the usually low or very low water-solubility of the derivatives of this invention, special attention will be paid to the selection of suitable carrier combinations that can assist in properly formulating them in view of the expected time release profile. Suitable pharmaceutical carriers include additives such as wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals.

The pharmaceutical compositions of the present invention may be prepared in any known manner, for instance by homogeneously mixing, dissolving, spray-drying, coating and/or grinding the active ingredients, in a one-step or a multi-steps procedure, with the selected carrier material and, where appropriate, the other additives such as surface-active agents. may also be prepared by micronisation, for instance in view to obtain them in the form of microspheres usually having a diameter of about 1 to 10 μm, namely for the manufacture of microcapsules for controlled or sustained release of the biologically active ingredient(s).

Suitable surface-active agents to be used in the pharmaceutical compositions of the present invention are non-ionic, cationic and/or anionic surfactants having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($W_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalenesulphonic acid/formaldehyde condensation product. Also suitable are the corresponding phosphates, e.g. salts of phosphoric acid ester and an adduct of p-nonylphenol with ethylene and / or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidylcholine, dipalmitoylphoshatidylcholine and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and / or 10 to 100 propyleneglycol ether groups. Such compounds usually contain from 1 to 5 ethyleneglycol units per propyleneglycol unit. Representative examples of non-ionic surfactants are nonylphenol-polyethoxyethanol, 1, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having four hydrocarbon radicals optionally substituted with halo, phenyl, substituted phenyl or hydroxy; for instance quaternary ammonium salts containing as N-substituent at least one $C_8$-$C_{22}$ alkyl radical (e.g. cetyl, lauryl, palmityl, myristyl, oleyl and the like) and, as further substituents, unsubstituted or halogenated lower alkyl, benzyl and / or hydroxy-$C_{1-4}$ alkyl radicals.

A more detailed description of surface-active agents suitable for this purpose may be found for instance in " McCutcheon's Detergents and Emulsifiers Annual " (MC Publishing Crop., Ridgewood, New Jersey, 1981), " Tensid-Taschenbuch ", 2nd ed. (Hanser Verlag, Vienna, 1981) and " Encyclopaedia of Surfactants (Chemical Publishing Co., New York, 1981).

Structure-forming, thickening or gel-forming agents may be included into the pharmaceutical compositions and combined preparations of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may be included into the pharmaceutical compositions and combined preparations of the present invention include, but are not limited to, cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof. Gelatin and modified celluloses represent a preferred class of gelling agents.

Other optional excipients which may be included in the pharmaceutical compositions and combined preparations of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium meta bisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions and combined preparations of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxy-methylcellulose, protamine sulfate and the like. The rate of drug release and duration of action may also be controlled by incorporating the active ingredient into particles, e.g. microcapsules, of a polymeric substance such as hydrogels, polylactic acid, hydroxymethyl-cellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition or combined preparation of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins and the like, and mixtures thereof.

In order to suitably use compounds disclosed in this invention for therapeutic or prophylactic purpose, such compounds are preferably administered so that a daily dose in the range of, for example, 0.1 mg to 75 mg per kg body weight is received, said daily dose being given if required in divided sub-doses. In general, lower doses will be administered when a parenteral route is employed. Thus, for example, for intravenous administration, a dose in the range of, for example, 0.5 mg to 30 mg per kg body weight will preferably be used. Similarly, for administration by inhalation, a dose in the range of, for example, 0.5 mg to 25 mg per kg body weight will preferably be used. According to a particular embodiment, the envisaged administration route for the compounds of the invention is oral administration, particularly in tablet form. Typically, unit dosage forms will contain about 1 mg to 500 mg of a compound of this invention.

Another embodiment of this invention includes the various precursor or " pro-drug " forms of the compounds of the present invention. It may be desirable to formulate the compounds of the present invention in the form of a chemical species which itself is not significantly biologically-active, but which when delivered to the body of a human being or higher mammal will undergo a chemical reaction catalysed by the normal function of the body, *inter alia,* enzymes present in the stomach or in blood serum, said chemical reaction having the effect of releasing a compound as defined herein. The term " pro-drug " thus relates to these species which are converted *in vivo* into the active pharmaceutical ingredient. The pro-drugs of the present invention can have any form suitable to the formulator, e.g. esters. In the present case, however, the pro-drug may necessarily exist in a form wherein a covalent bond is cleaved by the action of an enzyme present at the target *locus*. For example, a C-C covalent bond may be selectively cleaved by one or more enzymes at said target *locus* and, therefore, a pro-drug in a form other than an easily hydrolysable precursor, *inter alia* an ester, an amide, and the like, may be used.

For the purposes of the present invention the term " therapeutically suitable pro-drug " is defined herein as a compound modified in such a way as to be transformed *in vivo* to the therapeutically active form, whether by way of a single or by multiple biological transformations, when in contact with the tissues of humans or mammals to which the pro-drug has been administered, and without undue toxicity, irritation, or allergic response, and achieving the intended therapeutic outcome.

## BRIEF DESCRIPTION OF THE DRAWINGS

Figure 1 shows the sensitivity of an α-synuclein expressing neuroblastoma cell line to paraquat according to one embodiment of the invention.

Figure 2 shows the inhibition of α-synuclein mediated toxicity by two exemplary compounds of this invention, being N-(2,6-dimethylphenyl)-4-[3-(4-fluorobenzyl)-1,2,4-thiadiazol-5-yl]-2-methylpiperazine-1-carboxamide (compound 19, squares) and 2-phenyl-N-{2-[4-[3-(4-fluorobenzyl)-1,2,4-thiadiazol-5-yl]piperazin-1-yl]ethyl}butanamide (compound 111, triangles).

Figure 3 shows inhibition of paraquat-triggered degeneration of substantia nigra cells, using 1-[4-methoxyphenylsulfonyl]-4-[3-(3-methoxybenzyl)-1,2,4-thiadiazol-5-yl]piperazine (compound 53), *in vivo.*

## Example 1 - amidine formation

Illustrative compounds of the present invention have been prepared according to the synthetic pathway illustrated in schemes 1 and 2 herein-description.

Amidine formation according to step (a) of scheme 1, is schematically shown below:

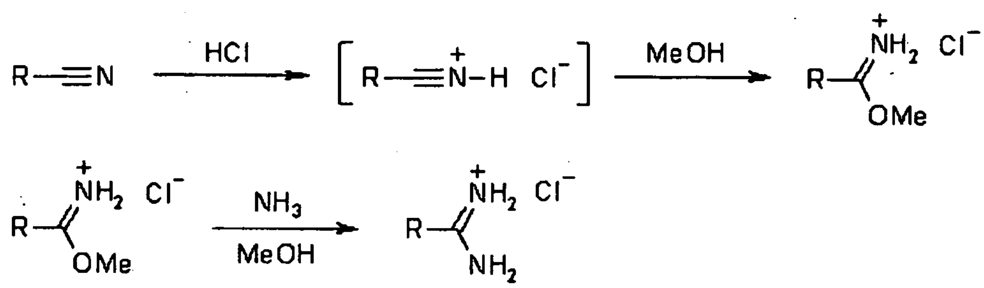


The nitrile compound is treated with gaseous HCl in a mixture of anhydrous chloroform and methanol to yield the imino ether hydrochloride. Subsequently, the mixture is treated with dry ammonia to yield the amidine compound.

Example 2 - synthesis of intermediate 3-benzyl-5-chloro-1,2,4-thiadiazole derivatives

The intermediate 3-benzyl-5-chloro-1,2,4-thiadiazole derivatives in step (b) of scheme 1 have been obtained according to the following procedure. In a three-necked 500mL flask equipped with a mechanical stirrer, a dropping funnel and a thermometer, dichloromethane (DCM) (130 ml) was charged and the appropriate amidine hydrochloride (0.1 mol) was suspended in it upon efficient stirring. Then perchloromethyl mercaptane (16.73 g) was added to the suspension. The stirred solution was cooled to -14˚C by using ammonium chloride-ice cooling bath. Then aqueous NaOH solution (0.5 mole dissolved in 30 ml distilled water) was added dropwise to the solution upon efficient stirring while keeping the inner temperature accurately below -8˚C. When the addition was finished the reaction mixture was stirred for another hour while the temperature was let to rise to room temperature. The precipitated NaCl was filtered off and washed with DCM. The organic phase of the filtrate was separated and saved. The aqueous phase was washed with 3x20 ml DCM. The collected organic phases including the previously saved solution were washed with water (4x20 ml). The organic phase was dried over anhydrous sodium sulphate and evaporated to dryness. The residue was distilled in high motor vacuum using a vacuum-jacketed Vigreaux-column.

For different intermediate species, the following data were obtained:

- 5-Chloro-3-(4-methylbenzyl)-[1,2,4]thiadiazole: starting from 31.8 g of 4-methylbenzyl amidine hydrochloride, 16.7 g of the title compound was obtained (yield : 48%); boiling point (b.p.): 130-135 ˚C/1 Hg mm.

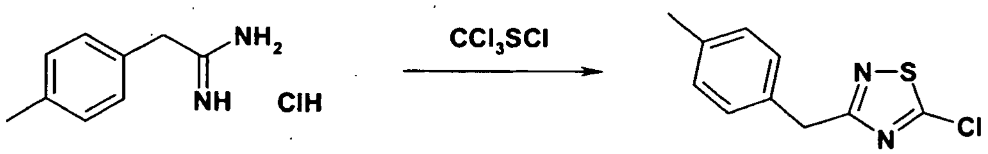


- 5-Chloro-3-(4-fluorobenzyl)-[1,2,4]thiadiazole: starting from 18.4 g (97.5 mmole) 4-fluorobenzyl amidine hydrochloride, 13.45 g of the title compound was obtained (yield : 67%); b.p.: 120-125 ˚C/1 Hg mm.

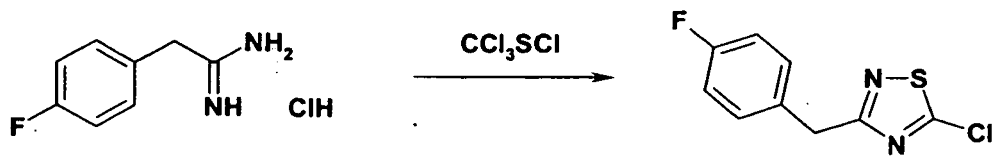


- 5-Chloro-3-(3-methoxybenzyl)-[1,2,4]thiadiazole: starting from 84.8 g of 3-methoxybenzyl amidine hydrochloride, 68.7 g of the title compound was obtained (yield : 75%); b.p.: 132-135 ˚C/1 Hg mm.

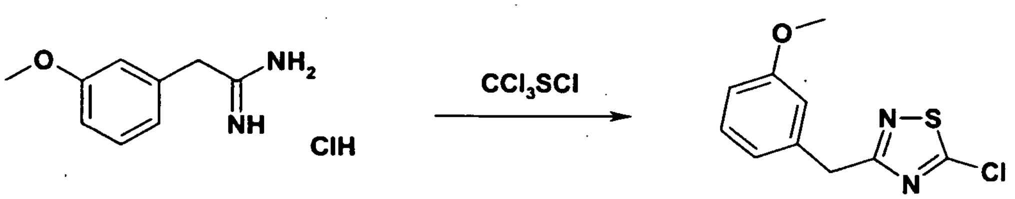


5-chloro-3-(4-chlorobenzyl)-[1,2,4] thiadiazole and 5-chloro-3-benzyl-[1,2,4] thiadiazole were also synthesised using these experimental conditions.

Example 3 - nucleophilic replacement of 5-chloro-3-(substituted benzyl)-thiadiazole derivatives with cyclic diamines

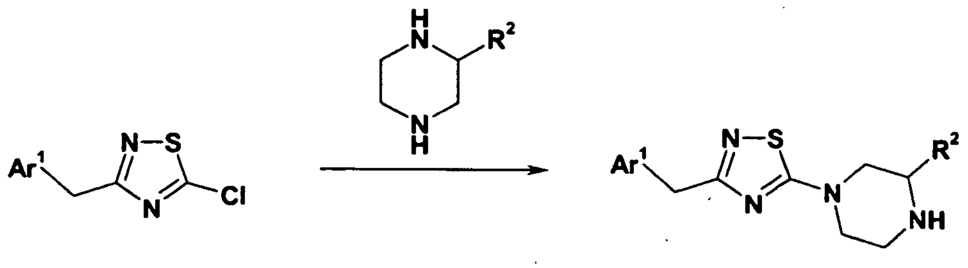

A piperazine derivative (50 mmole) was dissolved in ethanol (EtOH) (10-20 ml) and a chlorothiadiazole derivative (10 mmole) from example 2 was added in portions. The reaction mixture was refluxed until the reaction was complete. The course of the reaction was monitored by thin layered chromatography (TLC) in an eluent mixture of DCM-EtOH 5: 1. When reaction was complete (usually after 3 to 6 hours), the reaction mixture was evaporated to dryness. The residue was dissolved in water and the product was extracted with DCM. The organic phase was washed with water in order to remove the traces of the diamine then it was dried over $MgSO_4$ and evaporated to dryness. The purity of the product was checked by TLC in an eluent mixture DCM: EtOH 5:1 containing some drop of 25% aq. ammonium hydroxide solution. If the TLC showed apolar impurities the product was dissolved in 5% aqueous HCl solution and the impurities were washed away with ethyl-acetate. The aqueous phase was made alkaline (pH: 10-11) with 10% aqueous NaOH solution and the product was extracted with DCM.
The following data were obtained for certain species:

- 3-methyl-1-[3-(4-methyl-benzyl)-[1,2,4]thiadiazol-5-yl]-piperazine: starting from 3.37 g of 5-chloro-3-(4-methylbenzyl)-[1,2,4]thiadiazole (15 mmol) and a 5-fold excess of 2-methyl-piperazine, 4.05 g of the title compound was obtained after 3 hours reaction time (94% yield).

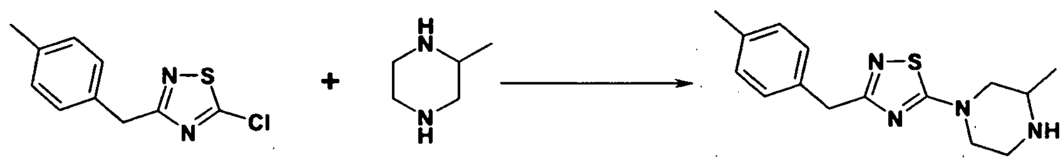

- 1-[3-(4-fluoro-benzyl)-[1,2,4]thiadiazol-5-yl]-piperazine: starting with 3.43 g of 5-chloro-3-(4-fluorobenzyl)-[1,2,4]thiadiazole (15 mmol), 3.73 g of the title compound was obtained after 6 hours reaction in the presence of a 5-fold excess of piperazine (89% yield).

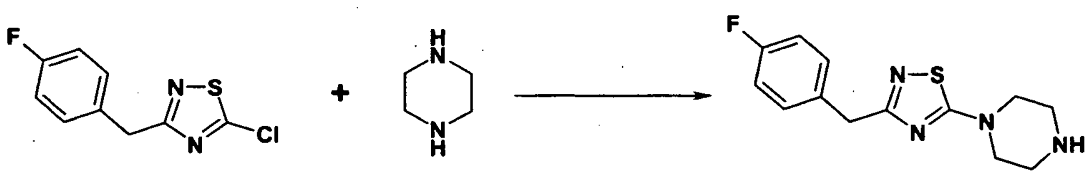

- 1-[3-(3-methoxy-benzyl)-[1,2,4]thiadiazol-5-yl]-piperazine: starting from 2.41 g 5-chloro-3-(4-fluorobenzyl)-[1,2,4] thiadiazole (10 mmol) and a 5-fold excess of piperazine (4.31 g), 2.65 g of the title compound was obtained after 5 hours reaction time (91% yield).

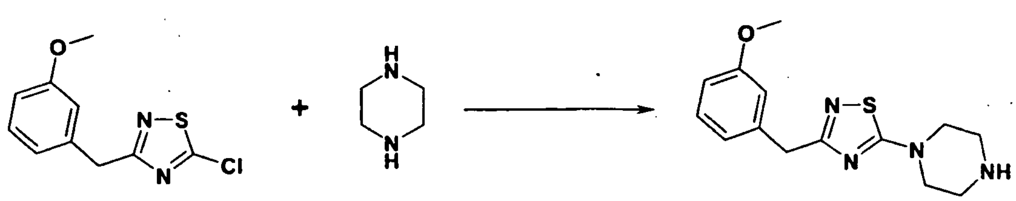

The following intermediate compounds have also been synthesised using these experimental conditions:

- 1-[3-(4-Fluoro-benzyl)-[1,2,4]thiadiazol-5-yl]-1,4-diazepine
- 3-Methyl-1-[3-(4-methyl-benzyl)-[1,2,4]thiadiazol-5-yl]-piperazine
- 1-[3-(3-Methoxy-benzyl)-[1,2,4]thiadiazol-5-yl]-piperazine
- 1-[3-(4-Fluoro-benzyl)-[1,2,4]thiadiazol-5-yl]-piperazine
- 3-Methyl-1-[3-(4-Fluoro-benzyl)-[1,2,4]thiadiazol-5-yl]-piperazine
- 3-Methyl-1-[3-(4-Chloro-benzyl)-[1,2,4]thiadiazol-5-yl]-piperazine
- 1-[3-(4-Chloro-benzyl)-[1,2,4]thiadiazol-5-yl]piperazine
- 1-[3-(4-methyl-benzyl)-[1,2,4]thiadiazol-5-yl]-piperazine
- 3-Methyl-1-[3-benzyl-[1,2,4]thiadiazol-5-yi]-piperazine
- 1-[3-benzyl-[1,2,4]thiadiazol-5-yl]-piperazine
- 2-{4-[3-(4-fluorobenzyl)-1,2,4-thiadiazol-5-yl]piperazin-1-yl}ethanamine
- 2-(4-[3-benzyl-1,2,4-thiadiazol-5-yl]piperazin-1-yl)ethanamine
- 2-(4-[3-(4-chlorobenzyl)-1,2,4-thiadiazol-5-yl]piperazin-1-yl)ethanamine, and
- 2-{4-[3-(4-methylbenzyl)-1,2,4-thiadiazol-5-yl]piperazin-1-yl}ethanamine.

In addition, compounds **113, 114** and **185** to **188** were synthesised according to these experimental conditions using N-substituted piperazine reagents (N-benzylpiperazine, N-(1,3-benzodioxol-5-ylmethyl)-piperazine, N-(2-methylphenyl)-piperazine, N-(2-ethoxyphenyl)-piperazine, N-(2-fluorophenyl)-piperazine and N-(3-trifluoromethylphenyl)-piperazine, respectively). However, for the isolation of the final product, the organic phase (DCM) was first washed with a 5% aqueous citric acid solution, water, a 5% aqueous Na2CO3 solution, and water, respectively. The organic phase was separated, dried over MgSO4, filtered and, evaporated to dryness. The residue was crystallised by diethyl ether to yield the desired compound.

<u>Example 4 - N-sulfonylation</u>

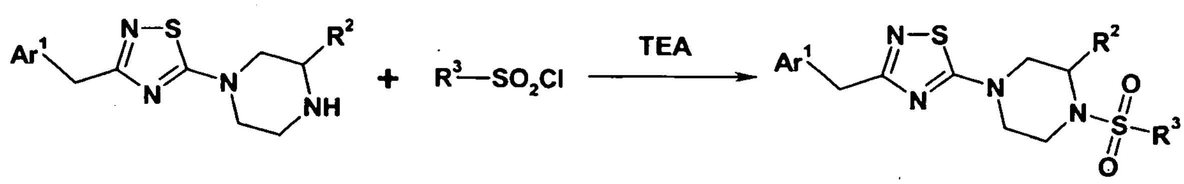


To the substituted piperazine derivative (250 μmole) dissolved in DCM (2-3 ml) TEA (500 μmole) was added. The reaction mixture was stirred at room temperature and then the appropriate sulfonyl chloride derivative (250 μmole) was added. The reaction mixture was further stirred at room temperature until total consumption of the starting products. The course of the reaction was monitored by TLC using dichloroethane (DCE)-EtOH 10:1 as an eluent mixture. The reaction time varied between 3 and 5 hours. When the reaction was complete DCM (2-3 ml) was added and the resulting solution was washed with a 5% aqueous citric acid solution (5 ml), water (5 ml), a 5% aqueous $Na_2CO_3$ solution, and water.(5 ml), respectively. The organic phase was separated, dried over $MgSO_4$, filtered and, evaporated to dryness. The residue was crystallised by diethyl ether to yield the desired compound.

The following data were obtained for the synthesized species:

- compound **60:** 1-[4-methoxyphenylsulfonyl]-4-[3-(4-methylbenzyl)-1,2,4-thiadiazol-5-yl]-2-methylpiperazine: starting from 3-methyl-1-[3-(4-methylbenzyl)-[1,2,4]thiadiazol-5-yl]-piperazine (1.25 mmole) and 1 molar equivalent of 4-methoxy-phenylsulphonyl chloride, after 4 hours reaction time the title compound was obtained in 99% yield:

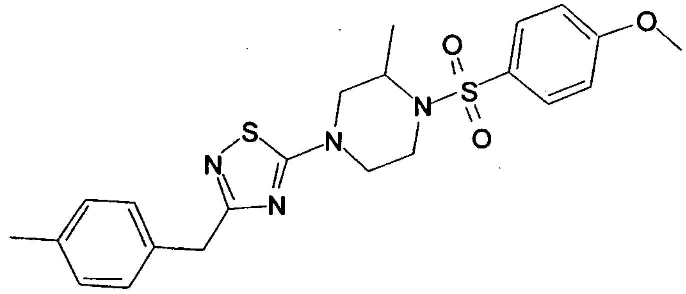

- compound **32:** 1-[4-methoxyphenylsulfonyl]-4-[3-(4-fluorobenzyl)-1,2,4-thiadiazol-5-yl]piperazine: starting from 400 mg 1-[3-(4-fluoro-benzyl)-[1,2,4]thiadiazol-5-yl]-piperazine (1.237 mmole) and 1 molar equivalent of 4-methoxyphenylsulphonyl chloride, 453 mg of the title compound was obtained after 3 hours of reaction (70 % yield).

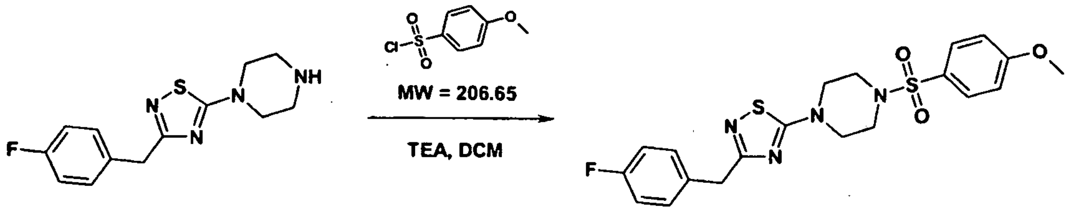


- compound **44:** 1-[3-methoxyphenylsulfonyl]-4-[3-(4-fluorobenzyl)-1,2,4-thiadiazol-5-yl]piperazine: starting from 80 mg 1-[3-(4-Fluoro-benzyl)-[1,2,4]thiadiazol-5-yl]-piperazine (0.287 mmol) and 1 molar equivalent of 3-methoxyphenylsulfonyl chloride, 102 mg of the title compound was obtained after **4** hours of reaction in 79% yield.

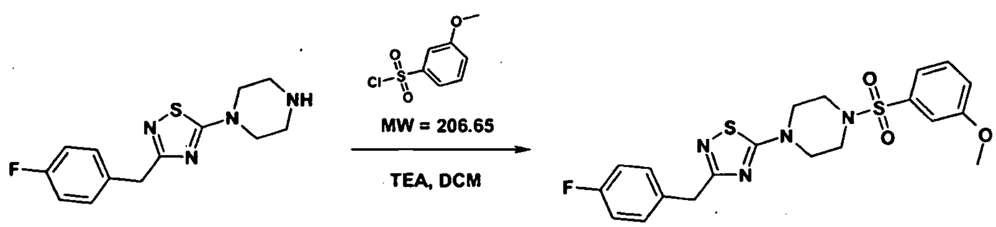


- compound **53:** 1-[4-methoxyphenylsulfonyl]-4-[3-(3-methoxybenzyl)-1,2,4-thiadiazol-5-yl]piperazine: starting from 150 mg 1-[3-(3-methoxybenzyl)-[1,2,4]thiadiazol-5-yl]-piperazine (0.517 mmol) and 1 molar equivalent of 4-methoxyphenylsulfonyl chloride, 156 mg of the title compound was obtained after 5 hours of reaction in 66% yield.

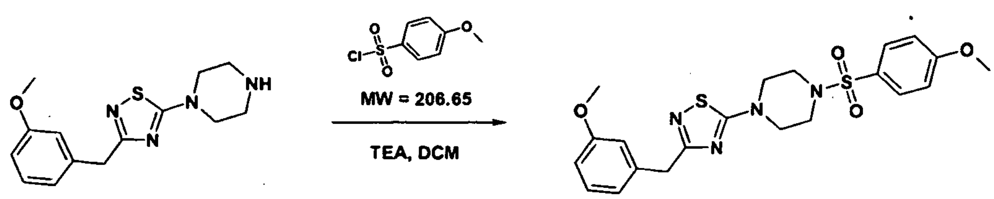

Compounds **30** to **85** were also synthesised using these experimental conditions.

Example 5 - N-acylation

To a substituted piperazine derivative (approximately 250 μmole) dissolved in DCM (2-3 ml) TEA (2.0 equiv.) was added. The reaction mixture was stirred at room temperature and then the appropriate acyl chloride derivative (1.0 equivalent) was added. The reaction mixture was further stirred at room temperature until total consumption of the starting products. The course of the reaction was monitored by TLC using dichloroethane(DCE)-EtOH 10:1 as an eluent mixture. The reaction time varied between 3-5 h in our practice. When the reaction was complete, DCM (2-3 ml) was added to it and the resulting solution was washed with a 5% aqueous citric acid solution (5 ml), water (5 ml), a 5% aqueous $Na_2CO_3$ solution, and water (5 ml), respectively. The organic phase was separated, dried over $MgSO_4$, filtered and, evaporated to dryness. The residue was crystallised by diethyl ether to yield the desired compound. 4-phenylacetyl-1-[3-(4-fluorobenzyl)-1,2,4-thiadiazol-5-yl]piperazine (compound **1**): starting from 1-[3-(4-fluorobenzyl)-1,2,4-thiadiazol-5-yl]piperazine and 1 equivalent of phenylacetyl chloride, the title compound was obtained and characterised by mass spectrometry: MS (m/z) : 397.18 ($[M+H]^+$, 100).

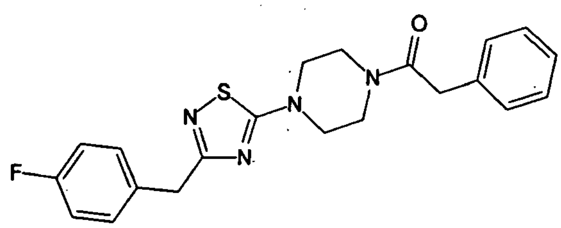

Compounds **1-8, 86-90, 94-112, 115-184** and **189-191** were synthesised using these experimental conditions.

Example 6 - N-alkylation

To a substituted piperazine derivative (approximately 250 μmole) dissolved in DCM (2-3 ml) TEA (2.0 equivalent) was added. The reaction mixture was stirred at room temperature and then the appropriate alkyl chloride derivative (1.0 equivalent) was added. The reaction mixture was further stirred at room temperature until total consumption of the starting products. The course of the reaction was monitored by TLC using dichloroethane(DCE)-EtOH 10:1 as an eluent mixture. The reaction time varied between 3 and 5 hours. When the reaction was complete, DCM (2-3 ml) was added to it and the resulting solution was washed with 5% aq. citric acid solution (5 ml), water (5 ml), 5% aq. $Na_2CO_3$ solution, and water (5 ml), respectively. The organic phase was separated, dried over $MgSO_4$, filtered and, evaporated to dryness. The residue was crystallized by diethyl ether to yield the desired compound. The final compounds **91-93** were synthesised using these experimental conditions and starting from cinnamyl chloride.

Example 7 - Urea-linkage formation

To a solution of isocyanate derivative (approximately 250 μmole) in tetrahydrofuran with a trace of DMF (2-3 ml) diisopropylethyl amine (2.2 equivalent) was added. Subsequently, 1 equivalent of a substituted piperazine derivative was added. The reaction mixture was stirred at room temperature until total consumption of the starting products. The course of the reaction was monitored by TLC using dichloroethane (DCE)-EtOH 10:1 as an eluent mixture. Suitable reaction times were between 3 and 5 hours. When reaction was complete,' the reaction mixture was evaporated until almost dry. DCM (2-3 ml) was added to it and the resulting mixture was washed with a 5% aqueous citric acid solution (5 ml), water (5 ml), a 5% aqueous $Na_2CO_3$ solution, and water (5 ml), respectively. The organic phase was separated, dried over $MgSO_4$, filtered and, evaporated to dryness. The residue was crystallised by diethyl ether to yield the desired compound. The following final compounds of the invention were synthesised using these experimental conditions.

Example 8 - Control of the structure of the synthesised compounds

A set of compounds falling within the scope of structural formula (A) has been synthesised. An overview of these exemplary compounds and their chemical structure is provided in Table 1. The structure of the compounds was checked using both mass spectra (hereinafter referred as MS) and high performance liquid chromatography (hereinafter referred as HPLC).

HPLC analysis was performed on a LaChrom™ HPLC system (Merck-Hitachi, using a LiChroCART™ 30-4 Purospher STAR RP-18, endcapped, 3μm column (Merck) and a gradient elution of eluant A (Acetonitrile-$H_2O$=5:95 with 20 mM, ammonium formate buffer, pH 7.4) and eluant B (Acetonitrile-$H_2O$=80:20 with 20 mM, ammonium formate buffer, pH 7.4, programmed as follows:

| Min. | A% | B% |
|---|---|---|
| 0.0 | 100 | 0 |
| 3.0 | 5 | 95 |
| 3.4 | 5 | 95 |
| 3.5 | 100 | 0 |
| 4.0 | 100 | 0 |

Elution was performed at room temperature with a flow rate of 2.0 mL/min. Samples were applied as 2.5μl of a solution of about 2mg/mL. Detection was performed at 220nm. Parameters of the gradient programs were occasionally adjusted to the properties of the analysed compound in order to reach the optimal separation power.

Mass spectrometry was performed on either a Mariner™ (Perspective Biosystems) ES/APCI (Atmospheric Pressure Chemical Ionization) interface or a Micromass® ZQ2000 MS instrument (Waters Corp.) with electrospray (ESI) interface integrated with MUX™ technology (Waters Corp.). The ionization mode used Electrospray in a positive mode, with centroid data analysis and mass in the range of 120-700 μ.

Example 9 - Construction of an α-synuclein over-expressing cell line

An α-synuclein expression plasmid was constructed by sub-cloning the *NcoI/XhoI* fragment from 212T-SYN (WT) (Griffioen et al., Biochem Biophys Acta (2006) 1762(3):312-318) containing the cDNA of human wild type α-synuclein correspondingly into a standard mammalian expression vector pcDNA3.1 resulting in plasmid pcDNA3.1-SYNwt. Plasmid pcDNA3.1 and pcDNA3.1-SYNwt were transfected to human neuroblastoma cells (ATCC No. CRL-2267) and independent clonal lines with the plasmids stably integrated into the genome were selected. These resulted in cell lines named M17 (transfected with pcDNA3.1) and M17-SYNwt (transfected with pcDNA3.1-SYNwt). Over-expression of α-synuclein in M17-SYNwt cell lines was confirmed by Western analysis.

Example 10: Use of α-synuclein expressing cells as a model for neuronal degradation

Due to the high levels of α-synuclein M17-SYNwt cells are exquisitely sensitivity to paraquat, a well-known risk factor of synuclein-dependent neuronal degeneration. In degenerated or dead cells lactate dehydrogenase (LDH) is leaked out of the cells into the extracellular environment due to a loss of plasma-membrane integrity. This principle was used to determine cytotoxicity by quantifying the level of leaked LDH into the growth medium.

The detailed method for determining α-synuclein cytotoxicity was as follows: From appropriate precultures of M17 and M17-SYN cells were seeded at 50000 cells/$cm^2$ in Optimem Reduced Serum without phenol red (InVitrogen, Cat. 31985-047) supplemented with 5% fetal calf serum, 1 mM sodium pyruvate, 1 x non-essential amino acids, 500 μg/ml G418 0,5 x antibiotic/antimycotic. After 3 hours of incubation at 37°C/5% $CO_2$ paraquat was added to the cells (final concentration of 32 mM), together with the test compound and the cells were further incubated for 40 hours. Subsequently, LDH activity was determined using Promega Cytotox 96 NonRadioactive cytotoxicity assay, (Cat. G1780) according the supplier's instructions.

Figure 1 shows that treatment of M17-SYNwt cells, but not of M17 cells with paraquat led to a relatively high level of LDH leaked into the medium demonstrating that α-synuclein mediates cellular degeneration or cell death in response to paraquat.

Example 11 - use of the α-synuclein expressing cells in the screening of exemplary compounds

This α-synuclein expressing neuroblastoma cells made it possible to assess the ability of novel compounds to counteract α-synuclein cytotoxicity. Active inhibitors of α-synuclein cytotoxicity were found to provoke a decrease of LDH leakage in paraquat-treated M17-SYNwt cells. Since this method monitors leaked LDH from degenerated or dead cells only non-toxic compounds will be identified as active inhibitors of α-synuclein-mediated cytotoxicity. Lack of toxicity is an important characteristic for compounds to be used as a medicament to patients in need. A compound was considered to be active in this test when it inhibits α-synuclein cytotoxicity by more than 25% relative to untreated M17-SYNwt cells

at a concentration of 20 μg/mL or lower. In the experiments, the control group consisted of M17-SYNwt cells treated with DMSO, the untreated paraquat group consisted of M17-SYNwt cells treated with paraquat and DMSO, and the treated paraquat group consisted of M17-SYNwt cells treated with paraquat and the test compound dissolved in DMSO.

In order to determine $EC_{50}$ compounds were tested at different concentrations ranging from non-effective (thus at a relatively low concentration) to an effective (relatively high) concentration of test compound. These data were also used for calculation of percent inhibition (% I). Percent inhibition was calculated as the synuclein toxicity inhibition by the compound in treated paraquat cells, relative to the synuclein cytotoxicity in untreated paraquat cells. This corresponds to the following equation:

**(LDH release of treated paraquat cells at non-effective concentration of test cmpd) -**
**(LDH release of treated paraquat cells at most effective concentration of test cmpd) /**
**(LDH release of untreated paraquat cells) - (LDH release control cells) *100%**

Compounds **19** (N-(2,6-dimethylphenyl)-4-[3-(4-fluorobenzyl)-1,2,4-thiadiazol-5-yl]-2-methylpiperazine-1-carboxamide) and **111.** (2-phenyl-N-{2-[4-[3-(4-fluorobenzyl)-1,2,4-thiadiazol-5-yl]piperazin-1-yl]ethyl}butanamide) were tested using the α-synuclein cytotoxicity assay as described above. Figure 2 shows that these compounds (compound **19** = squares; compound **111** = triangles) were able, in a dose-dependent manner, to reduce LDH activity in the medium demonstrating that the respective compounds alleviate α-synuclein-mediated cytotoxicity.

<u>Example 12 - inhibition of synuclein-mediated toxicity</u>

1,2,4-thiadazole compounds made according to the methods described herein were screened for activity using the α-synuclein cytotoxicity assay as described above. Dose responses were carried out on all compounds found to be active (10 point curves in duplicate). Although the pharmacological properties of the compounds disclosed in this invention vary with structural change as expected, active compounds most particularly possess $EC_{50}$ in a cell-based assay of synuclein cytotoxicity in a range from about 0.0001 to 10 μM. Data obtained for the compounds of Table 1 are presented in Table 2. Based on these results the novel class of inhibitors of α-synuclein cytotoxicity as claimed herein was identified.

<u>Example 13 : *in vivo* inhibition of synuclein-mediated instigated loss of substantia nigra neurons</u>

In order to model neuronal loss in the substantia nigra region of the brain, mice were treated with paraquat (intraperitoneal) at a dose not higher than 8 mg/kg/day for a continuous period of 15-100 days. These mice were also chronically co-treated during that period with:

- compound **60** (1-[4-methoxyphenylsulfonyl]-4-[3-(4-methylbenzyl)-1,2,4-thiadiazol-5-yl]-2-methylpiperazine), or
- compound **32** (1-[4-methoxyphenylsulfonyl]-4-[3-(4-fluorobenzyl)-1,2,4-thiadiazol-5-yl]piperazine), or
- compound **53** (1-[4-methoxyphenylsulfonyl]-4-[3-(3-methoxy-benzyl)-1,2,4-thiadiazol-5-yl]piperazine),

each being administered at a dose not higher than 20 mg/kg body weight/day), or by vehicle only (no active compound). Mice treatment by means of vehicle or a compound of the invention started 2 days before administration of paraquat.

At the end of the treatment period, mice were sacrificed and the corresponding brains were used for immunohistochemical analysis. The substantia nigra brain region has a relatively high percentage of cells with high levels of tyrosine hydroxylase. Using antibodies raised against tyrosin hydroxylase (anti-tyrosin hydroxylase), tyrosine hydroxylase containing neurons in the brains were detected. Quantitative and comparative analysis of the tyrosin hydroxylase-positive stained substantia nigra areas revealed a significantly larger TH-positive area in mice treated with compound (group 3) versus vehicle treated mice (group 2). The TH-positive area of group 3 is similar of that observed in mouse group 1 (not treated with paraquat), suggesting a strong and possibly complete inhibition of degeneration. These results indicate that the corresponding compound is able to inhibit paraquat-triggered degeneration of substantia nigra cells *in vivo.*

Figure 3 shows that compound **53** protects dopaminergic substantia nigra neurons against paraquat-triggered degeneration. Three groups of mice were treated with either paraquat and compound **53** (group 3; n=6), or paraquat and vehicle of compound **53** (group 2; n=4), or vehicle paraquat and vehicle of compound 53 (group 1; n=3). After 8 weeks treatment, brains were collected and used for immunohistochemistry (6 sections per brain per staining). Mouse brain were fixed overnight in 4% paraformaldehyde, and stored in PBS with 0.1% sodium azide. Free-floating vibratome-sections of 40 μm were prepared and stored in PBS containing 0.1% sodium azide. 6 midbrain sections of each brain

were selected for tyrosine hydroxylase (TH) immunohistochemistry. After quenching the endogenous peroxidase by using 1.5% $H_20_2$ in 1/1 PBS/methanol, sections were blocked in 10% fetal calf serum (30 minutes) and subsequently incubated overnight at 4°C with mouse anti-TH (1:10.000; MAB318; Chemicon International). After washing and incubation with a HRP-conjugated goat-anti-mouse secondary antibody (1:500; P 0447; DakoCytomation) for 60 minutes, immunostaining was visualized with 3,3'-diaminobenzidine. Sections were counterstained with Mayers hematoxylin. Per mouse, the median %TH-positive areas (of the substantia nigra regions) was determined. Results shown (Figure 3) are the mean ±SEM of the median %TH-positive areas of the different mouse brains of each group.

Example 14 - *in vivo* inhibition of 6-hydroxydopamine (6-OHDA) instigated loss of substantia nigra neurons

Unilateral substantia nigra lesions are obtained by stereotactic striatal injections of 6-hydroxydopamine in brains of living rats as described by Vercammen et al. in Molecular Therapy, 14(5) 716-723 (2006). These rats are also chronically co-treated with the same exemplary compounds and at the same dose as mentioned in example 13, or by vehicle only (no active compound). Daily treatment of compound or vehicle is started preferably 1 or 2 days before administration of 6-OHDA and lasts between 7 to 30 days after the 6-OHDA injection.

At the end of the treatment period, rats are sacrificed and the corresponding brains are used for immunohistochemical analysis. The substantia nigra brain region has a relatively high percentage of cells with high levels of tyrosine hydroxylase. Using antibodies raised against tyrosin hydroxylase (anti-tyrosine hydroxylase) tyrosine hydroxylase containing neurons in the brains are detected. The nigral lesion volumes and/or the tyrosine hydroxylase positive cell numbers are quantified as described in Vercammen et al. (cited *supra*). This analysis reveals that:

- the nigral lesion volumes are significantly reduced in rats treated with a compound according to this invention, as compared to vehicle treated rats, thus indicating that the compound is able to inhibit 6-OHDA triggered degeneration of substantia nigra cells *in vivo*; and
- tyrosine hydroxylase positive cell numbers are higher in rats treated with a compound according to this invention as compared to vehicle treated rats, thus providing confirmation that the compound is able to inhibit 6-OHDA triggered degeneration of substantia nigra cells *in vivo.*

Example 15 - *in vitro* inhibition of α-synuclein aggregation

α-synucleinopathies are characterised by aggregation of α-synuclein in neurons. Aggregation of purified α-synuclein is performed essentially as described by Gerard et al. FASEB. 20(3):524-6 (2006). 20-100 μg purified α-synuclein (Sigma; S7820) at a concentration of about 2.5 μg/mL is incubated in the presence of spermin (250 μM) or paraquat (32 mM) or 6-hydroxydopamine (400 μM) or vehicle in a 384 well plate. Spermin, paraquat and 6-hydroxydopamine promote the α-synuclein aggregation process. Aggregation kinetics is determined by measuring turbidity at 340 nm, every 1-15 minutes for at least one hour. The same exemplary compounds as mentioned in example 13, or vehicle only, is added to the different α-synuclein mixtures described above. This analysis reveals that, when a compound is present, the measured turbidity is lower compared to reactions containing vehicle only. This finding shows that the compound is able to inhibit aggregation of α-synuclein.

Table 1 - chemistry of the exemplary compounds synthesised

| No. | Compound name | Compound structure |
|---|---|---|
| 1 | 4-phenylacetyl-1-[3-(4-fluorobenzyl)-1,2,4-thiadiazol-5-yl] piperazine | 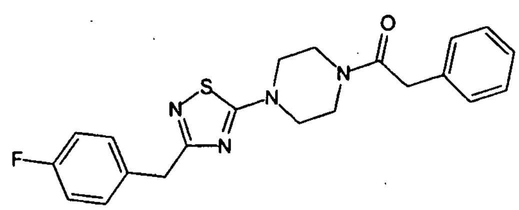 |
| 2 | 4-(4-fluorophenylacetyl),1-[3-(4-fluorobenzyl)-1,2,4-thiadiazol-5-yl] piperazine | 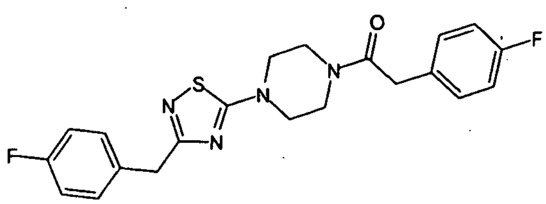 |

(continued)

| No. | Compound name | Compound structure |
|---|---|---|
| 3 | 1-(4-fluorophenylacetyl)-4-[3-(4-methylbenzyl)-1,2,4-thiadiazol-5-yl]-2-methylpiperazine | 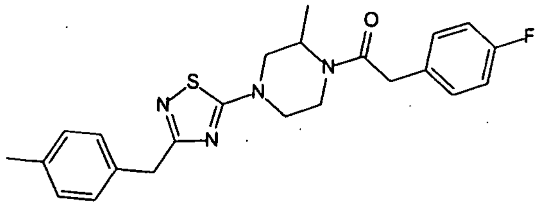 |
| 4 | 1-(4-methoxyphenylacetyl)-4-[3-(4-fluorobenzyl)-1,2,4-thiadiazol-5-yl]-2-methylpiperazine | 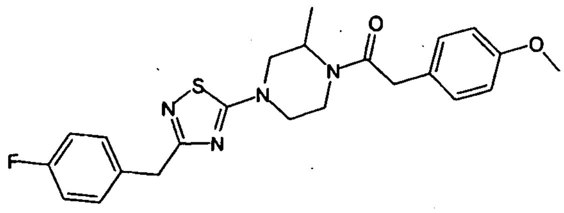 |
| 5 | 1-phenylacetyl-4-[3-(4-fluorobenzyl)-1,2,4-thiadiazol-5-yl]-2-methylpiperazine | 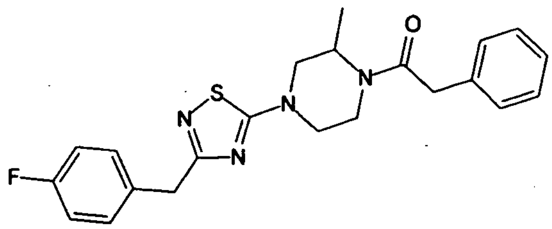 |
| 6 | 1-(4-fluorophenylacetyl)-4-[3-(4-fluorobenzyl)-1,2,4-thiadiazol-5-yl]-2-methylpiperazine | 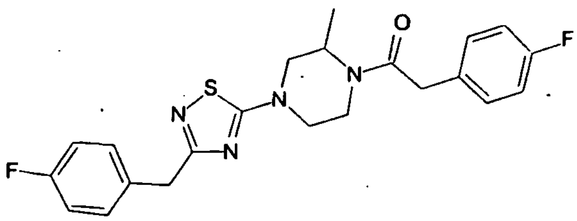 |
| 7 | 1-[chloro(phenyl)acetyl]-4-[3-(4-fluorobenzyl)-1,2,4-thiadiazol-5-yl]-2-methylpiperazine | 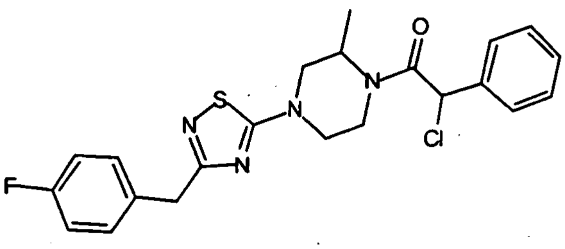 |
| 8 | 1-(2-phenylbutanoyl)-4-[3-(4-fluorobenzyl)-1,2,4-thiadiazol-5-yl]-2-methylpiperazine | 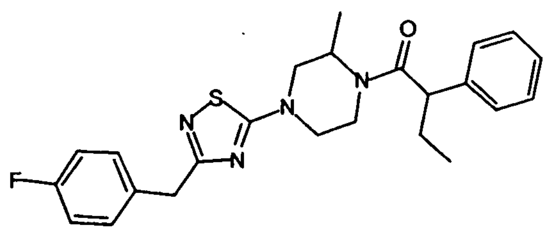 |

(continued)

| No. | Compound name | Compound structure |
|---|---|---|
| 9 | N-(3-fluorophenyl)-4-[3-(4-fluorobenzyl)-1,2,4-thiadiazol-5-yl] piperazine-1-carboxamide | 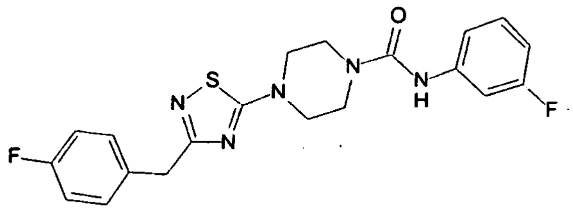 |
| 10 | N-(2-methylphenyl)-4-[3-(4-fluorobenzyl)-1,2,4-thiadiazol-5-yl]-2-methylpiperazine-1-carboxamide | 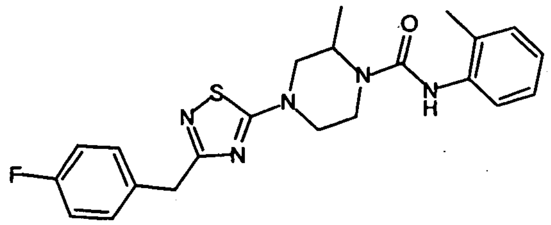 |
| 11 | N-(4-ethylphenyl)-4-[3-(4-fluorobenzyl)-1,2,4-thiadiazol-5-yl]-2-methylpiperazine-1-carboxamide | 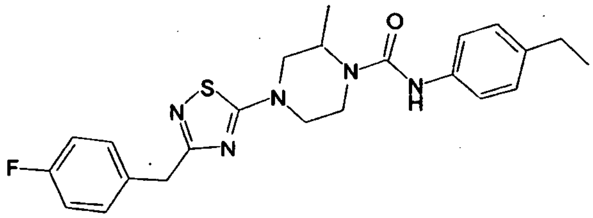 |
| 12 | N-(2-ethylphenyl)-4-[3-(4-fluorobenzyl)-1,2,4-thiadiazol-5-yl]-2-methylpiperazine-1-carboxamide | 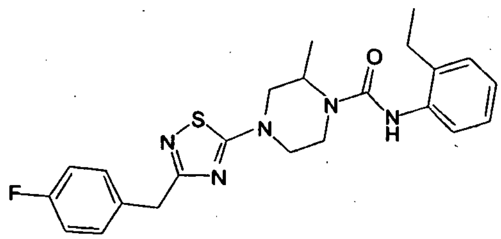 |
| 13 | N-phenyl-4-[3-(4-fluorobenzyl)-1,2,4-thiadiazol-5-yl]piperazine-1-carboxamide | 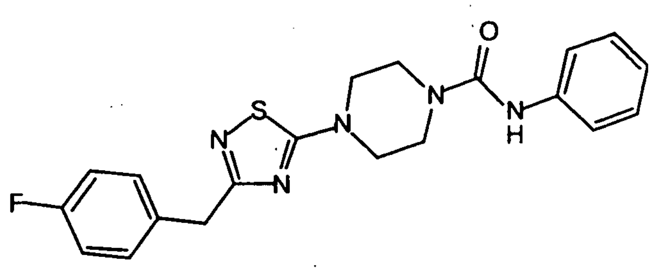 |
| 14 | N-(4-ethoxyphenyl)-4-[3-(4-fluorobenzyl)-1,2,4-thiadiazol-5-yl]-2-methylpiperazine-1-carboxamide | 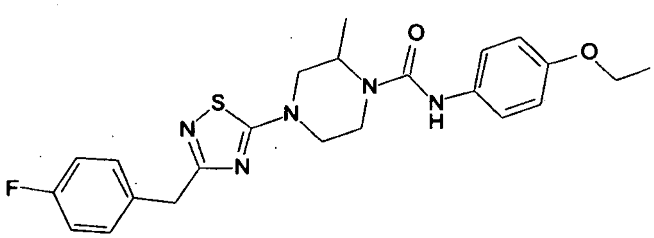 |

(continued)

| No. | Compound name | Compound structure |
|---|---|---|
| 15 | N-(2-methylphenyl)-4-[3-(4-fluorobenzyl)-1,2,4-thiadiazol-5-yl] piperazine-1-carboxamide | 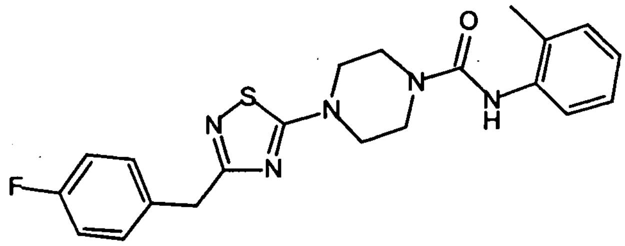 |
| 16 | N-(2-fluorophenyl)-4-[3-(4-fluorobenzyl)-1,2,4-thiadiazol-5-yl] piperazine-1-carboxamide | 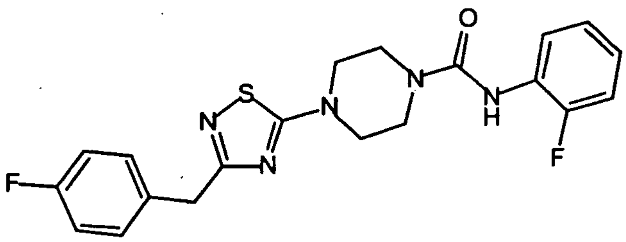 |
| 17 | N-(2-trifluoromethylphenyl)-4-[3-(4-fluorobenzyl)-1,2,4-thiadiazol-5-yl] piperazine-1-carboxamide | 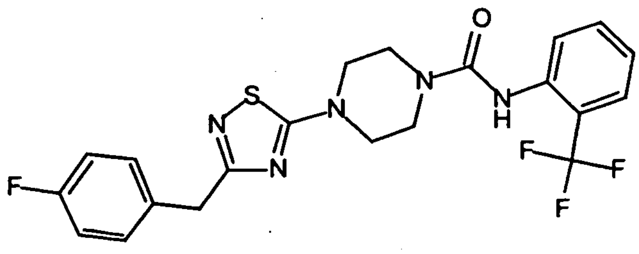 |
| 18 | N-(2-trifluoromethylphenyl)-4-[3-(4-fluorobenzyl)-1,2,4-thiadiazol-5-yl]-2-methylpiperazine-1-carboxamide | 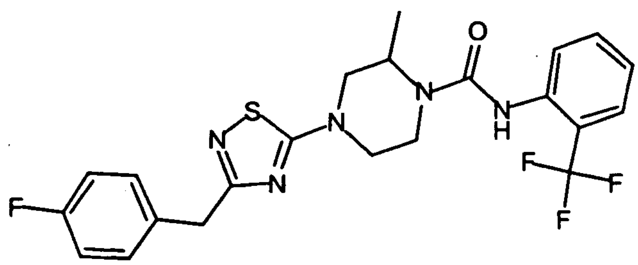 |
| 19 | N-(2,6-dimethylphenyl)-4-[3-(4-fluorobenzyl)-1,2,4-thiadiazol-5-yl]-2-methylpiperazine-1-carboxamide | 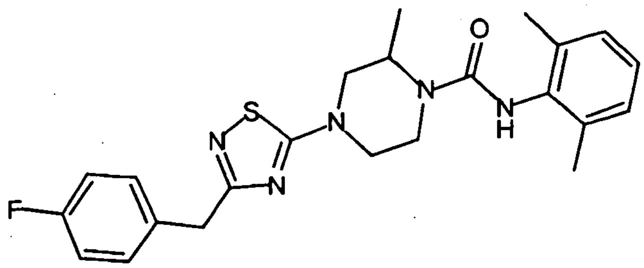 |

(continued)

| No. | Compound name | Compound structure |
|---|---|---|
| 20 | N-(2,4-dimethylphenyl)-4-[3-(4-fluorobenzyl)-1,2,4-thiadiazol-5-yl]-2-methylpiperazine-1-carboxamide | 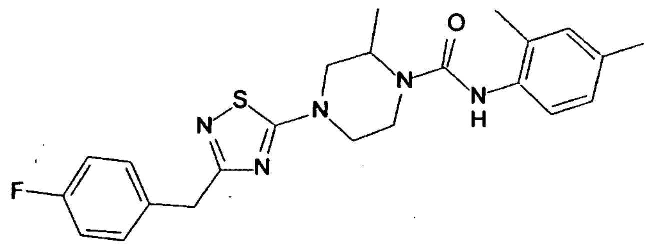 |
| 21 | N-(2,6-dichlorophenyl)-4-[3-(4-fluorobenzyl)-1,2,4-thiadiazol-5-yl]-2-methylpiperazine-1-carboxamide | 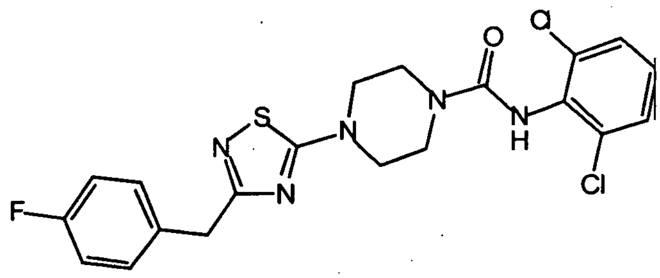 |
| 22 | N-(3-cyanophenyl)-4-[3-(4-fluorobenzyl)-1,2,4-thiadiazol-5-yl]-2-methylpiperazine-1-carboxamide | 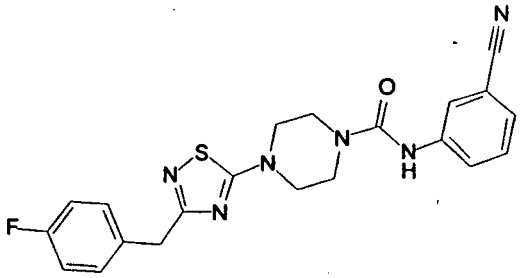 |
| 23 | N-(2,4-difluorophenyl)-4-[3-(4-fluorobenzyl)-1,2,4-thiadiazol-5-yl]-2-methylpiperazine-1-carboxamide | 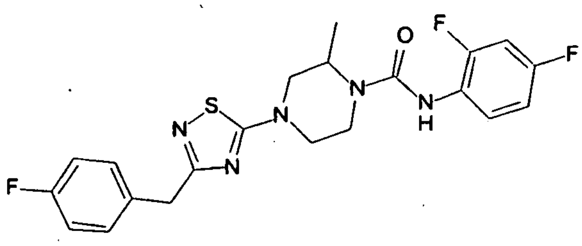 |
| 24 | N-(2,6-dimethylphenyl)-4-[3-(4-fluorobenzyl)-1,2,4-thiadiazol-5-yl] piperazine-1-carboxamide | 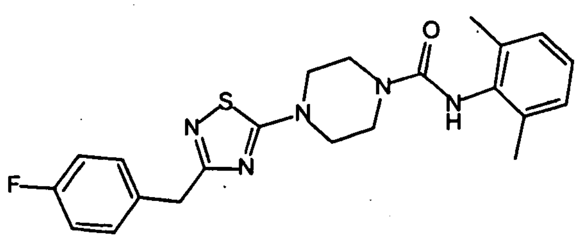 |
| 25 | N-1-naphtyl-4-[3-(4-fluorobenzyl)-1,2,4-thiadiazol-5-yl]-2-methylpiperazine-1-carboxamide | 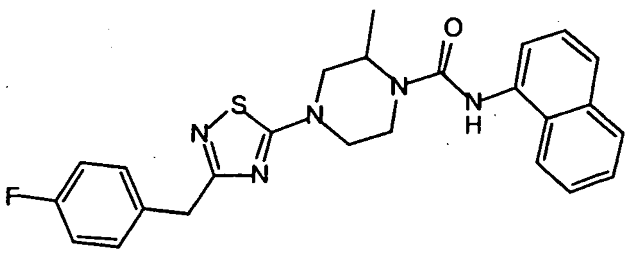 |

(continued)

| No. | Compound name | Compound structure |
|---|---|---|
| 26 | N-(3,4-difluorophenyl)-4-[3-(4-fluorobenzyl)-1,2,4-thiadiazol-5-yl]-2-methylpiperazine-1-carboxamide | 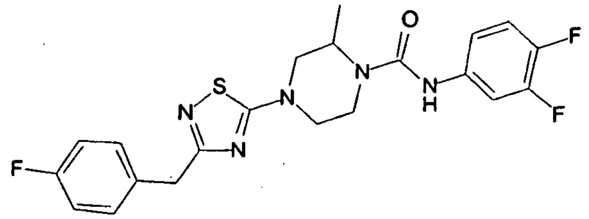 |
| 27 | N-(2,4-dimethoxyphenyl)-4-[3-(4-fluorobenzyl)-1,2,4-thiadiazol-5-yl] piperazine-1-carboxamide | 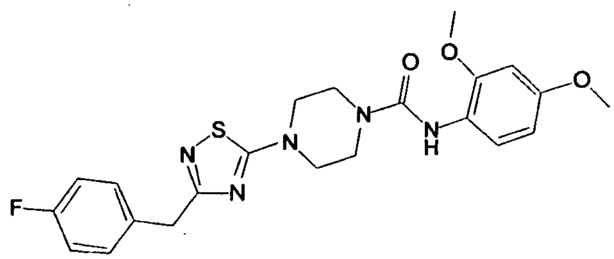 |
| 28 | N-(3,4-difluorophenyl)-4-[3-(4-fluorobenzyl)-1,2,4-thiadiazol-5-yl] piperazine-1-carboxamide | 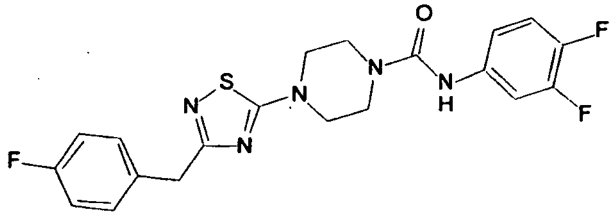 |
| 29 | N-(3,5-dimethoxyphenyl)-4-[3-(4-fluorobenzyl)-1,2,4-thiadiazol-5-yl]-2-methylpiperazine-1-carboxamide | 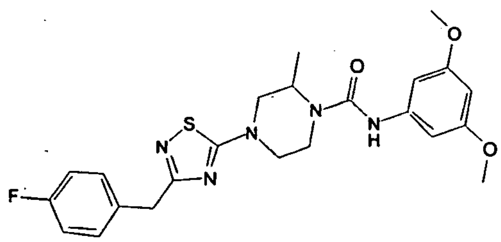 |
| 30 | 1-[4-tert-butylphenylsulfonyl]-4-[3-(4-fluorobenzyl)-1,2,4-thiad iazol-5-yl]piperazine | 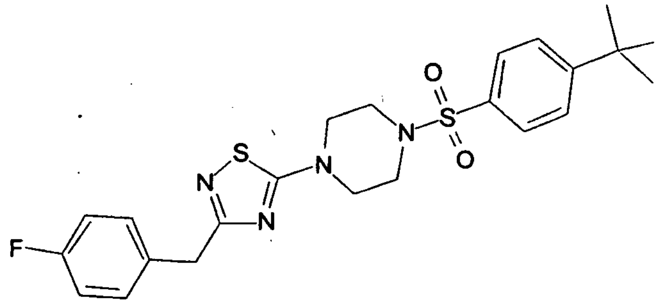 |
| 31 | 1-[4-methoxyphenylsulfonyl]-4-[3-(4-chlorobenzyl)-1,2,4-thiadiazol-5-yl]piperazine | 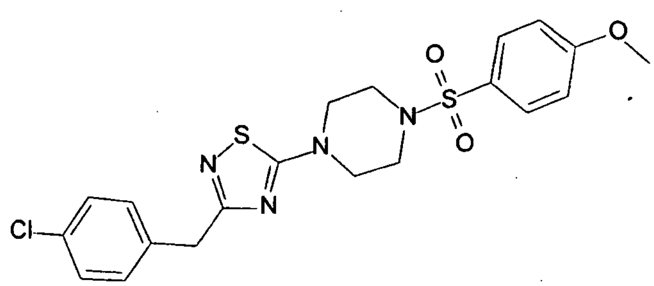 |

(continued)

| No. | Compound name | Compound structure |
|---|---|---|
| 32 | 1-[4-methoxyphenylsulfonyl]-4-[3-(4-fluorobenzyl)-1,2,4-thiadiazol-5-yl]piperazine | 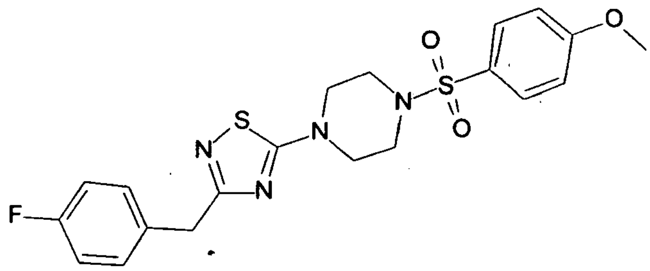 |
| 33 | 1-[4-fluorophenylsulfonyl]-4-[3-(4-chlorobenzyl)-1,2,4-thiadiazol-5-yl] piperazine | 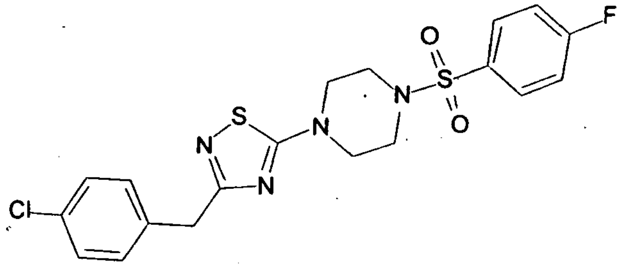 |
| 34 | 1-[4-chlorophenylsulfonyl]-4-[3-(4-methylbenzyl)-1,2,4-thiadiazol-5-yl]-2-methylpiperazine | 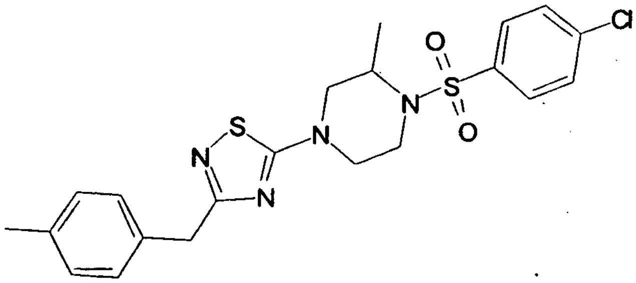 |
| 35 | 1-[1-naphtylsulfonyl]-4-[3-(4-methylbenzyl)-1,2,4-thiadiazol-5-yl]piperazine | 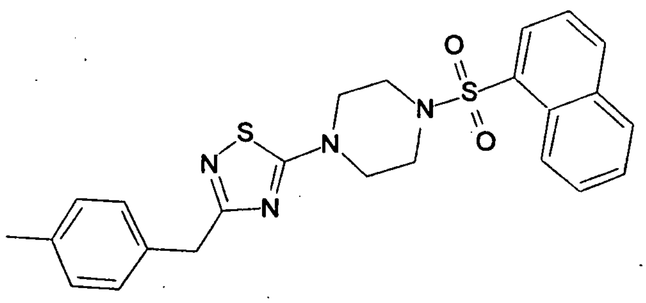 |
| 36 | 1-[2,5-dichlorophenylsulfonyl]-4-[3-(4-methylbenzyl)-1,2,4-thiadiazol-5-yl]-2-methylpiperazine | 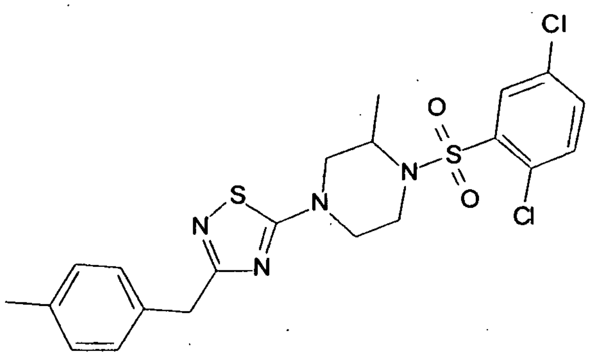 |

(continued)

| No. | Compound name | Compound structure |
|---|---|---|
| 37 | 1-[2,4,6-trimethylphenylsulfonyl]-4-[3-(4-methylbenzyl)-1,2,4-thiadiazol-5-yl]piperazine | 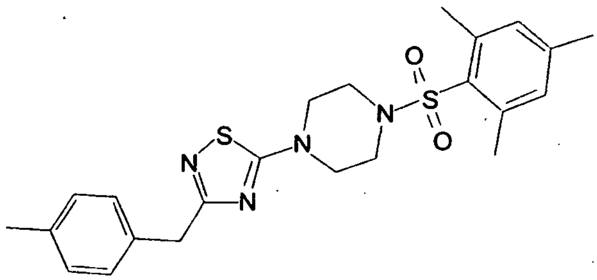 |
| 38 | 1-[2-naphtylsulfonyl]-4-[3-(4-methylbenzyl)-1,2,4-thiadiazol-5-yl]-2-methylpiperazine | 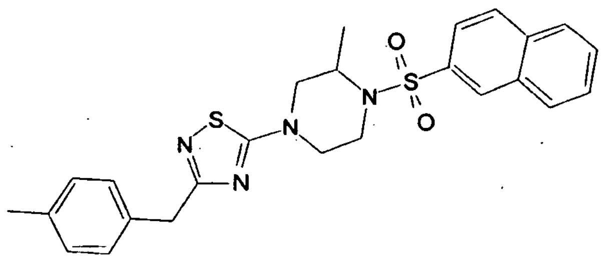 |
| 39 | 1-[2,5-dichlorophenylsulfonyl]-4-[3-(4-chlorobenzyl)-1,2,4-thiadiazol-5-yl]piperazine | 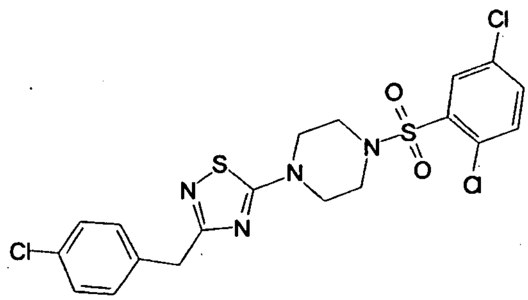 |
| 40 | 1-[4-bromophenylsulfonyl]-4-[3-(4-methylbenzyl)-1,2,4-thiadiazol-5-yl]-3-methylpiperazine | 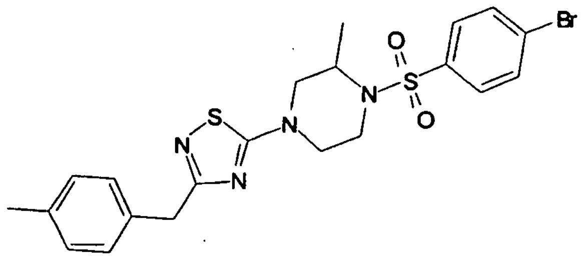 |
| 41 | 1-[1-naphtylsulfonyl]-4-[3-(4-methylbenzyl)-1,2,4-thiadiazol-5-yl]-3-methylpiperazine | 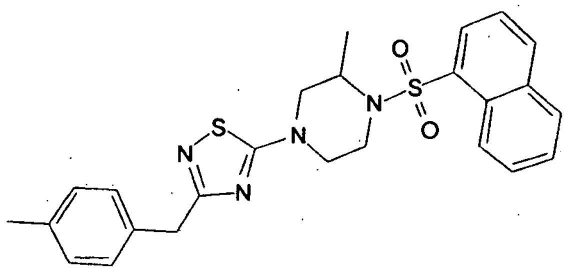 |

(continued)

| No. | Compound name | Compound structure |
|---|---|---|
| 42 | 1-[4-tert-butylphenylsulfonyl]-4-[3-(4-methylbenzyl)-1,2,4-thiadiazol-5-yl]-2-methylpiperazine | 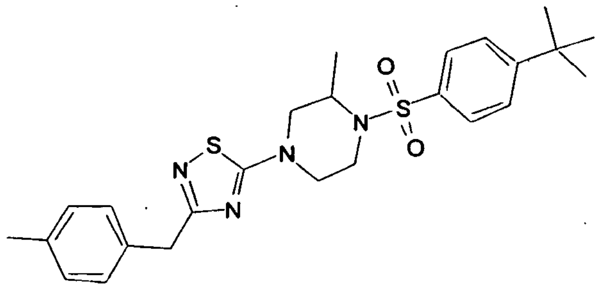 |
| 43 | 1-[1-naphtylsulfonyl]-4-[3-(4-chlorobenzyl)-1,2,4-thiadiazol-5-yl]-2-methylpiperazine | 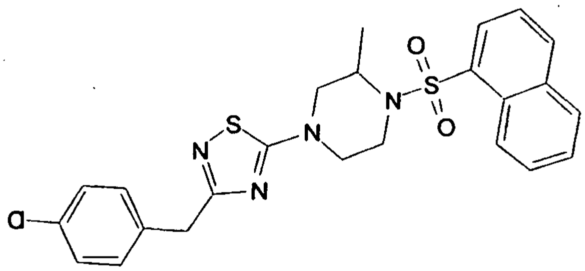 |
| 44 | 1-[3-methoxyphenylsulfonyl]-4-[3-(4-fluorobenzyl)-1,2,4-thiadiazol-5-yl]piperazine | 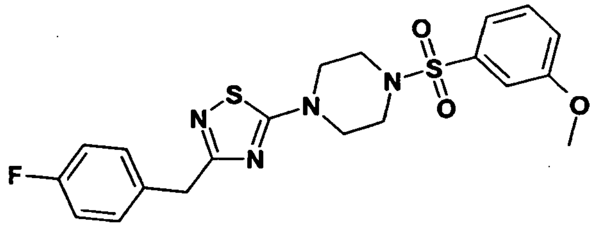 |
| 45 | 1-[2,4,6-trimethylphenylsulfonyl]-4-[3-(4-methylbenzyl)-1,2,4-thiadiazol-5-yl]-2-methylpiperazine | 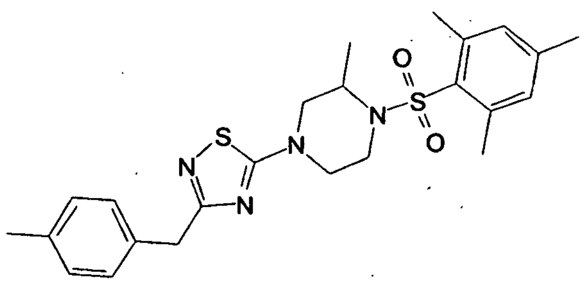 |
| 46 | 1-[3-trifluorophenylsulfonyl]-4-[3-(4-fluorobenzyl)-1,2,4-thiadiazol-5-yl]-2-methylpiperazine | 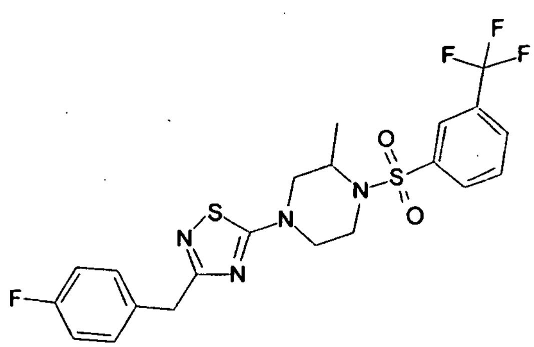 |

(continued)

| No. | Compound name | Compound structure |
|---|---|---|
| 47 | 1-phenylsulfonyl-4-[3-(4-fluorobenzyl)-1,2,4-thiadiazol-5-yl]-2-methylpiperazine | 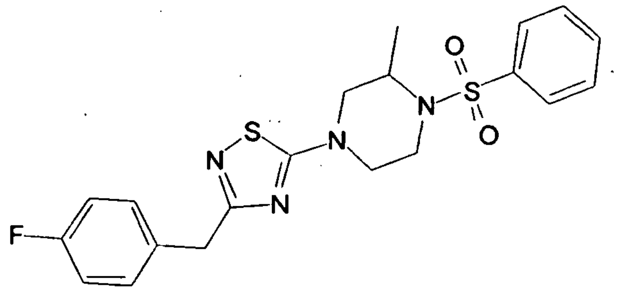 |
| 48 | 1-[4-acetamidophenylsulfonyl]-4-[3-(4-methylbenzyl)-1,2,4-thiadiazof-5-yl]-2-methylpiperazine | 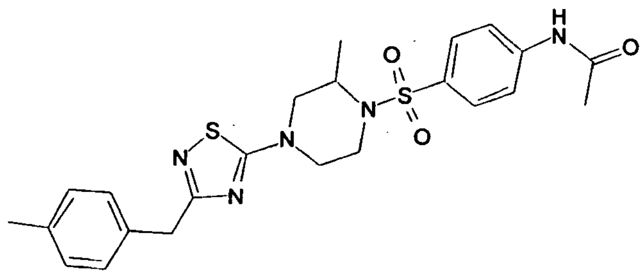 |
| 49 | 1-[4-acetamidophenylsulfonyl]-4-[3-(4-methylbenzyl)-1,2,4-thiadiazol-5-yl]piperazine | 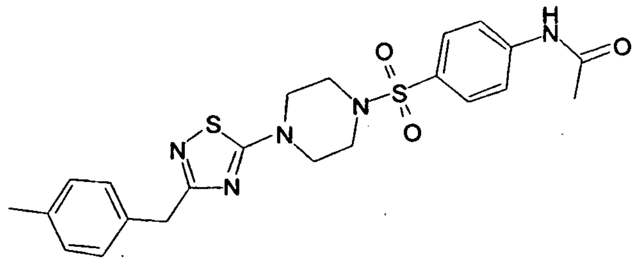 |
| 50 | 1-[4-methylphenylsulfonyl]-4-[3-(4-methylbenzyl)-1,2,4-thiadiazol-5-yl]-2-methylpiperazine | 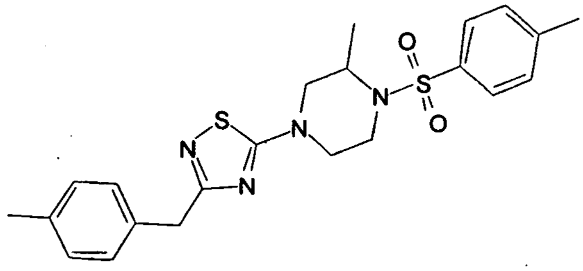 |
| 51 | 1-[4-methoxyphenylsulfonyl]-4-[3-(4-chlorobenzyl)-1,2,4-thiadiazol-5-yl]-2-methylpiperazine | 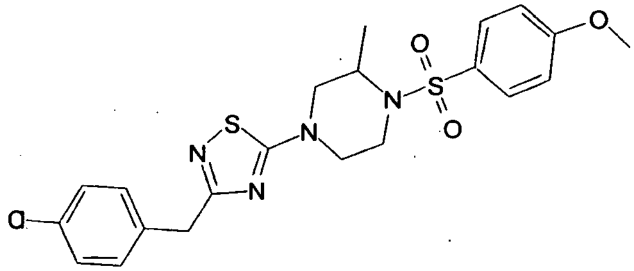 |

(continued)

| No. | Compound name | Compound structure |
|---|---|---|
| 52 | 1-[2-naphtylsulfonyl]-4-[3-(4-chlorobenzyl)-1,2,4-thiadiazol-5-yl] piperazine | 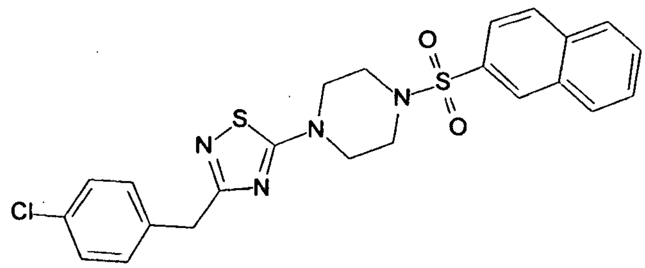 |
| 53 | 1-[4-methoxyphenylsulfonyl]-4-[3-(3-methoxybenzyl)-1,2,4-thiadiazol-5-yl]piperazine | 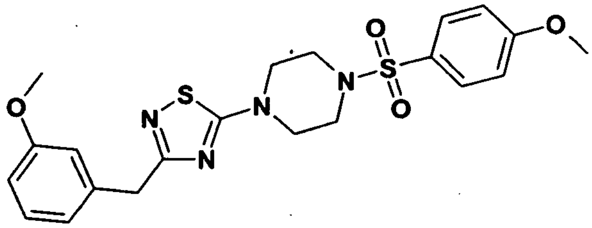 |
| 54 | 1-[4-tert-butylphenylsulfonyl]-4-[3-(4-fluorobenzyl)-1,2,4-thiadiazol-5-yl]-2-methylpiperazine | 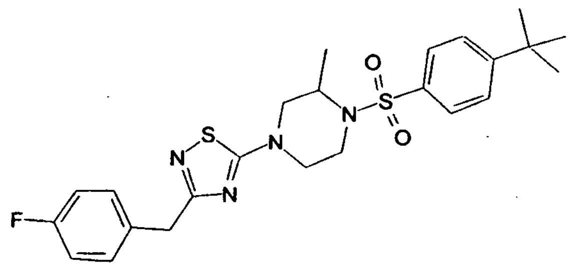 |
| 55 | 1-[4-acetamidophenylsulfonyl]-4-[3-(4-fluorobenzyl)-1,2,4-thiadiazol-5-yl]-2-methylpiperazine | 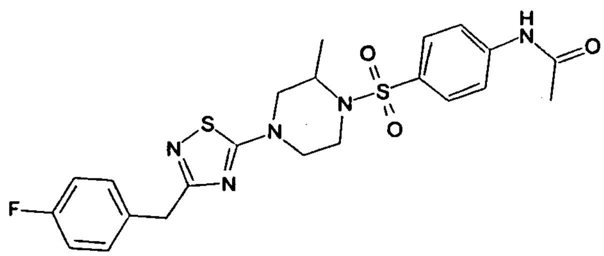 |
| 56 | 1-[3-trifluoromethylphenylsulfonyl]-4-[3-(4-methylbenzyl)-1,2,4-thiadiazol-5-yl]-2-methylpiperazine | 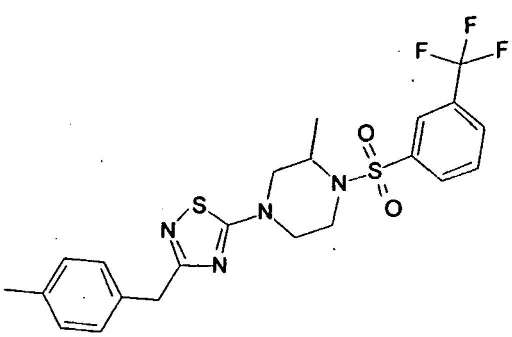 |

(continued)

| No. | Compound name | Compound structure |
|---|---|---|
| 57 | 1-[4-fluorophenylsulfonyl]-4-[3-(4-methylbenzyl)-1,2,4-thiadiazol-5-yl]piperazine | 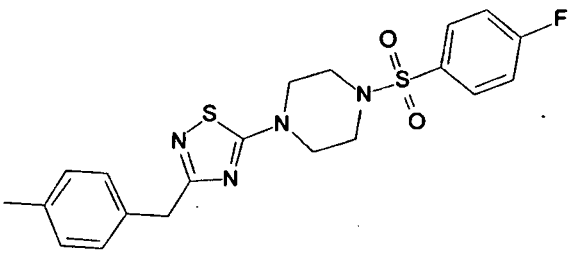 |
| 58 | 1-[4-fluorophenylsulfonyl]-4-[3-(4-methylbenzyl)-1,2,4-thiadiazol-5-yl]-2-methylpiperazine | 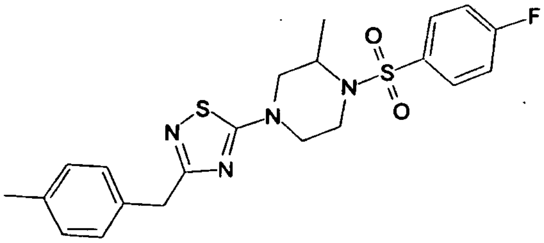 |
| 59 | 1-[4-fluorophenylsulfonyl]-4-[3-(4-fluorobenzyl)-1,2,4-thiadiazol-5-yl]-2-methylpiperazine | 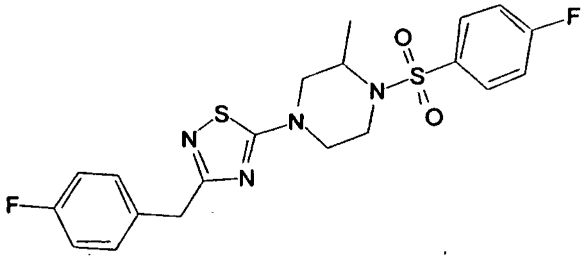 |
| 60 | 1-[4-methoxyphenylsulfonyl]-4-[3-(4-methylbenzyl)-1,2,4-thiadiazol-5-yl]-2-methylpiperazine | 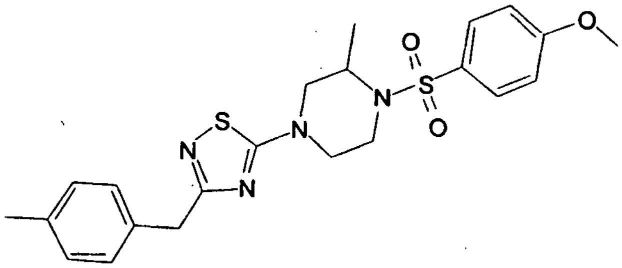 |
| 61 | 1-[4-ter-butylphenylsulfonyl]-4-[3-(4-fluorobenzyl)-1,2,4-thiadiazol-5-yl]piperazine | 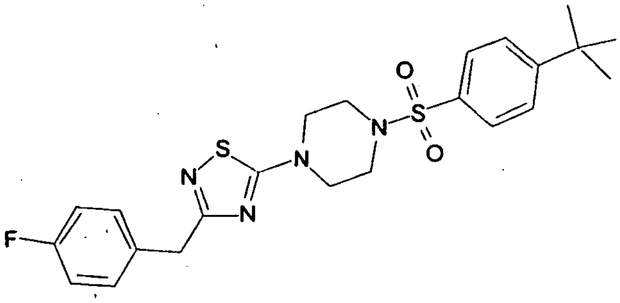 |

(continued)

| No. | Compound name | Compound structure |
|---|---|---|
| 62 | 1-[3-trifluoromethylphenylsulfonyl]-4-[3-(4-methylbenzyl)-1,2,4-thiadiazol-5-yl]piperazine | 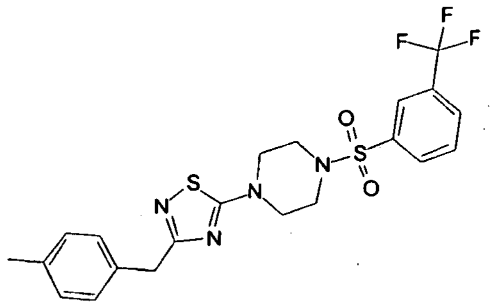 |
| 63 | 1-[4-methylphenylsulfonyl]-4-[3-(4-chlorobenzyl)-1,2,4-thiadiazol-5-yl] piperazine | 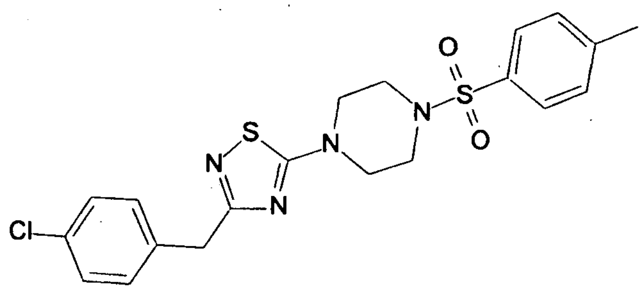 |
| 64 | 1-[4-bromophenylsulfonyl]-4-[3-(4-methylbenzyl)-1,2,4-thiadiazol-5-yl]piperazine | 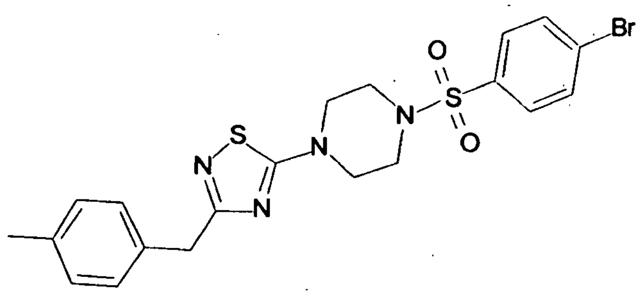 |
| 65 | 1-[2-naphtylsulfonyl]-4-[3-(4-methylbenzyl)-1,2,4-thiadiazol-5-yl]piperazine | 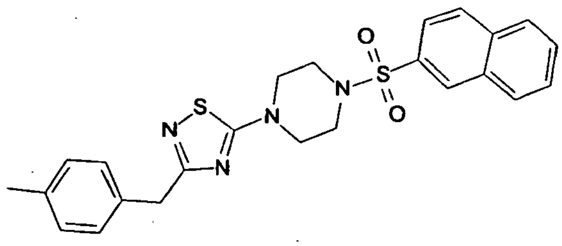 |
| 66 | 1-[4-chlorophenylsulfonyl]-4-[3-(4-methylbenzyl)-1,2,4-thiadiazol-5-yl]piperazine | 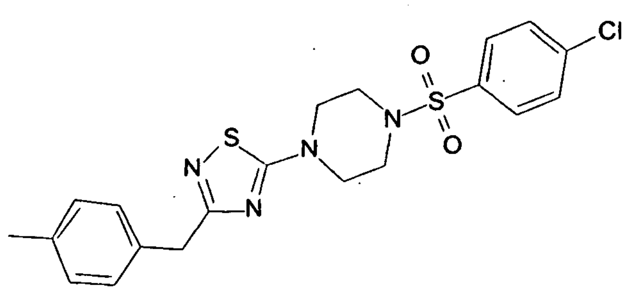 |

(continued)

| No. | Compound name | Compound structure |
|---|---|---|
| 67 | 1-[4-tert-butylphenylsulfonyl]-4-[3-(4-methylbenzyl)-1,2,4-thiadiazol-5-yl]piperazine | 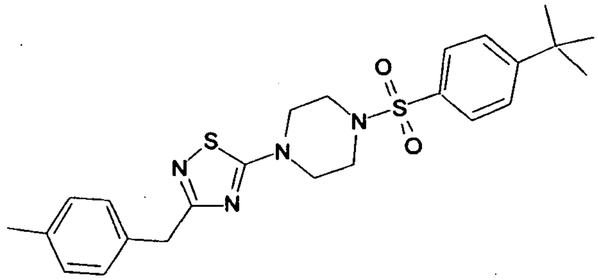 |
| 68 | 1-[4-methoxyphenylsulfonyl]-4-[3-(4-methylbenzyl)-1,2,4-thiadiazol-5-yl]piperazine | 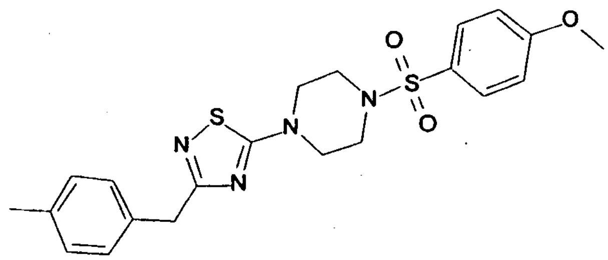 |
| 69 | 1-[4-tert-butylphenylsulfonyl]-4-[3-(4-chlorobenzyl)-1,2,4-thiadiazol-5-yl]piperazine | 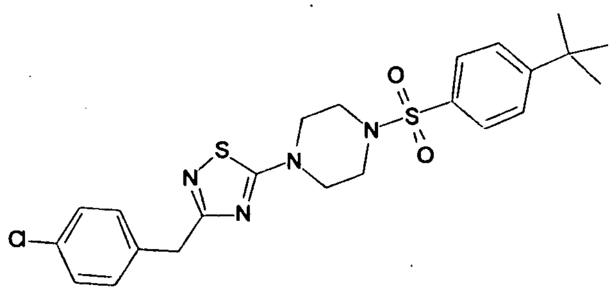 |
| 70 | 1-[quinoline-8-sulfonyl]-4-[3-(4-methylbenzyl)-1,2,4-thiadiazol-5-yl]piperazine | 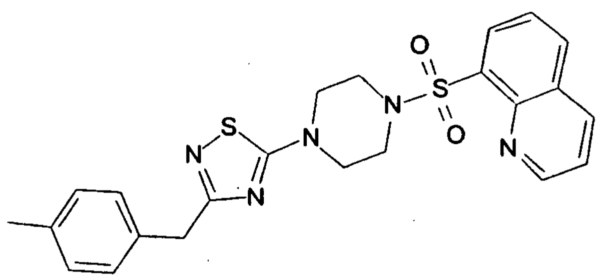 |
| 71 | 1-[4-nitrophenylsulfonyl]-4-[3-(4-methylbenzyl)-1,2,4-thiadiazol-5-yl]-2-methylpiperazine | 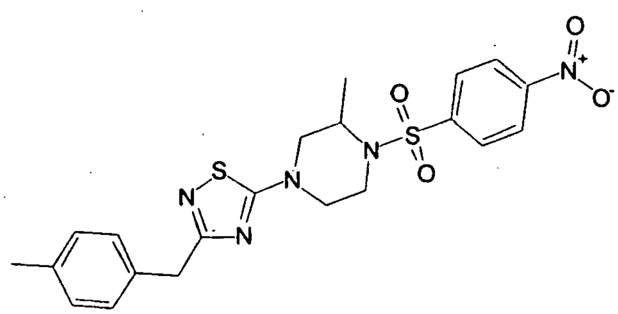 |

(continued)

| No. | Compound name | Compound structure |
|---|---|---|
| 72 | 1-[3-nitro-4-chlorophenylsulfonyl]-4-[3-(4-methylbenzyl)-1,2,4-thiadiazol-5-yl]-2-methylpiperazine | 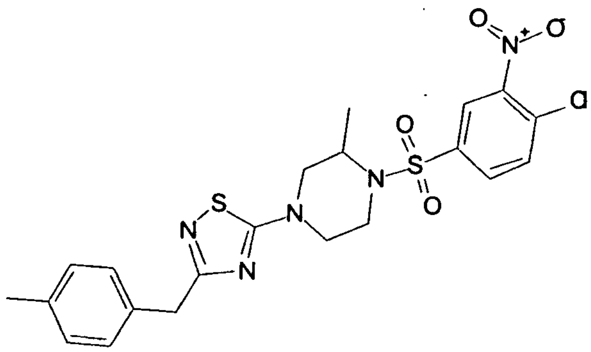 |
| 73 | 1-[4-nitrophenylsulfonyl]-4-[3-(4-fluorobenzyl)-1,2,4-thiadiazol-5-yl]-2-methylpiperazine | 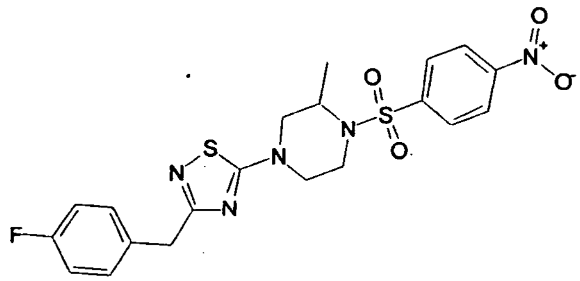 |
| 75 | 1-[3-nitro-4-chlorophenylsulfonyl]-4-[3-(4-methylbenzyl)-1,2,4-thiadiazol-5-yl]piperazine | 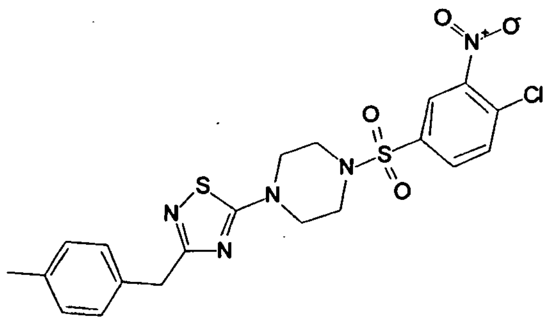 |
| 76 | 1-(benzylsulfonyl)-4-[3-(4-chlorobenzyl)-1,2,4-thiadiazol-5-yl] piperazine | 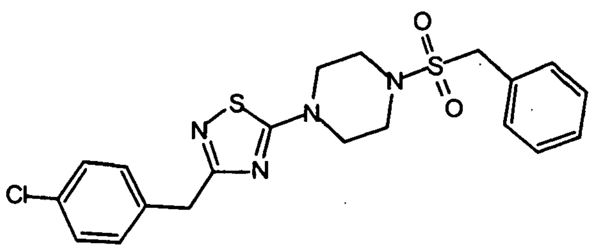 |
| 77 | 1-(benzylsulfonyl)-4-[3-(4-methylbenzyl)-1,2,4-thiadiazol-5-yl]piperazine | 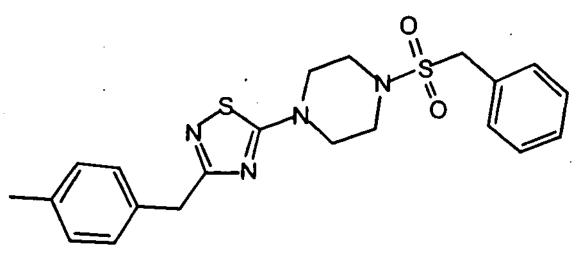 |

(continued)

| No. | Compound name | Compound structure |
|---|---|---|
| 78 | 1-(phenylprop-2-ensulfonyl)-4-[3-(4-fluorobenzyl)-1,2,4-thiadiazol-5-yl]piperazine | 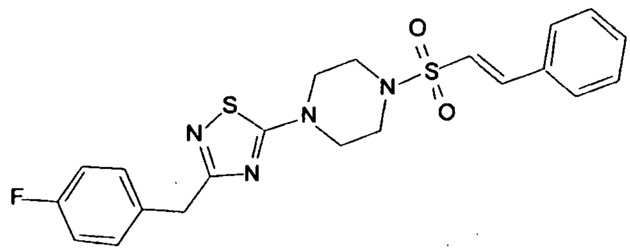 |
| 79 | 1-(phenylprop-2-ensulfonyl)-4-[3-(4-methylbenzyl)-1,2,4-thiadiazol-5-yl]-2-methylpiperazine | 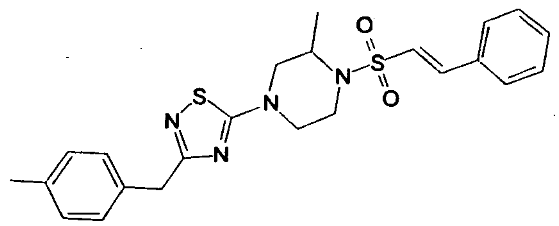 |
| 80 | 1-(phenylprop-2-ensulfonyl)-4-[3-(4-methylbenzyl)-1,2,4-thiadiazol-5-yl]piperazine | 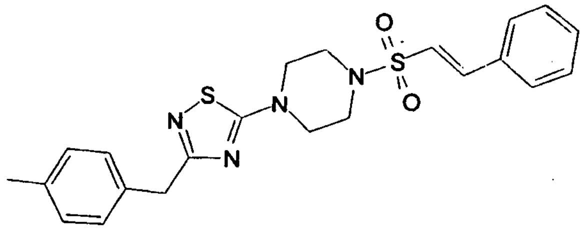 |
| 81 | 1-(butylsulfonyl)-4-[3-(4-chlorobenzyl)-1,2,4-thiadiazol-5-yl] piperazine | 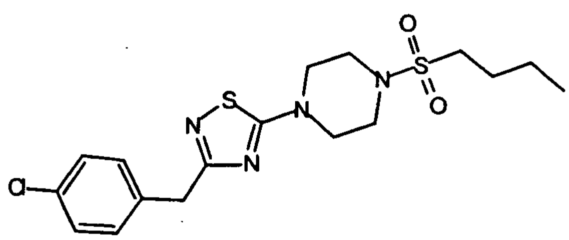 |
| 82 | 1-(octylsulfonyl)-4-[3-(4-methylbenzyl)-1,2,4-thiadiazol-5-yl]piperazine | 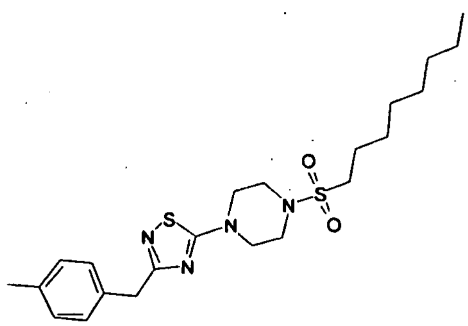 |
| 83 | 1-(butylsulfonyl)-4-[3-(4-methylbenzyl)-1,2,4-thiadiazol-5-yl]piperazine | 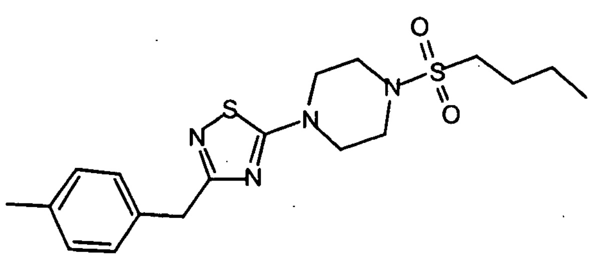 |

(continued)

| No. | Compound name | Compound structure |
|---|---|---|
| 84 | 1-(ethylsulfonyl)-4-[3-(4-chlorobenzyl)-1,2,4-thiadiazol-5-yl] piperazine | 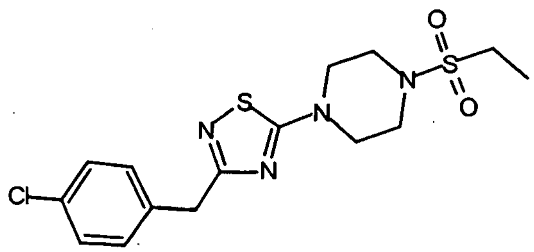 |
| 85 | 1-(isopropylsulfonyl)-4-[3-(4-methylbenzyl)-1,2,4-thiadiazol-5-yl]piperazine | 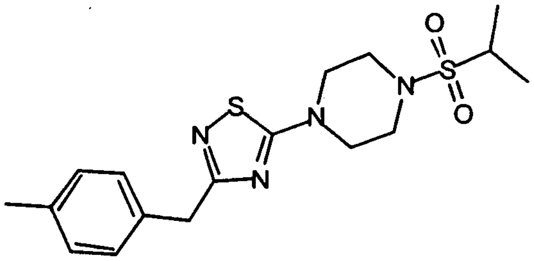 |
| 86 | 1-[(2E)-3-phenylprop-2-enoyl]-4-[3-(4-chlorobenzyl)-1,2,4-thiadiazol-5-yl]-2-methylpiperazine | 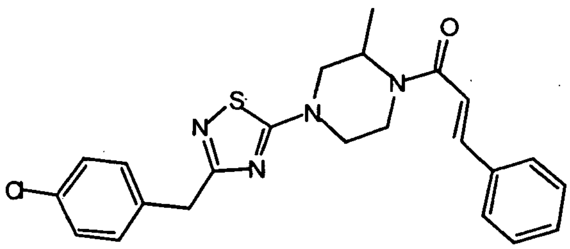 |
| 87 | 1-[(2E)-3-phenylprop-2-enoyl]-4-B103-2-methylpiperazine | 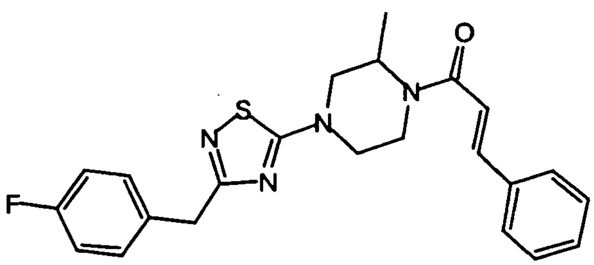 |
| 88 | 1-[(2E)-3-phenylprop-2-enoyl]-4-[3-(4-chlorobenzyl)-1,2,4-thiadiazol-5-yl]piperazine | 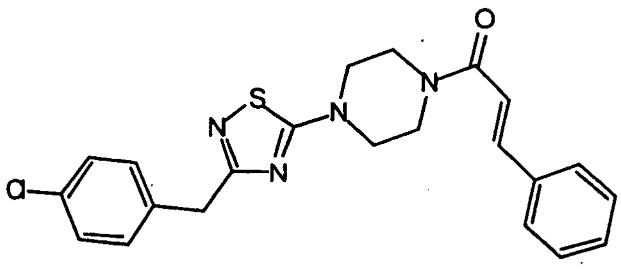 |

(continued)

| No. | Compound name | Compound structure |
|---|---|---|
| 89 | 1-(3-phenylpropanoyl)-4-[3-(4-fluorobenzyl)-1,2,4-thiadiazol-5-yl]-2-methylpiperazine | 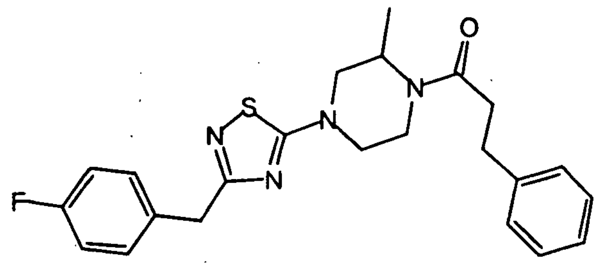 |
| 90 | 1-(3-phenylpropanoyl)-4-[3-(4-fluorobenzyl)-1,2,4-thiadiazol-5-yl] piperazine | 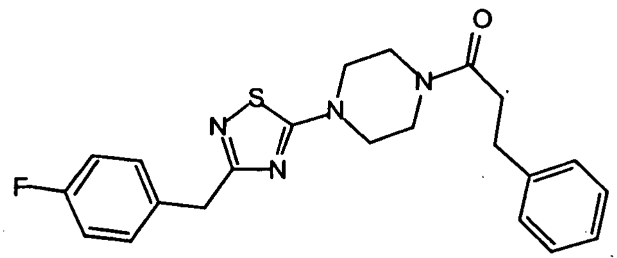 |
| 91 | 1-[3-phenylprop-2-enyl]-4-[3-(4-methylbenzyl)-1,2,4-thiadiazol-5-yl]piperazine | 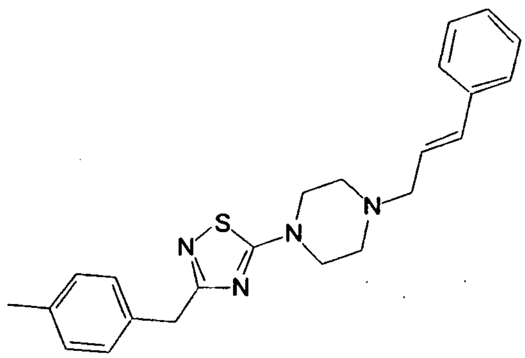 |
| 92 | 1-[3-phenylprop-2-enyl]-4-[3-(4-chlorobenzyl)-1,2,4-thiadiazol-5-yl] piperazine | 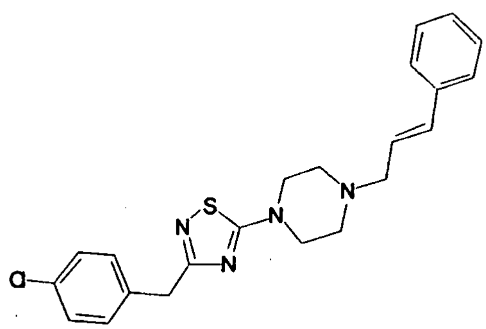 |
| 93 | 1-[3-phenylprop-2-enyl]-4-[3-(4-fluorobenzyl)-1,2,4-thiadiazol-5-yl] piperazine | 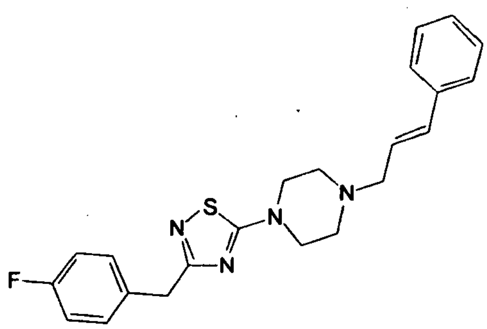 |

(continued)

| No. | Compound name | Compound structure |
|---|---|---|
| 94 | 4-pentyl-N-{2-[4-[3-(4-fluorobenzyl)-1,2,4-thiadiazol-5-yl]piperazin-1-yl]ethyl}benzamide | 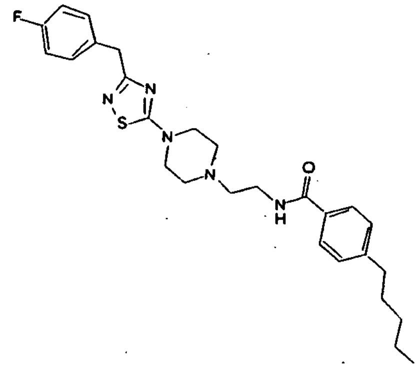 |
| 95 | 4-butyl-N-{2-[4-[3-(4-fluorobenzyl)-1,2,4-thiadiazol-5-yl]piperazin-1-yl]ethyl}benzamide | 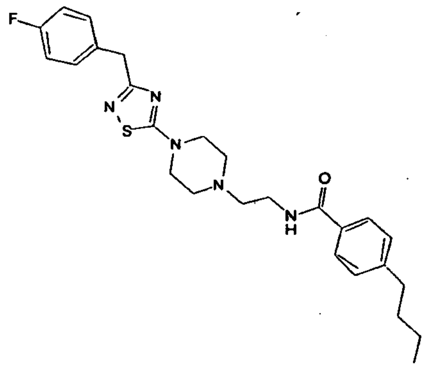 |
| 96 | 4-hexyl-N-{2-[4-[3-(4-fluorobenzyl)-1,2,4-thiadiazol-5-yl]piperazin-1-yl]ethyl}benzamide | 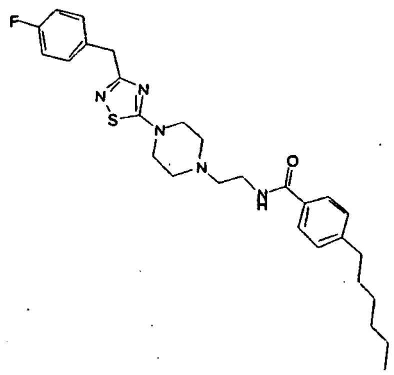 |
| 97 | 4-chloro-N-{2-[4-[3-(4-fluorobenzyl)-1,2,4-thiadiazol-5-yl]piperazin-1-yl]ethyl}benzamide | 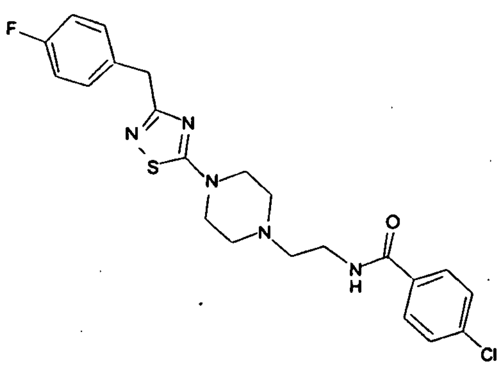 |

(continued)

| No. | Compound name | Compound structure |
|---|---|---|
| 98 | 3,5-dichloro-N-{2-[4-[3-(4-fluorobenzyl)-1,2,4-thiadiazol-5-yl] piperazin-1-yl]ethyl}benzamide | 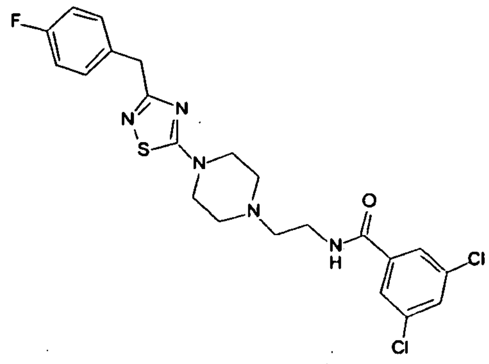 |
| 99 | 2-methyl-N-{2-[4-[3-(4-fluorobenzyl)-1,2,4-thiadiazol-5-yl] piperazin-1-yl]ethyl}benzamide | 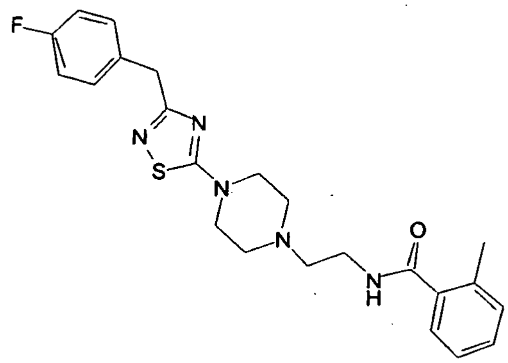 |
| 100 | 3-fluoro-N-(2-[4-[3-(4-chlorobenzyl)-1,2,4-thiadiazof-5-yl] piperazin-1-yl]ethyl)benzamide | 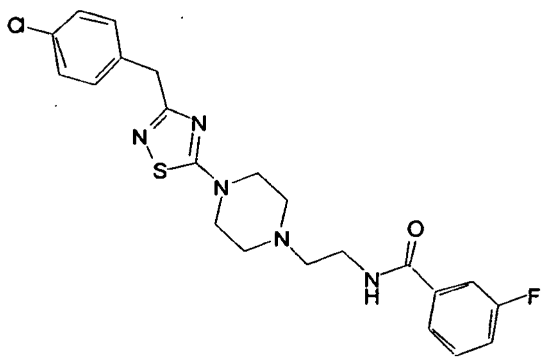 |
| 101 | 4-methyl-N-{2-[4-[3-(4-fluorobenzyl)-1,2,4-thiadiazol-5-yl] piperazin-1-yl]ethyl}benzamide | 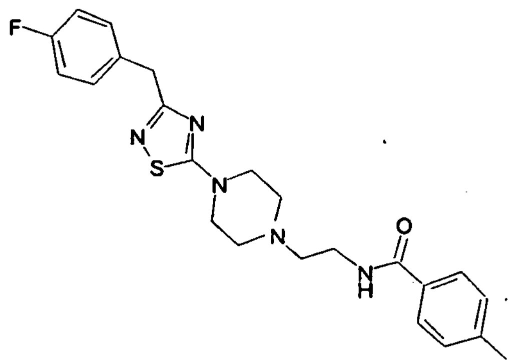 |

(continued)

| No. | Compound name | Compound structure |
|---|---|---|
| 102 | 4-fluoro-N-{2-[4-[3-(4-fluorobenzyl)-1,2,4-thiadiazol-5-yl]piperazin-1-yl]ethyl}benzamide | 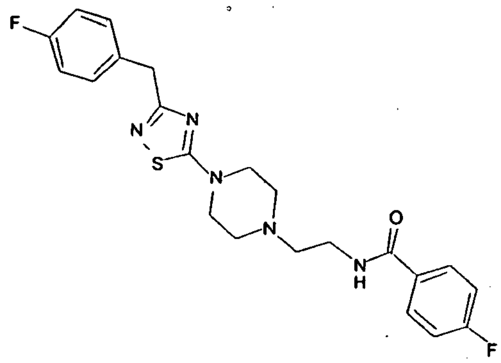 |
| 103 | 4-ethyl-N-{2-[4-[3-(4-fluorobenzyl)-1,2,4-thiadiazol-5-yl]piperazin-1-yl]ethyl}benzamide | 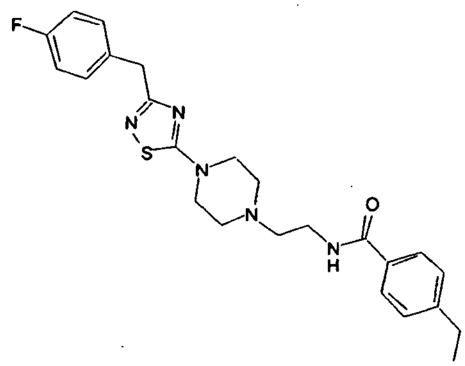 |
| 104 | N-{2-[4-[3-(4-fluorobenzyl)-1,2,4-thiadiazol-5-yl]piperazin-1-yl]ethyl}benzamide | 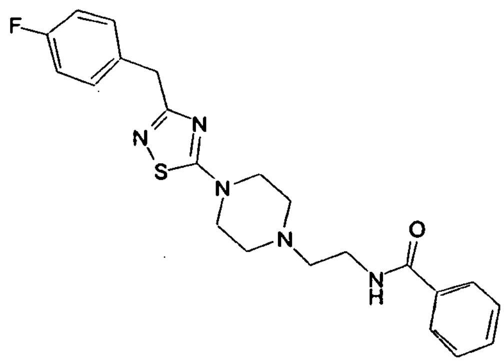 |
| 105 | 3-fluoro-N-{2-[4-[3-(4-fluorobenzyl)-1,2,4-thiadiazol-5-yl]piperazin-1-yl]ethyl}benzamide | 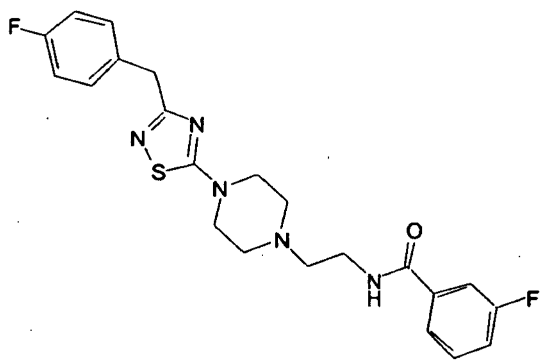 |

(continued)

| No. | Compound name | Compound structure |
|---|---|---|
| 106 | 3-fluoro-N-{2-[4-[3-benzyl-1,2,4-thiadiazol-5-yl]piperazin-1-yl]ethyl} benzamide | 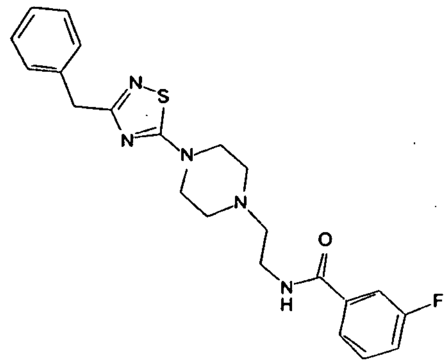 |
| 107 | 2-(4-fluorophenyl)-N-{2-[4-[3-(4-fluorobenzyl)-1,2,4-thiadiazol-5-yl] piperazin-1-yl]ethyl}acetamide | 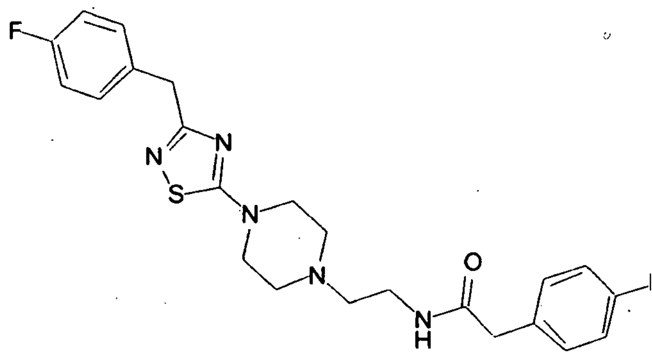 |
| 108 | 2-phenyl-N-{2-[4-[3-(4-fluorobenzyl)-1,2,4-thiadiazol-5-yl] piperazin-1-yl]ethyl}acetamide | 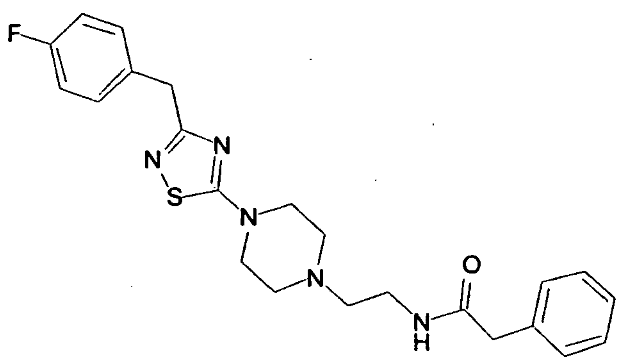 |
| 109 | 2-(4-fluorophenyl)-N-{2-[4-[3-(4-methylbenzyl)-1,2,4-thiadiazol-5-yl]piperazin-1-yl]ethyl}acetamide | 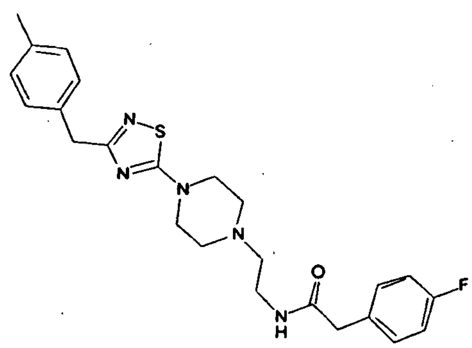 |

(continued)

| No. | Compound name | Compound structure |
|---|---|---|
| 110 | N-benzyl-N'-{2-[4-[3-(4-fluorobenzyl)-1,2,4-thiadiazol-5-yl]piperazin-1-yl]ethyl}urea | 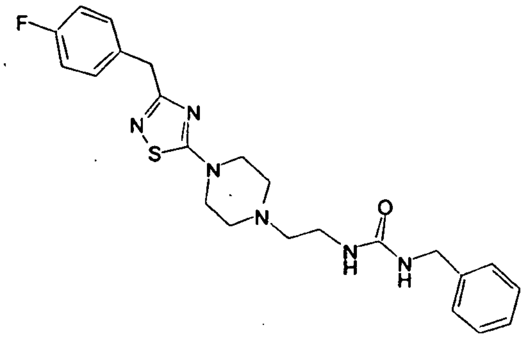 |
| 111 | 2-phenyl-N-{2-[4-[3-(4-fluorobenzyl)-1,2,4-thiadiazol-5-yl]piperazin-1-yl]ethyl}butanamide | 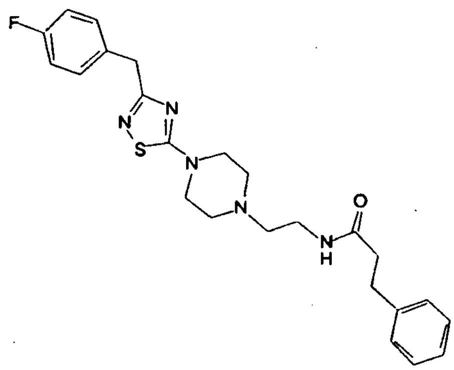 |
| 112 | 3-phenyl-N-{2-[4-[3-(4-fluorobenzyl)-1,2,4-thiadiazol-5-yl]piperazin-1-yl]ethyl}propanamide | 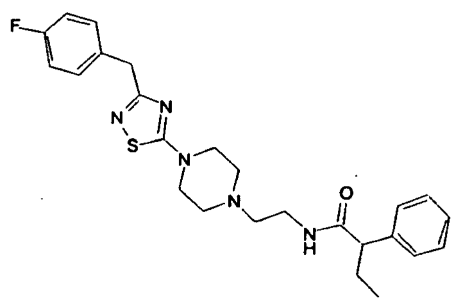 |
| 113 | 1-benzyl-4-[3-(4-fluorobenzyl)-1,2,4-thiadiazol-5-yl]piperazine | 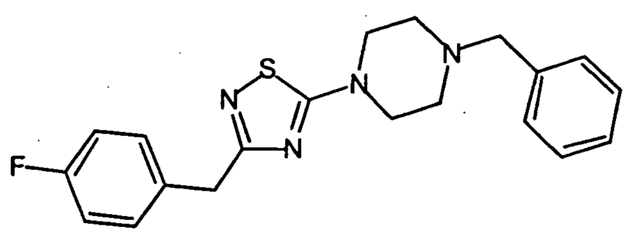 |
| 114 | 1-(1,3-benzodioxol-5-ylmethyl)-4-[3-(4-fluorobenzyl)-1,2,4-thiadiazol-5-yl]piperazine | 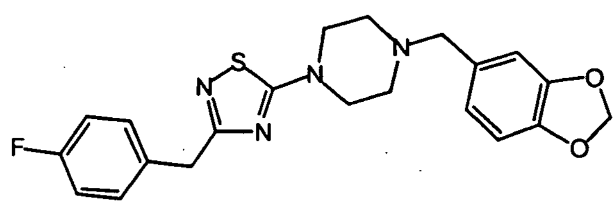 |

(continued)

| No. | Compound name | Compound structure |
|---|---|---|
| 115 | 1-(2-fluorobenzoyl)-4-[3-(3-methoxybenzyl)-1,2,4-thiadiazol-5-yl]-2-methylpiperazine | 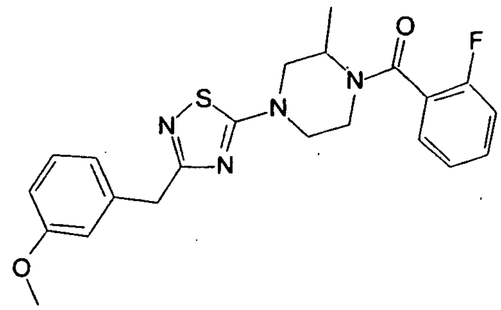 |
| 116 | 1-(4-ethylbenzoyl)-4-[3-(4-fluorobenzyl)-1,2,4-thiadiazol-5-yl]-2-methylpiperazine | 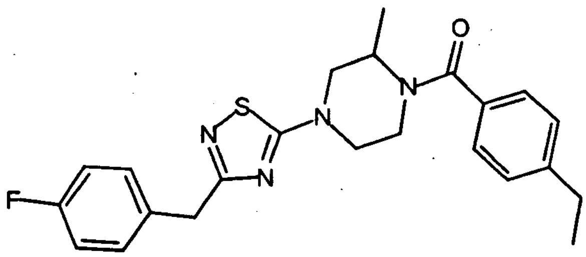 |
| 117 | 1-(4-butylbenzoyl)-4-[3-(4-methylbenzyl)-1,2,4-thiadiazol-5-yl]-2-methylpiperazine | 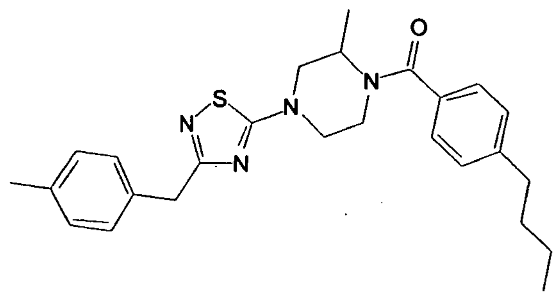 |
| 118 | 1-(4-methoxybenzoyl)-4-[3-(4-fluorobenzyl)-1,2,4-thiadiazol-5-yl]-2-methylpiperazine | 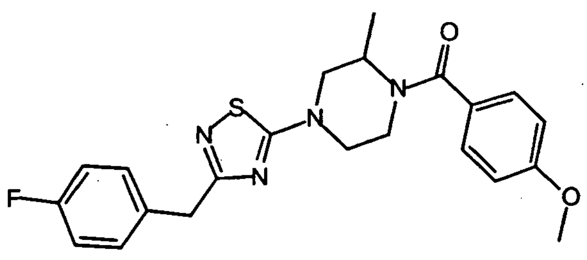 |
| 119 | 1-(1,3-benzodioxol-5-ylcarbonyl)-4-[3-(3-methoxybenzyl)-1,2,4-thiadiazol-5-yl]-2-methylpiperazine | 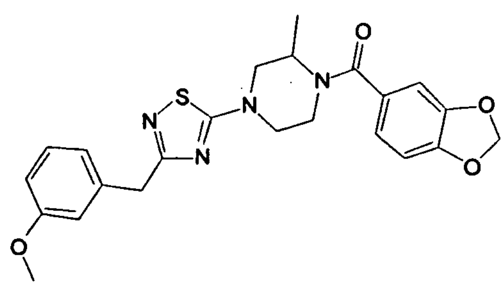 |

(continued)

| No. | Compound name | Compound structure |
|---|---|---|
| 120 | 1-(4-butylbenzoyl)-4-[3-(4-fluorobenzyl)-1,2,4-thiadiazol-5-yl]-2-methylpiperazine | 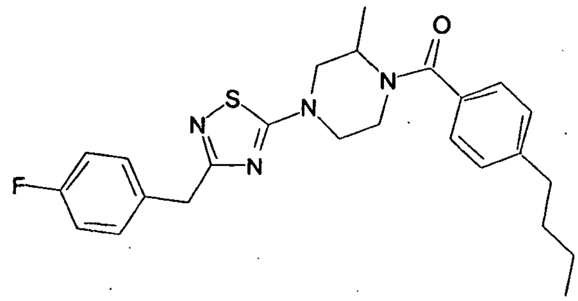 |
| 121 | 1-(4-hexylbenzoyl)-4-[3-(4-fluorobenzyl)-1,2,4-thiadiazol-5-yl] piperazine | 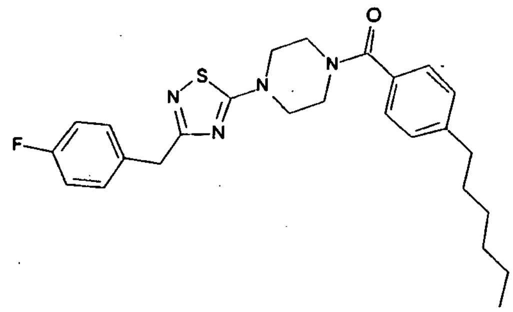 |
| 122 | 1-(3-chlorobenzoyl)-4-[3-(4-fluorobenzyl)-1,2,4-thiadiazol-5-yl]-2-methylpiperazine | 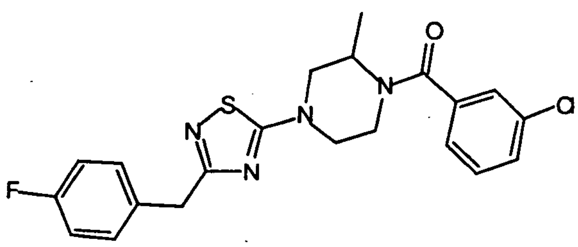 |
| 123 | 1-(4-fluorobenzoyl)-4-[3-(3-methoxybenzyl)-1,2,4-thiadiazol-5-yl]-2-methylpiperazine | 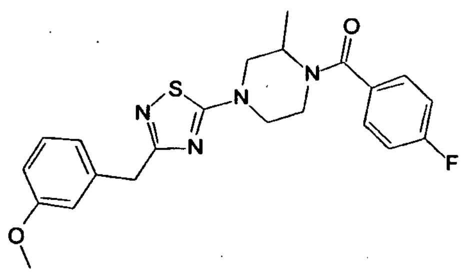 |
| 124 | 1-(4-methylbenzoyl)-4-[3-(3-methoxybenzyl)-1,2,4-thiadiazol-5-yl]-2-methylpiperazine | 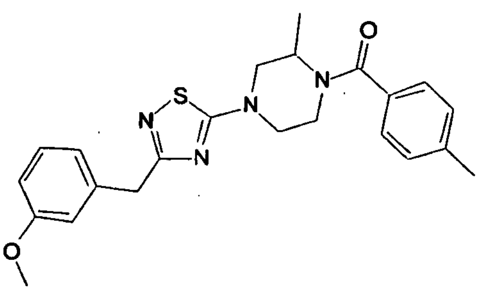 |

(continued)

| No. | Compound name | Compound structure |
|---|---|---|
| 125 | 1-(3-fluorobenzoyl)-4-[3-(4-chlorobenzyl)-1,2,4-thiadiazol-5-yl]-2-methylpiperazine | 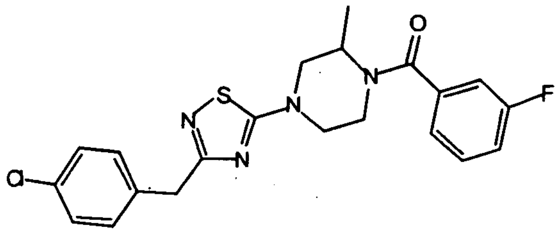 |
| 126 | 1-benzoyl-4-[3-(4-fluorobenzyl)-1,2,4-thiadiazol-5-yl]-2-methylpiperazine | 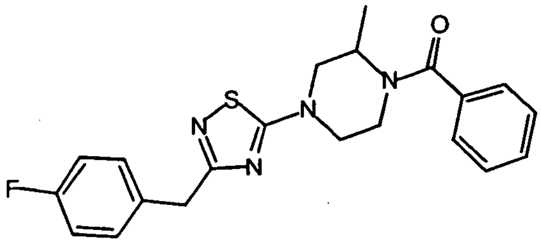 |
| 127 | 1-(4-fluorobenzoyl)-4-[3-(4-fluorobenzyl)-1,2,4-thiadiazol-5-yl]-2-methylpiperazine | 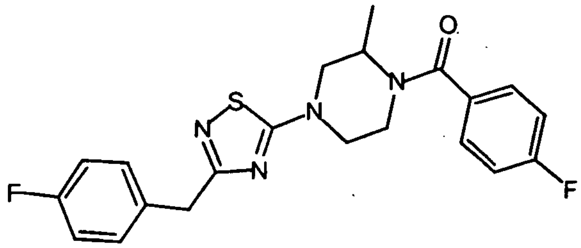 |
| 128 | 1-(4-tert-butylbenzoyl)-4-[3-(4-fluorobenzyl)-1,2,4-thiadiazol-5-yl]-2-methylpiperazine | 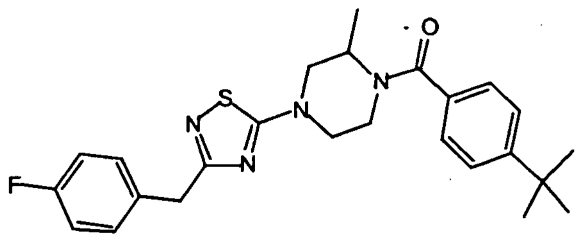 |
| 129 | 1-(1,1'-biphenyl-4-ylcarbonyl)-4-[3-(4-fluorobenzyl)-1,2,4-thiadiazol-5-yl]-2-methylpiperazine | 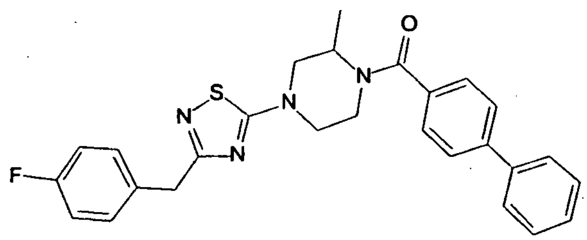 |
| 130 | 1-(4-methoxybenzoyl)-4-[3-(4-methylbenzyl)-1,2,4-thiadiazol-5-yl]-2-methylpiperazine | 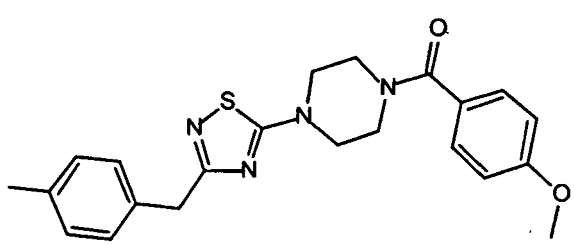 |

(continued)

| No. | Compound name | Compound structure |
|---|---|---|
| 131 | 1-(4-ethylbenzoyl)-4-[3-(4-methylbenzyl)-1,2,4-thiadiazol-5-yl]piperazine | 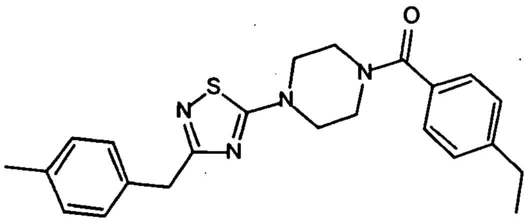 |
| 132 | 1-(2-naphthoyl)-4-[3-(4-fluorobenzyl)-1,2,4-thiadiazol-5-yl]-2-methylpiperazine | 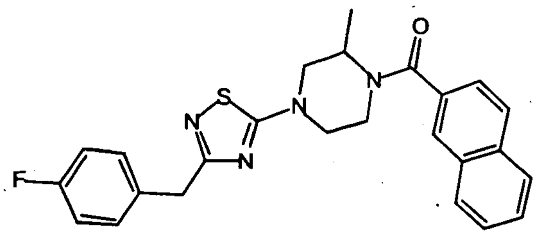 |
| 133 | 1-(2-methoxybenzoyl)-4-[3-(4-fluorobenzyl)-1,2,4-thiadiazol-5-yl]-2-methylpiperazine | 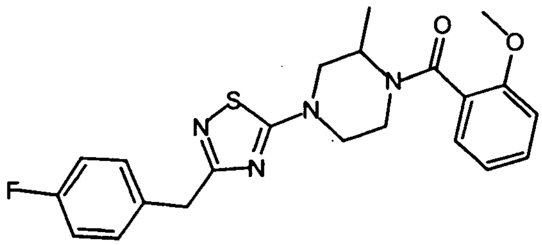 |
| 134 | 1-(4-pentylbenzoyl)-4-[3-(4-methylbenzyl)-1,2,4-thiadiazol-5-yl]-2-methylpiperazine | 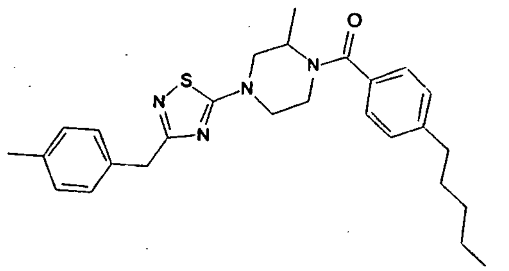 |
| 135 | 1-(4-bromobenzoyl)-4-[3-(4-fluorobenzyl)-1,2,4-thiadiazol-5-yl]-2-methylpiperazine | 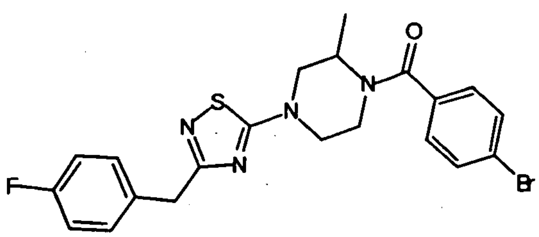 |
| 136 | 1-(2,4-dimethoxybenzoyl)-4-[3-(4-fluorobenzyl)-1,2,4-thiadiazol-5-yl]-2-methylpiperazine | 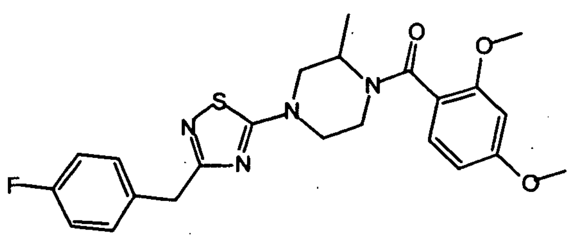 |

(continued)

| No. | Compound name | Compound structure |
|---|---|---|
| 137 | 1-(3,5-dichlorobenzoyl)-4-[3-(4-fluorobenzyl)-1,2,4-thiadiazol-5-yl] piperazine | 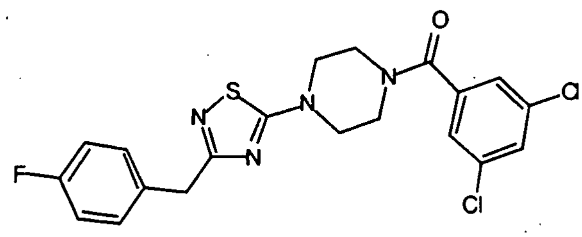 |
| 138 | 1-(3-chlorobenzoyl)-4-[3-(4-fluorobenzyl)-1,2,4-thiadiazol-5-yl] piperazine | 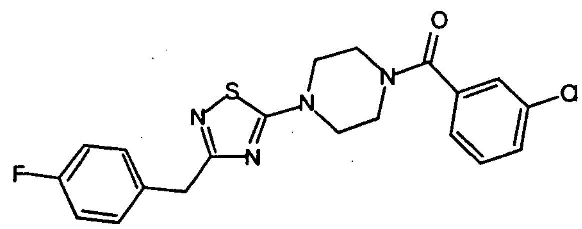 |
| 139 | 1-(4-methylbenzoyl)-4-[3-(4-fluorobenzyl)-1,2,4-thiadiazol-5-yl]-2-methylpiperazine | 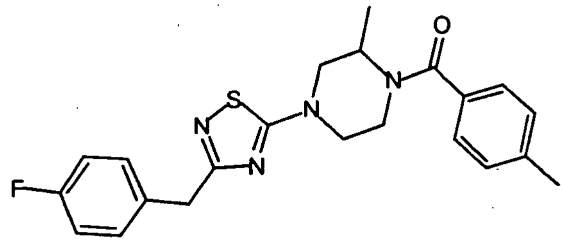 |
| 140 | 1-(2-methylbenzoyl)-4-[3-(4-fluorobenzyl)-1,2,4-thiadiazol-5-yl]-2-methylpiperazine | 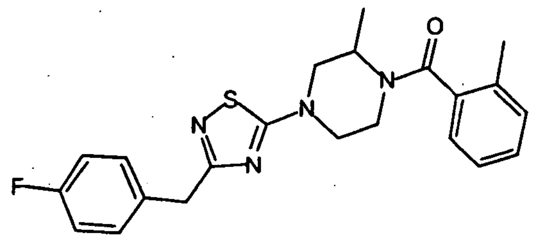 |
| 141 | 1-(4-methylbenzoyl)-4-[3-(4-fluorobenzyl)-1,2,4-thiadiazol-5-yl] piperazine | 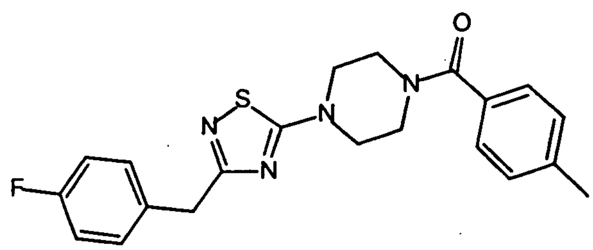 |
| 142 | 1-(3-bromobenzoyl)-4-[3-(4-fluorobenzyl)-1,2,4-thiadiazol-5-yl]-2-methylpiperazine | 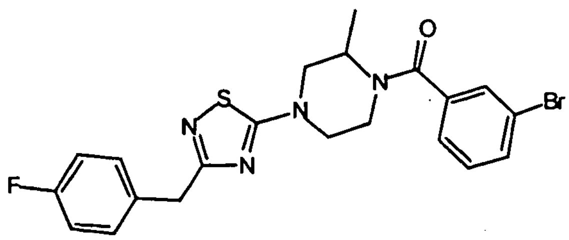 |

(continued)

| No. | Compound name | Compound structure |
|---|---|---|
| 143 | 1-(4-ethylbenzoyl)-4-[3-(3-methoxybenzyl)-1,2,4-thiadiazol-5-yl]-2-methylpiperazi ne | 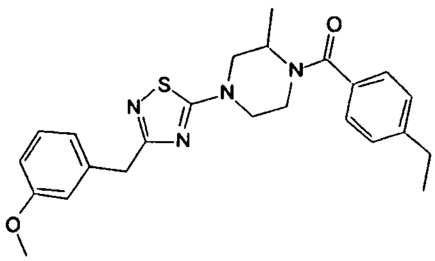 |
| 144 | 1-(3-methylbenzoyl)-4-[3-(4-methylbenzyl)-1,2,4-thiadiazol-5-yl]-2-methylpiperazine | 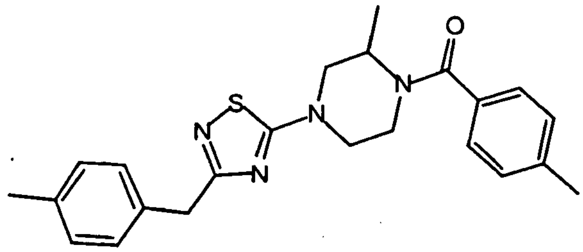 |
| 145 | 1-(3-trifluoromethylbenzoyl)-4-[3-(4-fluorobenzyl)-1,2,4-thiadiazol-5-yl]-2-methylpiperazine | 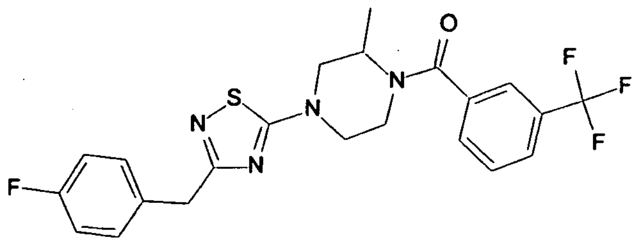 |
| 146 | 1-(4-tert-butylbenzoyl)-4-[3-(3-methoxybenzyl)-1,2,4-thiadiazol-5-yl]-2-methylpiperazine | 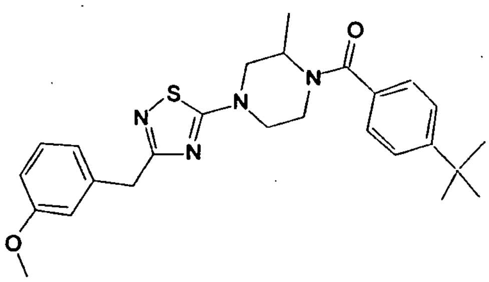 |
| 147 | 1-(4-ethylbenzoyl)-4-[3-(4-methylbenzyl)-1,2,4-thiadiazol-5-yl]-2-methylpiperazine | 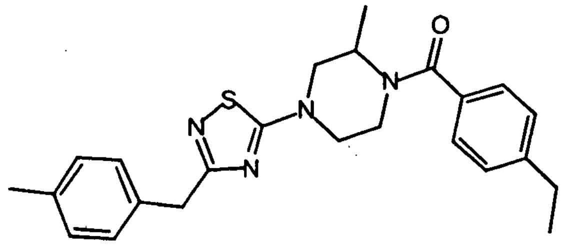 |
| 148 | 1-(2-bromobenzoyl)-4-[3-(4-fluorobenzyl)-1,2,4-thiadiazol-5-yl]-2-methylpiperazine | 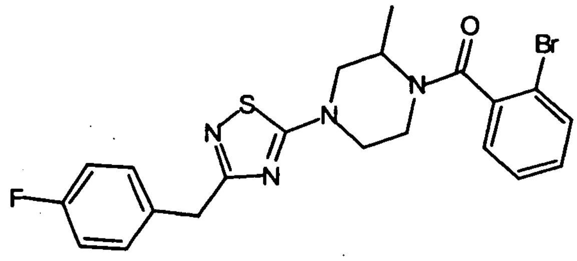 |

(continued)

| No. | Compound name | Compound structure |
|---|---|---|
| 149 | 1-(2-fluorobenzoyl)-4-[3-(4-methylbenzyl)-1,2,4-thiadiazol-5-yl]-2-methylpiperazine | 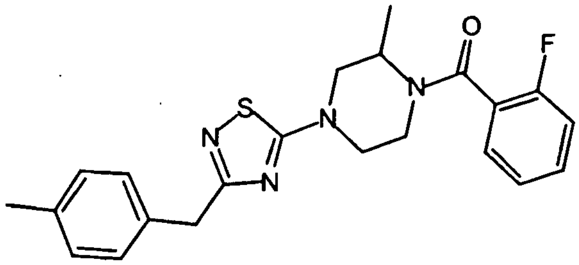 |
| 150 | 1-(3-fluorobenzoyl)-4-[3-benzyl-1,2,4-thiadiazol-5-yl]-2-methylpiperazine | 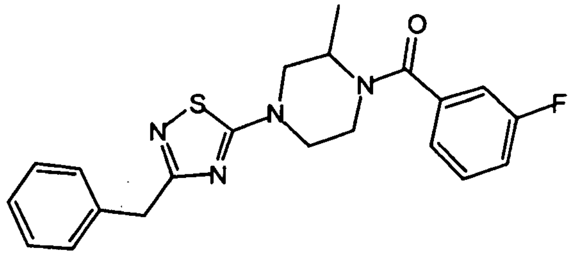 |
| 151 | 1-(4-fluorobenzoyl)-4-[3-(4-methylbenzyl)-1,2,4-thiadiazol-5-yl]-2-methylpiperazine | 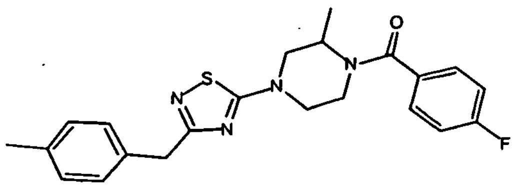 |
| 152 | 1-(2-methylbenzoyl)-4-[3-(4-fluorobenzyl)-1,2,4-thiadiazol-5-yl] piperazine | 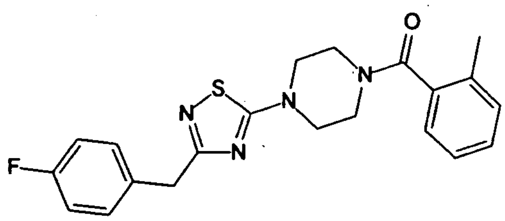 |
| 153 | 1-(4-fluorobenzoyl)-4-[3-(4-chlorobenzyl)-1,2,4-thiadiazol-5-yl] piperazine | 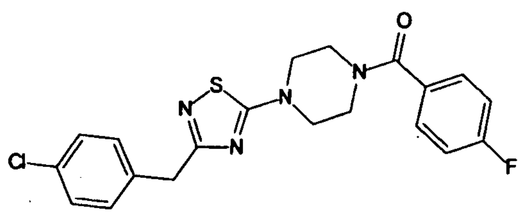 |
| 154 | 1-(4-fluorobenzoyl)-4-[3-benzyl-1,2,4-thiadiazol-5-yl]-2-methylpiperazine | 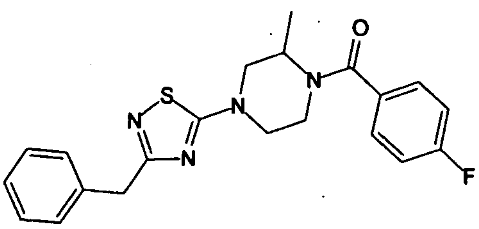 |

(continued)

| No. | Compound name | Compound structure |
|---|---|---|
| 155 | 1-(4-chlorobenzoyl)-4-[3-(4-methylbenzyl)-1,2,4-thiadiazol-5-yl]piperabine | 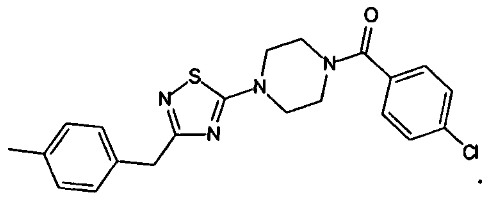 |
| 156 | 1-(3-fluorobenzoyl)-4-[3-(4-methylbenzyl)-1,2,4-thiadiazol-5-yl]-2-methylpiperazine | 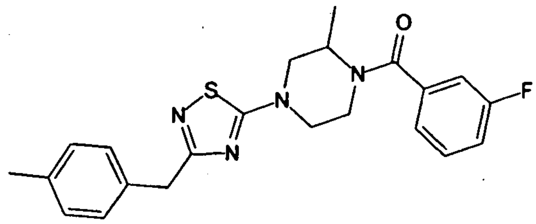 |
| 157 | 1-(3-fluorobenzoyl)-4-[3-(4-chlorobenzyl)-1,2,4-thiadiazol-5-yl] piperazine | 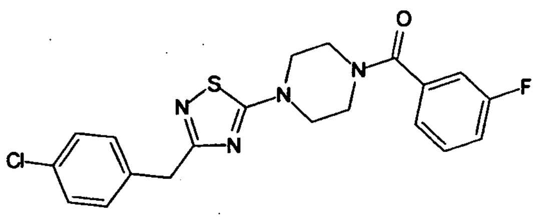 |
| 158 | 1-benzoyl-4-[3-(4-methylbenzyl)-1,2,4-thiadiazol-5-yl]piperazine | 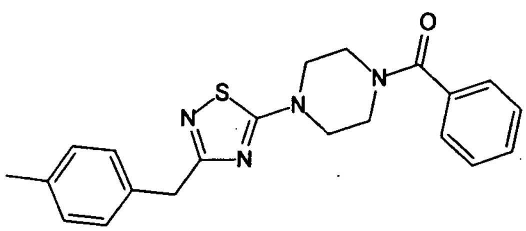 |
| 159 | 1-(2-fluorobenzoyl)-4-[3-(4-chlorobenzyl)-1,2,4-thiadiazol-5-yl] piperazine | 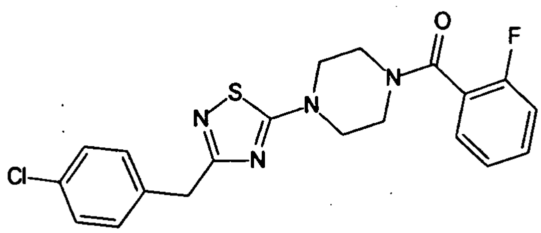 |
| 160 | 1-(4-fluorobenzoyl)-4-[3-(4-methylbenzyl)-1,2,4-thiadiazol-5-yl]piperazine | 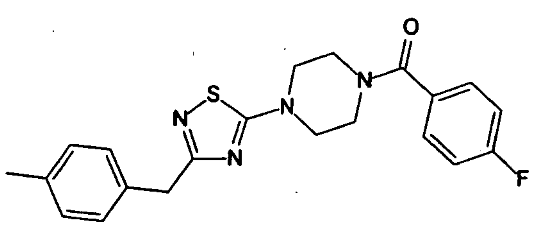 |

(continued)

| No. | Compound name | Compound structure |
|---|---|---|
| 161 | 1-(1,3-benzodioxol-5-ylcarbonyl)-4-[3-(4-methylbenzyl)-1,2,4-thiadiazol-5-yl]-2-methyl+B176piperazine | 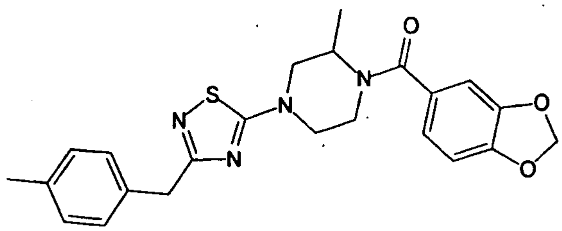 |
| 162 | 1-(3-flurorbenzoyl)-4-[3-(4-methylbenzyl)-1,2,4-thiadiazol-5-yl]piperazine | 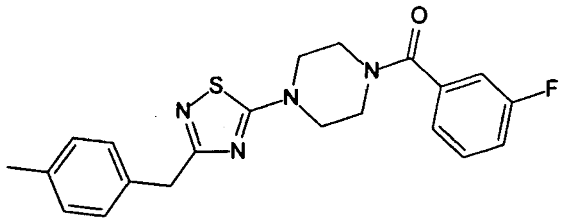 |
| 163 | 1-(4-fluorobenzoyl)-4-[3-benzyl-1,2,4-thiadiazol-5-yl]piperazine | 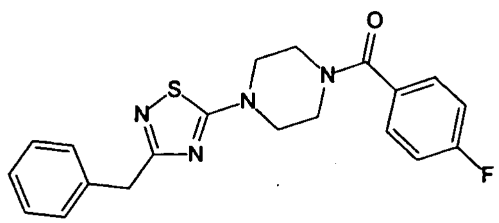 |
| 164 | 1-(4-bromobenzoyl)-4-[3-(4-methylbenzyl)-1,2,4-thiadiazol-5-yl]piperazine | 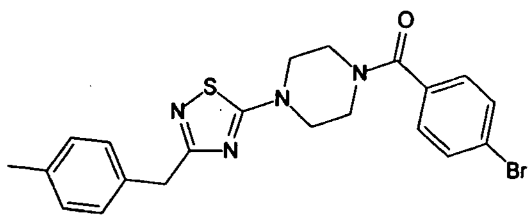 |
| 165 | 1-(4-ethylbenzoyl)-4-[3-(4-chlorobenzyl)-1,2,4-thiadiazol-5-yl] piperazine | 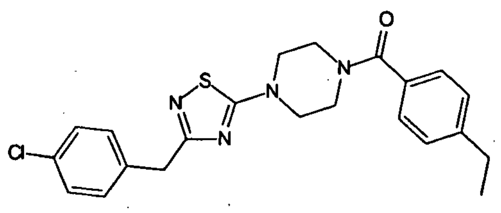 |
| 166 | 1-(2-chlorobenzoyl)-4-[3-(4-fluorobenzyl)-1,2,4-thiadiazol-5-yl]-2-methylpiperazine | 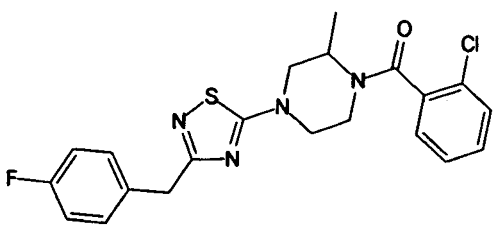 |

(continued)

| No. | Compound name | Compound structure |
|---|---|---|
| 167 | 1-(2-fluorobenzoyl)-4-[3-(4-chlorobenzyl)-1,2,4-thiadiazol-5-yl]-2-methylpiperazine | 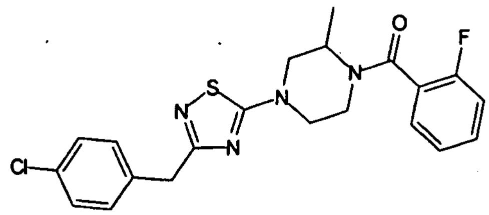 |
| 168 | 1-(4-trifluoromethylbenzoyl)-4-[3-(3-methoxybenzyl)-1,2,4-thiadiazol-5-yl]-2-methylpiperazine | 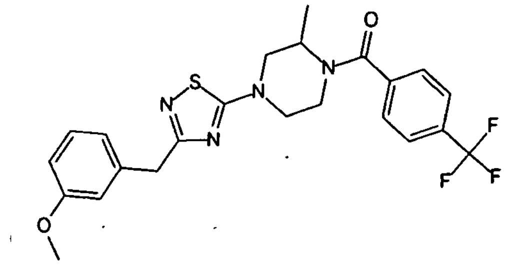 |
| 169 | 1-benzoyl-4-[3-(4-methylbenzyl)-1,2,4-thiadiazol-5-yl]-2-methylpiperazine | 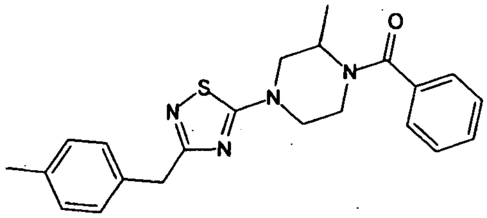 |
| 170 | 1-(4-bromobenzoyl)-4-[3-(3-methoxybenzyl)-1,2,4-thiadiazol-5-yl]-2-methylpiperazine | 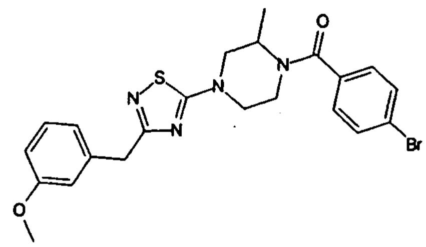 |
| 171 | 1-(4-trifluoromethylbenzoyl)-4-[3-(4-methylbenzyl)-1,2,4-thiadiazol-5-yl]piperazine | 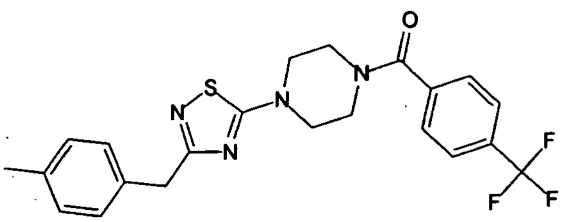 |
| 172 | 1-(3-nitro-4-methylbenzoyl)-4-[3-(4-fluorobenzyl)-1,2,4-thiadiazol-5-yl]-2-methylpiperazine | 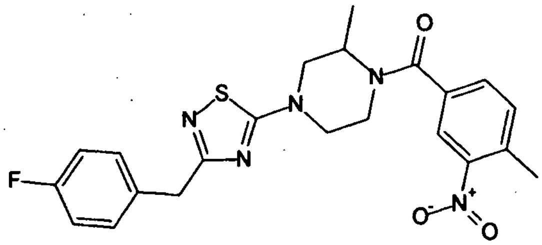 |

(continued)

| No. | Compound name | Compound structure |
|---|---|---|
| 173 | 1-benzoyl-4-[3-(3-methoxybenzyl)-1,2,4-thiadiazol-5-yl]-2-methylpiperazine | 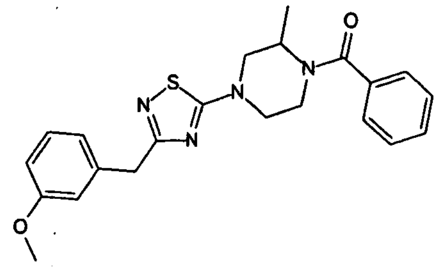 |
| 174 | 1-(4-chlorobenzoyl)-4-[3-(3-methoxybenzyl)-1,2,4-thiadiazol-5-yl]-2-methylpiperazine | 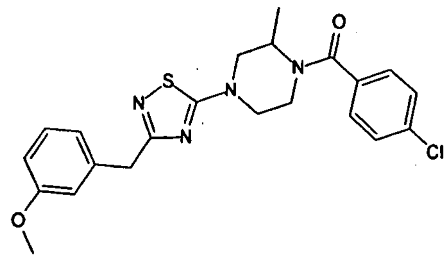 |
| 175 | 1-(2-fluorobenzoyl)-4-[3-(4-methylbenzyl)-1,2,4-thiadiazol-5-yl]piperazine | 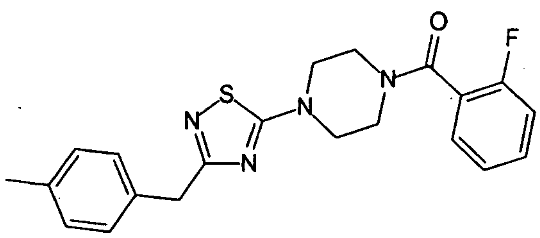 |
| 176 | 1-(4-hexylbenzoyl)-4-[3-(4-methylbenzyl)-1,2,4-thiadiazol-5-yl]piperazine | 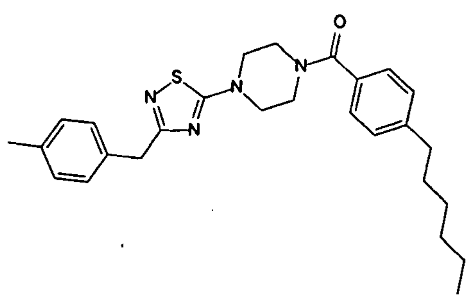 |
| 177 | 1-(2-chloro-4-nitrobenzoyl)-4-[3-(4-fluorobenzyl)-1,2,4-thiadiazol-5-yl]-2-methylpiperazine | 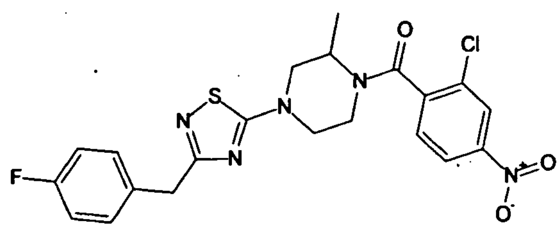 |
| 178 | 1-(1,3-benzodioxol-5-ylcarbonyl)-4-[3-(4-fluorobenzyl)-1,2,4-thiadiazol-5-yl]-2-methylpiperazine | 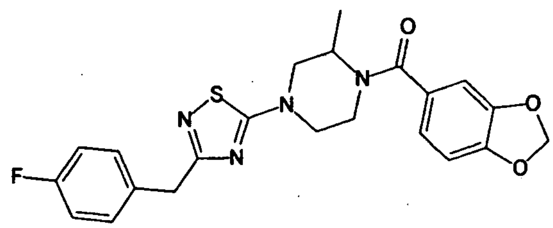 |

(continued)

| No. | Compound name | Compound structure |
|---|---|---|
| 179 | 1-(3-fluorobenzoyl)-4-[3-(4-fluorobenzyl)-1,2,4-thiadiazol-5-yl]-2-methylpiperazine | 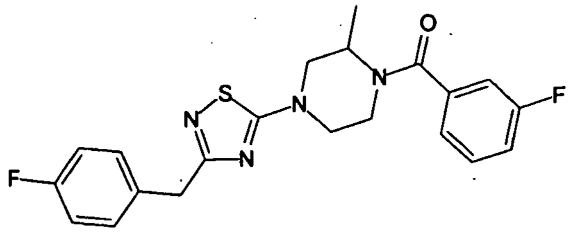 |
| 180 | 1-(4-tert-butylbenzoyl)-4-[3-(4-fluorobenzyl)-1,2,4-thiadiazol-5-yl] piperazine | 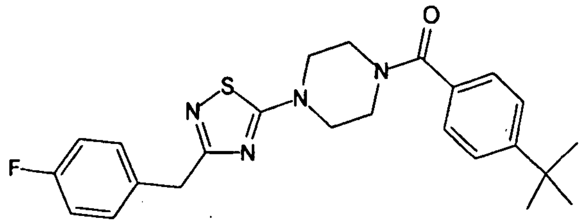 |
| 181 | 1-benzoyl-4-[3-(4-fluorobenzyl)-1,2,4-thiadiazol-5-yl]piperazine | 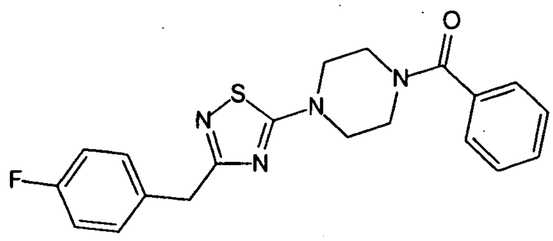 |
| 182 | 1-(4-butylbenzoyl)-4-[3-(4-methylbenzyl)-1,2,4-thiadiazol-5-yl]piperazine | 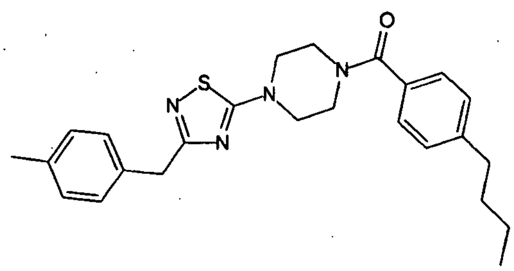 |
| 183 | 1-(4-tert-butylbenzoyl)-4-[3-(4-methylbenzyl)-1,2,4-thiadiazol-5-yl]piperazine | 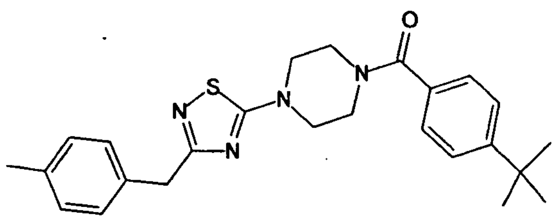 |
| 184 | 1-(4-nitrobenzoyl)-4-[3-(4-fluorobenzyl)-1,2,4-thiadiazol-5-yl]-2-methylpiperazine | 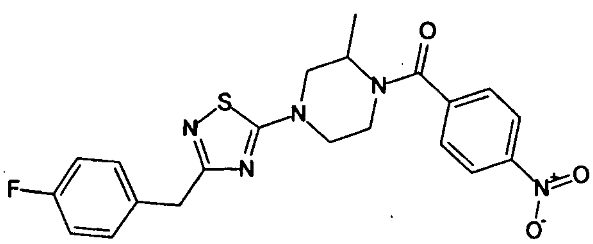 |

(continued)

| No. | Compound name | Compound structure |
|---|---|---|
| 185 | 1-(2-methylphenyl)-4-[3-(4-fluorobenzyl)-1,2,4-thiadiazol-5-yl] piperazine | 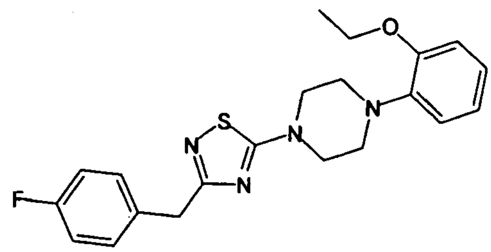 |
| 186 | 1-(2-ethoxyphenyl)-4-[3-(4-fluorobenzyl)-1,2,4-thiadiazol-5-yl] piperazine | 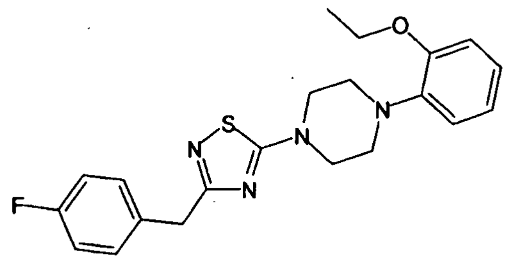 |
| 187 | 1-(2-fluorophenyl)-4-[3-(4-fluorobenzyl)-1,2,4-thiadiazol-5-yl] piperazine | 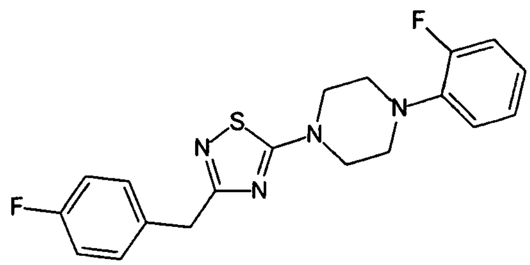 |
| 188 | 1-(3-trifluoromethylphenyl)-4-[3-(4-fluorobenzyl)-1,2,4-thiadiazol-5-yl] piperazine | 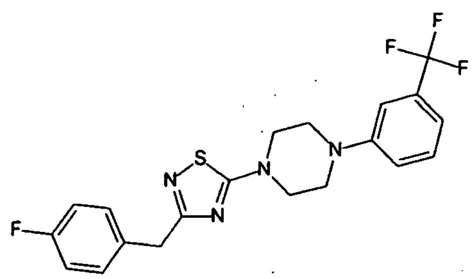 |
| 189 | 1-(4-chlorobenzoyl)-4-[3-(4-fluorobenzyl)-1,2,4-thiadiazol-5-yl]-1,4-diazepane | 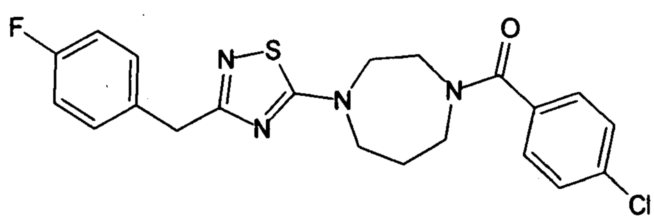 |
| 190 | 1-(4-methylbenzoyl)-4-[3-(4-fluorobenzyt)-1,2,4-thiadiazol-5-yl]-1,4-diazepane | 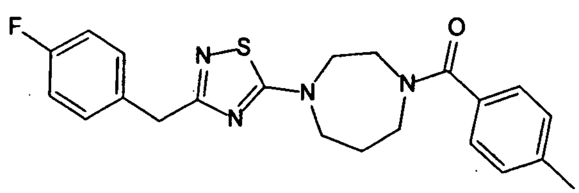 |

(continued)

| No. | Compound name | Compound structure |
|---|---|---|
| 191 | 1-(1,3-benzodioxol-5-ylcarbonyl)-4-[3-(4-fluorobenzyl)-1,2,4-thiadiazol-5-yl]-1,4-diazepane | 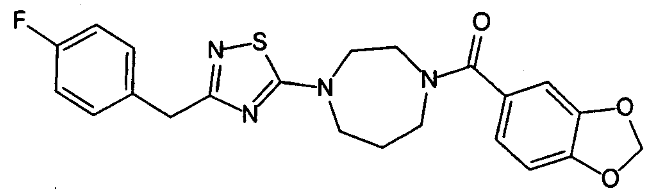 |
| 193 | 1-[4-methoxyphenylsulfonyl]-4-[3-(3-fluoro-benzyl)-1,2,4-thiadiazol-5-yl]piperazine | 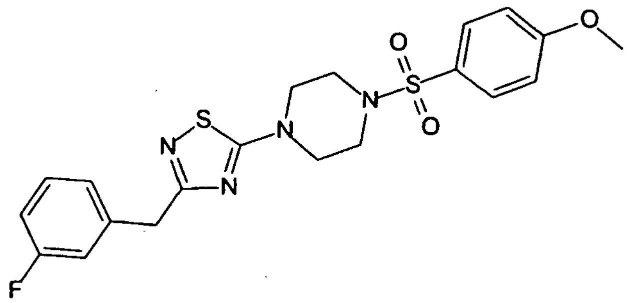 |
| 194 | 1-[4-methoxyphenylsulfonyl]-4-[3-(2-fluoro-benzyl)-1,2,4-thiadiazol-5-yl]piperazine | 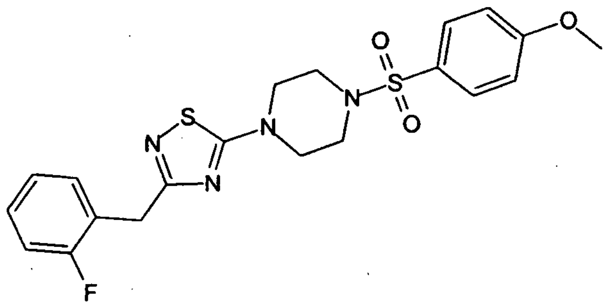 |
| 195 | 1-[2-methoxyphenylsulfonyl]-4-[3-(4-fluoro-benzyl)-1,2,4-thiadiazol-5-yl]piperazine | 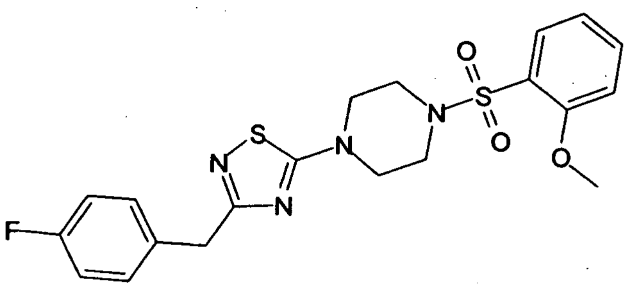 |
| 196 | 1-[4-ethoxyphenylsulfonyl]-4-[3-(4-fluoro-benzyl)-1,2,4-thiadiazol-5-yl] piperazine | 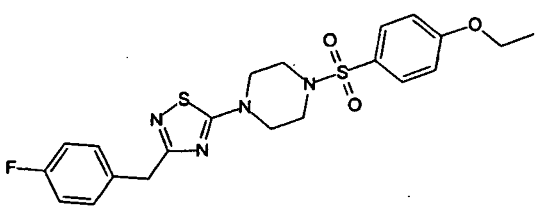 |

(continued)

| No. | Compound name | Compound structure |
|---|---|---|
| 197 | 1-[4-ethylphenylsulfonyl]-4-[3-(4-fluoro-benzyl)-1,2,4-thiadiazol-5-yl] piperazine | 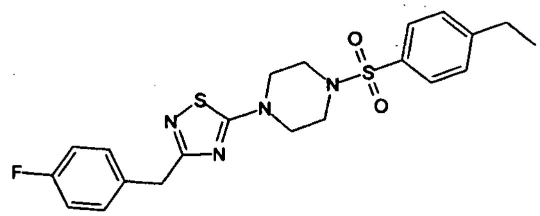 |
| 198 | 1-phenylsulfonyl-4-[3-(4-fluoro-benzyl)-1,2,4-thiadiazol-5-yl] piperazine | 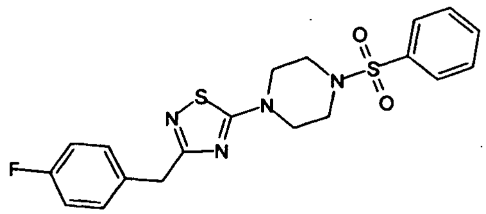 |
| 199 | 1-[4-methoxyphenylsulfonyl]-4-[3-benzyl-1,2,4-thiadiazol-5-yl] piperazine | 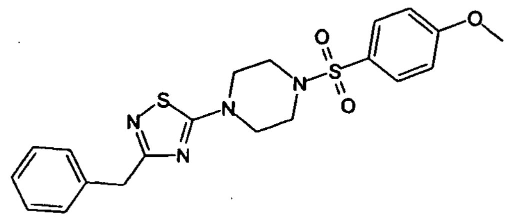 |
| 200 | 1-(4-propionyloxy)benzene-sulfonyl-4-[3-(4-fluoro-benzyl)-1,2,4-thiadiazol-5-yl]piperazine | 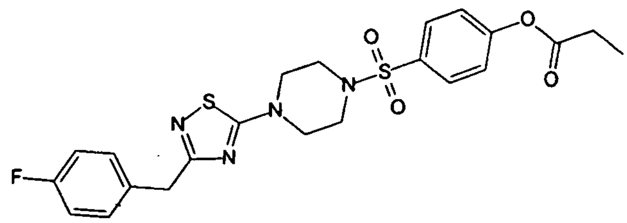 |
| 201 | 1-[4-methoxyphenylsulfonyl]-4-[3-(3-methyl-benzyl-1,2,4-thiadiazol-5-yl]piperazine | 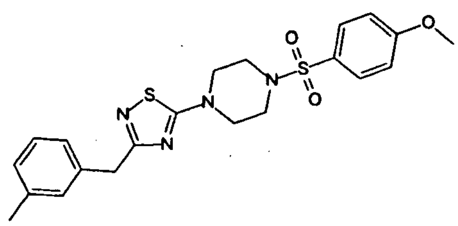 |
| 202 | 1-[4-methoxyphenylsulfonyl]-4-[3-(2-methyl-benzyl-1,2,4-thiadiazol-5-yl]piperazine | 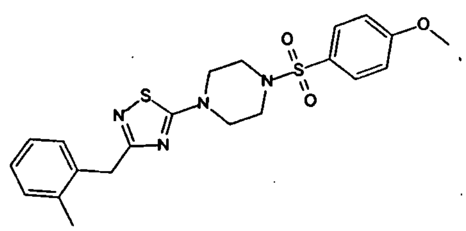 |

(continued)

| No. | Compound name | Compound structure |
|---|---|---|
| 203 | 1-[4-methoxyphenylsulfonyl]-4-[3-(2-methoxy-benzyl-1,2,4-thiadiazol-5-yl]piperazine | 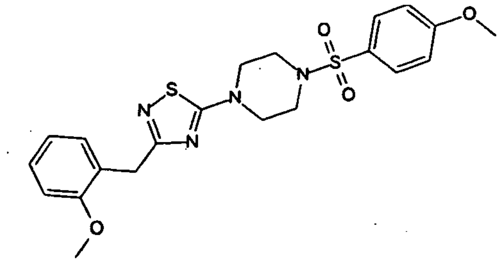 |
| 204 | 1-[4-methoxyphenylsulfonyl]-4-[3-(3-methyl-4-fluoro-benzyl-1,2,4-thiadiazol-5-yl]piperazine | 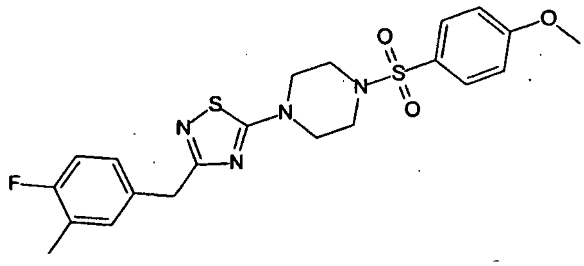 |
| 205 | 1-[4-methoxyphenylsulfonyl]-4-[3-(4-methoxy-benzyl-1,2,4-thiadiazol-5-yl]piperazine | 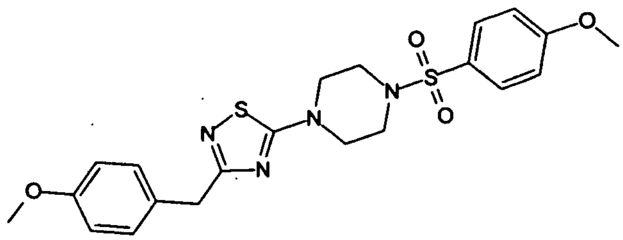 |
| | | |
| 207 | 1-[4-bromophenylsulfonyl]-4-[3-(3-methoxy-benzyl-1,2,4-thiadiazol-5-yl]piperazine | 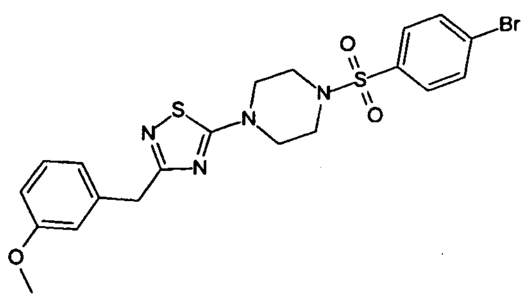 |
| 208 | 1-[4-methoxyphenylsulfonyl]-4-[3-(3-methoxybenzyl)-1,2,4-thiadiazol-5-yl]-2-methyl-piperazine | 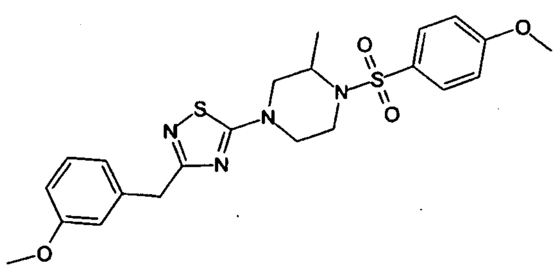 |

(continued)

| No. | Compound name | Compound structure |
|---|---|---|
| 217 | 1-(4-hydroxybenzenesulfonyl)-4-[3-(4-fluoro-benzyl)-1,2,4-thiadiazol-5-yl]piperazine | 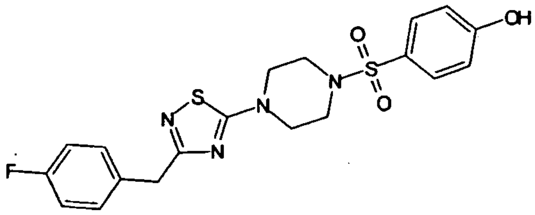 |

Table 2 - biological activity of the exemplary compounds

| No. | Name | %I | EC50 (μg/mL) |
|---|---|---|---|
| 60 | 1-[4-methoxyphenylsulfonyl]-4-[3-(4-methylbenzyl)-1,2,4-thiadiazol-5-yl]-2-methylpiperazine | 93 | 0,0013 |
| 49 | 1-[4-acetamidophenylsulfonyl]-4-[3-(4-methylbenzyl)-1,2,4-thiadiazol-5-yl] piperazine | 77 | 0,000106 |
| 31 | 1-[4-methoxyphenylsulfonyl]-4-[3-(4-chlorobenzyl)-1,2,4-thiadiazol-5-yl]piperazine | 59 | 0,000169 |
| 68 | 1-[4-methoxyphenylsulfonyl]-4-[3-(4-methylbenzyl)-1,2,4-thiadiazol-5-yl]piperazine | 53 | 0,000255 |
| 51 | 1-[4-methoxyphenylsulfonyl]-4-[3-(4-chlorobenzyl)-1,2,4-thiadiazol-5-yl]-2-methylpiperazine | 94 | 0,001346 |
| 33 | 1-[4-fluorophenylsulfonyl]-4-[3-(4-chlorobenzyl)-1,2,4-thiadiazol-5-yl]piperazine | 53 | 0,002818 |
| 48 | 1-[4-acetamidophenylsulfonyl]-4-[3-(4-methylbenzyl)-1,2,4-thiadiazol-5-yl]-2-methylpiperazine | 91 | 0,008439 |
| 57 | 1-[4-fluorophenylsulfonyl]-4-[3-(4-methylbenzyl)-1,2,4-thiadiazol-5-yl]piperazine | 88 | 0,010360 |
| 115 | 1-(2-fluorobenzoyl)-4-[3-(3-methoxybenzyl)-115 1,2,4-thiadiazol-5-yl]-2-methylpiperazine | 75 | 0,036080 |
| 65 | 1-[2-naphtylsulfonyl]-4-[3-(4-methylbenzyl)-1,2,4-thiadiazol-5-yl]piperazine | 78 | 0,039270 |
| 35 | 1-[1-naphtylsulfonyl]-4-[3-(4-methylbenzyl)-1,2,4-thiadiazol-5-yl]piperazine | 47 | 0,047235 |
| 116 | 1-(4-ethylbenzoyl)-4-[3-(4-fluorobenzyl)-1,2,4-thiadiazol-5-yl]-2-methylpiperazine | 71 | 0,052160 |
| 80 | 1-(phenylprop-2-ensulfonyl)-4-[3-(4-methylbenzyl)-1,2,4-thiadiazol-5-yl]piperazine | 70 | 0,056880 |
| 58 | 1-[4-fluorophenylsulfonyl]-4-[3-(4-methylbenzyl)-1,2,4-thiadiazol-5-yl]-2-methylpiperazine | 58 | 0,061030 |
| 34 | 1-[4-chlorophenylsulfonyl]-4-[3-(4-methylbenzyl)-1,2,4-thiadiazol-5-yl]-2-methylpiperazine | 66 | 0,065695 |
| 37 | 1-[2,4,6-trimethylphenylsulfonyl]-4-[3-(4-methylbenzyl)-1,2,4-thiadiazol-5-yl] piperazine | 48 | 0,073110 |
| 55 | 1-[4-acetamidophenylsulfonyl]-4-[3-(4-fluorobenzyl)-1,2,4-thiadiazol-5-yl]-2-methylpiperazine | 114 | 0,078150 |
| 63 | 1-[4-methylphenylsulfonyl]-4-[3-(4-chlorobenzyl)-1,2,4-thiadiazol-5-yl]piperazine | 83 | 0,080740 |
| 117 | 1-(4-butylbenzoyl)-4-[3-(4-methylbenzyl)-1,2,4-thiadiazol-5-yl]-2-methylpiperazine | 82 | 0,084110 |
| 118 | 1-(4-methoxybenzoyl)-4-[3-(4-fluorobenzyl)-1,2,4-thiadiazol-5-yl]-2-methylpiperazine | 64 | 0,091145 |
| 119 | 1-(1,3-benzodioxol-5-ylcarbonyl)-4-[3-(3-methoxybenzyl)-1,2,4-thiadiazol-5-yl]-2-methylpiperazine | 72 | 0,095050 |

(continued)

| No. | Name | %I | EC50 (μg/mL) |
|---|---|---|---|
| 120 | 1-(4-butylbenzoyl)-4-[3-(4-fluorobenzyl)-1,2,4-thiadiazol-5-yl]-2-methylpiperazine | 120 | 0,098730 |
| 40 | 1-[4-bromophenylsulfonyl]-4-[3-(4-methylbenzyl)-1,2,4-thiadiazol-5-yl]-3-methylpiperazine | 77 | 0,099855 |
| 36 | 1-[2,5-dichlorophenylsulfonyl]-4-[3-(4-methylbenzyl)-1,2,4-thiadiazol-5-yl]-2-methylpiperazine | 52 | 0,10 |
| 52 | 1-[2-naphtylsulfonyl]-4-[3-(4-chlorobenzyl)-1,2,4-thiadiazol-5-yl]piperazine | 104 | 0,10 |
| 121 | 1-(4-hexylbenzoyl)-4-[3-(4-fluorobenzyl)-1,2,4-thiadiazol-5-yl]piperazine | 64 | 0,12 |
| 78 | 1-(phenylprop-2-ensulfonyl)-4-[3-(4-fluorobenzyl)-1,2,4-thiadiazol-5-yl]piperazine | 75 | 0,13 |
| 38 | 1-[2-naphtylsulfonyl]-4-[3-(4-methylbenzyl)-1,2,4-thiadiazol-5-yl]-2-methylpiperazine | 78 | 0,13 |
| 122 | 1-(3-chlorobenzoyl)-4-[3-(4-fluorobenzyl)-1,2,4-thiadiazol-5-yl]-2-methylpiperazine | 92 | 0,14 |
| 123 | 1-(4-fluorobenzoyl)-4-[3-(3-methoxybenzyl)-1,2,4-thiadiazol-5-yl]-2-methylpiperazine | 95 | 0,14 |
| 124 | 1-(4-methylbenzoyl)-4-[3-(3-methoxybenzyl)-1,2,4-thiadiazol-5-yl]-2-methylpiperazine | 57 | 0,14 |
| 1 | 4-phenylacetyl-1-[3-(4-fluorobenzyl)-1,2,4-thiadiazol-5-yl]piperazine | 75 | 0,15 |
| 39 | 1-[2,5-dichlorophenylsulfonyl]-4-[3-(4-chlorobenzyl)-1,2,4-thiadiazol-5-yl] piperazine | 25 | 0,16 |
| 125 | 1-(3-fluorobenzoyl)-4-[3-(4-chlorobenzyl)-1,2,4-thiadiazol-5-yl]-2-methylpiperazine | 68 | 0,16 |
| 189 | 1-(4-chlorobenzoyl)-4-[3-(4-fluorobenzyl)-1,2,4-thiadiazol-5-yl]-1,4-diazepane | 85 | 0,17 |
| 79 | 1-(phenylprop-2-ensulfonyl)-4-[3-(4-methylbenzyl)-1,2,4-thiadiazol-5-yl]-2-methylpiperazine | 53 | 0,17 |
| 19 | N-(2,6-dimethylphenyl)-4-[3-(4-fluorobenzyl)-1,2,4-thiadiazol-5-yl]-2-methylpiperazine-1-carboxamide | 95 | 0,20 |
| 126 | 126 1-benzoyl-4-[3-(4-fluorobenzyl)-1,2,4-thiadiazol-5-yl]-2-methylpiperazine | 63 | 0,21 |
| 127 | 1-(4-fluorobenzoyl)-4-[3-(4-fluorobenzyl)-1,2,4-thiadiazol-5-yl]-2-methylpiperazine | 108 | 0,23 |
| 128 | 1-(4-tert-butylbenzoyl)-4-[3-(4-fluorobenzyl)-1,2,4-thiadiazol-5-yl]-2-methylpiperazine | 95 | 0,24 |
| 129 | 1-(1,1'-biphenyl-4-ylcarbonyl)-4-[3-(4-fluorobenzyl)-1,2,4-thiadiazol-5-yl]-2-methylpiperazine | 61 | 0,24 |
| 2 | 4-(4-fluorophenylacetyl),1-[3-(4-fluorobenzyl)-1,2,4-thiadiazol-5-yl]piperazine | 54 | 0,25 |
| 130 | 1-(4-methoxybenzoyl)-4-[3-(4-methylbenzyl)-1,2,4-thiadiazol-5-yl]-2-methylpiperazine | 118 | 0,25 |
| 131 | 1-(4-ethylbenzoyl)-4-[3-(4-methylbenzyl)-1,2,4-thiadiazol-5-yl]piperazine | 91 | 0,25 |
| 107 | 2-(4-fluorophenyl)-N-{2-[4-[3-(4-fluorobenzyl)- 1,2,4-thiadiazol-5-yl]piperazin-1-yl] ethyl}acetamide | 80 | 0,26 |
| 132 | 1-(2-naphthoyl)-4-[3-(4-fluorobenzyl)-1,2,4-thiadiazol-5-yl]-2-methylpiperazine | 74 | 0,26 |
| 133 | 1-(2-methoxybenzoyl)-4-[3-(4-fluorobenzyl)-1,2,4-thiadiazol-5-yl]-2-methylpiperazine | 118 | 0,29 |
| 134 | 1-(4-pentylbenzoyl)-4-[3-(4-methylbenzyl)-1,2,4-thiadiazol-5-yl]-2-methylpiperazine | 75 | 0,30 |
| 135 | 1-(4-bromobenzoyl)-4-[3-(4-fluorobenzyl)-1,2,4-thiadiazol-5-yl]-2-methylpiperazine | 73 | 0,31 |

(continued)

| No. | Name | %I | EC50 (μg/mL) |
|---|---|---|---|
| 136 | 1-(2,4-dimethoxybenzoyl)-4-[3-(4-fluorobenzyl)-1,2,4-thiadiazol-5-yl]-2-methylpiperazine | 83 | 0,34 |
| 137 | 1-(3,5-dichlorobenzoyl)-4-[3-(4-fluorobenzyl)-1,2,4-thiadiazol-5-yl]piperazine | 68 | 0,35 |
| 94 | 4-pentyl-N-{2-[4-[3-(4-fluorobenzyl)-1,2,4-thiadiazol-5-yl]piperazin-1-yl]ethyl} benzamide | 90 | 0,35 |
| 81 | 1-(butylsulfonyl)-4-[3-(4-chlorobenzyl)-1,2,4-thiadiazol-5-yl]piperazine | 80 | 0,35 |
| 138 | 1-(3-chlorobenzoyl)-4-[3-(4-fluorobenzyl)-1,2,4-thiadiazol-5-yl]piperazine | 81 | 0,36 |
| 185 | 1-(2-methylphenyl)-4-[3-(4-fluorobenzyl)-1,2,4-thiadiazol-5-yl]piperazine | 114 | 0,38 |
| 95 | 4-butyl-N-{2-[4-[3-(4-fluorobenzyl)-1,2,4-thiadiazol-5-yl]piperazin-1-yl]ethyl} benzamide | 78 | 0,38 |
| 139 | 1-(4-methylbenzoyl)-4-[3-(4-fluorobenzyl)-1,2,4-thiadiazol-5-yl]-2-methylpiperazine | 87 | 0,40 |
| 140 | 1-(2-methylbenzoyl)-4-[3-(4-fluorobenzyl)-1,2,4-thiadiazol-5-yl]-2-methylpiperazine | 56 | 0,45 |
| 141 | 1-(4-methylbenzoyl)-4-[3-(4-fluorobenzyl)-1,2,4-thiadiazol-5-yl]piperazine | 75 | 0,49 |
| 142 | 1-(3-bromobenzoyl)-4-[3-(4-fluorobenzyl)-1,2,4- thiadiazol-5-yl]-2-methylpiperazine | 72 | 0,50 |
| 9 | N-(3-fluorophenyl)-4-[3-(4-fluorobenzyl)-1,2,4-thiadiazol-5-yl]piperazine-1-carboxamide | 53 | 0,52 |
| 41 | 1-[1-naphtylsulfonyl]-4-[3-(4-methylbenzyl)-1,2,4-thiadiazol-5-yl]-3-methylpiperazine | 54 | 0,53 |
| 112 | 3-phenyl-N-{2-[4-[3-(4-fluorobenzyl)-1,2,4-thiadiazol-5-yl]piperazin-1-yl]ethyl} propanamide | 91 | 0,55 |
| 143 | 1-(4-ethylbenzoyl)-4-[3-(3-methoxybenzyl)-1,2,4-thiadiazol-5-yl]-2-methylpiperazine | 89 | 0,56 |
| 144 | 1-(3-methylbenzoyl)-4-[3-(4-methylbenzyl)-1,2,4-thiadiazol-5-yl]-2-methylpiperazine | 64 | 0,56 |
| 89 | 1-(3-phenylpropanoyl)-4-[3-(4-fluorobenzyl)-1,2,4-thiadiazol-5-yl]-2-methylpiperazine | 86 | 0,60 |
| 10 | N-(2-methylphenyl)-4-[3-(4-fluorobenzyl)-1,2,4-thiadiazol-5-yl]-2-methylpiperazine-1-carboxamide | 72 | 0,60 |
| 145 | 1-(3-trifluoromethylbenzoyl)-4-[3-(4-fluorobenzyl)-1,2,4-thiadiazol-5-yl]-2-methylpiperazine | 60 | 0,62 |
| 11 | N-(4-ethylphenyl)-4-[3-(4-fluorobenzyl)-1,2,4-thiadiazol-5-yl]-2-methylpiperazine-1-carboxamide | 73 | 0,69 |
| 91 | 1-[3-phenylprop-2-enyl]-4-[3-(4-methylbenzyl)-1,2,4-thiadiazol-5-yl]piperazine | 90 | 0,71 |
| 146 | 1-(4-tert-butylbenzoyl)-4-[3-(3-methoxybenzyl)-1,2,4-thiadiazol-5-yl]-2-methylpiperazine | 94 | 0,72 |
| 92 | 1-[3-phenylprop-2-enyl]-4-[3-(4-chlorobenzyl)-1,2,4-thiadiazol-5-yl]piperazine | 69 | 0,75 |
| 20 | N-(2,4-dimethylphenyl)-4-[3-(4-fluorobenzyl)-1,2,4-thiadiazol-5-yl]-2-methylpiperazine-1-carboxamide | 106 | 0,76 |
| 21 | N-(2,6-dichlorophenyl)-4-[3-(4-fluorobenzyl)-1,2,4-thiadiazol-5-yl]-2-methylpiperazine-1-carboxamide | 70 | 0,79 |

(continued)

| No. | Name | %I | EC50 (μg/mL) |
|---|---|---|---|
| 96 | 4-hexyl-N-{2-[4-[3-(4-fluorobenzyl)-1,2,4-thiadiazol-5-yl]piperazin-1-yl]ethyl} benzamide | 72 | 0,80 |
| 4 | 1-(4-methoxyphenylacetyl)-4-[3-(4-fluorobenzyl)-1,2,4-thiadiazol-5-yl]-2-methylpiperazine | 56 | 0,82 |
| 147 | 1-(4-ethylbenzoyl)-4-[3-(4-methylbenzyl)-1,2,4-thiadiazol-5-yl]-2-methylpiperazine | 88 | 0,84 |
| 148 | 1-(2-bromobenzoyl)-4-[3-(4-fluorobenzyl)-1,2,4-thiadiazol-5-yl]-2-methylpiperazine | 56 | 0,86 |
| 42 | 1-[4-tert-butylphenylsulfonyl]-4-[3-(4-methylbenzyl)-1,2,4-thiadiazol-5-yl]-2-methylpiperazine | 33 | 0,87 |
| 22 | N-(3-cyanophenyl)-4-[3-(4-fluorobenzyl)-1,2,4-thiadiazol-5-yl]-2-methylpiperazine-1-carboxamide | 59 | 0,88 |
| 43 | 1-[1-naphtylsulfonyl]-4-[3-(4-chlorobenzyl)-1,2,4-thiadiazol-5-yl]-2-methylpiperazine | 35 | 0,89 |
| 12 | N-(2-ethylphenyl)-4-[3-(4-fluorobenzyl)-1,2,4-thiadiazol-5-yl]-2-methylpiperazine-1-carboxamide | 92 | 0,89 |
| 23 | N-(2,4-difluorophenyl)-4-[3-(4-fluorobenzyl)-1,2,4-thiadiazol-5-yl]-2-methylpiperazine-1-carboxamide | 66 | 0,94 |
| 149 | 1-(2-fluorobenzoyl)-4-[3-(4-methylbenzyl)-1,2,4-thiadiazol-5-yl]-2-methylpiperazine | 105 | 0,98 |
| 86 | 1-[(2E)-3-phenylprop-2-enoyl]-4-[3-(4-chlorobenzyl)-1,2,4-thiadiazol-5-yl]-2-methylpiperazine | 81 | 1,13 |
| 150 | 1-(3-fluorobenzoyl)-4-[3-benzyl-1,2,4-thiadiazol-5-yl]-2-methylpiperazine | 99 | 1,14 |
| 151 | 1-(4-fluorobenzoyl)-4-[3-(4-methylbenzyl)-1,2,4-thiadiazol-5-yl]-2-methylpiperazine | 73 | 1,14 |
| 152 | 1-(2-methylbenzoyl)-4-[3-(4-fluorobenzyl)-1,2,4-thiadiazol-5-yl]piperazine | 66 | 1,15 |
| 45 | 1-[2,4,6-trimethylphenylsulfonyl]-4-[3-(4-methylbenzyl)-1,2,4-thiadiazol-5-yl]-2-methylpiperazine | 56 | 1,20 |
| 24 | N-(2,6-dimethylphenyl)-4-[3-(4-fluorobenzyl)-1,2,4-thiadiazol-5-yl]piperazine-1-carboxamide | 124 | 1,20 |
| 13 | N-phenyl-4-[3-(4-fluorobenzyl)-1,2,4-thiadiazol-5-yl]piperazine-1-carboxamide | 71 | 1,23 |
| 97 | 4-chloro-N-{2-[4-[3-(4-fluorobenzyl)-1,2,4-thiadiazol-5-yl]piperazin-1-yl]ethyl} benzamide | 68 | 1,29 |
| 46 | 1-[3-trifluorophenylsulfonyl]-4-[3-(4-fluorobenzyl)-1,2,4-thiadiazol-5-yl]-2-methylpiperazine | 64 | 1,34 |
| 90 | 1-(3-phenylpropanoyl)-4-[3-(4-fluorobenzyl)-1,2,4-thiadiazol-5-yl]piperazine | 73 | 1,41 |
| 98 | 3,5-dichloro-N-{2-[4-[3-(4-fluorobenzyl)-1,2,4-thiadiazol-5-yl]piperazin-1-yl]ethyl} benzamide | 102 | 1,42 |
| 153 | 1-(4-fluorobenzoyl)-4-[3-(4-chlorobenzyl)-1,2,4-thiadiazol-5-yl]piperazine | 53 | 1,44 |
| 8 | 1-(2-phenylbutanoyl)-4-[3-(4-fluorobenzyl)-1,2,4-thiadiazol-5-yl]-2-methylpiperazine | 71 | 1,49 |
| 113 | 1-benzyl-4-[3-(4-fluorobenzyl)-1,2,4-thiadiazol-5-yl]piperazine | 65 | 1,54 |
| 99 | 2-methyl-N-{2-[4-[3-(4-fluorobenzyl)-1,2,4-thiadiazol-5-yl]piperazin-1-yl]ethyl} benzamide | 97 | 1,58 |
| 154 | 1-(4-fluorobenzoyl)-4-[3-benzyl-1,2,4-thiadiazol-5-yl]-2-methylpiperazine | 75 | 1,58 |

(continued)

| No. | Name | %I | EC50 (μg/mL) |
|---|---|---|---|
| 82 | 1-(octylsulfonyl)-4-[3-(4-methylbenzyl)-1,2,4-thiadiazol-5-yl]piperazihe | 50 | 1,58 |
| 155 | 1-(4-chlorobenzoyl)-4-[3-(4-methylbenzyl)-1,2,4-thiadiazol-5-yl]piperazine | 76 | 1,64 |
| 156 | 1-(3-fluorobenzoyl)-4-[3-(4-methylbenzyl)-1,2,4-thiadiazol-5-yl]-2-methylpiperazine | 86 | 1,67 |
| 25 | *N*-1-naphtyl-4-[3-(4-fluorobenzyl)-1,2,4-thiadiazol-5-yl]-2-methylpiperazine-1-carboxamide | 97 | 1,69 |
| 157 | 1-(3-fluorobenzoyl)-4-[3-(4-chlorobenzyl)-1,2,4-thiadiazol-5-yl]piperazine | 70 | 1,70 |
| 14 | N-(4-ethoxyphenyl)-4-[3-(4-fluorobenzyl)-1,2,4-thiadiazol-5-yl]-2-methylpiperazine-1-carboxamide | 70 | 1,73 |
| 111 | 2-phenyl-N-{2-[4-[3-(4-fluorobenzyl)-1,2,4-thiadiazol-5-yl]piperazin-1-yl]ethyl} butanamide | 79 | 1,79 |
| 100 | 3-fluoro-N-{2-[4-[3-(4-chlorobenzyl)-1,2,4-thiadiazol-5-yl]piperazin-1-yl]ethyl} benzamide | 75 | 1,79 |
| 101 | 4-methyl-N-{2-[4-[3-(4-fluorobenzyl)-1,2,4-thiadiazol-5-yl]piperazin-1-yl]ethyl} benzamide | 68 | 1,94 |
| 102 | 4-fluoro-N-{2-[4-[3-(4-fluorobenzyl)-1,2,4-thiadiazol-5-yl]piperazin-1-yl]ethyl} benzamide | 80 | 1,99 |
| 93 | 1-[3-phenylprop-2-enyl]-4-[3-(4-fluorobenzyl)-1,2,4-thiadiazol-5-yl]piperazine | 91 | 1,99 |
| 186 | 1-(2-ethoxyphenyl)-4-[3-(4-fluorobenzyl)-1,2,4-thiadiazol-5-yl]piperazine | 94 | 2,05 |
| 108 | 2-phenyl-N-{2-[4-[3-(4-fluorobenzyl)-1,2,4-thiadiazol-5-yl]piperazin-1-yl]ethyl} acetamide | 63 | 2,06 |
| 158 | 1-benzoyl-4-[3-(4-methylbenzyl)-1,2,4-thiadiazol-5-yl]piperazine | 57 | 2,10 |
| 3 | 1-(4-fluorophenylacetyl)-4-[3-(4-methylbenzyl)-1,2,4-thiadiazol-5-yl]-2-methylpiperazine | 76 | 2,20 |
| 103 | 4-ethyl-N-{2-[4-[3-(4-fluorobenzyl)-1,2,4-thiadiazol-5-yl]piperazin-1-yl]ethyl} benzamide | 139 | 2,32 |
| 87 | 1-[(2E)-3-phenylprop-2-enoyl]-4-B103-2-methylpiperazine | 94 | 2,49 |
| 83 | 1-(butylsulfonyl)-4-[3-(4-methylbenzyl)-1,2,4-thiadiazol-5-yl]piperazine | 36 | 2,64 |
| 190 | 1-(4-methylbenzoyl)-4-[3-(4-fluorobenzyl)-1,2,4-thiadiazol-5-yl]-1,4-diazepane | 87 | 2,77 |
| 187 | 1-(2-fluorophenyl)-4-[3-(4-fluorobenzyl)-1,2,4-thiadiazol-5-yl]piperazine | 83 | 2,81 |
| 159 | 1-(2-fluorobenzoyl)-4-[3-(4-chlorobenzyl)-1,2,4-thiadiazol-5-yl]piperazine | 48 | 2,85. |
| 160 | 1-(4-fluorobenzoyl)-4-[3-(4-methylbenzyl)-1,2,4-thiadiazol-5-yl]piperazine | 88 | 2,87 |
| 161 | 1-(1,3-benzodioxol-5-ylcarbonyl)-4-[3-(4-methylbenzyl)-1,2,4-thiadiazol-5-yl]-2-methyl+B 176piperazine | 101 | 3,36 |
| 162 | 1-(3-flurorbenzoyl)-4-[3-(4-methylbenzyl)-1,2,4-thiadiazol-5-yl]piperazine | 101 | 3,40 |
| 84 | 1-(ethylsulfonyl)-4-[3-(4-chlorobenzyl)-1,2,4-thiadiazol-5-yl]piperazine | 53 | 3,48 |
| 109 | 2-(4-fluorophenyl)-N-{2-[4-[3-(4-methylbenzyl)-1,2,4-thiadiazol-5-yl]piperazin-1-yl] ethyl}acetamide | 93 | 3,52 |
| 163 | 1-(4-fluorobenzoyl)-4-[3-benzyl-1,2,4-thiadiazol-5-yl]piperazine | 67 | 3,61 |
| 85 | 1-(isopropylsulfonyl)-4-[3-(4-methylbenzyl)-1,2,4-thiadiazol-5-yl]piperazine | 69 | 3,65 |
| 104 | N-{2-[4-[3-(4-fluorobenzyl)-1,2,4-thiadiazol-5-yl]piperazin-1-yl]ethyl}benzamide | 65 | 3,82 |

(continued)

| No. | Name | %I | EC50 (μg/mL) |
|---|---|---|---|
| 164 | 1-(4-bromobenzoyl)-4-[3-(4-methylbenzyl)-1,2,4-thiadiazol-5-yl]piperazine | 60 | 3,86 |
| 15 | N-(2-methylphenyl)-4-[3-(4-fluorobenzyl)-1,2,4-thiadiazol-5-yl]piperazine-1-carboxamide | 65 | 5,01 |
| 16 | N-(2-fluorophenyl)-4-[3-(4-fluorobenzyl)-1,2,4-thiadiazol-5-yl]piperazine-1-carboxamide | 72 | 5,68 |
| 30 | 1-[4-tert-butylphenylsulfonyl]-4-[3-(4-fluorobenzyl)-1,2,4-thiadiazol-5-yl]piperazine | 51 | |
| 77 | 1-(benzylsulfonyl)-4-[3-(4-methylbenzyl)-1,2,4-thiadiazol-5-yl]piperazine | 81 | |
| 5 | 1-phenylacetyl-4-[3-(4-fluorobenzyl)-1,2,4-thiadiazol-5-yl]-2-methylpiperazine | 83 | |
| 6 | 1-(4-fluorophenylacetyl)-4-[3-(4-fluorobenzyl)-1,2,4-thiadiazol-5-yl]-2-methylpiperazine | 75 | |
| 7 | 1-[chloro(phenyl)acetyl]-4-[3-(4-fluorobenzyl)-1,2,4-thiadiazol-5-yl]-2-methylpiperazine | 69 | |
| 17 | N-(2-trifluoromethylphenyl)-4-[3-(4-fluorobenzyl)-1,2,4-thiadiazol-5-yl]piperazine-1-carboxamide | 46 | |
| 18 | N-(2-trifluoromethylphenyl)-4-[3-(4-fluorobenzyl)-1,2,4-thiadiazol-5-yl]-2-methylpiperazine-1-carboxamide | 41 | |
| 26 | N-(3,4-d ifluorophenyl)-4-[3-(4-fluorobenzyl)-1,2,4-thiadiazol-5-yl]-2-methylpiperazine-1-carboxamide | 70 | |
| 27 | N-(2;4-dimethoxyphenyl)-4-[3-(4-fluorobenzyl)-1,2,4-thiadiazol-5-yl]piperazine-1-carboxamide | 67 | |
| 28 | N-(3,4-difluorophenyl)-4-[3-(4-fluorobenzyl)-1,2,4-thiadiazol-5-yl]piperazine-1-carboxamide | 64 | |
| 29 | N-(3,5-dimethoxyphenyl)-4-[3-(4-fluorobenzyl)-1,2,4-thiadiazol-5-yl]-2-methylpiperazine-1-carboxamide | 51 | |
| 47 | 1-Phenylsulfonyl-4-[3-(4-fluorobenzyl)-1,2,4-thiadiazol-5-yl]-2-methylpiperazine | 129 | |
| 50 | 1-[4-methylphenylsulfonyl]-4-[3-(4-methylbenzyl)-1,2,4-thiadiazol-5-yl]-2-methylpiperazine | 105 | |
| 54 | 1-[4-tert-butylphenylsulfonyl]-4-[3-(4-fluorobenzyl)-1,2,4-thiadiazol-5-yl]-2-methylpiperazine | 60 | |
| 56 | 1-[3-trifluoromethylphenylsulfonyl]-4-[3-(4-methylbenzyl)-1,2,4-thiadiazol-5-yl]-2-methylpiperazine | 87 | |
| 59 | 1-[4-fluorophenylsulfonyl]-4-[3-(4-fluorobenzyl)-59 1,2,4-thiadiazol-5-yl]-2-methylpiperazine | 42 | |
| 61 | 1-[4-ter-butylphenylsulfonyl]-4-[3-(4-fluorobenzyl)-1,2,4-thiadiazol-5-yl]piperazine | 47 | |
| 62 | 1-[3-trifluoromethylphenylsulfonyl]-4-[3-(4-methylbenzyl)-1,2,4-thiadiazol-5-yl] piperazine | 45 | |
| 64 | 1-[4-bromophenylsulfonyl]-4-[3-(4-methylbenzyl)- . 1,2,4-thiadiazol-5-yl]piperazine | 58 | |
| 66 | 1-[4-chlorophenylsulfonyl]-4-[3-(4-methylbenzyl)-1,2,4-thiadiazol-5-yl]piperazine | 81 | |
| 67 | 1-[4-tert-butylphenylsulfonyl]-4-[3-(4-methylbenzyl)-1,2,4-thiadiazol-5-yl] piperazine | 41 | |
| 69 | 1-[4-fert-butylphenylsulfonyl]-4-[3-(4-chlorobenzyl)-1,2,4-thiadiazol-5-yl]piperazine | 31 | |
| 70 | 1-[quinoline-8-sulfonyl]-4-[3-(4-methylbenzyl)-1,2,4-thiadiazol-5-yl]piperazine | 44 | |

(continued)

| No. | Name | %I | EC50 (μg/mL) |
|---|---|---|---|
| 71 | 1-[4-nitrophenylsulfonyl]-4-[3-(4-methylbenzyl)-1,2,4-thiadiazol-5-yl]-2-methylpiperazine | 33 | |
| 72 | 1-[3-nitro-4-chlorophenylsulfonyl]-4-[3-(4-methylbenzyl)-1,2,4-thiadiazol-5-yl]-2-methylpiperazine | | |
| 73 | 1-[4-nitrophenylsulfonyl]-4-[3-(4-fluorobenzyl)-1,2,4-thiadiazol-5-yl]-2-methylpiperazine | 33 | |
| 75 | 1-[3-nitro-4-chlorophenylsulfonyl]-4-[3-(4-methylbenzyl)-1,2,4-thiadiazol-5-yl] piperazine | | |
| 76 | 1-(benzylsulfonyl)-4-[3-(4-chlorobenzyl)-1,2,4-thiadiazol-5-yl]piperazine | 40 | |
| 88 | 1-[(2E)-3-phenylprop-2-enoyl]-4-[3-(4-chlorobenzyl)-1,2,4-thiadiazol-5-yl] piperazine | 72 | |
| 105 | 3-fluoro-N-(2-[4-[3-(4-fluorobenzyl)-1,2,4-thiadiazol-5-yl]piperazin-1-yl]ethyl) benzamide | 115 | |
| 106 | 3-fluoro-N-{2-[4-[3-benzyl-1,2,4-thiadiazol-5-yl]piperazin-1-yl]ethyl}benzamide | 49 | |
| 110 | N-benzyl-N'-{2-[4-[3-(4-fluorobenzyl)-1,2,4-thiadiazol-5-yl]piperazin-1-yl]ethyl}urea | 77 | |
| 114 | 1-(1,3-benzodioxol-5-ylmethyl)-4-[3-(4-fluorobenzyl)-1,2,4-thiadiazol-5-yl] piperazine | 86 | |
| 165 | 1-(4-ethylbenzoyl)-4-[3-(4-chlorobenzyl)-1,2,4-thiadiazol-5-yl]piperazine | 133 | |
| 166 | 1-(2-chlorobenzoyl)-4-[3-(4-fluorobenzyl)-1,2,4-thiadiazol-5-yl]-2-methylpiperazine | 123 | |
| 167 | 1-(2-fluorobenzoyl)-4-[3-(4-chlorobenzyl)-1,2,4-thiadiazol-5-yl]-2-methylpiperazine | 117 | |
| 168 | 1-(4-trifluoromethylbenzoyl)-4-[3-(3-methoxybenzyl)-1,2,4-thiadiazol-5-yl]-2-methylpiperazine | 91 | |
| 169 | 1-benzoyl-4-[3-(4-methylbenzyl)-1,2,4-thiadiazol-5-yl]-2-methylpiperazine | 91 | |
| 170 | 1-(4-bromobenzoyl)-4-[3-(3-methoxybenzyl)-1,2,4-thiadiazol-5-yl]-2-methylpiperazine | 89 | |
| 171 | 1-(4-trifluoromethylbenzoyl)-4-[3-(4-methylbenzyl)-1,2,4-thiadiazol-5-yl]piperazine | 86 | |
| 172 | 1-(3-nitro-4-methylbenzoyl)-4-[3-(4-fluorobenzyl)-1,2,4-thiadiazol-5-yl]-2-methylpiperazine | 86 | |
| 173 | 1-benzoyl-4-[3-(3-methoxybenzyl)-1,2,4-thiadiazol-5-yl]-2-methylpiperazine | 85 | |
| 174 | 1-(4-chlorobenzoyl)-4-[3-(3-methoxybenzyl)-1,2,4-thiadiazol-5-yl]-2-methylpiperazine | 83 | |
| 175 | 1-(2-fluorobenzoyl)-4-[3-(4-methylbenzyl)-1,2,4-thiadiazol-5-yl]piperazine | 78 | |
| 176 | 1-(4-hexylbenzoyl)-4-[3-(4-methylbenzyl)-1,2,4-thiadiazol-5-yl]piperazine | 77 | |
| 177 | 1-(2-chloro-4-nitrobenzoyl)-4-[3-(4-fluorobenzyl)-1,2,4-thiadiazol-5-yl]-2-methylpiperazine | 77 | |
| 178 | 1-(1,3-benzodioxol-5-ylcarbonyl)-4-[3-(4-fluorobenzyl)-1,2,4-thiadiazol-5-yl]-2-methylpiperazine | 68 | |
| 179 | 1-(3-fluorobenzoyl)-4-[3-(4-fluorobenzyl)-1,2,4-thiadiazol-5-yl]-2-methylpiperazine | 68 | |
| 180 | 1-(4-tert-butylbenzoyl)-4-[3-(4-fluorobenzyl)-1,2,4-thiadiazol-5-yl]piperazine | 62 | |
| 181 | 1-benzoyl-4-[3-(4-fluorobenzyl)-1,2,4-thiadiazol-5-yl]piperazine | 60 | |
| 182 | 1-(4-butylbenzoyl)-4-[3-(4-methylbenzyl)-1,2,4-thiadiazol-5-yl]piperazine | 57 | |
| 183 | 1-(4-tert-butylbenzoyl)-4-[3-(4-methylbenzyl)-1,2,4-thiadiazol-5-yl]piperazine | 52 | |

(continued)

| No. | Name | %I | EC50 (μg/mL) |
|---|---|---|---|
| 184 | 1-(4-nitrobenzoyl)-4-[3-(4-fluorobenzyl)-1,2,4-thiadiazol-5-yl]-2-methylpiperazine | 52 | |
| 188 | 1-(3-trifluoromethylphenyl)-4-[3-(4-fluorobenzyl)-1,2,4-thiadiazol-5-yl]piperazine | 96 | |
| 191 | 1-(1,3-benzodioxol-5-ylcarbonyl)-4-[3-(4-fluorobenzyl)-1,2,4-thiadiazol-5-yl]-1,4-diazepane | 63 | |
| 44 | 1-[3-methoxyphenylsulfonyl]-4-[3-(4-fluorobenzyl)-1,2,4-thiadiazol-5-yl]piperazine | 99 | 0.0571 |
| 53 | 1-[4-methoxyphenylsulfonyl]-4-[3-(3-methoxybenzyl)-1,2,4-thiadiazol-5-yl] piperazine | 83 | 0.00066 |
| 32 | 1-[4-methoxyphenylsulfonyl]-4-[3-(4-fluorobenzyl)-1,2,4-thiadiazol-5-yl]piperazine | 78 | 0.0024 |
| 193 | 1-[4-methoxyphenylsulfonyl]-4-[3-(3-fluoro-benzyl)-1,2,4-thiadiazol-5-yl]piperazine | 68 | 0.0048 |
| 194 | 1-[4-methoxyphenylsulfonyl]-4-[3-(2-fluoro-benzyl)-1,2,4-thiadiazol-5-yl]piperazine | 102 | 0.0012 |
| 195 | 1-[2-methoxyphenylsulfonyl]-4-[3-(4-fluoro-benzyl)-1,2,4-thiadiazol-5-yl]piperazine | 99 | 0.025 |
| 196 | 1-[4-ethoxyphenylsulfonyl]-4-[3-(4-fluoro-benzyl)-1,2,4-thiadiazol-5-yl]piperazine | 42 | 0.0081 |
| 197 | 1-[4-ethylphenylsulfonyl]-4-[3-(4-fluoro-benzyl)-1,2,4-thiadiazol-5-yl]piperazine | 84 | 0.239 |
| 198 | 1-phenylsulfonyl-4-[3-(4-fluoro-benzyl)-1,2,4-thiadiazol-5-yl]piperazine | 25 | 0.146 |
| 199 | 1-[4-methoxyphenylsulfonyl]-4-[3-benzyl-1,2,4-thiadiazol-5-yl]piperazine | 38 | 0.0016 |
| 200 | 1-(4-propionyloxy)benzene-sulfonyl-4-[3-(4-fluoro-benzyl)-1,2,4-thiadiazol-5-yl] piperazine | 80 | 0.051 |
| 201 | 1-[4-methoxyphenylsulfonyl]-4-[3-(3-methyl-benzyl-1,2,4-thiadiazol-5-yl]piperazine | 97 | 0.0021 |
| 202 | 1-[4-methoxyphenylsulfonyl]-4-[3-(2-methyl-benzyl-1,2,4-thiadiazol-5-yl]piperazine | 63 | 0.0075 |
| 203 | 1-[4-methoxyphenylsulfonyl]-4-[3-(2-methoxy-benzyl-1,2,4-thiadiazol-5-yl] piperazine | 61 | 0.0062 |
| 204 | 1-[4-methoxyphenylsulfonyl]-4-[3-(3-methyl-4-fluoro-benzyl-1,2,4-thiadiazol-5-yl] piperazine | 90 | 0.0010 |
| 205 | 1-[4-methoxyphenylsulfonyl]-4-[3-(4-methoxy-benzyl-1,2,4-thiadiazol-5-yl] piperazine | 53 | 0.0011 |
| 207 | 1-[4-bromophenylsulfonyl]-4-[3-(3-methoxy-benzyl-1,2,4-thiadiazol-5-yl]piperazine | 83 | 0.028 |
| 208 | 1-[4-methoxyphenylsulfonyl]-4-[3-(3-methoxybenzyl)-1,2,4-thia-diazol-5-yl]-2-methyl-piperazine | 92 | 0.0034 |
| 217 | 1-(4-hydroxybenzenesulfonyl)-4-[3-(4-fluoro-benzyl)-1,2,4-thiadiazol-5-yl] piperazine | 77 | 0..033 |

## Claims

**1.** A pharmaceutical composition comprising a therapeutically effective amount of a 1,2,4-thiadiazole derivative according to the structural formula (A)

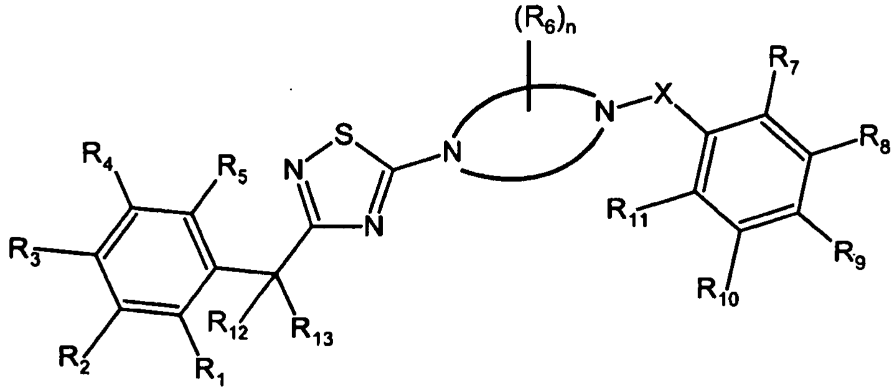

(A)

wherein the divalent group schematically represented by the structural formula (A')

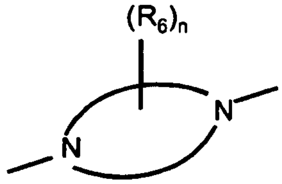

(A')

includes an optionally mono-substituted or poly-substituted, saturated or partly unsaturated heterocyclic ring with at least two nitrogen atoms in the said heterocyclic ring and with a total of 5 to 7 atoms in the said heterocyclic ring, and further wherein:

- $R_6$ is a substituent independently selected from the group consisting of oxo and $C_{1-4}$ alkyl;
- n is selected from the group consisting of 0, 1, 2 and 3;
- $R_1$, $R_2$, $R_3$, $R_4$ and $R_5$ are each independently selected from the group consisting of hydrogen, $C_{1-4}$ alkyl, $C_{1-4}$ alkoxy, aryl, aryloxy, aryl-$C_{1-4}$ alkyloxy, heteroaryloxy, benzenesulfonate, amino, hydroxy, nitro, trifluoromethyl, trifluoromethoxy and halogen, or any two adjacent substituents selected from the group consisting of $R_1$, $R_2$, $R_3$, $R_4$ and $R_5$ form, together with the phenyl ring carbon atoms to which they are attached, a saturated or unsaturated ring fused to said phenyl ring and having from 5 to 7 ring members, said saturated or unsaturated ring optionally comprising one or two oxygen atoms and being optionally substituted with one or more halogen atoms;
- $R_7$, $R_8$, $R_9$, $R_{10}$ and $R_{11}$ are each independently selected from the group consisting of hydrogen, $C_{1-10}$ alkyl, $C_{1-6}$ alkoxy, aryl, hydroxy, acetyl, nitro, trifluoromethyl and halogen; or any two adjacent substituents selected from the group consisting of $R_7$, $R_8$, $R_9$, $R_{10}$ and $R_{11}$ form, together with the phenyl ring carbon atoms to which they are attached, a saturated or unsaturated ring fused to said phenyl ring and having from 5 to 7 ring members, said saturated or unsaturated ring optionally comprising one or two heteroatoms independently selected from the group consisting of oxygen, sulfur and nitrogen; and each of said $C_{1-6}$ alkyl, $C_{1-6}$ alkoxy, aryl or fused ring is optionally substituted with one or more halogen atoms;
- $R_{12}$ and $R_{13}$ are each independently selected from the group consisting of hydrogen, $C_{1-6}$ alkyl, aryl-$C_{1-4}$ alkyl, aryl and N-containing heterocyclic rings, or $R_{12}$ and $R_{13}$ together form a $C_{3-6}$ cycloalkyl or heterocyclic group; and
- X is a linking moiety selected from the group consisting of a single bond; - C(=O)-; -S(=O)$_2$-; divalent saturated non-cyclic hydrocarbon groups comprising from 1 to 6 atoms in the main chain, each of said atoms in the main chain being independently selected from the group consisting of carbon, nitrogen and sulfur, and each of said carbon atoms in the main chain being optionally substituted with one or more substituents independently selected from the group consisting of oxo, thioxo, $C_{1-4}$ alkyl and halogen, provided that the number of heteroatoms in the main chain of said divalent saturated or unsaturated non-cyclic hydrocarbon group is 0, 1 or 2; and divalent saturated or unsaturated heterocyclic groups comprising from 2 to 6 carbon atoms and from 1 to 3 heteroatoms independently selected from the group consisting of oxygen, sulfur and nitrogen in the said heterocyclic group; or X together with one of $R_7$ and $R_{11}$ forms a saturated or unsaturated ring having from 5 to 7 ring members

and being fused to the phenyl ring bearing said one of $R_7$ and $R_{11}$, said saturated or unsaturated ring optionally comprising one or two heteroatoms independently selected from the group consisting of oxygen, sulfur and nitrogen, and said saturated or unsaturated ring optionally comprising one or more substituents independently selected from the group consisting of $C_{1-4}$ alkyl and trifluoromethyl or is selected from the group consisting of - CO-CH=CH-, $-CH_2$-CH=CH- and $-SO_2$-CH=CH-;

or a stereoisomer or solvate thereof, or a pharmaceutically acceptable salt thereof, in combination with one or more pharmaceutically acceptable excipients.

**2.** The pharmaceutical composition according to claim 1, wherein the 1,2,4-thiadiazole derivative has the structural formula (B)

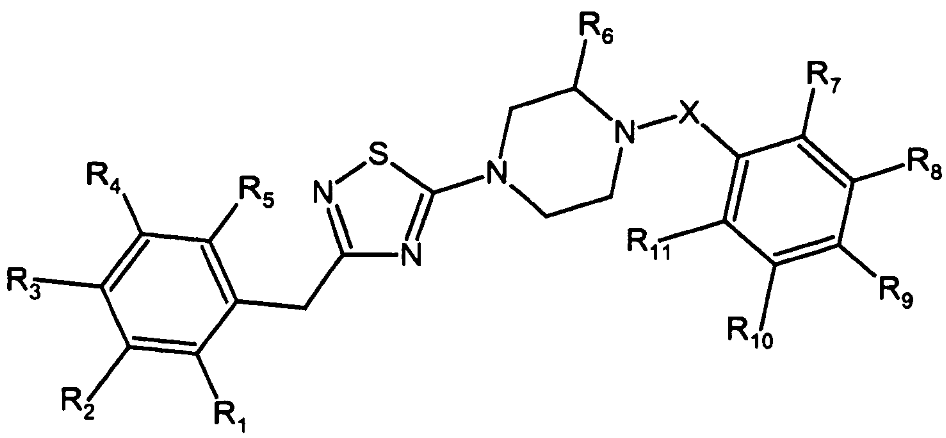

**(B)**

wherein X, $R_1$, $R_2$, $R_3$, $R_4$, $R_5$, $R_6$, $R_7$, $R_8$. $R_9$, $R_{10}$ and $R_{11}$ are as defined in claim 1, or a stereoisomer or a solvate thereof, or a pharmaceutically acceptable salt thereof.

**3.** The pharmaceutical composition according to claim 1 or claim 2, wherein X is a divalent saturated or unsaturated hydrocarbon group comprising from 1 to 3 carbon atoms but no heteroatom in the main chain, and wherein each of said carbon atoms in the main chain is optionally substituted with one or more substituents independently selected from the group consisting of oxo, thioxo, $C_{1-4}$ alkyl and halogen.

**4.** The pharmaceutical composition according to claim 1 or claim 2, wherein X is selected from the group consisting of $-CO-CH_2-$, $-CO-(CH_2)_2-$, $-CO-CHR_{14}-$, and - CO-CHX'-, wherein $R_{14}$ is $C_{1-4}$ alkyl and wherein X' is halogen.

**5.** The pharmaceutical composition according to claim 1 or claim 2, wherein X is a divalent saturated or unsaturated hydrocarbon group comprising from 1 to 5 carbon atoms and one single nitrogen, oxygen or sulfur atom in the main chain, and wherein each of said carbon atoms in the main chain is optionally substituted with one or more substituents independently selected from the group consisting of oxo, thioxo and $C_{1-4}$ alkyl.

**6.** The pharmaceutical composition according to claim 1 or claim 2, wherein three of $R_1$, $R_2$, $R_3$, $R_4$ and $R_5$ are hydrogen, and wherein two adjacent substituents selected from the group consisting of $R_1$, $R_2$, $R_3$, $R_4$ and $R_5$ form, together with the phenyl ring carbon atoms to which they are attached, a phenyl or methylenedioxy ring fused to said phenyl ring.

**7.** The pharmaceutical composition according to claim 1 or claim 2, wherein four of $R_1$, $R_2$, $R_3$, $R_4$ and $R_5$ are hydrogen, and wherein one of $R_1$, $R_2$, $R_3$, $R_4$ and $R_5$ is selected from the group consisting of $C_{1-4}$ alkyl, $C_{1-4}$ alkoxy, aryl, aryloxy, aryl-$C_{1-4}$ alkyloxy, heteroaryloxy, benzenesulfonate, amino, hydroxy, nitro, trifluoromethyl, trifluoromethoxy and halogen.

**8.** The pharmaceutical composition according to any of claims 1 to 7, wherein $R_{12}$ is hydrogen and $R_{13}$ is selected from the group consisting of piperidinyl and morpholinyl.

**9.** The pharmaceutical composition according to any of claims 1 to 8, wherein two adjacent substituents selected from the group consisting of $R_7$, $R_8$, $R_9$, $R_{10}$ and $R_{11}$ form, together with the phenyl ring carbon atoms to which they are attached, a methylenedioxy group fused to said phenyl ring.

**10.** The pharmaceutical composition according to claim 1, wherein said 1,2,4-thiadiazole derivative is selected from the group consisting of:

4-phenylacetyl-1-[3-(4-fluorobenzy)-1,2,4-thiadiazol-5-yl]piperazine 4-(4-fluorophenylacetyl),1-[3-(4-fluorobenzyl)-1,2, 4-thiadiazol-5-yl]piperazine
1-(4-fluorophenylacetyl)-4-[3-(4-methylbenzyl)-1,2,4-thiadiazol-5-yl]-2-methylpiperazine
1-(4-methoxyphenylacetyl)-4-[3-(4-fluorobenzyl)-1,2,4-thiadiazol-5-yl]-2-methylpiperazine
1-phenylacetyl-4-[3-(4-tluorobenzyl)-1,2,4-thiadiazol-5-yl]-2-methylpiperazine
1-(4-fluorophenylacetyl)-4-[3-(4-fluorobenzyl)-1,2,4-thiadiazol-5-yl]-2-methylpiperazine
1-[chloro(phenyl)acetyl]-4-[3-(4-fluorobenzyl)-1,2,4-thiadiazol-5-yl]-2-methylpiperazine
1-(2-phenylbutanoyl)-4-[3-(4-fluorobenzyl)-1,2,4-thiadiazol-5-yl]-2-methylpiperazine
N-(3-fluorophenyl)-4-[3-(4-fluorobenzyl)-1,2,4-thiadiazol-5-yl]piperazine-1-carboxamide N-(2-methylphenyl)-4-[3-(4-fluorobenzyl)-1,2,4-thiadiazol-5-yl]-2-methylpiperazine-1-carboxamide
N-(4-ethylphenyl)-4-[3-(4-fluorobenzyl)-1,2,4-thiadiazol-5-yl]-2-methylpiperazine-1-carboxamide
N-(2-ethylphenyl)-4-[3-(4-fluorobenzyl)-1,2,4-thiadiazol-5-yl]-2-methylpiperazine-1-carboxamide
N-phenyl-4-[3-(4-fluorobenzyl)-1,2,4-thiadiazol-5-yl]piperazine-1-carboxamide
N-(4-ethoxyphenyl)-4-[3-(4-fluorobenzyl)-1,2,4-thiadiazol-5-yl]-2-methylpiperazine-1-carboxamide
N-(2-methylphenyl)-4-[3-(4-fluorobenzyl)-1,2,4-thiadiazol-5-yl]piperazine-1-carboxamide
N-(2-fluorophenyl)-4-[3-(4-fluorobenzyl)-1,2,4-thiadiazol-5-yl]piperazine-1-carboxamide N-(2-trifluoromethylphenyl)-4-[3-(4-fluorobenzyl)-1,2,4-thiadiazol-5-yl]piperazine-1-carboxamide
N-(2-trifluoromethylphenyl)-4-[3-(4-fluorobenzyl)-1,2,4-thiadiazol-5-yl]-2-methylpiperazine-1-carboxamide
N-(2,6-dimethylphenyl)-4-[3-(4-fluorobenzyl)-1,2,4-thiadiazol-5-yl]-2-methylpiperazine-1-carboxamide
N-(2,4-dimethylphenyl)-4-[3-(4-fluorobenzyl)-1,2,4-thiadiazol-5-yl]-2-methylpiperazine-1-carboxamide
N-(2,6-dichlorophenyl)-4-[3-(4-fluorobenzyl)-1,2,4-thiadiazol-5-yl]-2-methylpiperazine-1-carboxamide
N-(3-cyanophenyl)-4-[3-(4-fluorobenzyl)-1,2,4-thiadiazol-5-yl]-2-methylpiperazine-1-carboxamide
N-(2,4-difluorophenyl)-4-[3-(4-fluorobenzyl)-1,2,4-thiadiazol-5-yl]-2-methylpiperazine-1-carboxamide
N-(2,6-dimethylphenyl)-4-[3-(4-fluorobenzyl)-1,2,4-thiadiazol-5-yl]piperazine-1-carboxamide
N-1-naphtyl-4-[3-(4-fluorobenzyl)-1,2,4-thiadiazol-5-yl]-2-methylpiperazine-1-carboxamide
N-(3,4-difluorophenyl)-4-[3-(4-fluorobenzyl)-1,2,4-thiadiazol-5-yl]-2-methylpiperazine-1-carboxamide
N-(2,4-dimethoxyphenyl)-4-[3-(4-fluorobenzyl)-1,2,4-thiadiazol-5-yl]piperazine-1-carboxamide
N-(3,4-difluorophenyl)-4-[3-(4-fluorobenzyl)-1,2,4-thiadiazol-5-yl]piperazine-1-carboxamide
N-(3,5-dimethoxyphenyl)-4-[3-(4-fluorobenzyl)-1,2,4-thiadiazol-5-yl]-2-methylpiperazine-1-carboxamide
1-[4-tert-butylphenylsulfonyl]-4-[3-(4-fluorobenzyl)-1,2,4-thiadiazol-5-yl]piperazine
1-[4-methoxyphenylsulfonyl]-4-[3-(4-chlorobenzyl)-1,2,4-thiadiazol-5-yl]piperazine
1-[4-methoxyphenylsulfonyl]-4-[3-(4-fluorobenzyl)-1,2,4-thiadiazol-5-yl]piperazine
1-[4-fluorophenylsulfonyl]-4-[3-(4-chlorobenzyl)-1,2,4-thiadiazol-5-yl]piperazine 1-[4-chlorophenylsulfonyl]-4-[3-(4-methylbenzyl)-1,2,4-thiadiazol-5-yl]-2-methylpiperazine
1-[1-naphtylsulfonyl]-4-[3-(4-methylbenzyl)-1,2,4-thiadiazol-5-yl]piperazine
1-[2,5-dichlorophenylsulfonyl]-4-[3-(4-methylbenzyl)-1,2,4-thiadiazol-5-yl]-2-methylpiperazine
1-[2,4,6-trimethylphenylsulfonyl]-4-[3-(4-methylbenzyl)-1,2,4-thiadiazol-5-yl]piperazine
1-[2-naphtylsulfonyl]-4-[3-(4-methylbenzyl)-1,2,4-thiadiazol-5-yl]-2-methylpiperazine
1-[2,5-dichlorophenylsulfonyl]-4-[3-(4-chlorobenzyl)-1,2,4-thiadiazol-5-yl]piperazine 1-[4-bromophenylsulfonyl]-4-[3-(4-methylbenzyl)-1,2,4-thiadiazol-5-yl]-3-methylpiperazine
1-[1-naphtylsulfonyl]-4-[3-(4-methylbenzyl)-1,2,4-thiadiazol-5-yl]-3-methylpiperazine
1-[4-tert-butylphenylsulfonyl]-4-[3-(4-methylbenzyl)-1,2,4-thiadiazol-5-yl]-2-methylpiperazine
1-[1-naphtylsulfonyl]-4-[3-(4-chlorobenzyl)-1,2,4-thiadiazol-5-yl]-2-methylpiperazine
1-[2,4,6-trimethylphenylsulfonyl]-4-[3-(4-methylbenzyl)-1,2,4-thiadiazol-5-yl]-2-methylpiperazine
1-[3-trifluorophenylsulfonyl]-4-[3-(4-fluorobenzyl)-1,2,4-thiadiazol-5-yl]-2-methylpiperazine
1-phenylsulfonyl-4-[3-(4-fluorobenzyl)-1,2,4-thiadiazol-5-yl]-2-methylpiperazine
1-[4-acetamidophenylsulfonyl]-4-[3-(4-methylbenzyl)-1,2,4-thiadiazol-5-yl]-2-methylpiperazine
1-[4-acetamidophenylsulfonyl]-4-[3-(4-methylbenzyl)-1,2,4-thiadiazol-5-yl]piperazine
1-[4-methylphenylsulfonyl]-4-[3-(4-methylbenzyl)-1,2,4-thiadiazol-5-yl]-2-methylpiperazine
1-[4-methoxyphenylsulfonyl]-4-[3-(4-chlorobenzyl)-1,2,4-thiadiazol-5-yl]-2-methylpiperazine
1-[2-naphtylsulfonyl]-4-[3-(4-chlorobenzyl)-1,2,4-thiadiazol-5-yl]piperazine

1-[4-tert-butylphenylsulfonyl]-4-[3-(4-fluorobenzyl)-1,2,4-thiadiazol-5-yl]-2-methylpiperazine
1-[4-acetamidophenylsulfonyl]-4-[3-(4-fluorobenzyl)-1,2,4-thiadiazol-5-yl]-2-methylpiperazine
1-[3-trifluoromethylphenylsulfonyl]-4-[3-(4-methylbenzyl)-1,2,4-thiadiazol-5-yl]-2-methylpiperazine
1-[4-fluorophenylsulfonyl]-4-[3-(4-methylbenzyl)-1,2,4-thiadiazol-5-yl]piperazine
1-[4-fluorophenylsulfonyl]-4-[3-(4-methylbenzyl)-1,2,4-thiadiazol-5-yl]-2-methylpiperazine
1-[4-fluorophenylsulfonyl]-4-[3-(4-fluorobenzyl)-1,2,4-thiadiazol-5-yl]-2-methylpiperazine
1-[4-methoxyphenylsulfonyl]-4-[3-(4-methylbenzyl)-1,2,4-thiadiazol-5-yl]-2-methylpiperazine
1-[4-ter-butylphenylsulfonyl]-4-[3-(4-fluorobenzyl)-1,2,4-thiadiazol-5-yl]piperazine
1-[3-trifluoromethylphenylsulfonyl]-4-[3-(4-methylbenzyl)-1,2,4-thiadiazol-5-yl]piperazine
1-[4-methylphenylsulfonyl]-4-[3-(4-chlorobenzyl)-1,2,4-thiadiazol-5-yl]piperazine
1-[4-bromophenylsulfonyl]-4-[3-(4-methylbenzyl)-1,2,4-thiadiazol-5-yl]piperazine
1-[2-naphtylsulfonyl]-4-[3-(4-methylbenzyl)-1,2,4-thiadiazol-5-yl]piperazine
1-[4-chlorophenylsulfonyl]-4-[3-(4-methylbenzyl)-1,2,4-thiadiazol-5-yl]piperazine
1-[4-tert-butylphenylsulfonyl]-4-[3-(4-methylbenzyl)-1,2,4-thiadiazol-5-yl]piperazine
1-[4-methoxyphenylsulfonyl]-4-[3-(4-methylbenzyl)-1,2,4-thiadiazol-5-yl]piperazine
1-[4-tert-butylphenylsulfonyl]-4-[3-(4-chlorobenzyl)-1,2,4-thiadiazol-5-yl]piperazine
1-[quinoline-8-sulfonyl]-4-[3-(4-methylbenzyl)-1,2,4-thiadiazol-5-yl]piperazine 1-[4-nitrophenylsulfonyl]-4-[3-(4-methylbenzyl)-1,2,4-thiadiazol-5-yl]-2-methylpiperazine
1-[3-nitro-4-chlorophenylsulfonyl]-4-[3-(4-methylbenzyl)-1,2,4-thiadiazol-5-yl]-2-methylpiperazine
1-[4-nitrophenylsulfonyl]-4-[3-(4-fluorobenzyl)-1,2,4-thiadiazol-5-yl]-2-methylpiperazine
1-[4-methoxyphenylsulfonyl]-4-[3-(4-fluorobenzyl)-1,2,4-thiadiazol-5-yl]piperazine
1-[3-nitro-4-chlorophenylsulfonyl]-4-[3-(4-methylbenzyl)-1,2,4-thiadiazol-5-yl]piperazine
1-(benzylsulfonyl)-4-[3-(4-chlorobenzyl)-1,2,4-thiadiazol-5-yl]piperazine
1-(benzylsulfonyl)-4-[3-(4-methylbenzyl)-1,2,4-thiadiazol-5-yl]piperazine
1-(phenylprop-2-ensulfonyl)-4-[3-(4-fluorobenzyl)-1,2,4-thiadiazol-5-yl]piperazine
1-(phenylprop-2-ensulfonyl)-4-[3-(4-methylbenzyl)-1,2,4-thiadiazol-5-yl]-2-methylpiperazine
1-(phenylprop-2-ensulfonyl)-4-[3-(4-methylbenzyl)-1,2,4-thiadiazol-5-yl]piperazine
1-(butylsulfonyl)-4-[3-(4-chlorobenzyl)-1,2,4-thiadiazol-5-yl]piperazine
1-(octylsulfonyl)-4-[3-(4-methylbenzyl)-1,2,4-thiadiazol-5-yl]piperazine
1-(butylsulfonyl)-4-[3-(4-methylbenzyl)-1,2,4-thiadiazol-5-yl]piperazine
1-(ethylsulfonyl)-4-[3-(4-chlorobenzyl)-1,2,4-thiadiazol-5-yl]piperazine
1-(isopropylsulfonyl)-4-[3-(4-methylbenzyl)-1,2,4-thiadiazol-5-yl]piperazine
1-[(2E)-3-phenylprop-2-enoyl]-4-[3-(4-chlorobenzyl)-1,2,4-thiadiazol-5-yl]-2-methylpiperazine
1-[(2E)-3-phenylprop-2-enoyl]-4-B103-2-methylpiperazine
1-[(2E)-3-phenylprop-2-enoyl]-4-[3-(4-chlorobenzyl)-1,2,4-thiadiazol-5-yl]piperazine
1-(3-phenylpropanoyl)-4-[3-(4-fluorobenzyl)-1,2,4-thiadiazol-5-yl]-2-methylpiperazine
1-(3-phenylpropanoyl)-4-[3-(4-fluorobenzyl)-1,2,4-thiadiazol-5-yl]piperazine
1-[3-phenylprop-2-enyl]-4-[3-(4-methylbenzyl)-1,2,4-thiadiazol-5-yl]piperazine
1-[3-phenylprop-2-enyl]-4-[3-(4-chlorobenzyl)-1,2,4-thiadiazol-5-yl]piperazine
1-[3-phenylprop-2-enyl]-4-[3-(4-fluorobenzyl)-1,2,4-thiadiazol-5-yl]piperazine
4-pentyl-N-{2-[4-[3-(4-fluorobenzyl)-1,2,4-thiadiazol-5-yl]piperazin-1-yl]ethyl}benzamide
4-butyl-N-{2-[4-[3-(4-fluorobenzyl)-1,2,4-thiadiazol-5-yl]piperazin-1-yl]ethyl}benzamide
4-hexyl-N-{2-[4-[3-(4-fluorobenzyl)-1,2,4-thiadiazol-5-yl]piperazin-1-yl]ethyl}benzamide
4-chloro-N-{2-[4-[3-(4-fluorobenzyl)-1,2,4-thiadiazol-5-yl]piperazin-1-yl]ethyl}benzamide
3,5-dichloro-N-{2-[4-[3-(4-fluorobenzyl)-1,2,4-thiadiazol-5-yl]piperazin-1-yl]ethyl}benzamide
2-methyl-N-{2-[4-[3-(4-fluorobenzyl)-1,2,4-thiadiazol-5-yl]piperazin-1-yl]ethyl}benzamide
3-fluoro-N-{2-[4-[3-(4-chlorobenzyl)-1,2,4-thiadiazol-5-yl]piperazin-1-yl]ethyl}benzamide
4-methyl-N-{2-[4-[3-(4-fluorobenzyl)-1,2,4-thiadiazol-5-yl]piperazin-1-yl]ethyl}benzamide
4-fluoro-N-{2-[4-[3-(4-fluorobenzyl)-1,2,4-thiadiazol-5-yl]piperazin-1-yl]ethyl}benzamide
4-ethyl-N-{2-[4-[3-(4-fluorobenzyl)-1,2,4-thiadiazol-5-yl]piperazin-1-yl]ethyl}benzamide
N-{2-[4-[3-(4-fluorobenzyl)-1,2,4-thiadiazol-5-yl]piperazin-1-yl]ethyl}benzamide
3-fluoro-N-{2-[4-[3-(4-fluorobenzyl)-1,2,4-thiadiazol-5-yl]piperazin-1-yl]ethyl}benzamide
3-fluoro-N-{2-[4-[3-benzyl-1,2,4-thiadiazol-5-yl]piperazin-1-yl]ethyl}benzamide 2-(4-fluorophenyl)-N-{2-[4-[3-(4-fluorobenzyl)-1,2,4-thiadiazol-5-yl]piperazin-1-yl]ethyl}acetamide
2-phenyl-N-{2-[4-[3-(4-fluorobenzyl)-1,2,4-thiadiazol-5-yl]piperazin-1-yl]ethyl}acetamide
2-(4-fluorophenyl)-N-{2-[4-[3-(4-methylbenzyl)-1,2,4-thiadiazol-5-yl]piperazin-1-yl]ethyl}acetamide
N-benzyl-N'-{2-[4-[3-(4-fluorobenzyl)-1,2,4-thiadiazol-5-yl]piperazin-1-yl]ethyl}urea
2-phenyl-N-{2-[4-[3-(4-fluorobenzyl)-1,2,4-thiadiazol-5-yl]piperazin-1-yl]ethyl}butanamide

3-phenyl-N-{2-[4-[3-(4-fluorobenzyl)-1,2,4-thiadiazol-5-yl]piperazin-1-yl]ethyl}propanamide
1-benzyl-4-[3-(4-fluorobenzyl)-1,2,4-thiadiazol-5-yl]piperazine
1-(1,3-benzodioxol-5-ylmethyl)-4-[3-(4-fluorobenzyl)-1,2,4-thiadiazol-5-yl]piperazine
1-(2-fluorobenzoyl)-4-[3-(3-methoxybenzyl)-1,2,4-thiadiazol-5-yl]-2-methylpiperazine
1-(4-ethylbenzoyl)-4-[3-(4-fluorobenzyl)-1,2,4-thiadiazol-5-yl]-2-methylpiperazine
1-(4-butylbenzoyl)-4-[3-(4-methylbenzyl)-1, 2,4-thiadiazol-5-yl]-2-methylpiperazine
1-(4-methoxybenzoyl)-4-[3-(4-fluorobenzyl)-1,2,4-thiadiazol-5-yl]-2-methylpiperazine
1-(1,3-benzodioxol-5-ylcarbonyl)-4-[3-(3-methoxybenzyl)-1,2,4-thiadiazol-5-yl]-2-methylpiperazine
1-(4-butylbenzoyl)-4-[3-(4-fluorobenzyl)-1,2,4-thiadiazol-5-yl]-2-methylpiperazine
1-(4-hexylbenzoyl)-4-[3-(4-fluorobenzyl)-1,2,4-thiadiazol-5-yl]piperazine
1-(3-chlorobenzoyl)-4-[3-(4-fluorobenzyl)-1,2,4-thiadiazol-5-yl]-2-methylpiperazine
1-(4-fluorobenzoyl)-4-[3-(3-methoxybenzyl)-1,2,4-thiadiazol-5-yl]-2-methylpiperazine
1-(4-methylbenzoyl)-4-[3-(3-methoxybenzyl)-1,2,4-thiadiazol-5-yl]-2-methylpiperazine
1-(3-fluorobenzoyl)-4-[3-(4-chlorobenzyl)-1,2,4-thiadiazol-5-yl]-2-methylpiperazine
1-benzoyl-4-[3-(4-fluorobenzyl)-1,2,4-thiadiazol-5-yl]-2-methylpiperazine
1-(4-fluorobenzoyl)-4-[3-(4-fluorobenzyl)-1,2,4-thiadiazol-5-yl]-2-methylpiperazine
1-(4-tert-butylbenzoyl)-4-[3-(4-fluorobenzyl)-1,2,4-thiadiazol-5-yl]-2-methylpiperazine 1-(1,1'-biphenyl-4-ylcarbonyl)-4-[3-(4-fluorobenzyl)-1,2,4-thiadiazol-5-yl]-2-methylpiperazine
1-(4-methoxybenzoyl)-4-[3-(4-methylbenzyl)-1,2,4-thiadiazol-5-yl]-2-methylpiperazine
1-(4-ethylbenzoyl)-4-[3-(4-methylbenzyl)-1,2,4-thiadiazol-5-yl]piperazine
1-(2-naphthoyl)-4-[3-(4-fluorobenzyl)-1,2,4-thiadiazol-5-yl]-2-methylpiperazine
1-(2-methoxybenzoyl)-4-[3-(4-fluorobenzyl)-1,2,4-thiadiazol-5-yl]-2-methylpiperazine
1-(4-pentylbenzoyl)-4-[3-(4-methylbenzyl)-1,2,4-thiadiazol-5-yl]-2-methylpiperazine
1-(4-bromobenzoyl)-4-[3-(4-fluorobenzyl)-1,2,4-thiadiazol-5-yl]-2-methylpiperazine 1-(2,4-dimethoxybenzoyl)-4-[3-(4-fluorobenzyl)-1,2,4-thiadiazol-5-yl]-2-methylpiperazine
1-(3,5-dichlorobenzoyl)-4-[3-(4-fluorobenzyl)-1,2,4-thiadiazol-5-yl]piperazine
1-(3-chlorobenzoyl)-4-[3-(4-fluorobenzyl)-1,2,4-thiadiazol-5-yl]piperazine
1-(4-methylbenzoyl)-4-[3-(4-fluorobenzyl)-1,2,4-thiadiazol-5-yl]-2-methylpiperazine
1-(2-methylbenzoyl)-4-[3-(4-fluorobenzyl)-1,2,4-thiadiazol-5-yl]-2-methylpiperazine
1-(4-methylbenzoyl)-4-[3-(4-fluorobenzyl)-1,2,4-thiadiazol-5-yl]piperazine
1-(3-bromobenzoyl)-4-[3-(4-fluorobenzyl)-1,2,4-thiadiazol-5-yl]-2-methylpiperazine
1-(4-ethylbenzoyl)-4-[3-(3-methoxybenzyl)-1,2,4-thiadiazol-5-yl]-2-methylpiperazine
1-(3-methylbenzoyl)-4-[3-(4-methylbenzyl)-1,2,4-thiadiazol-5-yl]-2-methylpiperazine 1-(3-trifluoromethylbenzoyl)-4-[3-(4-fluorobenzyl)-1,2,4-thiadiazol-5-yl]-2-methylpiperazine
1-(4-tert-butylbenzoyl)-4-[3-(3-methoxybenzyl)-1,2,4-thiadiazol-5-yl]-2-methylpiperazine
1-(4-ethylbenzoyl)-4-[3-(4-methylbenzyl)-1,2,4-thiadiazol-5-yl]-2-methylpiperazine
1-(2-bromobenzoyl)-4-[3-(4-fluorobenzyl)-1,2,4-thiadiazol-5-yl]-2-methylpiperazine
1-(2-fluorobenzoyl)-4-[3-(4-methylbenzyl)-1,2,4-thiadiazol-5-yl]-2-methylpiperazine
1-(3-fluorobenzoyl)-4-[3-benzyl-1,2,4-thiadiazol-5-yl]-2-methylpiperazine
1-(4-fluorobenzoyl)-4-[3-(4-methytbenzyl)-1,2,4-thiadiazol-5-yl]-2-methylpiperazine
1-(2-methylbenzoyl)-4-[3-(4-fluorobenzyl)-1,2,4-thiadiazol-5-yl]piperazine
1-(4-fluorobenzoyl)-4-[3-(4-chlorobenzyl)-1,2,4-thiadiazol-5-yl]piperazine
1-(4-fluorobenzoyl)-4-[3-benzyl-1,2,4-thiadiazol-5-yl]-2-methylpiperazine
1-(4-chlorobenzoyl)-4-[3-(4-methylbenzyl)-1,2,4-thiadiazol-5-yl]piperazine
1-(3-fluorobenzoyl)-4-[3-(4-methylbenzyl)-1,2,4-thiadiazol-5-yl]-2-methylpiperazine
1-(3-fluorobenzoyl)-4-[3-(4-chlorobenzyl)-1,2,4-thiadiazol-5-yl]piperazine
1-benzoyl-4-[3-(4-methylbenzyl)-1,2,4-thiadiazol-5-yl]piperazine
1-(2-fluorobenzoyl)-4-[3-(4-chlorobenzyl)-1,2,4-thiadiazol-5-yl]piperazine
1-(4-fluorobenzoyl)-4-[3-(4-methylbenzyl)-1,2,4-thiadiazol-5-yl]piperazine 1-(1,3-benzodioxol-5-ylcarbonyl)-4-[3-(4-methylbenzyl)-1,2,4-thiadiazol-5-yl]-2-methylpiperazine
1-(3-flurorbenzoyl)-4-[3-(4-methylbenzyl)-1,2,4-thiadiazol-5-yl]piperazine
1-(4-fluorobenzoyl)-4-[3-benzyl-1,2,4-thiadiazol-5-yl]piperazine
1-(4-bromobenzoyl)-4-[3-(4-methylbenzyl)-1,2,4-thiadiazol-5-yl]piperazine
1-(4-ethylbenzoyl)-4-[3-(4-chlorobenzyl)-1,2,4-thiadiazol-5-yl]piperazine
1-(2-chlorobenzoyl)-4-[3-(4-fluorobenzyl)-1,2,4-thiadiazol-5-yl]-2-methylpiperazine
1-(2-fluorobenzoyl)-4-[3-(4-chlorobenzyl)-1,2,4-thiadiazol-5-yl]-2-methylpiperazine
1-(4-trifluoromethylbenzoyl)-4-[3-(3-methoxybenzyl)-1,2,4-thiadiazol-5-yl]-2-methylpiperazine
1-benzoyl-4-[3-(4-methylbenzyl)-1,2,4-thiadiazol-5-yl]-2-methylpiperazine

1-(4-bromobenzoyl)-4-[3-(3-methoxybenzyl)-1,2,4-thiadiazol-5-yl]-2-methylpiperazine
1-(4-trifluoromethylbenzoyl)-4-[3-(4-methylbenzyl)-1,2,4-thiadiazol-5-yl]piperazine
1-(3-nitro-4-methylbenzoyl)-4-[3-(4-fluorobenzyl)-1,2,4-thiadiazol-5-yl]-2-methylpiperazine
1-benzoyl-4-[3-(3-methoxybenzyl)-1,2,4-thiadiazol-5-yl]-2-methylpiperazine
1-(4-chlorobenzoyl)-4-[3-(3-methoxybenzyl)-1,2,4-thiadiazol-5-yl]-2-methylpiperazine
1-(2-fluorobenzoyl)-4-[3-(4-methylbenzyl)-1,2,4-thiadiazol-5-yl]piperazine
1-(4-hexylbenzoyl)-4-[3-(4-methylbenzyl)-1,2,4-thiadiazol-5-yl]piperazine
1-(2-chloro-4-nitrobenzoyl)-4-[3-(4-fluorobenzyl)-1,2,4-thiadiazol-5-yl]-2-methylpiperazine
1-(1,3-benzodioxol-5-ylcarbonyl)-4-[3-(4-fluorobenzyl)-1,2,4-thiadiazol-5-yl]-2-methylpiperazine
1-(3-fluorobenzoyl)-4-[3-(4-fluorobenzyl)-1,2,4-thiadiazol-5-yl]-2-methylpiperazine
1-(4-tert-butylbenzoyl)-4-[3-(4-fluorobenzyl)-1,2,4-thiadiazol-5-yl]piperazine
1-benzoyl-4-[3-(4-fluorobenzyl)-1,2,4-thiadiazol-5-yl]piperazine
1-(4-butylbenzoyl)-4-[3-(4-methylbenzyl)-1,2,4-thiadiazol-5-yl]piperazine
1-(4-tert-butylbenzoyl)-4-[3-(4-methylbenzyl)-1,2,4-thiadiazol-5-yl]piperazine
1-(4-nitrobenzoyl)-4-[3-(4-fluorobenzyl)-1,2,4-thiadiazol-5-yl]-2-methylpiperazine
1-(2-methylphenyl)-4-[3-(4-fluorobenzyl)-1,2,4-thiadiazol-5-yl]piperazine
1-(2-ethoxyphenyl)-4-[3-(4-fluorobenzyl)-1,2,4-thiadiazol-5-yl]piperazine
1-(2-fluorophenyl)-4-[3-(4-fluorobenzyl)-1,2,4-thiadiazol-5-yl]piperazine
1-(3-trifluoromethylphenyl)-4-[3-(4-fluorobenzyl)-1,2,4-thiadiazol-5-yl]piperazine
1-(4-chlorobenzoyl)-4-[3-(4-fluorobenzyl)-1,2,4-thiadiazol-5-yl]-1,4-diazepane
1-(4-methylbenzoyl)-4-[3-(4-fluorobenzyl)-1,2,4-thiadiazol-5-yl]-1,4-diazepane
1-(1,3-benzodioxol-5-ylcarbonyl)-4-[3-(4-fluorobenzyl)-1,2,4-thiadiazol-5-yl]-1,4-diazepane,
1-[4-methoxyphenylsulfonyl]-4-[3-(3-fluoro-benzyl)-1,2,4-thiadiazol-5-yl]piperazine;
1-[4-methoxyphenylsulfonyl]-4-[3-(2-fluoro-benzyl)-1,2,4-thiadiazol-5-yl]piperazine;
1-[2-methoxyphenylsulfonyl]-4-[3-(4-fluoro-benzyl)-1,2,4-thiadiazol-5-yl]piperazine;
1-[4-ethoxyphenylsulfonyl]-4-[3-(4-fluoro-benzyl)-1,2,4-thiadiazol-5-yl]piperazine;
1-[4-ethylphenylsulfonyl]-4-[3-(4-fluoro-benzyl)-1,2,4-thiadiazol-5-yl]piperazine;
1-phenylsulfonyl-4-[3-(4-fluoro-benzyl)-1,2,4-thiadiazol-5-yl]piperazine;
1-[4-methoxyphenylsulfonyl]-4-[3-benzyl-1,2,4-thiadiazol-5-yl]piperazine;
1-(4-propionyloxy)benzene-sulfonyl-4-[3-(4-fluoro-benzyl)-1,2,4-thiadiazol-5-yl]piperazine;
1-[4-methoxyphenylsulfonyl]-4-[3-(3-methyl-benzyl-1,2,4-thiadiazol-5-yl]piperazine;
1-[4-methoxyphenylsulfonyl]-4-[3-(2-methyl-benzyl-1,2,4-thiadiazol-5-yl]piperazine;
1-[4-methoxyphenylsulfonyl]-4-[3-(2-methoxy-benzyl-1,2,4-thiadiazol-5-yl]piperazine;
1-[4-methoxyphenylsulfonyl]-4-[3-(3-methyl-4-fluoro-benzyl-1,2,4-thiadiazol-5-yl]piperazine;
1-[4-methoxyphenylsulfonyl]-4-[3-(4-methoxy-benzyl-1,2,4-thiadiazol-5-yl]piperazine;
1-[4-bromophenylsulfonyl]-4-[3-(3-methoxy-benzyl-1,2,4-thiadiazol-5-yl]piperazine;
1-[4-methoxyphenylsulfonyl]-4-[3-(3-methoxybenzyl)-1,2,4-thia-diazol-5-yl]-2-methyl-piperazine; and
1-(4-hydroxybenzenesulfonyl)-4-[3-(4-fluoro-benzyl)-1,2,4-thiadiazol-5-yl]piperazine.

**11.** A 1,2,4-thiadiazole derivative selected from the group consisting of:

- 1-[3-methoxyphenylsulfonyl]-4-[3-(4-fluorobenzyl)-1,2,4-thiadiazol-5-yl]piperazine;
- 1-[4-methoxyphenylsulfonyl]-4-[3-(3-methoxybenzyl)-1,2,4-thiadiazol-5-yl]piperazine;
- 1-[4-methoxyphenylsulfonyl]-4-[3-(3-fluoro-benzyl)-1,2,4-thiadiazol-5-yl]piperazine;
- 1-[4-methoxyphenylsulfonyl]-4-[3-(2-fluoro-benzyl)-1,2,4-thiadiazol-5-yl]piperazine;
- 1-[2-methoxyphenylsulfonyl]-4-[3-(4-fluoro-benzyl)-1,2,4-thiadiazol-5-yl]piperazine;
- 1-[4-ethoxyphenylsulfonyl]-4-[3-(4-fluoro-benzyl)-1,2,4-thiadiazol-5-yl]piperazine;
- 1-[4-ethylphenylsulfonyl]-4-[3-(4-fluoro-benzyl)-1,2,4-thiadiazol-5-yl]piperazine;
- 1-phenylsulfonyl-4-[3-(4-fluoro-benzyl)-1,2,4-thiadiazol-5-yl]piperazine;
- 1-[4-methoxyphenylsulfonyl]-4-[3-benzyl-1,2,4-thiadiazol-5-yl]piperazine;
- 1-(4-propionyloxy)benzene-sulfonyl-4-[3-(4-fluoro-benzyl)-1,2,4-thiadiazol-5-yl]piperazine;
- 1-[4-methoxyphenylsulfonyl]-4-[3-(3-methyl-benzyl-1,2,4-thiadiazol-5-yl]piperazine;
- 1-[4-methoxyphenylsulfonyl]-4-[3-(2-methyl-benzyl-1,2,4-thiadiazol-5-yl]piperazine;
- 1-[4-methoxyphenylsulfonyl]-4-[3-(2-methoxy-benzyl-1,2,4-thiadiazol-5-yl]piperazine;
- 1-[4-methoxyphenylsulfonyl]-4-[3-(3-methyl-4-fluoro-benzyl-1,2,4-thiadiazol-5-yl]piperazine;
- 1-[4-methoxyphenylsulfonyl]-4-[3-(4-methoxy-benzyl-1,2,4-thiadiazol-5-yl]piperazine;
- 1-[4-bromophenylsulfonyl]-4-[3-(3-methoxy-benzyl-1,2,4-thiadiazol-5-yl]piperazine;
- 1-[4-methoxyphenylsulfonyl]-4-[3-(3-methoxybenzyl)-1,2,4-thia-diazol-5-yl]-2-methyl-piperazine; and

- 1-(4-hydroxybenzenesulfonyl)-4-[3-(4-fluoro-benzyl)-1,2,4-thiadiazol-5-yl]piperazine.

**12.** The pharmaceutical composition according to any of claims 1 to 10, further comprising a therapeutic amount of one or more drugs selected from the group consisting of neuro-protective agents and α-synuclein deposition inhibitors.

**13.** A 1,2,4-thiadiazole derivative as defined in any of claims 1 to 10 for use in the treatment of an α-synucleopathy.

**14.** The 1,2,4-thiadiazole derivative according to claim 13, wherein said α-synucleopathy is selected from the group consisting of Parkinson's disease, diffuse Lewy body disease, traumatic brain injury, amyotrophic lateral sclerosis, Niemann-Pick disease, Hallervorden-Spatz syndrome, Down syndrome, neuroaxonal dystrophy, multiple system atrophy and Alzheimer's disease.

**Patentansprüche**

**1.** Pharmazeutische Zusammensetzung umfassend eine therapeutisch wirksame Menge eines 1,2,4-Thiadiazolderivats nach der Strukturformel: (A)

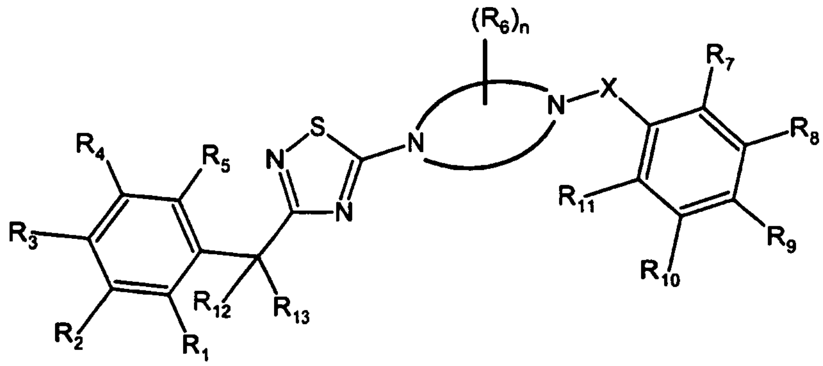

(A)

wobei die divalente Gruppe, die schematisch durch die Strukturformel (A')

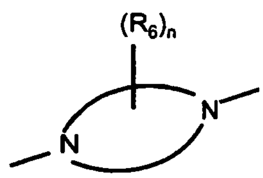

(A')

dargestellt ist, einen gegebenenfalls monosubstituierten oder polysubstituierten, gesättigten oder teilweise ungesättigten heterozyklischen Ring mit mindestens zwei Stickstoffatomen in dem heterozyklischen Ring und mit insgesamt 5 bis 7 Atomen in dem heterozyklischen Ring enthält, und wobei des Weiteren:

- $R_6$ ein Substituent ist, der unabhängig ausgewählt ist aus der Gruppe bestehend aus Oxo und $C_{1-4}$-Alkyl;
- n ausgewählt ist aus der Gruppe bestehend aus 0, 1, 2 und 3;
- $R_1$, $R_2$, $R_3$, $R_4$ und $R_5$ jeweils unabhängig ausgewählt sind aus der Gruppe bestehend aus Wasserstoff, $C_{1-4}$-Alkyl, $C_{1-4}$-Alkoxy, Aryl, Aryloxy, Aryl-$C_{1-4}$-Alkyloxy, Heteroaryloxy, Benzolsulfonat, Amino, Hydroxy, Nitro, Trifluormethyl, Trifluormethoxy und Halogen, oder beliebige zwei benachbarte Substituenten, die ausgewählt sind aus der Gruppe bestehend aus $R_1$, $R_2$, $R_3$, $R_4$ und $R_5$, gemeinsam mit den Phenylring- Kohlenstoffatomen, an welche sie angehängt sind, einen gesättigten oder ungesättigten Ring bilden, der an den Phenylring fusioniert ist und 5 bis 7 Ringelemente aufweist, wobei der gesättigte oder ungesättigte Ring gegebenenfalls ein oder zwei Sauerstoffatome umfasst und gegebenenfalls mit einem oder mehreren Halogenatomen substituiert ist;

- $R_7$, $R_8$, $R_9$, $R_{10}$ und $R_{11}$ jeweils unabhängig ausgewählt sind aus der Gruppe bestehend aus Wasserstoff, $C_{1-10}$-Alkyl, $C_{1-6}$-Alkoxy, Aryl, Hydroxy, Acetyl, Nitro, Trifluormethyl und Halogen; oder beliebige zwei benachbarte Substituenten, die ausgewählt sind aus der Gruppe bestehend aus $R_7$, $R_8$, $R_9$, $R_{10}$ und $R_{11}$, gemeinsam mit den Phenylring-Kohlenstoffatomen, an welche sie angehängt sind, einen gesättigten oder ungesättigten Ring bilden, der an den Phenylring fusioniert ist und 5 bis 7 Ringelemente aufweist, wobei der gesättigte oder ungesättigte Ring gegebenenfalls ein oder zwei Heteroatome umfasst, die unabhängig ausgewählt sind aus der Gruppe bestehend aus Sauerstoff, Schwefel und Stickstoff; und jedes von dem $C_{1-6}$-Alkyl, $C_{1-6}$-Alkoxy, Aryl oder dem fusionierten Ring gegebenenfalls mit einem oder mehreren Halogenatomen substituiert ist;
- $R_{12}$ und $R_{13}$ jeweils unabhängig ausgewählt sind aus der Gruppe bestehend aus Wasserstoff, $C_{1-6}$-Alkyl, Aryl-$C_{1-4}$-Alkyl, Aryl und N-enthaltenden heterozyklischen Ringen, oder $R_{12}$ und $R_{13}$ gemeinsam ein $C_{3-6}$-Cycloalkyl oder eine heterozyklische Gruppe bilden; und
- X eine Bindungseinheit ist, die ausgewählt ist aus der Gruppe bestehend aus einer Einzelbindung; -C(=O)-; -S(=O)$_2$-; divalenten gesättigten nicht-zyklischen Kohlenwasserstoffgruppen, umfassend 1 bis 6 Atome in der Hauptkette, wobei jedes der Atome in der Hauptkette unabhängig ausgewählt ist aus der Gruppe bestehend aus Kohlenstoff, Stickstoff und Schwefel, wobei jedes der Kohlenstoffatome in der Hauptkette gegebenenfalls mit einem oder mehreren Substituenten substituiert ist, die unabhängig ausgewählt sind aus der Gruppe bestehend aus Oxo, Thioxo, $C_{1-4}$-Alkyl und Halogen, vorausgesetzt, dass die Anzahl von Heteroatomen in der Hauptkette der divalenten gesättigten oder ungesättigten nicht-zyklischen Kohlenwasserstoffgruppe 0, 1 oder 2 ist; und divalenten gesättigten oder ungesättigten heterozyklischen Gruppen, umfassend 2 bis 6 Kohlenstoffatome und 1 bis 3 Heteroatome, die unabhängig ausgewählt sind aus der Gruppe bestehend aus Sauerstoff, Schwefel und Stickstoff in der heterozyklischen Gruppe; oder X gemeinsam mit einem von $R_7$ und $R_{11}$ einen gesättigten oder ungesättigten Ring mit 5 bis 7 Ringelementen bildet und an den Phenylring fusioniert ist, der das eine von $R_7$ und $R_{11}$ trägt, wobei der gesättigte oder ungesättigte Ring gegebenenfalls ein oder zwei Heteroatome umfasst, die unabhängig ausgewählt sind aus der Gruppe bestehend aus Sauerstoff, Schwefel und Stickstoff, und der gesättigte oder ungesättigte Ring gegebenenfalls einen oder mehrere Substituenten umfasst, die unabhängig ausgewählt sind aus der Gruppe bestehend aus $C_{1-4}$-Alkyl und Trifluormethyl, oder ausgewählt ist aus der Gruppe bestehend aus -CO-CH=CH-, -$CH_2$-CH=CH- und -$SO_2$-CH=CH-;

oder ein Stereoisomer oder Solvat davon, oder ein pharmazeutisch annehmbares Salz davon, in Kombination mit einem oder mehreren pharmazeutisch annehmbaren Trägern.

2. Pharmazeutische Zusammensetzung nach Anspruch 1, wobei das 1,2,4-Thiadiazolderivat die Strukturformel (B)

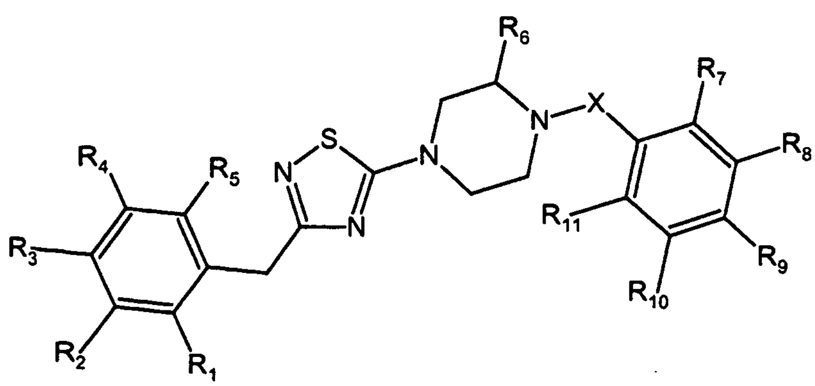

(B)

hat, wobei X, $R_1$, $R_2$, $R_3$, $R_4$, $R_5$, $R_6$, $R_7$, $R_8$, $R_9$, $R_{10}$ und $R_{11}$ wie in Anspruch 1, definiert sind, oder ein Stereoisomer oder ein Solvat davon oder ein pharmazeutisch annehmbares Salz davon sind.

3. Pharmazeutische Zusammensetzung nach Anspruch 1 oder Anspruch 2, wobei X eine divalente gesättigte oder ungesättigte Kohlenwasserstoffgruppe ist, umfassend 1 bis 3 Kohlenstoffatome aber kein Heteroatom in der Hauptkette, und wobei jedes der Kohlenstoffatome in der Hauptkette gegebenenfalls mit einem oder mehreren Substituenten substituiert ist, die unabhängig ausgewählt sind aus der Gruppe bestehend aus Oxo, Thioxo, $C_{1-4}$-Alkyl und Halogen.

4. Pharmazeutische Zusammensetzung nach Anspruch 1 oder Anspruch 2, wobei X ausgewählt ist aus der Gruppe bestehend aus $-CO-CH_2-$, $-CO-(CH_2)_2-$, $-CO-CHR_{14}-$ und -CO-CHX'-, wobei $R_{14}$ $C_{1-4}$-Alkyl ist und wobei X' Halogen ist.

5. Pharmazeutische Zusammensetzung nach Anspruch 1 oder Anspruch 2, wobei X eine divalente gesättigte oder ungesättigte Kohlenwasserstoffgruppe ist, umfassend 1 bis 5 Kohlenstoffatomen und ein einziges Stickstoff-, Sauerstoff- oder Schwefelatom in der Hauptkette, und wobei jedes der Kohlenstoffatome in der Hauptkette gegebenenfalls mit einem oder mehreren Substituenten substituiert ist, die unabhängig ausgewählt sind aus der Gruppe bestehend aus Oxo, Thioxo und $C_{1-4}$-Alkyl.

6. Pharmazeutische Zusammensetzung nach Anspruch 1 oder Anspruch 2, wobei drei von $R_1$, $R_2$, $R_3$, $R_4$ und $R_5$ Wasserstoff sind, und wobei zwei benachbarte Substituenten, die ausgewählt sind aus der Gruppe bestehend aus $R_1$, $R_2$, $R_3$, $R_4$ und $R_5$, gemeinsam mit den Phenylring-Kohlenstoffatomen, an welche sie angehängt sind, einen Phenyl- oder Methylendioxyring bilden, der an den Phenylring fusioniert ist.

7. Pharmazeutische Zusammensetzung nach Anspruch 1 oder Anspruch 2, wobei vier von $R_1$, $R_2$, $R_3$, $R_4$ und $R_5$ Wasserstoff sind, und wobei eines von $R_1$, $R_2$, $R_3$, $R_4$ und $R_5$ ausgewählt ist aus der Gruppe bestehend aus $C_{1-4}$ -Alkyl, $C_{1-4}$-Alkoxy, Aryl, Aryloxy, Aryl-$C_{1-4}$-Alkyloxy, Heteroaryloxy, Benzolsulfonat, Amino, Hydroxy, Nitro, Trifluormethyl, Trifluormethoxy und Halogen.

8. Pharmazeutische Zusammensetzung nach einem der Ansprüche 1 bis 7, wobei $R_{12}$ Wasserstoff ist und $R_{13}$ ausgewählt ist aus der Gruppe bestehend aus Piperidinyl und Morpholinyl.

9. Pharmazeutische Zusammensetzung nach einem der Ansprüche 1 bis 8, wobei zwei benachbarte Substituenten, die ausgewählt sind aus der Gruppe bestehend aus $R_7$, $R_8$, $R_9$, $R_{10}$ und $R_{11}$, gemeinsam mit den Phenylring-Kohlenstoffatomen, an welche sie angehängt sind, eine Methylendioxygruppe bilden, die an den Phenylring fusioniert ist.

10. Pharmazeutische Zusammensetzung nach Anspruch 1, wobei das 1,2,4-Thiadiazolderivat ausgewählt ist aus der Gruppe bestehend aus:

4-Phenylacetyl-1-[3-(4-fluorbenzyl)-1,2,4-thiadiazol-5-yl]piperazin,
4-(4-Fluorphenylacetyl),1-[3-(4-Fluorbenzyl)-1,2,4-thiadiazol-5-yl]piperazin,
1-(4-Fluorphenylacetyl)-4-[3-(4-methylbenzyl)-1,2,4-thiadiazol-5-yl]-2-methylpiperazin,
1-(4-Methoxyphenylacetyl)-4-[3-(4-fluorbenzyl)-1,2,4-thiadiazol-5-yl]-2-methylpiperazin,
1-Phenylacetyl-4-[3-(4-fluorbenzyl)-1,2,4-thiadiazol-5-yl]-2-methylpiperazin,
1-(4-Fluorphenylacetyl)-4-[3-(4-fluorbenzyl)-1,2,4-thiadiazol-5-yl]-2-methylpiperazin,
1-[Chlor(phenyl)acetyl]-4-[3-(4-fluorbenzyl)-1,2,4-thiadiazol-5-yl]-2-methylpiperazin,
1-(2-Phenylbutanoyl)-4-[3-(4-fluorbenzyl)-1,2,4-thiadiazol-5-yl]-2-methylpiperazin,
N-(3-Fluorphenyl)-4-[3-(4-fluorbenzyl)-1,2,4-thiadiazol-5-yl]piperazin-1-carboxamid,
N-(2-Methylphenyl)-4-[3-(4-fluorbenzyl)-1,2,4-thiadiazol-5-yl]-2-methylpiperazin-1-carboxamid,
N-(4-Ethylphenyl)-4-[3-(4-fluorbenzyl)-1,2,4-thiadiaol-5-yl]-2-methylpiperazin-1-carboxamid,
N-(2-Ethylphenyl)-4-[3-(4-fluorbenzyl)-1,2,4-thiadiazol-5-yl]-2-methylpiperazin-1-carboxamid,
N-Phenyl-4-[3-(4-fluorbenzyl)-1,2,4-thiadiazol-5-yl]piperazin-1-carboxamid,
N-(4-Ethoxyphenyl)-4-[3-(4-fluorbenzyl)-1,2,4-thiadiazol-5-yl]-2-methylpiperazin-1-carboxamid,
N-(2-Methylphenyl)-4-[3-(4-fluorbenzyl)-1,2,4-thiadiazol-5-yl]piperazin-1-carboxamid,
N-(2-fluorphenyl)-4-[3-(4-fluorbenzyl)-1-2,4-thiadiazol-5-yl]piperazin-1-carboxamid,
N-(2-Trifluormethylphenyl)-4-[3-(4-fluorbenzyl)-1,2,4-thiadiazol-5-yl]piperazin-1-carboxamid,
N-(2-Trifluormethylphenyl)-4-[3-(4-fluorbenzyl)-1,2,4-thiadiazol-5-yl]-2-methylpiperazin-1-carboxamid,
N-(2,6-Dimethylphenyl)-4-[3-(4-fluorbenzyl)-1,2,4-thiadiazol-5-yl]-2-methylpiperazin-1-carboxamid,
N-(2,4-Dimethylphenyl)-4-[3-(4-fluorbenzyl)-1,2,4-thiadiazol-5-yl]-2-methylpiperazin-1-carboxamid,
N-(2,6-Dichlorphenyl)-4-[3-(4-fluorbenzyl)-1,2,4-thiadiazol-5-yl]-2-methylpiperazin-1-carboxamid,
N-(3-Cyanophenyl)-4-[3-(4-fluorbenzyl)-1,2,4-thiadiazol-5-yl]-2-methylpiperazin-1-carboxamid,
N-(2,4-Difluorphenyl)-4-[3-(4-fluorbenzyl)-1,2,4-thiadiazol-5-yl]-2-methylpiperazin-1-carboxamid,
N-(2,6-Dimethylphenyl)-4-[3-(4-fluorbenzyl)-1,2,4-thiadiazol-5-yl]piperazin-1-carboxamid,
N-1-Naphtyl-4-[3-(4-fluorbenzyl)-1,2,4-thiadiazol-5-yl]-2-methylpiperazin-1-carboxamid,
N-(3,4-Difluorphenyl)-4-[3-(4-fluorbenzyl)-1,2,4-thiadiazol-5-yl]-2-methylpiperazin-1-carboxamid,
N-(2,4-Dimethoxyphenyl)-4-[3-(4-fluorbenzyl)-1,2,4-thiadiazol-5-yl]piperazin-1-carboxamid, N-(3,4-Difluorphenyl)-4-[3-(4-fluorbenzyl)-1,2,4-thiadiazol-5-yl]piperazin-1-carboxamid,
N-(3,5-Dimethoxyphenyl)-4-[3-(4-fluorbenzyl)-1,2,4-thiadiazol-5-yl]-2-methylpiperazin-1-carboxamid,
1-[4-tert-Butylphenylsulfonyl]-4-[3-(4-fluorbenzyl)-1,2,4-thiadiazol-5-yl]piperazin,
1-[4-Methoxyphenylsulfonyl]-4-[3-(4-chlorbenzyl)-1,2,4-thiadiazol-5-yl]piperazin,
1-[4-Methoxyphenylsulfonyl]-4-[3-(4-fluorbenzyl)-1,2,4-thiadiazol-5-yl]piperazin,
1-[4-Fluorphenylsulfonyl]-4-[3-(4-chlorbenzyl)-1,2,4-thiadiazol-5-yl]piperazin,
1-[4-Chlorphenylsulfonyl]-4-[3-(4-methylbenzyl)-1,2,4-thiadiazol-5-yl]-2-methylpiperazin,
1-[1-Naphtylsulfonyl]-4-[3-(4-methylbenzyl)-1,2,4-thiadiazol-5-yl]piperazin,
1-[2,5-Dichlorphenylsulfonyl]-4-[3-(4-methylbenzyl)-1,2,4-thiadiazol-5-yl]-2-methylpiperazin,
1-[2,4,6-Trimethylphenylsulfonyl]-4-[3-(4-methylbenzyl)-1,2,4-thiadiazol-5-yl]-piperazin,
1-[2-Naphtylsulfonyl]-4-[3-(4-methylbenzyl)-1,2,4-thiadiazol-5-yl]-2-methylpiperazin,
1-[2,5-Dichlorphenylsulfonyl]-4-[3-(4-chlorbenzyl)-1,2,4-thiadiazol-5-yl]-piperazin,
1-[4-Bromphenylsulfonyl]-4-[3-(4-methylbenzyl)-1,2,4-thiadiazol-5-yl]-3-methylpiperazin,
1-[1-Naphtylsulfonyl]-4-[3-(4-methylbenzyl)-1,2,4-thiadiazol-5-yl]-3-methylpiperazin,
1-[4-tert-Butylphenylsulfonyl]-4-[3-(4-methylbenzyl)-1,2,4-thiadiazol-5-yl]-2-methylpiperazin,
1-[1-Naphtylsulfonyl]-4-[3-(4-chlorbenzyl)-1,2,4-thiadiazol-5-yl]-2-methylpiperazin,
1-[2,4,6-Trimethylphenylsulfonyl]-4-[3-(4-methylbenzyl)-1,2,4-thiadiazol-5-yl]-2-methylpiperazin,
1-[3-Trifluorphenylsulfonyl]-4-[3-(4-fluorbenzyl)-1,2,4-thiadiazol-5-yl]-2-methylpiperazin,
1-Phenylsulfonyl-4-[3-(4-fluorbenzyl)-1,2,4-thiadiazol-5-yl]-2-methylpiperazin,
1-[4-Acetamidophenylsulfonyl]-4-[3-(4-methylbenzyl)-1,2,4-thiadiazol-5-yl]-2-methylpiperazin,
1-[4-Acetamidophenylsulfonyl]-4-[3-(4-methylbenzyl)-1,2,4-thiadiazol-5-yl]-piperazin,
1-[4-Methylphenylsulfonyl]-4-[3-(4-methylbenzyl)-1,2,4-thiadiazol-5-yl]-2-methylpiperazin,
1-[4-Methoxyphenylsulfonyl]-4-[3-(4-chlorbenzyl)-1,2,4-thiadiazol-5-yl]-2-methylpiperazin,
1-[2-Naphtylsulfonyl]-4-[3-(4-chlorbenzyl)-1,2,4-thiadiazol-5-yl]piperazin,
1-[4-tert-Butylphenylsulfonyl]-4-[3-(4-fluorbenzyl)-1,2,4-thiadiazol-5-yl]-2-methylpiperazin,
1-[4-Acetamidophenylsulfonyl]-4-[3-(4-fluorbenzyl)-1,2,4-thiadiazol-5-yl]-2-methylpiperazin,
1-[3-Trifluormethylphenylsulfonyl]-4-[3-(4-methylbenzyl)-1,2,4-thiadiazol-5-yl]-2-methylpiperazin,
1-[4-Fluorphenylsulfonyl]-4-[3-(4-methylbenzyl)-1,2,4-thiadiazol-5-yl-]piperazin,
1-[4-Fluorphenylsulfonyl]-4-[3-(4-methylbenzyl)-1,2,4-thiadiazol-5-yl]-2-methylpiperazin,
1-[4-Fluorphenylsulfonyl]-4-[3-(4-fluorbenzyl)-1,2,4-thiadiazol-5-yl]-2-methylpiperazin,
1-[4-Methoxyphenylsulfonyl]-4-[3-(4-methylbenzyl)-1,2,4-thiadiazol-5-yl]-2-methylpiperazin,
1-[4-tert-Butylphenylsulfonyl]-4-[3-(4-fluorbenzyl)-1,2,4-thiadiazol-5-yl]piperazin,
1-[3-Trifluormethylphenylsulfonyl]-4-[3-(4-methylbenzyl)-1,2,4-thiadiazol-5-yl]-piperazin,
1-[4-Methylphenylsulfonyl]-4-[3-(4-chlorbenzyl)-1,2,4-thiadiazol-5-yl]piperazin,
1-[4-Bromphenylsulfonyl]-4-[3-(4-methylbenzyl)-1,2,4-thiadiazol-5-yl]piperazin,
1-[2-Naphtylsulfonyl]-4-[3-(4-methylbenzyl)-1,2,4-thiadiazol-5-yl]piperazin,
1-[4-Chlorphenylsulfonyl]-4-[3-(4-methylbenzyl)-1,2,4-thiadiazol-5-yl]piperazin,
1-[4-tert-Butylphenylsulfonyl]-4-[3-(4-methylbenzyl)-1,2,4-thiadiazol-5-yl]-piperazin,
1-[4-Methoxyphenylsulfonyl]-4-[3-(4-methylbenzyl)-1,2,4-thiadiazol-5-yl]piperazin,
1-[4-tert-Butylphenylsulfonyl]-4-[3-(4-chlorbenzyl)-1,2,4-thiadiazol-5-yl]piperazin,
1-[Chinolin-8-sulfonyl]-4-[3-(4-methylbenzyl)-1,2,4-thiadiazol-5-yl]piperazin,
1-[4-Nitrophenylsulfonyl]-4-[3-(4-methylbenzyl)-1,2,4-thiadiazol-5-yl]-2-methylpiperazin,
1-[3-Nitro-4-chlorphenylsulfonyl]-4-[3-(4-methylbenzyl)-1,2,4-thiadiazol-5-yl]-2-methylpiperazin,
1-[4-Nitrophenylsulfonyl]-4-[3-(4-fluorbenzyl)-1,2,4-thiadiazol-5-yl]-2-methylpiperazin,
1-[4-Methoxyphenylsulfonyl]-4-[3-(4-fluorbenzyl)-1,2,4-thiadiazol-5-yl]piperazin,
1-[3-Nitro-4-chlorphenylsulfonyl]-4-[3-(4-methylbenzyl)-1,2,4-thiadiazol-5-yl]-piperazin
1-(Benzylsulfonyl)-4-[3-(4-chlorbenzyl)-1,2,4-thiadiazol-5-yl]piperazin,
1-(Benzylsulfonyl)-4-[3-(4-methylbenzyl)-1,2,4-thiadiazol-5-yl]piperazin,
1-(Phenylprop-2-ensulfonyl)-4-[3-(4-fluorbenzyl)-1,2,4-thiadiazol-5-yl]piperazin,
1-(Phenylprop-2-ensulfonyl)-4-[3-(4-methylbenzyl)-1,2,4-thiadiazol-5-yl]-2-methylpiperazin,
1-(Phenylprop-2-ensulfonyl)-4-[3-(4-methylbenzyl)-1,2,4-thiadiazol-5-yl]piperazin,
1-(Butylsulfonyl)-4-[3-(4-chlorbenzyl)-1,2,4-thiadiazol-5-yl]piperazin,
1-(Octylsulfonyl)-4-[3-(4-methylbenzyl)-1,2,4-thiadiazol-5-yl]piperazin,
1-(Butylsulfonyl)-4-[3-(4-methylbenzyl)-1,2,4-thiadiazol-5-yl]piperazin,
1-(Ethylsulfonyl)-4-[3-(4-chlorbenzyl)-1,2,4-thiadiazol-5-yl]piperazin,
1-(Isopropylsulfonyl)-4-[3-(4-methylbenzyl)-1,2,4-thiadiazol-5-yl]piperazin,
1-[(2E)-3-Phenylprop-2-enoyl]-4-[3-(4-chlorbenzyl)-1,2,4-thiadiazol-5-yl]-2-methylpiperazin,
1-[(2E)-3-Phenylprop-2-enoyl]-4-B103-2-methylpiperazin,

1-[(2E)-3-Phenylprop-2-enoyl]-4-[3-(4-chlorbenzyl)-1,2,4-thiadiazol-5-yl]-piperazin,
1-(3-Phenylpropanoyl)-4-[3-(4-fluorbenzyl)-1,2,4-thiadiazol-5-yl]-2-methylpiperazin,
1-(3-Phenylpropanoyl)-4-[3-(4-fluorbenzyl)-1,2,4-thiadiazol-5-yl]piperazin,
1-[3-Phenylprop-2-enyl]-4-[3-(4-methylbenzyl)-1,2,4-thiadiazol-5-yl]-piperazin,
1-[3-Phenylprop-2-enyl]-4-[3-(4-chlorbenzyl)-1,2,4-thiadiazol-5-yl]piperazin,
1-[3-Phenylprop-2-enyl]-4-[3-(4-fluorbenzyl)-1,2,4-thiadiazol-5-yl]piperazin,
4-Pentyl-N-{2-[4-[3-(4-fluorbenzyl)-1,2,4-thiadiazol--5-yl]piperazin-1-yl]ethyl}-benzamid,
4-Butyl-N-{2-[4-[3-(4-fluorbenzyl)-1,2,4-thiadiazol-5-yl]piperazin-1-yl]ethyl}-benzamid,
4-Hexyl-N-{2-[4-[3-(4-fluorbenzyl)-1,2,4-thiadiazol-5-yl]piperazin-1-yl]ethyl}-benzamid,
4-Chlor-N-{2-[4-[3-(4-fluorbenzyl)-1,2,4-thiadiazol-5-yl]piperazin-l-yl]ethy- 1}-benzamid,
3,5-Dichlor-N-[2-{4-[3-(4-fluorbenzyl)-1,2,4-thiadiazol-5-yl]piperazin-1-yl]ethyl}-benzamid,
2-Methyl-N-{2-[4-[3-(4-fluorbenzyl)-1,2,4-thiadiazol-5-yl]piperazin-1-yl]ethyl}-benzamid,
3-Fluor-N-{2-[4-[3-(4-chlorbenzyl)-1,2,4-thiadiazol-5-yl]piperazin-1-yl]ethyl}-benzamid,
4-Methyl-N-{2-[4-[3-(4-fluorbenzyl)-1,2,4-thiadiazol-5-yl]piperazin-1-yl]ethyl}-benzamid,
4-Fluor-N-{2-[4-[3-(4-fluorbenzyl)-1,2,4-thiadiazol-5-yl]piperazin-1-yl]ethyl}-benzamid,
4-Ethyl-N-{2-[4-[3-(4-fluorbenzyl)-1,2,4-thiadiazol-5-yl]piperazin-1-yl]ethyl}-benzamid,
N-{2-[4-[3-(4-Fluorbenzyl)-1,2,4-thiadiazol-5-yl]piperazin-1-yl]ethyl}benzamid,
3-Fluor-N-{2-[4-[3-(4-fluorbenzyl)-1,2,4-thiadiazol-5-yl]piperazin-1-yl]ethyl}-benzamid,
3-Fluor-N-{2-[4-[3-benzyl-1,2,4-thiadiazol-5-yl]piperazin-1-yl]ethyl}benzamid,
2-(4-Fluorphenyl)-N-{2-[4-[3-(4-fluorbenzyl)-1,2,4-thiadiazol-5-yl]piperazin-1-yl]ethyl}acetamid,
2-Phenyl-N-{2-[4-[3-(4-fluorbenzyl)-1,2,4-thiadiazol-5-yl]piperazin-1-yl]ethyl}-acetamid,
2-(4-Fluorphenyl)-N-{2-[4-[3-(4-methylbenzyl)-1,2,4-thiadiazol-5-yl]piperazin-1-yl]ethyl}acetamid,
N-Benzyl-N'-{2-[4-[3-(4-fluorbenzyl)-1,2,4-thiadiazol-5-yl]piperazin-1-yl]ethyl}harnstoff,
2-Phenyl-N-{2-[4-[3-(4-fluorbenzyl)-1,2,4-thiadiazol-5-yl]piperazin-1-yl]ethyl}-butanamid,
3-Phenyl-N-{2-[4-[3-(4-fluorbenzyl)-1,2,4-thiadiazol-5-yl]piperazin-1-yl]ethyl}-propanamid,
1-Benzyl-4-[3-(4-fluorbenzyl)-1,2,4-thiadiazol-5-yl]piperazin,
1-(1,3-Benzodioxol-5-ylmethyl)-4-[3-(4-fluorbenzyl)-1,2,4-thiadiazol-5-yl]-piperazin,
1-(2-Fluorbenzoyl)-4-[3-(3-methoxybenzyl)-1,2,4-thiadiazol-5-yl]-2-methylpiperazin,
1-(4-Ethylbenzoyl)-4-[3-(4-fluorbenzyl)-1,2,4-thiadiazol-5-yl]-2-methylpiperazin,
1-(4-Butylbenzoyl)-4-[3-(4-methylbenzyl)-1,2,4-thiadiazol-5-yl]-2-methylpiperazin,
1-(4-Methoxybenzoyl)-4-[3-(4-fluorbenzyl)-1,2,4-thiadiazol-5-yl]-2-methylpiperazin,
1-(1,3-Benzodioxol-5-ylcarbonyl)-4-[3-(3-methoxybenzyl)-1,2,4-thiadiazol-5-yl]-2-methylpiperazin,
1-(4-Butylbenzoyl)-4-[3-(4-fluorbenzyl)-1,2,4-thiadiazol-5-yl]-2-methylpiperazin,
1-(4-Hexylbenzoyl)-4-[3-(4-fluorbenzyl)-1,2,4-thiadiazol-5-yl]piperazin,
1-(3-Chlorbenzoyl)-4-[3-(4-fluorbenzyl)-1,2,4-thiadiazol-5-yl]-2-methylpiperazin,
1-(4-Fluorbenzoyl)-4-[3-(3-methoxybenzyl)-1,2,4-thiadiazol-5-yl]-2-methylpiperazin,
1-(4-Methylbenzoyl)-4-[3-(3-methoxybenzyl)-1,2,4-thiadiazol-5-yl]-2-methylpiperazin,
1-(3-Fluorbenzoyl)-4-[3-(4-chlorbenzyl)-1,2,4-thiadiazol-5-yl]-2-methylpiperazin,
1-Benzoyl-4-[3-(4-fluorbenzyl)-1,2,4-thiadiazol-5-yl]-2-methylpiperazin,
1-(4-Fluorbenzoyl)-4-[3-(4-fluorbenzyl)-1,2,4-thiadiazol-5-yl]-2-methylpiperazin,
1-(4-tert-Butylbenzoyl)-4-[3-(4-fluorbenzyl)-1,2,4-thiadiazol-5-yl]-2-methylpiperazin,
1-(1,1'-Biphenyl-4-ylcarbonyl)-4-[3-(4-fluorbenzyl)-1,2,4-thiadiazol-5-yl]-2-methylpiperazin,
1-(4-Methoxybenzoyl)-4-[3-(4-methylbenzyl)-1,2,4-thiadiazol-5-yl]-2-methylpiperazin,
1-(4-Ethylbenzoyl)-4-[3-(4-methylbenzyl)-1,2,4-thiadiazol-5-yl]piperazin,
1-(2-Naphthoyl)-4-[3-(4-fluorbenzyl)-1,2,4-thiadiazol-5-yl]-2-methylpiperazin,
1-(2-Methoxybenzoyl)-4-[3-(4-fluorbenzyl)-1,2,4-thiadiazol-5-yl]-2-methylpiperazin,
1-(4-Pentylbenzoyl)-4-[3-(4-methylbenzyl)-1,2,4-thiadiazol-5-yl]-2-methylpiperazin,
1-(4-Bromobenzoyl)-4-[3-(4-fluorbenzyl)-1,2,4-thiadiazol-5-yl]-2-methylpiperazin,
1-(2,4-Dimethoxybenzoyl)-4-[3-(4-fluorbenzyl)-1,2,4-thiadiazol-5-yl]-2-methylpiperazin,
1-(3,5-Dichlorbenzoyl)-4-[3-(4-fluorbenzyl)-1,2,4-thiadiazol-5-yl]piperazin,
1-(3-Chlorbenzoyl)-4-[3-(4-fluorbenzyl)-1,2,4-thiadiazol-5-yl]piperazin,
1-(4-Methylbenzoyl)-4-[3-(4-fluorbenzyl)-1,2,4-thiadiazol-5-yl]-2-methylpiperazin,
1-(2-Methylbenzoyl)-4-[3-(4-fluorbenzyl)-1,2,4-thiadiazol-5-yl]-2-methylpiperazin,
1-(4-Methylbenzoyl)-4-[3-(4-fluorbenzyl)-1,2,4-thiadiazol-5-yl]piperazin,
1-(3-Bromobenzoyl)-4-[3-(4-fluorbenzyl)-1,2,4-thiadiazol-5-yl]-2-methylpiperazin,
1-(4-Ethylbenzoyl)-4-[3-(3-methoxybenzyl)-1,2,4-thiadiazol-5-yl]-2-methylpiperazin,
1-(3-Methylbenzoyl)-4-[3-(4-methylbenzyl)-1,2,4-thiadiazol-5-yl]-2-methylpiperazin,
1-(3-Trifluormethylbenzoyl)-4-[3-(4-fluorbenzyl)-1,2,4-thiadiazol-5-yl]-2-methylpiperazin,

1-(4-tert-Butylbenzoyl)-4-[3-(3-methoxybenzyl)-1,2,4-thiadiazol-5-yl]-2-methylpiperazin,
1-(4-Ethylbenzoyl)-4-[3-(4-methylbenzyl)-1,2,4-thiadiazol-5-yl]-2-methylpiperazin,
1-(2-Bromobenzoyl)-4-[3-(4-fluorbenzyl)-1,2,4-thiadiazol-5-yl]-2-methylpiperazin,
1-(2-Fluorbenzoyl)-4-[3-(4-methylbenzyl)-1,2,4-thiadiazol-5-yl]-2-methylpiperazin,
1-(3-Fluorbenzoyl)-4-[3-benzyl-1,2,4-thiadiazol-5-yl]-2-methylpiperazin,
1-(4-Fluorbenzoyl)-4-[3-(4-methylbenzyl)-1,2,4-thiadiazol-5-yl]-2-methylpiperazin,
1-(2-Methylbenzoyl)-4-[3-(4-fluorbenzyl)-1,2,4-thiadiazol-5-yl]piperazin,
1-(4-Fluorbenzoyl)-4-[3-(4-chlorbenzyl)-1,2,4-thiadiazol-5-yl]piperazin,
1-(4-Fluorbenzoyl)-4-[3-benzyl-1,2,4-thiadiazol-5-yl]-2-methylpiperazin,
1-(4-Chlorbenzoyl)-4-[3-(4-methylbenzyl)-1,2,4-thiadiazol-5-yl]piperazin,
1-(3-Fluorbenzoyl)-4-[3-(4-methylbenzyl)-1,2,4-thiadiazol-5-yl]-2-methylpiperazin,
1-(3-Fluorbenzoyl)-4-[3-(4-chlorbenzyl)-1,2,4-thiadiazol-5-yl]piperazin,
1-Benzoyl-4-[3-(4-methylbenzyl)-1,2,4-thiadiazol-5-yl]piperazin,
1-(2-Fluorbenzoyl)-4-[3-(4-chlorbenzyl)-1,2,4-thiadiazol-5-yl]piperazin,
1-(4-Fluorbenzoyl)-4-[3-(4-methylbenzyl)-1,2,4-thiadiazol-5-yl]piperazin,
1-(1,3-Benzodioxol-5-ylcarbonyl)-4-[3-(4-methylbenzyl)-1,2,4-thiadiazol-5-yl]-2-methylpiperazin,
1-(3-Fluorbenzoyl)-4-[3-(4-methylbenzyl)-1,2,4-thiadiazol-5-yl]piperazin,
1-(4-Fluorbenzoyl)-4-[3-benzyl-1,2,4-thiadiazol-5-yl]piperazin,
1-(4-Bromobenzoyl)-4-[3-(4-methylbenzyl)-1,2,4-thiadiazol-5-yl]piperazin,
1-(4-Ethylbenzoyl)-4-[3-(4-chlorbenzyl)-1,2,4-thiadiazol-5-yl]piperazin,
1-(2-Chlorbenzoyl)-4-[3-(4-fluorbenzyl)-1,2,4-thiadiazol-5-yl]-2-methylpiperazin,
1-(2-Fluorbenzoyl)-4-[3-(4-chlorbenzyl)-1,2,4-thiadiazol-5-yl]-2-methylpiperazin,
1-(4-Trifluormethylbenzoyl)-4-[3-(3-methoxybenzyl)-1,2,4-thiadiazol-5-yl]-2-methylpiperazin,
1-Benzoyl-4-[3-(4-methylbenzyl)-1,2,4-thiadiazol-5-yl]-2-methylpiperazin,
1-(4-Bromobenzoyl)-4-[3-(3-methoxybenzyl)-1,2,4-thiadiazol-5-yl]-2-methylpiperazin,
1-(4-Trifluormethylbenzoyl)-4-[3-(4-methylbenzyl)-1,2,4-thiadiazol-5-yl]piperazin,
1-(3-Nitro-4-methylbenzoyl)-4-[3-(4-fluorbenzyl)-1,2,4-thiadiazol-5-yl]-2-methylpiperazin,
1-Benzoyl-4-[3-(3-methoxybenzyl)-1,2,4-thiadiazol-5-yl]-2-methylpiperazin,
1-(4-Chlorbenzoyl)-4-[3-(3-methoxybenzyl)-1,2,4-thiadiazol-5-yl]-2-methylpiperazin,
1-(2-Fluorbenzoyl)-4-[3-(4-methylbenzyl)-1,2,4-thiadiazol-5-yl]piperazin,
1-(4-hexylbenzoyl)-4-[3-(4-methylbenzyl)-1,2,4-thiadiazol-5-yl]piperazin,
1-(2-Chlor-4-nitrobenzoyl)-4-[3-(4-fluorbenzyl)-1,2,4-thiadiazol-5-yl]-2-methylpiperazine,
1-(1,3-Benzodioxol-5-ylcarbonyl)-4-[3-(4-fluorbenzyl)-1,2,4-thiadiazol-5-yl]-2-methylpiperazin,
1-(3-Fluorbenzoyl)-4-[3-(4-fluorbenzyl)-1,2,4-thiadiazol-5-yl]-2-methylpiperazin,
1-(4-tert-Butylbenzoyl)-4-[3-(4-fluorbenzyl)-1,2,4-thiadiazol-5-yl]piperazin,
1-Benzoyl-4-[3-(4-fluorbenzyl)-1,2,4-thiadiazol-5-yl]piperazin,
1-(4-Butylbenzoyl)-4-[3-(4-methylbenzyl)-1,2,4-thiadiazol-5-yl]piperazin,
1-(4-tert-Butylbenzoyl)-4-[3-(4-methylbenzyl)-1,2,4-thiadiazol-5-yl]piperazin,
1-(4-Nitrobenzoyl)-4-[3-(4-fluorbenzyl)-1,2,4-thiadiazol-5-yl]-2-methylpiperazin,
1-(2-Methylphenyl)-4-[3-(4-fluorbenzyl)-1,2,4-thiadiazol-5-yl]piperazin,
1-(2-Ethoxyphenyl)-4-[3-(4-fluorbenzyl)-1,2,4-thiadiazol-5-yl]piperazin,
1-(2-fluorphenyl)-4-[3-(4-fluorbenzyl)-1,2,4-thiadiazol-5-yl]piperazin,
1-(3-Trifluormethylphenyl)-4-[3-(4-fluorbenzyl)-1,2,4-thiadiazol-5-yl]piperazin,
1-(4-Chlorbenzoyl)-4-[3-(4-fluorbenzyl)-1,2,4-thiadiazol-5-yl]-1,4-diazepan,
1-(4-Methylbenzoyl)-4-[3-(4-fluorbenzyl)-1,2,4-thiadiazol-5-yl]-1,4-diazepan,
1-(1,3-Benzodioxol-5-ylcarbonyl)-4-[3-(4-fluorbenzyl)-1,2,4-thiadiazol-5-yl]-1,4-diazepan,
1-[4-Methoxyphenylsulfonyl]-4-[3-(3-fluorbenzyl)-1,2,4-thiadiazol-5-yl]piperazin;
1-[4-Methoxyphenylsulfonyl]-4-[3-(2-fluorbenzyl)-1,2,4-thiadiazol-5-yl]piperazin;
1-[2-Methoxyphenylsulfonyl]-4-[3-(4-fluorbenzyl)-1,2,4-thiadiazol-5-yl]piperazin;
1-[4-Ethoxyphenylsulfonyl]-4-[3-(4-fluorbenzyl)-1,2,4-thiadiazol-5-yl]piperazin;
1-[4-Ethylphenylsulfonyl]-4-[3-(4-fluorbenzyl)-1,2,4-thiadiazol-5-yl]piperazin;
1-Phenylsulfonyl-4-[3-(4-fluorbenzyl)-1,2,4-thiadiazol-5-yl]piperazin;
1-[4-Methoxyphenylsulfonyl]-4-[3-benzyl-1,2,4-thiadiazol-5-yl]piperazin;
1-(4-Propionyloxy)benzol-sulfonyl-4-[3-(4-fluorbenzyl)-1,2,4-thiadiazol-5-yl]-piperazin;
1-[4-Methoxyphenylsulfonyl]-4-[3-(3-methylbenzyl-1,2,4-thiadiazol-5-yl]piperazin;
1-[4-Methoxyphenylsulfonyl]-4-[3-(2-methylbenzyl-1,2,4-thiadiazol-5-yl]piperazin;
1-[4-Methoxyphenylsulfonyl]-4-[3-(2-methoxy-benzyl-1,2,4-thiadiazol-5-yl]piperazin;
1-[4-Methoxyphenylsulfonyl]-4-[3-(3-methyl-4-fluorbenzyl-1,2,4-thiadiazol-5-yl]-piperazin;

1-[4-Methoxyphenylsulfonyl]-4-[3-(4-methoxybenzyl-1,2,4-thiadiazol-5-yl]piperazin;
1-[4-Bromophenylsulfonyl]-4-[3-(3-methoxybenzyl-1,2,4-thiadiazol-5-yl]piperazin;
1-[4-Methoxyphenylsulfonyl]-4-[3-(3-methoxybenzyl)-1,2,4-thiadiazol-5-yl]-2-methylpiperazin; und
1-(4-Hydroxybenzolsulfonyl)-4-[3-(4-fluorbenzyl)-1,2,4-thiadiazol-5-yl]piperazin.

**11.** 1,2,4-Thiadiazolderivat, das ausgewählt ist aus der Gruppe bestehend aus:

- 1-[3-Methoxyphenylsulfonyl]-4-[3-(4-fluorbenzyl)-1,2,4-thiadiazol-5-yl]piperazin;
- 1-[4-Methoxyphenylsulfonyl]-4-[3-(3-methoxybenzyl)-1,2,4-thiadiazol-5-yl]piperazin;
- 1-[4-Methoxyphenylsulfonyl]-4-[3-(3-fluorbenzyl)-1,2,4-thiadiazol-5-yl]piperazin;
- 1-[4-Methoxyphenylsulfonyl]-4-[3-(2-fluorbenzyl)-1,2,4-thiadiazol-5-yl]piperazin;
- 1-[2-Methoxyphenylsulfonyl]-4-[3-(4-fluorbenzyl)-1,2,4-thiadiazol-5-yl]piperazin;
- 1-[4-Ethoxyphenylsulfonyl]-4-[3-(4-fluorbenzyl)-1,2,4-thiadiazol-5-yl]piperazin;
- 1-[4-Ethylphenylsulfonyl]-4-[3-(4-fluorbenzyl)-1,2,4-thiadiazol-5-yl]piperazin;
- 1-Phenylsulfonyl-4-[3-(4-fluorbenzyl)-1,2,4-thiadiazol-5-yl]piperazin;
- 1-[4-Methoxyphenylsulfonyl]-4-[3-benzyl-1,2,4-thiadiazol-5-yl]piperazin;
- 1-(4-Propionyloxy)benzol-sulfonyl-4-[3-(4-fluorbenzyl)-1,2,4-thiadiazol-5-yl]-piperazin;
- 1-[4-Methoxyphenylsulfonyl]-4-[3-(3-methylbenzyl-1,2,4-thiadiazol-5-yl]piperazin;
- 1-[4-Methoxyphenylsulfonyl]-4-[3-(2-methylbenzyl-1,2,4-thiadiazol-5-yl]piperazin;
- 1-[4-Methoxyphenylsulfonyl]-4-[3-(2-methoxybenzyl-1,2,4-thiadiazol-5-yl]piperazin;
- 1-[4-Methoxyphenylsulfonyl]-4-[3-(3-methyl-4-fluorbenzyl-1,2,4-thiadiazol-5-yl]-piperazin;
- 1-[4-Methoxyphenylsulfonyl]-4-[3-(4-methoxybenzyl-1,2,4-thiadiazol-5-yl]piperazin;
- 1-[4-Bromophenylsulfonyl]-4-[3-(3-methoxybenzyl-1,2,4-thiadiazol-5-yl]piperazin;
- 1-[4-Methoxyphenylsulfonyl]-4-[3-(3-methoxybenzyl)-1,2,4-thiadiazol-5-yl]-2-methylpiperazin; und
- 1-(4-Hydroxybenzolsulfonyl)-4-[3-(4-fluorbenzyl)-1,2,4-thiadiazol-5-yl]piperazin.

**12.** Pharmazeutische Zusammensetzung nach einem der Ansprüche 1 bis 10, des Weiteren umfassend eine therapeutische Menge eines oder mehrerer Arzneimittel, die ausgewählt sind aus der Gruppe bestehend aus neuroprotektiven Mitteln und Inhibitoren einer α-Synuclein-Ablagerung.

**13.** 1,2,4-Thiadiazolderivat nach einem der Ansprüche 1 bis 10 zur Verwendung in der Behandlung einer α-Synucleopathie.

**14.** 1,2,4-Thiadiazolderivat nach Anspruch 13, wobei die α-Synucleopathie ausgewählt ist aus der Gruppe bestehend aus Parkinson, Lewy-Körperchen Demenz, traumatischer Gehirnverletzung, amyotropher lateraler Sklerose, Niemann-Pick-Krankheit, Hallervorden-Spatz-Syndrom, Down-Syndrom, neuroaxonaler Dystrophie, multipler Systematrophie und Alzheimer Krankheit.

## Revendications

**1.** Composition pharmaceutique comprenant une quantité thérapeutiquement efficace d'un dérivé 1,2,4-thiadiazole selon la formule développée (A)

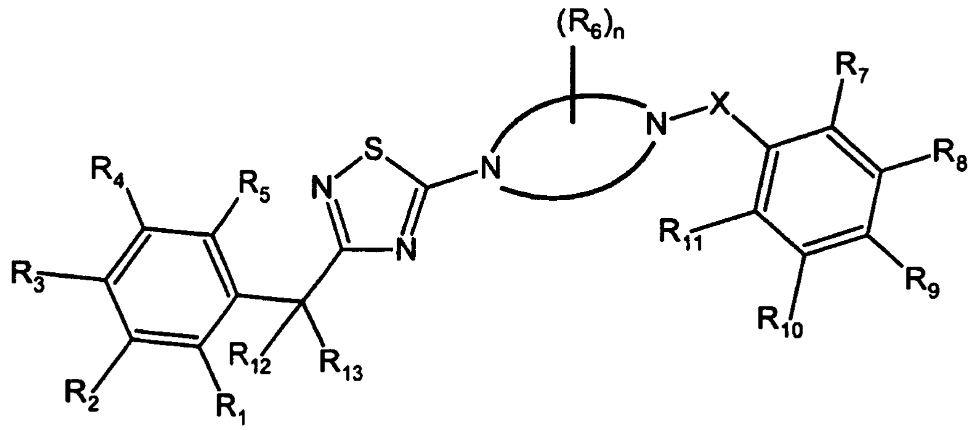

(A)

dans laquelle le groupe divalent représenté de manière schématique par la formule développée (A')

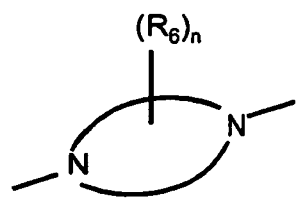

(A')

inclut un cycle hétérocyclique saturé ou partiellement insaturé, éventuellement mono-substitué ou poly-substitué ayant au moins deux atomes d'azote dans ledit cycle hétérocyclique et ayant un total de 5 à 7 atomes dans ledit cycle hétérocyclique, et dans lequel, en outre :

- $R_6$ représente un substituant choisi de manière indépendante dans le groupe constitué par un groupe oxo et un groupe alkyle en $C_{1-4}$ ;
- n est choisi dans le groupe constitué par 0, 1, 2 et 3 ;
- $R_1$, $R_2$, $R_3$, $R_4$ et $R_5$ sont choisis chacun de manière indépendante dans le groupe constitué par un atome d'hydrogène, un groupe alkyle en $C_{1-4}$, alcoxy en $C_{1-4}$, aryle, aryloxy, arylalkyloxy en $C_{1-4}$, hétéroaryloxy, benzènesulfonate, amino, hydroxy, nitro, trifluorométhyle, trifluorométhoxy et un atome d'halogène, ou bien n'importe lesquels de deux substituants adjacents choisis dans le groupe constitué par $R_1$, $R_2$, $R_3$, $R_4$ et $R_5$ forment, conjointement avec les atomes de carbone du cycle phényle auxquels ils sont attachés, un cycle saturé ou insaturé condensé audit cycle phényle et ayant entre 5 et 7 membres composant le cycle, ledit cycle saturé ou insaturé comprenant éventuellement un ou deux atomes d'oxygène et étant éventuellement substitué par un ou plusieurs atomes d'halogène ;
- $R_7$, $R_8$, $R_9$, $R_{10}$ et $R_{11}$ sont choisis chacun de manière indépendante dans le groupe constitué par un atome d'hydrogène, un groupe alkyle en $C_{1-10}$, alcoxy en $C_{1-6}$, aryle, hydroxy, acétyle, nitro, trifluorométhyle et un atome d'halogène ; ou bien n'importe lesquels de deux substituants adjacents choisis dans le groupe constitué par $R_7$, $R_8$, $R_9$, $R_{10}$ et $R_{11}$ forment, conjointement avec les atomes de carbone d'un cycle phényle auxquels ils sont attachés, un cycle saturé ou insaturé condensé audit cycle phényle et ayant entre 5 et 7 membres composant le cycle, ledit cycle saturé ou insaturé comprenant éventuellement un ou deux hétéroatomes choisis de manière indépendante dans le groupe constitué par un atome d'oxygène, un atome de soufre et un atome d'azote ; et chacun desdits groupes alkyle en $C_{1-6}$, alcoxy en $C_{1-6}$, aryle ou cycle condensé est éventuellement substitué par un ou plusieurs atomes d'halogène ;
- $R_{12}$ et $R_{13}$ sont choisis chacun de manière indépendante dans le groupe constitué par un atome d'hydrogène, un groupe alkyle en $C_{1-6}$, arylalkyle en $C_{1-4}$, aryle et des cycles hétérocycliques azotés, ou bien $R_{12}$ et $R_{13}$ forment conjointement un groupe cycloalkyle en $C_{3-6}$ ou un groupe hétérocyclique ; et
- X représente un segment de liaison choisi dans le groupe constitué par une liaison simple ; -C(=O)- ; -S(=O)$_2$- ;

des groupes hydrocarbonés non cycliques saturés divalents comprenant entre 1 et 6 atomes dans la chaîne principale, chacun desdits atomes de la chaîne principale étant choisi de manière indépendante dans le groupe constitué par un atome de carbone, un atome d'azote et un atome de soufre, et chacun desdits atomes de carbone de la chaîne principale étant éventuellement substitué par un ou plusieurs substituants choisi (s) de manière indépendante dans le groupe constitué par un groupe oxo, thioxo, alkyle en $C_{1-4}$ et un atome d'halogène, à la condition que le nombre d'hétéroatomes de la chaîne principale dudit groupe hydrocarboné non cyclique saturé divalent ou insaturé soit égal à 0, 1 ou 2 ; et des groupes hétérocycliques saturés ou insaturés divalents comprenant entre 2 et 6 atomes de carbone et entre 1 et 3 hétéroatomes choisis de manière indépendante dans le groupe constitué par un atome d'oxygène, un atome de soufre et un atome d'azote dans ledit groupe hétérocyclique ; ou bien X, conjointement avec l'un parmi $R_7$ et $R_{11}$ forme un cycle saturé ou insaturé ayant entre 5 et 7 membres composant le cycle et étant condensé au cycle phényle portant ledit un parmi $R_7$ et $R_{11}$, ledit cycle saturé ou insaturé comprenant éventuellement un ou deux hétéroatomes choisis de manière indépendante dans le groupe constitué par un atome d'oxygène, un atome de soufre et un atome d'azote, et ledit cycle saturé ou insaturé comprenant éventuellement un ou plusieurs substituants choisis de manière indépendante dans le groupe constitué par un groupe alkyle en $C_{1-4}$ et un groupe trifluorométhyle ou est choisi dans le groupe constitué par -CO-CH=CH-, $-CH_2$-CH=CH- et $-SO_2$-CH=CH- ;

ou un stéréoisomère ou un solvate de celui-ci, ou un sel pharmaceutiquement acceptable de celui-ci, en association avec un ou plusieurs excipients pharmaceutiquement acceptables.

**2.** Composition pharmaceutique selon la revendication 1, dans laquelle le dérivé 1,2,4- thiadiazole a la formule développée (B)

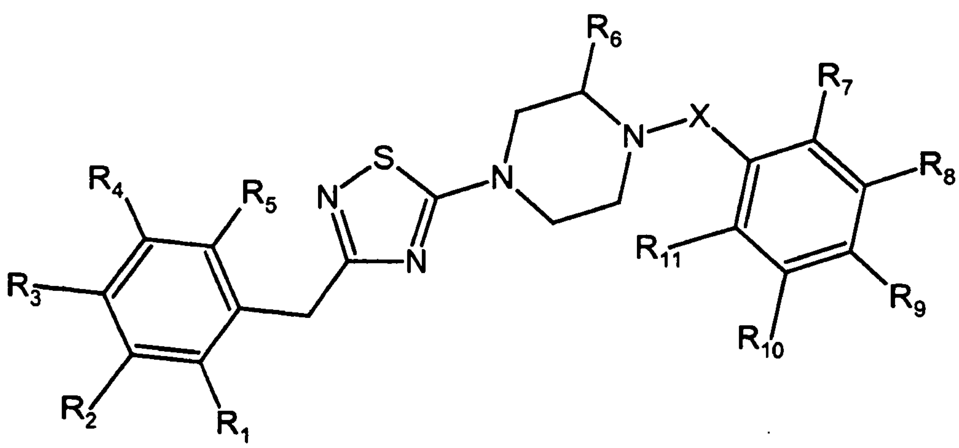

(B)

dans laquelle X, $R_1$, $R_2$, $R_3$, $R_4$, $R_5$, $R_6$, $R_7$, $R_8$, $R_9$, $R_{10}$ et $R_{11}$ sont tels que définis dans la revendication 1, ou un stéréoisomère ou un solvate de celui-ci, ou un sel pharmaceutiquement acceptable de celui-ci.

**3.** Composition pharmaceutique selon la revendication 1 ou la revendication 2, dans laquelle X est un groupe hydrocarboné saturé ou insaturé divalent comprenant entre 1 et 3 atomes de carbone mais pas d'hétéroatome dans la chaîne principale, et dans laquelle chacun parmi lesdits atomes de carbone de la chaîne principale est éventuellement substitué par un ou plusieurs substituants choisis de manière indépendante dans le groupe constitué par un groupe oxo, un groupe thioxo, un groupe alkyle en $C_{1-4}$ et un atome d'halogène.

**4.** Composition pharmaceutique selon la revendication 1 ou la revendication 2, dans laquelle X est choisi dans le groupe constitué par $-CO-CH_2-$, $-CO-(CH_2)_2-$, $CO-CHR_{14}-$, et -CO-CHX'-, dans laquelle $R_{14}$ représente un groupe alkyle en $C_{1-4}$ et dans laquelle X' représente un atome d'halogène.

**5.** Composition pharmaceutique selon la revendication 1 ou la revendication 2, dans laquelle X représente un groupe hydrocarboné saturé ou insaturé divalent comprenant entre 1 et 5 atomes de carbone et un seul atome d'azote, d'oxygène ou de soufre dans la chaîne principale, et dans laquelle chacun desdits atomes de carbone de la chaîne

principale est éventuellement substitué par un ou plusieurs substituants choisis de manière indépendante dans le groupe constitué par un groupe oxo, un groupe thioxo et un groupe alkyle en $C_{1-4}$.

**6.** Composition pharmaceutique selon la revendication 1 ou la revendication 2, dans laquelle trois parmi $R_1$, $R_2$, $R_3$, $R_4$ et $R_5$ sont un atome d'hydrogène, et dans laquelle deux substituants adjacents choisis dans le groupe constitué par $R_1$, $R_2$, $R_3$, $R_4$ et $R_5$ forment, conjointement avec les atomes de carbone du cycle phényle auxquels ils sont attachés, un cycle phényle ou méthylènedioxy condensé audit cycle phényle.

**7.** Composition pharmaceutique selon la revendication 1 ou la revendication 2, dans laquelle quatre parmi $R_1$, $R_2$, $R_3$, $R_4$ et $R_5$ sont un atome d'hydrogène, et dans laquelle un parmi $R_1$, $R_2$, $R_3$, $R_4$ et $R_5$ est choisi dans le groupe constitué par un groupe alkyle en $C_{1-4}$, alcoxy en $C_{1-4}$, aryle, aryloxy, arylalkyloxy en $C_{1-4}$, hétéroaryloxy, benzènesulfonate, amino, hydroxy, nitro, trifluorométhyle, trifluorométhoxy et un atome d'halogène.

**8.** Composition pharmaceutique selon l'une quelconque des revendications 1 à 7, dans laquelle $R_{12}$ est un atome d'hydrogène et $R_{13}$ est choisi dans le groupe constitué par un groupe pipéridinyle et morpholinyle.

**9.** Composition pharmaceutique selon l'une quelconque des revendications 1 à 8, dans laquelle deux substituants adjacents choisis dans le groupe constitué par $R_7$, $R_8$, $R_9$, $R_{10}$ et $R_{11}$ forment, conjointement avec les atomes de carbone du cycle phényle auxquels ils sont attachés, un groupe méthylènedioxy condensé audit cycle phényle.

**10.** Composition pharmaceutique selon la revendication 1, dans laquelle ledit dérivé 1,2,4-thiadiazole est choisi dans le groupe constitué par :

la 4-phénylacétyl-1-[3-(4-fluorobenzyl)-1,2,4-thiadiazol-5-yl]pipérazine ;
la 4-(4-fluorophénylacétyl)-1-[3-(4-fluorobenzyl)-1,2,4-thiadiazol-5-yl]-pipérazine ;
la 1-(4-fluorophénylacétyl)-4-[3-(4-méthylbenzyl)-1,2,4-thiadiazol-5-yl]-2-méthylpipérazine ;
la 1-(4-méthoxyphénylacétyl)-4-[3-(4-fluorobenzyl)-1,2,4-thiadiazol-5-yl]-2-méthylpipérazine ;
la 1-phénylacétyl-4-[3-(4-fluorobenzyl)-1,2,4-thiadiazol-5-yl]-2-méthyl-pipérazine ;
la 1-(4-fluorophénylacétyl)-4-[3-(4-fluorobenzyl)-1,2,4-thiadiazol-5-yl]-2-méthylpipérazine ;
la 1-[chloro(phényl)acétyl]-4-[3-(4-fluorobenzyl)-1,2,4-thiadiazol-5-yl]-2-méthylpipérazine ;
la 1-(2-phénylbutanoyl)-4-[3-(4-fluorobenzyl)-1,2,4-thiadiazol-5-yl]-2-méthylpipérazine ;
le N-(3-fluorophényl)-4-[3-(4-fluorobenzyl)-1,2,4-thiadiazol-5-yl]-pipérazine-1-carboxamide ;
le N-(2-méthylphényl)-4-[3-(4-fluorobenzyl)-1,2,4-thiadiazol-5-yl]-2-méthylpipérazine-1-carboxamide ;
le N-(4-éthylphényl)-4-[3-(4-fluorobenzyl)-1,2,4-thiadiazol-5-yl]-2-méthylpipérazine-1-carboxamide ;
le N-(2-éthylphényl)-4-[3-(4-fluorobenzyl)-1,2,4-thiadiazol-5-yl]-2-méthylpipérazine-1-carboxamide ;
le N-phényl-4-[3-(4-fluorobenzyl)-1,2,4-thiadiazol-5-yl]pipérazine-1-carboxamide ;
le N-(4-éthoxyphényl)-4-[3-(4-fluorobenzyl)-1,2,4-thiadiazol-5-yl]-2-méthylpipérazine-1-carboxamide ;
le N-(2-méthylphényl)-4-[3-(4-fluorobenzyl)-1,2,4-thiadiazol-5-yl]-pipérazine-1-carboxamide ;
le N-(2-fluorophényl)-4-[3-(4-fluorobenzyl)-1,2,4-thiadiazol-5-yl]-pipérazine-1-carboxamide ;
le N-(2-trifluorométhylphényl)-4-[3-(4-fluorobenzyl)-1,2,4-thiadiazol-5-yl]-pipérazine-1-carboxamide ;
le N-(2-trifluorométhylphényl)-4-[3-(4-fluorobenzyl)-1,2,4-thiadiazol-5-yl]-2-méthylpipérazine-1-carboxamide ;
le N-(2,6-diméthylphényl)-4-[3-(4-fluorobenzyl)-1,2,4-thiadiazol-5-yl]-2-méthylpipérazine-1-carboxamide ;
le N-(2,4-diméthylphényl)-4-[3-(4-fluorobenzyl)-1,2,4-thiadiazol-5-yl]-2-méthylpipérazine-1-carboxamide ;
le N-(2,6-dichlorophényl)-4-[3-(4-fluorobenzyl)-1,2 ,4-thiadiazol-5-yl]-2-méthylpipérazine-1-carboxamide ;
le N-(3-cyanophényl)-4-[3-(4-fluorobenzyl)-'1,2,4-thiadiazol-5-yl]-2-méthylpipérazine-1-carboxamide ;
le N-(2,4-difluorophényl)-4-[3-(4-fluorobenzyl)-1,2,4-thiadiazol-5-yl]-2-méthylpipérazine-1-carboxamide ;
le N-(2,6-diméthylphényl)-4-[3-(4-fluorobenzyl)-1,2,4-thiadiazol-5-yl]-pipérazine-1-carboxamide ;
le N-1-naphtyl-4-[3-(4-fluorobenzyl)-1,2,4-thiadiazol-5-yl]-2-méthyl-pipérazine-1-carboxamide ;
le N-(3,4-difluorophényl)-4-[3-(4-fluorobenzyl)-1,2,4-thiadiazol-5-yl]-2-méthylpipérazine-1-carboxamide ;
le N-(2,4-diméthoxyphényl)-4-[3-(4-fluorobenzyl)-1,2,4-thiadiazol-5-yl]-pipérazine-1-carboxamide ;
le N-(3,4-difluorophényl)-4-[3-(4-fluorobenzyl)-1,2,4-thiadiazol-5-yl]-pipérazine-1-carboxamide ;
le N-(3,5-diméthoxyphényl)-4-[3-(4-fluorobenzyl)-1,2,4-thiadiazol-5-yl]-2-méthylpipérazine-1-carboxamide ;
la 1-[4-tert-butylphénylsulfonyl]-4-[3-(4-fluorobenzyl)-1,2,4-thiadiazol-5-yl]pipérazine ;
la 1-[4-méthoxyphénylsulfonyl]-4-[3-(4-chlorobenzyl)-1,2,4-thiadiazol-5-yl]pipérazine ;
la 1-[4-méthoxyphénylsulfonyl]-4-[3-(4-fluorobenzyl)-1,2,4-thiadiazol-5-yl]pipérazine ;
la 1-[4-fluorophénylsulfonyl]-4-[3-(4-chlorobenzyl)-1,2,4-thiadiazol-5-yl]pipérazine ;
la 1-[4-chlorophénylsulfonyl]-4-[3-(4-méthylbenzyl)-1,2,4-thiadiazol-5-yl]-2-méthylpipérazine ;
la 1-[1-naphtylsulfonyl]-4-[3-(4-méthylbenzyl)-1,2,4-thiadiazol-5-yl]-pipérazine ;

la 1-[2,5-dichlorophénylsulfonyl]-4-[3-(4-méthylbenzyl)-1,2,4-thiadiazol-5-yl]-2-méthylpipérazine ;
la 1-[2,4,6-triméthylphénylsulfonyl]-4-[3-(4-méthylbenzyl)-1,2,4-thiadiazol-5-yl]pipérazine ;
la 1-[2-naphtylsulfonyl]-4-[3-(4-méthylbenzyl)-1,2,4-thiadiazol-5-yl]-2-méthylpipérazine ;
la 1-[2,5-dichlorophénylsulfonyl]-4-[3-(4-chlorobenzyl)-1,2,4-thiadiazol-5-yl]pipérazine ;
la 1-[4-bromophénylsulfonyl]-4-[3-(4-méthylbenzyl)-1,2,4-thiadiazol-5-yl]-3-méthylpipérazine ;
la 1-[1-naphtylsulfonyl]-4-[3-(4-méthylbenzyl)-1,2,4-thiadiazol-5-yl]-3-méthylpipérazine ;
la 1-[4-tert-butylphénylsulfonyl]-4-[3-(4-méthylbenzyl)-1,2,4-thiadiazol-5-yl]-2-méthylpipérazine ;
la 1-[1-naphtylsulfonyl]-4-[3-(4-chlorobenzyl)-1,2 ,4-thiadiazol-5-yl]-2-méthylpipérazine ;
la 1-[2,4,6-triméthylphénylsulfonyl]-4-[3-(4-méthylbenzyl)-1,2,4-thiadiazol-5-yl]-2-méthylpipérazine ;
la 1-[3-trifluorophénylsulfonyl]-4-[3-(4-fluorobenzyl)-1,2,4-thiadiazol-5-yl]-2-méthylpipérazine ;
la 1-phénylsulfonyl-4-[3-(4-fluorobenzyl)-1,2,4-thiadiazol-5-yl]-2-méthylpipérazine ;
la 1-[4-acétamidophénylsulfonyl]-4-[3-(4-méthylbenzyl)-1,2,4-thiadiazol-5-yl]-2-méthylpipérazine ;
la 1-[4-acétamidophénylsulfonyl]-4-[3-(4-méthylbenzyl)-1,2,4-thiadiazol-5-yl]pipérazine ;
la 1-[4-méthylphénylsulfonyl]-4-[3-(4-méthylbenzyl)-1,2,4-thiadiazol-5-yl]-2-méthylpipérazine ;
la 1-[4-méthoxyphénylsulfonyl]-4-[3-(4-chlorobenzyl)-1,2,4-thiadiazol-5-yl]-2-méthylpipérazine ;
la 1-[2-naphtylsulfonyl]-4-[3-(4-chlorobenzyl)-1,2,4-thiadiazol-5-yl]-pipérazine ;
la 1-[4-tert-butylphénylsulfonyl]-4-[3-(4-fluorobenzyl)-1,2,4-thiadiazol-5-yl]-2-méthylpipérazine ;
la 1-[4-acétamidophénylsulfonyl]-4-[3-(4-fluorobenzyl)-1,2,4-thiadiazol-5-yl]-2-méthylpipérazine ;
la 1-[3-trifluorométhylphénylsulfonyl]-4-[3-(4-méthylbenzyl)-1,2,4-thiadiazol-5-yl]-2-méthylpipérazine ;
la 1-[4-fluorophénylsulfonyl]-4-[3-(4-méthylbenzyl)-1,2,4-thiadiazol-5-yl]pipérazine ;
la 1-[4-fluorophénylsulfonyl]-4-[3-(4-méthylbenzyl)-1,2,4-thiadiazol-5-yl]-2-méthylpipérazine ;
la 1-[4-fluorophénylsulfonyl]-4-[3-(4-fluorobenzyl)-1,2,4-thiadiazol-5-yl]-2-méthylpipérazine ;
la 1-[4-méthoxyphénylsulfonyl]-4-[3-(4-méthylbenzyl)-1,2,4-thiadiazol-5-yl]-2-méthylpipérazine ;
la 1-[4-ter-butylphénylsulfonyl]-4-[3-(4-fluorobenzyl)-1,2,4-thiadiazol-5-yl]pipérazine ;
la 1-[3-trifluorométhylphénylsulfonyl]-4-[3-(4-méthylbenzyl)-1,2,4-thiadiazol-5-yl]pipérazine ;
la 1-[4-méthylphénylsulfonyl]-4-[3-(4-chlorobenzyl)-1,2,4-thiadiazol-5-yl]pipérazine ;
la 1-[4-bromophénylsulfonyl]-4-[3-(4-méthylbenzyl)-1,2,4-thiadiazol-5-yl]pipérazine ;
la 1-[2-naphtylsulfonyl]-4-[3-(4-méthylbenzyl)-1,2,4-thiadiazol-5-yl]pipérazine ;
la 1-[4-chlorophénylsulfonyl]-4-[3-(4-méthylbenzyl)-1,2,4-thiadiazol-5-yl]pipérazine ;
la 1-[4-tert-butylphénylsulfonyl]-4-[3-(4-méthylbenzyl)-1,2,4-thiadiazol-5-yl]pipérazine ;
la 1-[4-méthoxyphénylsulfonyl]-4-[3-(4-méthylbenzyl)-1,2,4-thiadiazol-5-yl]pipérazine ;
la 1-[4-tert-butylphénylsulfonyl]-4-[3-(4-chlorobenzyl)-1,2,4-thiadiazol-5-yl]pipérazine ;
la 1-[quinoline-8-sulfonyl]-4-[3-(4-méthylbenzyl)-1,2,4-thiadiazol-5-yl]-pipérazine ;
la 1-[4-nitrophénylsulfonyl]-4-[3-(4-méthylbenzyl)-1,2,4-thiadiazol-5-yl]-2-méthylpipérazine ;
la 1-[3-nitro-4-chlorophénylsulfonyl]-4-[3-(méthylbenzyl)-1,2,4-thiadiazol-5-yl]-2-méthylpipérazine ;
la 1-[4-nitrophénylsulfonyl]-4-[3-(4-fluorobenzyl)-1,2,4-thiadiazol-5-yl]-2-méthylpipérazine ;
la 1-[4-méthoxyphénylsulfonyl]-4-[3-(4-fluorobenzyl)-1,2,4-thiadiazol-5-yl]pipérazine ;
la 1-[3-nitro-4-chlorophénylsulfonyl]-4-[3-(méthylbenzyl)-1,2,4-thiadiazol-5-yl]pipérazine ;
la 1-(benzylsulfonyl)-4-[3-(4-chlorobenzyl)-1,2,4-thiadiazol-5-yl]-pipérazine ;
la 1-(benzylsulfonyl)-4-[3-(4-méthylbenzyl)-1,2,4-thiadiazol-5-yl]-pipérazine ;
la 1-(phénylprop-2-énsulfonyl)-4-[3-(4-fluorobenzyl)-1,2,4-thiadiazol-5-yl]-pipérazine ;
la 1-(phénylprop-2-énsulfonyl)-4-[3-(4-méthylbenzyl)-1,2,4-thiadiazol-5-yl]-2-méthylpipérazine ;
la 1-(phénylprop-2-énsulfonyl)-4-[3-(4-méthylbenzyl)-1,2,4-thiadiazol-5-yl]pipérazine ;
la 1-(butylsulfonyl)-4-[3-(4-chlorobenzyl)-1,2,4-thiadiazol-5-yl]pipérazine ; la 1-(octylsulfonyl)-4-[3-(4-méthylbenzyl)-1,2,4-thiadiazol-5-yl]pipérazine ; la 1-(butylsulfonyl)-4-[3-(4-méthylbenzyl)-1,2,4-thiadiazol-5-yl] pipérazine ; la 1-(éthylsulfonyl)-4-[3-(4-chlorobenzyl)-1,2,4-thiadiazol-5-yl]pipérazine ; la 1-(isopropylsulfonyl)-4-[3-(4-méthylbenzyl)-1,2,4-thiadiazol-5-yl]-pipérazine ;
la 1-[(2E)-3-phénylprop-2-énoyl]-4-[3-(4-chlorobenzyl)-1,2,4-thiadiazol-5-yl]-2-méthylpipérazine ;
la 1-[(2E)-3-phénylprop-2-énoyl]-4-B103-2-méthylpipérazine ;
la 1-[(2E)-3-phénylprop-2-énoyl]-4-[3-(4-chlorobenzyl)-1,2,4-thiadiazol-5-yl]pipérazine ;
la 1-(3-phénylpropanoyl)-4-[3-(4-fluorobenzyl)-1,2,4-thiadiazol-5-yl]-2-méthylpipérazine ;
la 1-(3-phénylpropanoyl)-4-[3-(4-fluorobenzyl)-1,2,4-thiadiazol-5-yl]-pipérazine ;
la 1-[3-phénylprop-2-ényl]-4-[3-(4-méthylbenzyl)-1,2,4-thiadiazol-5-yl]-pipérazine ;
la 1-[3-phénylprop-2-ényl]-4-[3-(4-chlorobenzyl)-1,2,4-thiadiazol-5-yl]-pipérazine ;
la 1-[3-phénylprop-2-ényl]-4-[3-(4-fluorobenzyl)-1,2,4-thiadiazol-5-yl]-pipérazine ;
le 4-pentyl-N-{2-[4-[3-(4-fluorobenzyl)-1,2,4-thiadiazol-5-yl]pipérazin-1-yl]-éthyl}benzamide ;
le 4-butyl-N-{2-[4-[3-(4-fluorobenzyl)-1,2,4-thiadiazol-5-yl]pipérazin-1-yl]-éthyl}benzamide ;
le 4-hexyl-N-{2-[4-[3-(4-fluorobenzyl)-1,2,4-thiadiazol-5-yl]pipérazin-1-yl]-éthyl}benzamide ;

le 4-chloro-N-{2-[4-[3-(4-fluorobenzyl)-1,2,4-thiadiazol-5-yl]pipérazin-1-yl]éthyl}benzamide ;
le 3,5-dichloro-N-{2-[4-[3-(4-fluorobenzyl)-1,2,4-thiadiazol-5-yl]pipérazin-1-yl]éthyl}benzamide ;
le 2-méthyl-N-{2-[4-[3-(4-fluorobenzyl)-1,2,4-thiadiazol-5-yl]pipérazin-1-yl]éthyl}benzamide ;
le 3-fluoro-N-{2-[4-[3-(4-fluorobenzyl)-1,2,4-thiadiazol-5-yl]pipérazin-1-yl]éthyl}benzamide ;
le 4-méthyl-N-{2-[4-[3-(4-fluorobenzyl)-1,2,4-thiadiazol-5-yl]pipérazin-1-yl]éthyl}benzamide ;
le 4-fluoro-N-{2-[4-[3-(4-fluorobenzyl)-1,2,4-thiadiazol-5-yl]pipérazin-1-yl]-éthyl}benzamide ;
le 4-éthyl-N-{2-[4-[3-(4-fluorobenzyl)-1,2,4-thiadiazol-5-yl]pipérazin-1-yl]-éthyl}benzamide ;
le N-{2-[4-[3-(4-fluorobenzyl)-1,2,4-thiadiazol-5-yl]pipérazin-1-yl]-éthyl}benzamide ;
le 3-fluoro-N-{2-[4-[3-(4-fluorobenzyl)-1,2,4-thiadiazol-5-yl]pipérazin-1-yl]-éthyl}benzamide ;
le 3-fluoro-N-{2-[4-[3-benzyl-1,2,4-thiadiazol-5-yl]pipérazin-1-yl]-éthyl}benzamide ;
le 2-(4-fluorophényl)-N-{2-[4-[3-(4-fluorobenzyl)-1,2,4-thiadiazol-5-yl]-pipérazin-1-yl]éthyl}acétamide ;
le 2-phényl-N-{2-[4-[3-(4-fluorobenzyl)-1,2,4-thiadiazol-5-yl]pipérazin-1-yl]éthyl}acétamide ;
le 2-(4-fluorophényl)-N-{2-[4-[3-(4-méthylbenzyl)-1,2,4-thiadiazol-5-yl]-pipérazin-1-yl]éthyl}acétamide ;
le N-benzyl-N'-{2-[4-[3-(4-fluorobenzyl)-1,2,4-thiadiazol-5-yl]pipérazin-1-yl]éthyl}urée ;
le 2-phényl)-N-{2-[4-[3-(4-fluorobenzyl)-1,2,4-thiadiazol-5-yl]pipérazin-1-yl]éthyl}butanamide ;
le 3-phényl)-N-{2-[4-[3-(4-fluorobenzyl)-1,2,4-thiadiazol-5-yl]pipérazin-1-yl]éthyl}propanamide ;
la 1-benzyl-4-[3-(4-fluorobenzyl)-1,2,4-thiadiazol-5-yl]pipérazine ;
la 1-(1,3-benzodioxol-5-ylméthyl)-4-[3-(4-fluorobenzyl)-1,2,4-thiadiazol-5-yl]pipérazine ;
la 1-(2-fluorobenzoyl)-4-[3-(3-méthoxybenzyl)-1,2,4-thiadiazol-5-yl]-2-méthylpipérazine ;
la 1-(4-éthylbenzoyl)-4-[3-(4-fluorobenzyl)-1,2,4-thiadiazol-5-yl]-2-méthylpipérazine ;
la 1-(4-butylbenzoyl)-4-[3-(4-méthylbenzyl)-1,2,4-thiadiazol-5-yl]-2-méthylpipérazine ;
la 1-(4-méthoxybenzoyl)-4-[3-(4-fluorobenzyl)-1,2,4-thiadiazol-5-yl]-2-méthylpipérazine ;
la 1-(1,3-benzodioxol-5-ylcarbonyl)-4-[3-(3-méthoxybenzyl)-1,2,4-thiadiazol-5-yl]-2-méthylpipérazine ;
la 1-(4-butylbenzoyl)-4-[3-(4-fluorobenzyl)-1,2,4-thiadiazol-5-yl]-2-méthylpipérazine ;
la 1-(4-hexylbenzoyl)-4-[3-(4-fluorobenzyl)-1,2,4-thiadiazol-5-yl]-pipérazine ;
la 1-(3-chlorobenzoyl)-4-[3-(4-fluorobenzyl)-1,2,4-thiadiazol-5-yl]-2-méthylpipérazine ;
la 1-(4-fluorobenzoyl)-4-[3-(3-méthoxybenzyl)-1,2,4-thiadiazol-5-yl]-2-méthylpipérazine ;
la 1-(4-méthylbenzoyl)-4-[3-(3-méthoxybenzyl)-1,2,4-thiadiazol-5-yl]-2-méthylpipérazine ;
la 1-(3-fluorobenzoyl)-4-[3-(4-chlorobenzyl)-1,2,4-thiadiazol-5-yl]-2-méthylpipérazine ;
la 1-benzoyl-4-[3-(4-fluorobenzyl)-1,2,4-thiadiazol-5-yl]-2-méthylpipérazine ;
la 1-(4-fluorobenzoyl)-4-[3-(4-fluorobenzyl)-1,2,4-thiadiazol-5-yl]-2-méthylpipérazine ;
la 1-(4-tert-butylbenzoyl)-4-[3-(4-fluorobenzyl)-1,2,4-thiadiazol-5-yl]-2-méthylpipérazine ;
la 1-(1,1'-biphényl-4-ylcarbonyl)-4-[3-(4-fluorobenzyl)-1,2,4-thiadiazol-5-yl]-2-méthylpipérazine ;
la 1-(4-méthoxybenzoyl)-4-[3-(4-méthylbenzyl)-1,2,4-thiadiazol-5-yl]-2-méthylpipérazine ;
la 1-(4-éthylbenzoyl)-4-[3-(4-méthylbenzyl)-1,2,4-thiadiazol-5-yl]-pipérazine ;
la 1-(2-naphtoyl)-4-[3-(4-fluorobenzyl)-1,2,4-thiadiazol-5-yl]-2-méthylpipérazine ;
la 1-(2-méthoxybenzoyl)-4-[3-(4-fluorobenzyl-1,2,4-thiadiazol-5-yl]-2-méthylpipérazine ;
la 1-(4-pentylbenzoyl)-4-[3-(4-méthylbenzyl)-1,2,4-thiadiazol-5-yl]-2-méthylpipérazine ;
la 1-(4-bromobenzoyl)-4-(3-(4-fluorobenzyl)-1,2,4-thiadiazol-5-yl]-2-méthylpipérazine ;
la 1-(2,4-diméthoxybenzoyl)-4-[3-(4-fluorobenzyl)-1,2,4-thiadiazol-5-yl]-2-méthylpipérazine ;
la 1-(3,5-dichlorobenzoyl)-4-[3-(4-fluorobenzyl)-1,2,4-thiadiazol-5-yl]pipérazine ;
la 1-(3-chlorobenzoyl)-4-[3-(4-fluorobenzyl)-1,2,4-thiadiazol-5-yl]-pipérazine ;
la 1-(4-méthylbenzoyl)-4-[3-(4-fluorobenzyl)-1,2,4-thiadiazol-5-yl]-2-méthylpipérazine ;
la 1-(2-méthylbenzoyl)-4-[3-(4-fluorobenzyl)-1,2,4-thiadiazol-5-yl]-2-méthylpipérazine ;
la 1-(4-méthylbenzoyl)-4-[3-(4-fluorobenzyl)-1,2,4-thiadiazol-5-yl]-pipérazine;
la 1-(3-bromobenzoyl)-4-[3-(4-fluorobenzyl)-1,2,4-thiadiazol-5-yl]-2-méthylpipérazine;
la 1-(4-éthylbenzoyl)-4-[3-(3-méthoxybenzyl)-1,2,4-thiadiazol-5-yl]-2-méthylpipérazine ;
la 1-(3-méthylbenzoyl)-4-[3-(4-méthylbenzyl)-1,2,4-thiadiazol-5-yl]-2-méthylpipérazine ;
la 1-(3-trifluorométhylbenzoyl)-4-[3-(4-fluorobenzyl)-1,2,4-thiadiazol-5-yl]-2-méthylpipérazine ;
la 1-(4-tert-butylbenzoyl)-4-[3-(3-méthoxybenzyl)-1,2,4-thiadiazol-5-yl]-2-méthylpipérazine ;
la 1-(4-éthylbenzoyl)-4-[3-(4-méthylbenzyl)-1,2,4-thiadiazol-5-yl]-2-méthylpipérazine ;
la 1-(2-bromobenzoyl)-4-[3-(4-fluorobenzyl)-1,2,4-thiadiazol-5-yl]-2-méthylpipérazine ;
la 1-(2-fluorobenzoyl)-4-[3-(4-méthylbenzyl)-1,2,4-thiadiazol-5-yl]-2-méthylpipérazine ;
la 1-(fluorobenzoyl)-4-[3-benzyl-1,2,4-thiadiazol-5-yl]-2-méthylpipérazine ;
la 1-(4-fluorobenzoyl)-4-[3-(4-méthylbenzyl)-1,2,4-thiadiazol-5-yl]-2-méthylpipérazine ;
la 1-(2-méthylbenzoyl)-4-[3-(4-fluorobenzyl)-1,2,4-thiadiazol-5-yl]-pipérazine ;
la 1-(4-fluorobenzoyl)-4-[3-(4chlorobenzyl)-1,2,4-thiadiazol-5-yl]-pipérazine ;
la 1-(4-fluorobenzoyl)-4-[3-benzyl-1,2,4-thiadiazol-5-yl]-2-méthylpipérazine ;

la 1-(4-chlorobenzoyl)-4-[3-(4-méthylbenzyl)-1,2,4-thiadiazol-5-yl]-pipérazine ;
la 1-(3-fluorobenzoyl)-4-[3-(4-méthylbenzyl)-1,2,4-thiadiazol-5-yl]-2-méthylpipérazine ;
la 1-(3-fluorobenzoyl)-4-[3-(4-chlorobenzyl)-1,2,4-thiadiazol-5-yl]-pipérazine ;
la 1-benzoyl-4-[3-(4-méthylbenzyl)-1,2,4-thiadiazol-5-yl]pipérazine ;
la 1-(2-fluorobenzoyl)-4-[3-(4-chlorobenzyl)-1,2,4-thiadiazol-5-yl]-pipérazine ;
la 1-(4-fluorobenzoyl)-4-[3-(4-méthylbenzyl)-1,2,4-thiadiazol-5-yl]-pipérazine ;
la 1-(1,3-benzodioxol-5-ylcarbonyl)-4-[3-(4-méthylbenzyl)-1,2,4-thiadiazol-5-yl]-2-méthylpipérazine ;
la 1-(3-fluorobenzoyl)-4-[3-(4-méthylbenzyl)-1,2,4-thiadiazol-5-yl]-pipérazine ;
la 1-(4-fluorobenzoyl)-4-[3-benzyl-1 ,2,4-thiadiazol-5-yl]pipérazine ;
la 1-(4-bromobenzoyl)-4-[3-(4-méthylbenzyl)-1,2,4-thiadiazol-5-yl]-pipérazine ;
la 1-(4-éthylbenzoyl)-4-[3-(4-chlorobenzyl)-1,2,4-thiadiazol-5-yl]-pipérazine ;
la 1-(2-chlorobenzoyl)-4-[3-(4-fluorobenzyl)-1,2,4-thiadiazol-5-yl]-2-méthylpipérazine ;
la 1-(2-fluorobenzoyl)-4-[3-(4-chlorobenzyl)-1,2,4-thiadiazol-5-yl]-2-méthylpipérazine ;
la 1-(4-trifluorométhylbenzoyl)-4-[3-(3-méthoxybenzyl)-1,2,4-thiadiazol-5-yl]-2-méthylpipérazine ;
la 1-benzoyl-4-[3-(4-méthylbenzyl)-1,2,4-thiadiazol-5-yl]-2-méthylpipérazine ;
la 1-(4-bromobenzoyl)-4-[3-(3-méthoxybenzyl)-1,2,4-thiadiazol-5-yl]-2-méthylpipérazine ;
la 1-(4-trifluorométhylbenzoyl)-4-[3-(4-méthylbenzyl)-1,2,4-thiadiazol-5-yl]pipérazine ;
la 1-(3-nitro-4-méthylbenzoyl)-4-[3-(4-fluorobenzyl)-1,2,4-thiadiazol-5-yl]-2-méthylpipérazine ;
la 1-benzoyl-4-[3-(3-méthoxybenzyl)-1,2,4-thiadiazol-5-yl]-2-méthylpipérazine ;
la 1-(4-chlorobenzoyl)-4-[3-(3-méthoxybenzyl)-1,2,4-thiadiazol-5-yl]-2-méthylpipérazine ;
la 1-(2-fluorobenzoyl)-4-[3-(4-méthylbenzyl)-1,2,4-thiadiazol-5-yl]-pipérazine ;
la 1-(4-hexylbenzoyl)-4-[3-(4-méthylbenzyl)-1,2,4-thiadiazol-5-yl]-pipérazine ;
la 1-(2-chloro-4-nitrobenzoyl)-4-[3-(4-fluorobenzyl)-1,2,4-thiadiazol-5-yl]-2-méthylpipérazine ;
la 1-(1,3-benzodioxol-5-ylcarbonyl)-4-[3-(4-fluorobenzyl)-1,2,4-thiadiazol-5-yl]-2-méthylpipérazine ;
la 1-(3-fluorobenzoyl)-4-[3-(4-fluorobenzyl)-1,2,4-thiadiazol-5-yl]-2-méthylpipérazine ;
la 1-(4-tert-butylbenzoyl)-4-[3-(4-fluorobenzyl)-1,2,4-thiadiazol-5-yl]-pipérazine ;
la 1-benzoyl-4-[3-(4-fluorobenzyl)-1,2,4-thiadiazol-5-yl]pipérazine ;
la 1-(4-butylbenzoyl)-4-[3-(4-méthylbenzyl)-1,2,4-thiadiazol-5-yl]-pipérazine ;
la 1-(4-tert-butylbenzoyl)-4-[3-(4-méthylbenzyl)-1,2,4-thiadiazol-5-yl]-pipérazine ;
la 1-(4-nitrobenzoyl)-4-[3-(4-fluorobenzyl)-1,2,4-thiadiazol-5-yl]-2-méthylpipérazine ;
la 1-(2-méthylphényl)-4-[3-(4-fluorobenzyl)-1,2,4-thiadiazol-5-yl]-pipérazine ;
la 1-(2-éthoxyphényl)-4-[3-(4-fluorobenzyl)-1,2,4-thiadiazol-5-yl]-pipérazine ;
la 1-(2-fluorophényl)-4-[3-(4-fluorobenzyl)-1,2,4-thiadiazol-5-yl]-pipérazine ;
la 1-(3-trifluorométhylphényl)-4-[3-(4-fluorobenzyl)-1,2,4-thiadiazol-5-yl]pipérazine ;
le 1-(4-chlorobenzoyl)-4-[3-(4-fluorobenzyl)-1,2,4-thiadiazol-5-yl]-1,4-diazépane ;
le 1-(4-méthylbenzoyl)-4-[3-(4-fluorobenzyl)-1,2,4-thiadiazol-5-yl]-1,4-diazépane;
le 1-(1,3-benzodioxol-5-ylcarbonyl)-4-[3-(4-fluorobenzyl)-1,2,4-thiadiazol-5-yl]-1,4-diazépane ;
la 1-[4-méthoxyphénylsulfonyl]-4-[3-(3-fluoro-benzyl)-1,2,4-thiadiazol-5-yl]pipérazine ;
la 1-[4-méthoxyphénylsulfonyl]-4-[3-(2-fluoro-benzyl)-1,2,4-thiadiazol-5-yl]pipérazine ;
la 1-[2-méthoxyphénylsulfonyl]-4-[3-(4-fluoro-benzyl)-1,2,4-thiadiazol-5-yl]pipérazine ;
la 1-[4-éthoxyphénylsulfonyl]-4-[3-(4-fluoro-benzyl)-1,2,4-thiadiazol-5-yl]pipérazine ;
la 1-[4-éthylphénylsulfonyl]-4-[3-(4-fluoro-benzyl)-1,2,4-thiadiazol-5-yl]pipérazine ;
la 1-phénylsulfonyl-4-[3-(4-fluoro-benzyl)-1 ,2,4-thiadiazol-5-yl]pipérazine ; la 1-[4-méthoxyphénylsulfonyl]-4-[3-benzyl-1,2,4-thiadiazol-5-yl]-pipérazine ;
la 1-(4-propionyloxy)benzène-sulfonyl-4-[3-(4-fluoro-benzyl)-1,2,4-thiadiazol-5-yl]pipérazine ;
la 1-[4-méthoxyphénylsulfonyl]-4-[3-(3-méthyl-benzyl-1,2,4-thiadiazol-5-yl]pipérazine ;
la 1-[4-méthoxyphénylsulfonyl]-4-[3-(2-méthyl-benzyl-1,2,4-thiadiazol-5-yl]pipérazine ;
la 1-[4-méthoxyphénylsulfonyl]-4-[3-(2-méthoxy-benzyl-1,2,4-thiadiazol-5-yl]pipérazine ;
la 1-[4-méthoxyphénylsulfonyl]-4-[3-(3-méthyl-4-fluoro-benzyl-1,2,4-thiadiazol-5-yl]pipérazine ;
la 1-[4-méthoxyphénylsulfonyl]-4-[3-(4-méthoxy-benzyl-1,2,4-thiadiazol-5-yl]pipérazine ;
la 1-[4-bromophénylsulfonyl]-4-[3-(3-méthoxy-benzyl-1,2,4-thiadiazol-5-yl]pipérazine ;
la 1-[4-méthoxyphénylsulfonyl]-4-[3-(3-méthoxybenzyl)-1,2,4-thia-diazol-5-yl]-2-méthyl-pipérazine ; et
la 1-(4-hydroxybenzènesulfonyl)-4-[3-(4-fluoro-benzyl)-1,2,4-thiadiazol-5-yl]pipérazine.

**11.** Dérivé 1,2,4-thiadiazole choisi dans le groupe constitué par :

- la 1-[3-méthoxyphénylsulfonyl]-4-[3-(4-fluorobenzyl)-1,2,4-thiadiazol-5-yl]pipérazine ;
- la 1-[4-méthoxyphénylsulfonyl]-4-[3-(3-méthoxybenzyl)-1,2,4-thiadiazol-5-yl]pipérazine ;

- la 1-[4-méthoxyphénylsulfonyl]-4-[3-(3-fluoro-benzyl)-1,2,4-thiadiazol-5-yl]pipérazine ;
- la 1-[4-méthoxyphénylsulfonyl]-4-[3-(2-fluoro-benzyl)-1,2,4-thiadiazol-5-yl]pipérazine ;
- la 1-[2-méthoxyphénylsulfonyl]-4-[3-(4-fluoro-benzyl)-1,2,4-thiadiazol-5-yl]pipérazine ;
- la 1-[4-éthoxyphénylsulfonyl]-4-[3-(4-fluoro-benzyl)-1,2,4-thiadiazol-5-yl]pipérazine ;
- la 1-[4-éthylphénylsulfonyl]-4-[3-(4-fluoro-benzyl)-1,2,4-thiadiazol-5-yl]pipérazine ;
- 1-phénylsulfonyl-4-[3-(4-fluoro-benzyl)-1,2,4-thiadiazol-5-yl]-pipérazine ;
- la 1-[4-méthoxyphénylsulfonyl]-4-[3-benzyl-1,2,4-thiadiazol-5-yl]-pipérazine ;
- la 1-(4-propionyloxy)benzène-sulfonyl-4-[3-(4-fluoro-benzyl)-1,2,4-thiadiazol-5-yl]pipérazine ;
- la 1-[4-méthoxyphénylsulfonyl]-4-[3-(3-méthyl-benzyl-1,2,4-thiadiazol-5-yl]pipérazine ;
- la 1-[4-méthoxyphénylsulfonyl]-4-[3-(2-méthyl-benzyl-1,2,4-thiadiazol-5-yl]pipérazine ;
- la 1-[4-méthoxyphénylsulfonyl]-4-[3-(2-méthoxy-benzyl-1,2,4-thiadiazol-5-yl]pipérazine ;
- la 1-[4-méthoxyphénylsulfonyl]-4-[3-(3-méthyl-4-fluoro-benzyl-1,2,4-thiadiazol-5-yl]pipérazine ;
- la 1-[4-méthoxyphénylsulfonyl]-4-[3-(4-méthoxy-benzyl-1,2,4-thiadiazol-5-yl]pipérazine ;
- la 1-[4-bromophénylsulfonyl]-4-[3-(3-méthoxy-benzyl-1,2,4-thiadiazol-5-yl]pipérazine ;
- la 1-[4-méthoxyphénylsulfonyl]-4-[3-(3-méthoxybenzyl)-1,2,4-thiadiazol-5-yl]-2-méthyl-pipérazine ; et
- la 1-(4-hydroxybenzènesulfonyl)-4-[3-(4-fluoro-benzyl)-1,2,4-thiadiazol-5-yl]pipérazine.

**12.** Composition pharmaceutique selon l'une quelconque des revendications 1 à 10, comprenant en outre une quantité thérapeutique d'un ou plusieurs médicaments choisis dans le groupe constitué par des agents neuroprotecteurs et des inhibiteurs de dépôt d'$\alpha$-synucléine.

**13.** Dérivé 1,2,4-thiadiazole tel que défini dans l'une quelconque des revendications 1 à 10, destiné à être utilisé dans le traitement d'une $\alpha$-synucléopathie.

**14.** Dérivé 1,2,4-thiadiazole selon la revendication 13, dans lequel ladite $\alpha$-synucléopathie est choisie dans le groupe constitué par la maladie de Parkinson, la maladie à corps de Lewy diffus, une lésion cérébrale traumatique, la sclérose latérale amyotrophique, la maladie de Niemann-Pick, le syndrome de Hallervorden-Spatz, le syndrome de Down (trisomie 21), une dystrophie neuroaxonale, les atrophies multi-systématisées et la maladie d'Alzheimer.

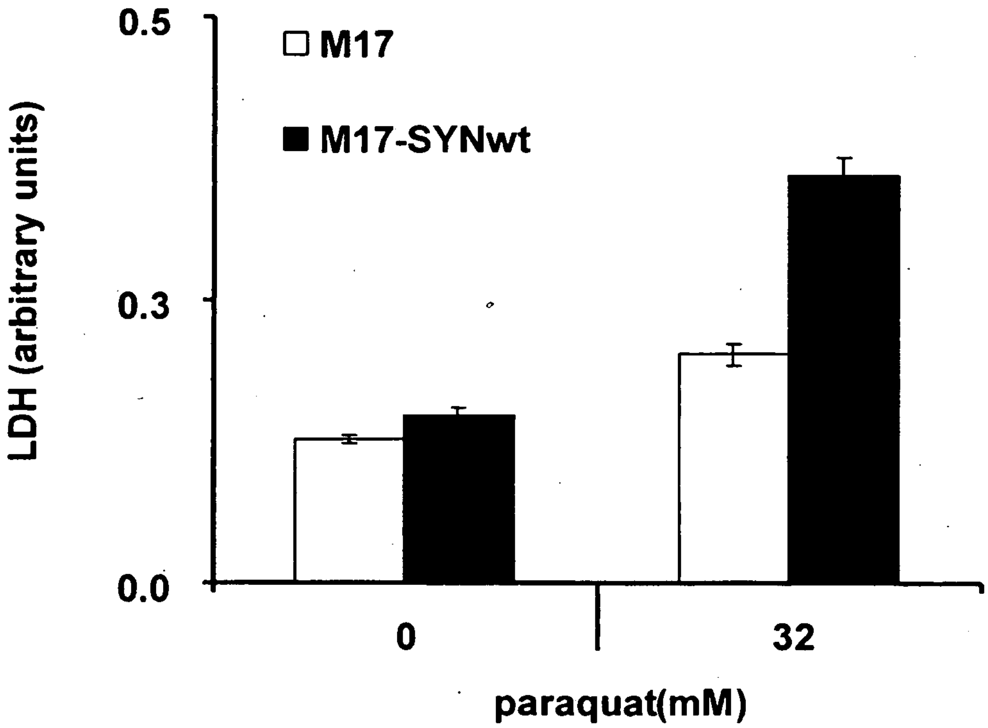


Figure 1

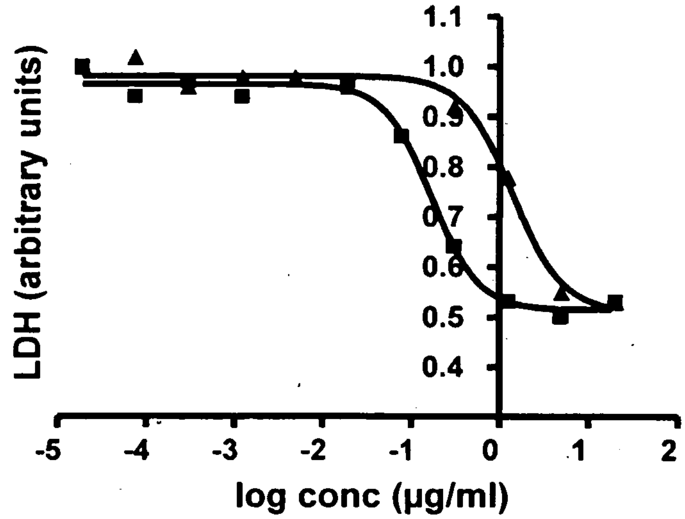
LDH (arbitrary units)
1.1
1.0
0.9
0.8
0.7
0.6
0.5
0.4
-5
-4
-3
-2
-1
0
1
2
log conc (µg/ml)

Figure 2

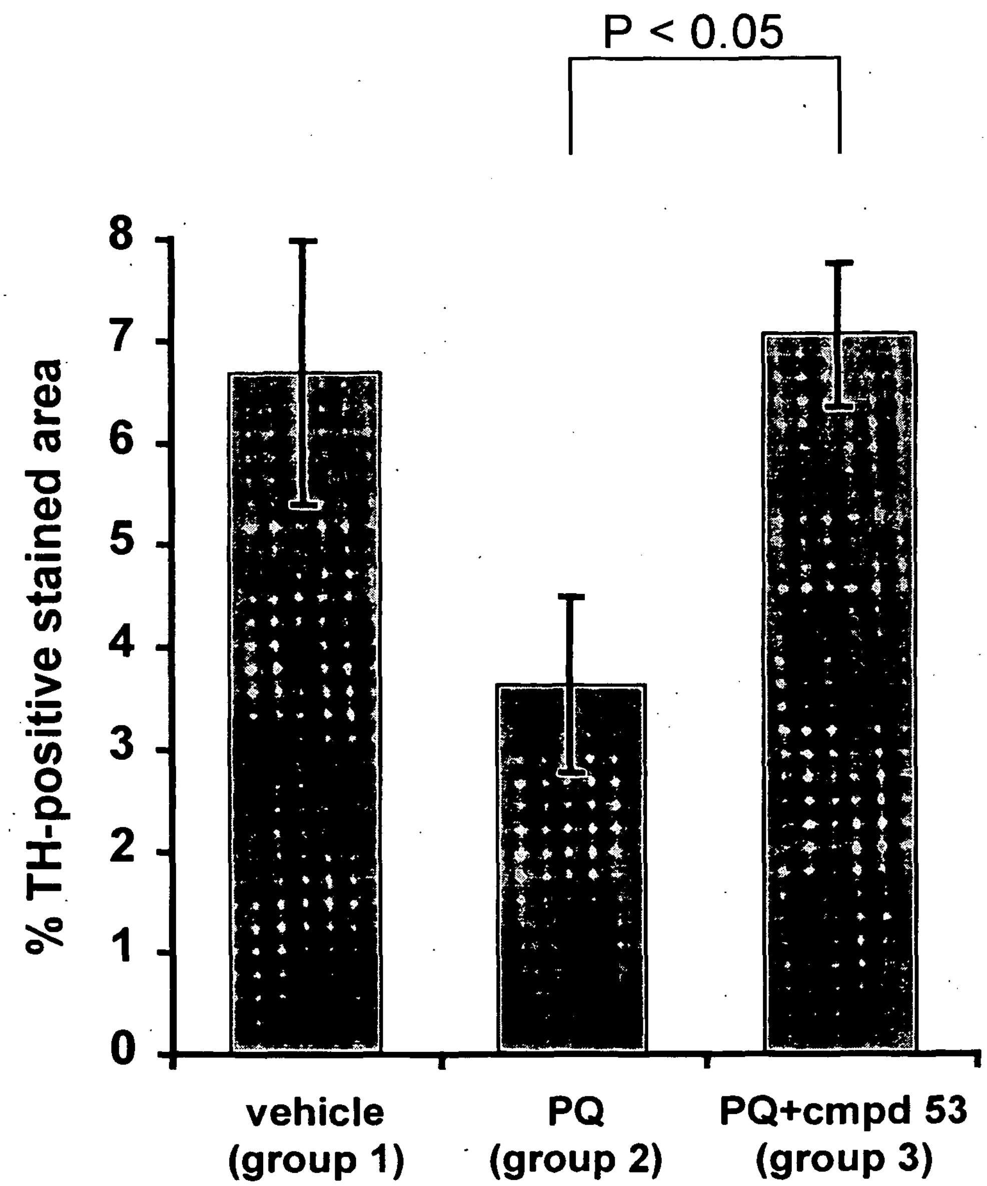
P < 0.05
% TH-positive stained area
8
7
6
5
4
3
2
1
0
vehicle
(group 1)
PQ
(group 2)
PQ+cmpd 53
(group 3)

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9951584 A


### Non-patent literature cited in the description


- **Chou et al.** *Adv. Enzyme Reg.,* 1984, vol. 22, 27
- McCutcheon's Detergents and Emulsifiers Annual. MC Publishing Crop, 1981
- Tensid-Taschenbuch. Hanser Verlag, 1981
- Encyclopaedia of Surfactants. Chemical Publishing Co, 1981
- **Griffioen et al.** *Biochem Biophys Acta,* 2006, vol. 1762 (3), 312-318
- **Vercammen et al.** *Molecular Therapy,* 2006, vol. 14 (5), 716-723
- **Gerard et al.** *FASEB.,* 2006, vol. 20 (3), 524-6